# EUROPEAN PATENT APPLICATION

(11) **EP 4 610 263 A1**
(43) Date of publication of application: **03.09.2025**
(21) Application number: 23890797.6
(22) Date of filing: 15.11.2023
(51) Int. Cl.: C07D 519/00, A61P 35/00, A61K 31/501, A61K 47/50, A61K 31/519

(54) **PAN-KRAS INHIBITOR COMPOUND**

(30) Priority: 16.11.2022 CN 202211438020; 06.12.2022 CN 202211619913; 19.12.2022 CN 202211636230
(71) Applicant: Adlai Nortye Biopharma Co., Ltd., Yuhang District Hangzhou Zhejiang 311121 (CN)
(72) Inventor: LV, Meng, Hangzhou, Zhejiang 311121 (CN); CHEN, Yufeng, Hangzhou, Zhejiang 311121 (CN); LI, Feifan, Hangzhou, Zhejiang 311121 (CN); LIU, Canfeng, Hangzhou, Zhejiang 311121 (CN); CHENG, Wanli, Hangzhou, Zhejiang 311121 (CN); CHEN, Kaixuan, Hangzhou, Zhejiang 311121 (CN); WU, Peng, Hangzhou, Zhejiang 311121 (CN); YANG, Han, Hangzhou, Zhejiang 311121 (CN); JIN, Chaofan, Hangzhou, Zhejiang 311121 (CN); SUN, Zhao, Hangzhou, Zhejiang 311121 (CN); LIU, Shuaishuai, Hangzhou, Zhejiang 311121 (CN); HE, Nanhai, Hangzhou, Zhejiang 311121 (CN); YU, Zhiyong, Hangzhou, Zhejiang 311121 (CN)
(74) Representative: Michalski Hüttermann & Partner Patentanwälte mbB
(86) International application number: PCT/CN2023/131674
(87) International publication number: WO 2024/104364

(57) **Abstract**

The present disclosure relates to a pan-KRAS inhibitor compound represented by Formula (I), a pharmaceutical composition comprising the compound, and the use of the compound of Formula (I) for the prevention and/or treatment of cancer, tumors, inflammatory diseases, autoimmune diseases, or immune-mediated diseases.

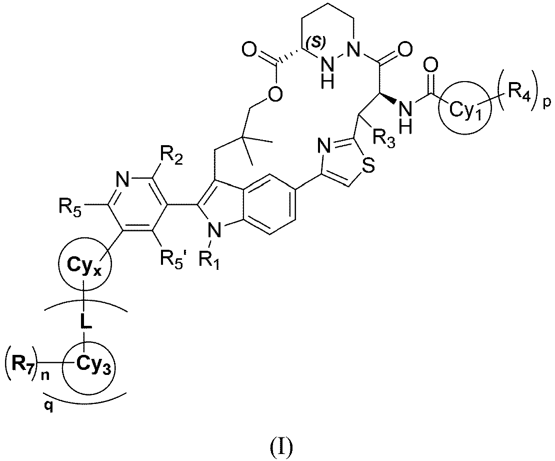

## Description

### Technical field

The present disclosure relates to a compound, in particular to a highly active pan-KRAS inhibitor and its use.

### Background technique

RAS is one of the most frequently mutated genes in human tumors, and its mutation occurs in about 30% of tumor patients, among which KRAS accounts for about 85% of RAS mutations. KRAS mutations exist in 88% of pancreatic cancer, 50% of colorectal adenocarcinoma, and 32% of lung adenocarcinoma. The development of targeted KRAS inhibitors has great clinical significance and value.

KRAS is a membrane-bound protein with GTPase activity, which cycles between a GDP-bound inactive conformation and a GTP-bound active conformation through nucleotide exchange, performing the function of a "molecular switch". KRAS in the GTP-bound state can activate multiple downstream signaling pathways including RAF-MEK-ERK and PI3K-AKT, and regulate life processes such as cell growth, proliferation, differentiation and apoptosis.

KRAS mutations (such as G12C, G12D, G12V, G13D, etc.) will affect the hydrolysis of GTP mediated by GTPase activating proteins (GAPs), increase KRAS in the GTP-bound activated state, and over-activate downstream signaling pathways, eventually lead to the occurrence and development of tumors. However, since the KRAS protein lacks a corresponding hydrophobic pocket suitable for drug binding, and its affinity with GTP and GDP is at the picomolar level (~20pM), it is very difficult to develop inhibitors that competitively bind to KRAS. KRAS has been considered an undruggable target for the past decades.

In May 2021, AMG510 was approved by the FDA for the treatment of locally advanced or metastatic non-small cell lung cancer carrying the KRAS^{G12C} mutation, breaking the history of KRAS being "undruggable". However, the G12C mutation only accounts for a small portion of KRAS mutations. For mutations at other sites of KRAS, there is still a lack of satisfactory and effective inhibitor compounds, and a large number of clinical needs have not been met. Therefore, the development of effective pan- KRAS inhibitor compounds are a need in the art.

### Contents of the disclosure

The disclosure provides a pan-KRAS inhibitor. Such structures are different from existing KRAS^{G12C} inhibitors that act through covalent binding, but instead mediate the formation of a ternary complex between ubiquitous intracellular chaperones (such as Cyclophilin A) and KRAS proteins. The formation of the ternary complex can block the combination of KRAS and its downstream effector molecules (such as RAF) through steric blockage, inhibit the activation of MAPK and PI3K-AKT signaling pathways, thereby inhibiting the occurrence and development of tumors, and play a role in the treatment of tumors and other diseases.

In one aspect, the present disclosure provides a compound having a structure of Formula (I) or Formula (II), or a pharmaceutically acceptable salt, isotope derivative, or stereoisomer thereof: 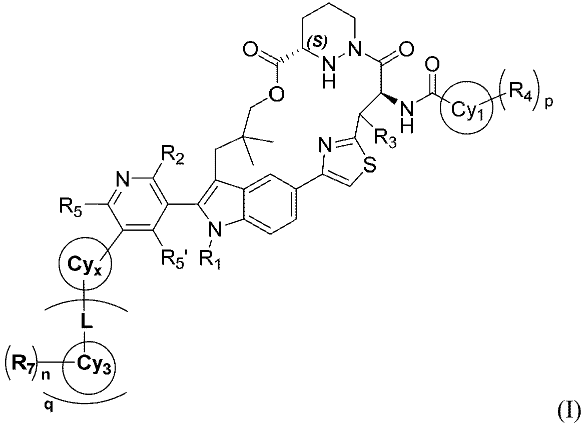
wherein, R₁ represents C₁-C₆ alkyl, -(C₁-C₆ alkylene)-(C₃-C₈ cycloalkyl), -(C₁-C₆ alkylene)-(4-8 membered heterocycloalkyl), -(C₁-C₆ alkylene)-ORa, -(C₁-C₆ alkylene)-SRa or -(C₁-C₆ alkylene)-NRaRa';
R₂ represents halogen, cyano, C₁-C₆ alkyl, -(C₀-C₆ alkylene)-(C₃-C₈ cycloalkyl), or -(C₀-C₆ alkylene)-(4-8 membered heterocycloalkyl), which is optionally substituted with 0, 1 or 2 substituents selected from ORa, -SRa, or NRaRa';
R₃ represents hydrogen, -O(C₀-C₆ alkylene)Ra, -S(C₀-C₆ alkylene)Ra, -N(C₀-C₆ alkylene)Ra(C₀-C₆ alkylene)Rₐ^{'}, which is optionally substituted with 0, 1, or 2 substituents selected from -ORa, -SRa, or -NRaRa';
Cy₁ represents C₃-C₁₂ cycloalkyl or 4-12 membered heterocycloalkyl;
R₄ each independently represents hydrogen, halogen, oxo, C₁-C₆ alkyl, -(C₀-C₆ alkylene)(C₃-C₆) cycloalkyl, -(C₀-C₆ alkylene)(4-8 membered) heterocycloalkyl, -(C₀-C₆ alkylene)ORa, -(C₀-C₆ alkylene)SRa, -(C₀-C₆ alkylene)NRaRa', -(C₀-C₆ alkylene)CORa, -(C₀-C₆ alkylene)COORa, -(C₀-C₆ alkylene)CONRaRa', -(C₀-C₆ alkylene) NRaCORa', -(C₀-C₆ alkylene) OCONRaRa', -(C₀-C₆ alkylene) NRaCONRaRa', -(C₀-C₆ alkylene)SORa, -(C₀-C₆ alkylene)S(O)₂Ra, -(C₀-C₆ alkylene)NRaS(O)₂Ra', -(C₀-C₆ alkylene)CN, -(C₀-C₆ alkylene)(C6-C10 aryl) or -(C₀-C₆ alkylene)(5-12 membered heteroaryl) ; wherein two R₄ groups on two carbon atoms of Cy₁, together with the carbon atoms to which they are attached and the atoms between these two carbon atoms, may form a 3-8 membered ring, and the 3-8 membered ring may optionally contain 0, 1, 2 or 3 heteroatoms selected from N, O or S ; or two R₄ groups on the same carbon atom of Cy₁, together with the carbon atom to which they are attached, may form a 3-8 membered ring, and the 3-8 membered ring may optionally contain 0, 1, 2 or 3 heteroatoms selected from N, O or S;
R₅, R₅' each independently represents hydrogen, halogen, C₁-C₆ alkyl, -(C₀-C₆ alkylene)(C₃-C₈) cycloalkyl or -(C₀-C₆ alkylene)CN;
Cyₓ represents a Cy₂ optionally substituted with m R₆;
Cy₂ is selected from a C₆-C₁₂ aryl, a 5-12 membered heteroaryl, or an oxo-substituted saturated or partially saturated 5-12 membered heterocycloalkyl, wherein the C₆-C₁₂ aryl, the 5-12 membered heteroaryl, or the 5-12 membered heterocycloalkyl may be monocyclic or polycyclic fused rings;
R₆ is each independently selected from: hydrogen, halogen, oxo, C₁-C₆ alkyl, -(C₀-C₆ alkylene)(C₃-C₈ cycloalkyl), -(C₀-C₆ alkylene)(4-8 membered heterocycloalkyl), - (C₀-C₆) alkyleneCN, -(C₀-C₆ alkylene)C(O)NRa Ra', -(C₀-C₆ alkylene) NRaC(O) Ra', -(C₀-C₆ alkylene)C(O)Ra, -(C₀-C₆ alkylene)C(O)ORa, -(C₀-C₆ alkylene)OC(O)NRaRa', -(C₀-C₆ alkylene)NRaC(O)ORa', -(C₀-C₆ alkylene)NRaC(O)NRaRa', -(C₀-C₆ alkylene)S(O)₂Ra, -P(O)RaRa'; or -(C₀-C₆ alkylene)-ORa, -(C₀-C₆ alkylene)-SRa, or -(C₀-C₆ alkylene)-NRaRa', each optionally substituted with 0, 1, or 2 substituents; or two R₆ groups on two adjacent ring atoms of Cy₂ may together with said two ring atoms form a ring, the ring optionally comprising 0, 1, 2, 3, or 4 heteroatoms selected from N, O, and S, and optionally comprising 0, 1, 2, or 3 unsaturated bonds;
L represents -(C₀-C₆) alkylene-, -(C₀-C₃) alkylene-O-(C₀-C₃) alkylene-, -(C₀-C₃) alkylene-S-(C₀-C₃) alkylene-, or -(C₀-C₃) alkylene-NRa-(C₀-C₃) alkylene-; wherein, the -(C₀-C₆)alkylene- or -(C₀-C₃)alkylene- is optionally substituted with 0, 1, 2, or 3 C₁-C₃ alkyl groups, or two C₁-C₃ alkyl substituents on the same carbon atom may form a 3-8 membered ring, which optionally contains 0, 1, 2, or 3 heteroatoms selected from N, O, or S;
Cy₃ is selected from C₃-C₁₂ cycloalkyl and 4- to 12-membered heterocycloalkyl, wherein when Cy₃ is a 4- to 12-membered heterocycloalkyl, Cy₃ may be a spiro ring, bridged ring, or fused ring, and when the ring contains a S or P atom, the S or P atom may optionally be oxidized to -S(O)₂-, -S(O)(NRa)- or -P(O)Ra;
R₇ is each independently selected from: hydrogen, halogen, oxo, C₁-C₆ alkyl, -(C₀-C₆ alkylene)(C₃-C₆ cycloalkyl), -(C₀-C₆ alkylene)(4-8 membered heterocycloalkyl), - (C₀-C₆) alkyleneCN, -(C₀-C₆ alkylene)ORa, -(C₀-C₆ alkylene)SRa, -(C₀-C₆ alkylene)NRaRa', -(C₀-C₆ alkylene)C(O)Ra, -(C₀-C₆ alkylene)C(O)ORa, -(C₀-C₆ alkylene)OC(O)NRaRa', -(C₀-C₆ alkylene)NRaC(O)ORa', -(C₀-C₆ alkylene)NRaC(O)NRaRa', -(C₀-C₆ alkylene)C(O)NRa Ra', -(C₀-C₆ alkylene)S(O)₂Ra, -(C₀-C₆ alkylene)S(O)(NH)Ra; alternatively, two R₇ groups on the same carbon atom of Cy₃ may form a spiro ring, which may optionally contain 0, 1, 2, 3, or 4 heteroatoms selected from N, O, and S; or two R₇ groups on adjacent ring atoms of Cy₃ may form a fused ring with said two ring atoms, which may optionally contain 0, 1, 2, 3, or 4 heteroatoms selected from N, O, and S; or two R₇ groups on non-adjacent ring atoms of Cy₃ may form a bridged ring with said two ring atoms, which may optionally contain 0, 1, 2, 3, or 4 heteroatoms selected from N, O, and S; and optionally, said spiro ring, fused ring, or bridged ring may contain 0, 1, 2, or 3 unsaturated bonds;
alternatively, R₆ and R₇ may together with L form a 4- to 8-membered ring, which may optionally contain 0, 1, 2, or 3 heteroatoms selected from N, O, and S;
wherein, p represents 0, 1, 2, 3 or 4;
q represents 0, 1 or 2;
m, n each independently represent 0, 1, 2, 3 or 4;
Ra and Ra' each independently represent hydrogen, C₁-C₆ alkyl, C₃-C₈ cycloalkyl, or 4-8 membered heterocycloalkyl; wherein, when Ra and Ra' are bonded to the same nitrogen atom, Ra, Ra', and the nitrogen atom together may form a 4- to 8-membered ring, which may optionally contain 0, 1, 2, or 3 heteroatoms selected from N, O, or S;
the alkyl, cycloalkyl, heterocycloalkyl and alkylene may be substituted by 0, 1, 2, 3, 4, 5 or 6 halogen atoms independently.

In some embodiments, R₁ represents C₁-C₆ alkyl, -(C₁-C₆ alkylene)-(C₃-C₈ cycloalkyl) or -(C₁-C₆ alkylene)-(4-8 membered heterocycloalkyl); preferably, R₁ represents C₁-C₆ alkyl; more preferably, R₁ represents C₁-C₃ alkyl.

In some embodiments, R₂ represents C₁-C₆ alkyl, which may be optionally substituted by 0, 1 or 2 -ORa substituents; preferably, R₂ represents 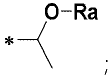 ; more preferably, R₂ represents 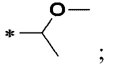 more preferably, R₂ represents 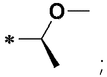 wherein * represents the site where R₂ is attached to the corresponding position in formula (I).

In some embodiments, R₃ represents hydrogen or -O(C₁-C₆)alkyl, -O(C₀-C₆ alkylene)(C₃-C₈)cycloalkyl, or -O(C₀-C₆ alkylene)(4-8 membered)heterocycloalkyl, which is optionally substituted with 0 or 1 substituent selected from ORa, -SRa, or NRaRa'.

In some embodiments, Cy₁ represents C₃-C₈ cycloalkyl or 4-8 membered heterocycloalkyl.

In some embodiments, R₄ each independently represents hydrogen, halogen, C₁-C₆ alkyl, -(C₀-C₆ alkylene)ORₐ, -(C₀-C₆ alkylene)SRₐ, -(C₀-C₆ alkylene)NRₐRₐ', -(Co-C₆ alkylene)CONRₐRₐ', -(C₀-C₆ alkylene)NRₐCORₐ', -(C₀-C₆ alkylene)OCONRₐRₐ', - (C₀-C₆ alkylene)CN, or -(C₀-C₆ alkylene)(5-12 membered heteroaryl); or two R₄ groups on two carbon atoms of Cy₁, together with the carbon atoms to which they are attached and the atoms between these two carbon atoms, may form a 3-8 membered ring, which optionally contains 0, 1, 2, or 3 heteroatoms selected from N, O, or S; or two R₄ groups on the same carbon atom of Cy₁, together with the carbon atom to which they are attached, may form a 3-8 membered ring, which optionally contains 0, 1, 2, or 3 heteroatoms selected from N, O, or S.

In some embodiments, R₄ each independently represents hydrogen, halogen, C₁-C₆ alkyl, -(C₀-C₆ alkylene)CONRₐRₐ', or -(C₀-C₆ alkylene)(5-12 membered heteroaryl); or two R₄ groups on two carbon atoms of Cy₁, together with the carbon atoms to which they are attached and the atoms between these two carbon atoms, may form a 3-8 membered ring, which optionally contains 0, 1, 2, or 3 heteroatoms selected from N, O, or S; or two R₄ groups on the same carbon atom of Cy₁, together with the carbon atom to which they are attached, may form a 3-8 membered ring, which optionally contains 0, 1, 2, or 3 heteroatoms selected from N, O, or S.

In some embodiments, R₅, R₅' each independently represents hydrogen or C₁-C₆ alkyl; more preferably, R₅, R₅' each independently represents hydrogen or methyl.

In some embodiments, Cy₂ is selected from a benzene ring, a 5-6 membered heteroaryl, or an oxo-substituted saturated or partially saturated 5-6 membered heterocyclic group; wherein the 5-6 membered heterocyclic group preferably contains a nitrogen atom.

In some embodiments, when Cy₂ represents a six-membered ring, Cy₂ is selected from:

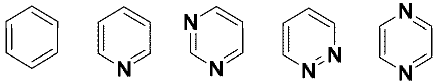

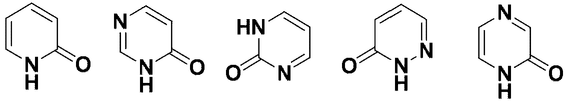

In some embodiments, Cy₂ is selected from a 5-membered monocyclic heteroaromatic ring or a 9-membered fused heteroaromatic ring; wherein the 5-membered or 9-membered heterocyclic group preferably contains a nitrogen atom.

In some embodiments, R₆ is each independently selected from: hydrogen, halogen, oxo, C₁-C₆ alkyl, -(C₀-C₆ alkylene)(C₃-C₈ cycloalkyl), -(C₀-C₆) alkylene-CN, -(C₀-C₆ alkylene)(4- to 8-membered heterocycloalkyl), -(C₀-C₆ alkylene)-C(O)NRaRa', -(C₀-C₆ alkylene)-NRaC(O)Ra', -(C₀-C₆ alkylene)-S(O)₂Ra, or -P(O)RaRa'; or -(C₀-C₆ alkylene)-ORa, -(C₀-C₆ alkylene)-SRa, or -(C₀-C₆ alkylene)-NRaRa', each optionally substituted with 0, 1, or 2 substituents; or as described in claim 1, two R₆ groups on adjacent ring atoms of Cy₂ may together with the ring atoms to which they are attached form a ring, which optionally contains 0, 1, 2, 3, or 4 heteroatoms selected from N, O, and S, and optionally contains 0, 1, 2, or 3 unsaturated bonds; when two R₆ groups form a ring containing unsaturated bonds, the ring is preferably an aromatic heterocycle.

In some embodiments, L represents -(C₀-C₃) alkylene-, optionally substituted with 0, 1, 2, or 3 C₁-C₃ alkyl groups, or two C₁-C₃ alkyl substituents on the same carbon atom may form a 3- to 4-membered ring.

In some embodiments, L represents -(C₀-C₁) alkylene-O-(C₀-C₁) alkylene-, -(C₀-C₁) alkylene-S-(C₀-C₁) alkylene-, or -(C₀-C₁) alkylene-NRa-(C₀-C₁) alkylene-, wherein the C₁ alkylene is optionally substituted with 0, 1, or 2 C₁-C₃ alkyl groups, or two C₁-C₃ alkyl substituents on the same carbon atom may form a 3- to 4-membered ring; more preferably, L represents -O-, -S-, or -NRa-.

In some embodiments, Cy₃ is selected from 4- to 8-membered heterocycloalkyl, which may be a spirocyclic, bridged, or fused ring system, and when the ring contains an S or P atom, the S or P atom is optionally oxidized to -S(O)₂-, -S(O)(NRa)-, or - P(O)Ra-.

In some embodiments, R₇ is each independently selected from: hydrogen, halogen, oxo, C₁-C₆ alkyl, -(C₀-C₆ alkylene)(4- to 8-membered heterocycloalkyl), -(C₀-C₆) alkylene-CN, -(C₀-C₆ alkylene)-ORa, -(C₀-C₆ alkylene)-SRa, -(C₀-C₆ alkylene)-NRaRa', -(C₀-C₆ alkylene)-C(O)Ra, -(C₀-C₆ alkylene)-OC(O)NRaRa', -(C₀-C₆ alkylene)-NRaC(O)ORa', -(C₀-C₆ alkylene)-NRaC(O)NRaRa', -(C₀-C₆ alkylene)-C(O)NRaRa', -(C₀-C₆ alkylene)-S(O)₂Ra, or -(C₀-C₆ alkylene)-S(O)(NH)Ra; or as described in claim 1, two R₇ groups may form a spirocyclic, bridged, or fused ring system, which optionally contains 0, 1, 2, 3, or 4 heteroatoms selected from N, O, and S, and optionally contains 0, 1, 2, or 3 unsaturated bonds; when two R' groups form a ring containing unsaturated bonds, the ring is preferably an aromatic heterocycle.

In some embodiments, when q is 0, one of R₆ contains at least one amino group, preferably a secondary amine or a tertiary amine, more preferably a tertiary amine.

In some embodiments, p is preferably 0, 1 or 2; or q is preferably 0 or 1; or m and n are each independently preferably 0, 1 or 2.

In some embodiments, 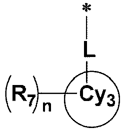 includes at least one secondary or tertiary amine; preferably, includes at least one tertiary amine; wherein * represents the site at which 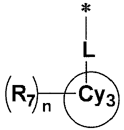 is connected to the corresponding position in formula (I).

In some embodiments, the structure in formula (I) -Cy₁-(R₄)pis selected from the following: 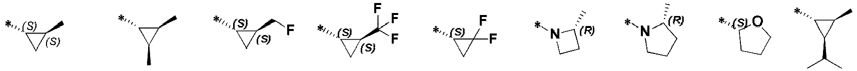 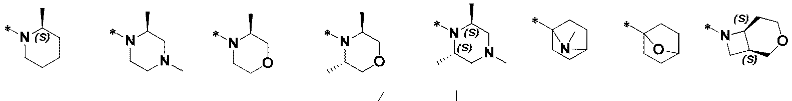 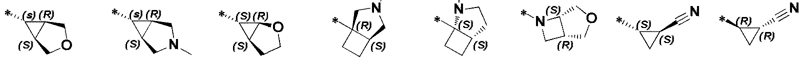 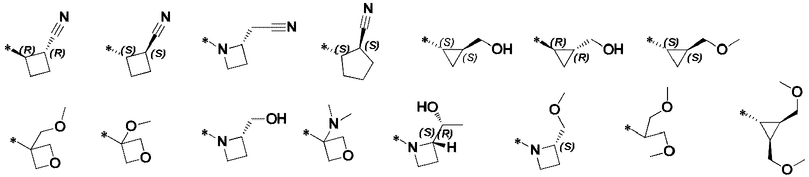 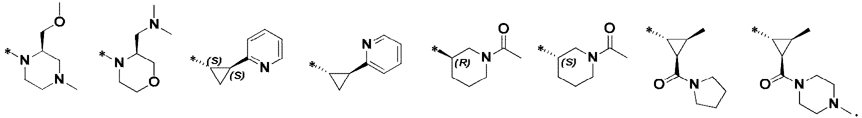 wherein * represents the site at which -Cy₁-(R₄)p is connected to the corresponding position in formula (I).

In some embodiments, the structure in formula (I) 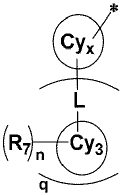 is selected from the following: 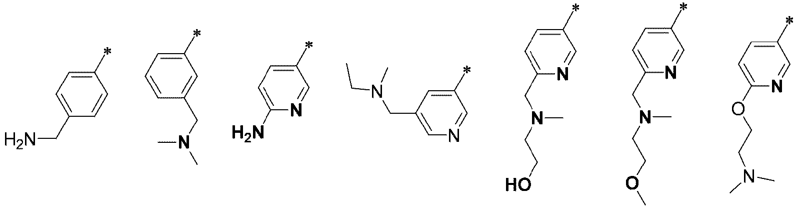 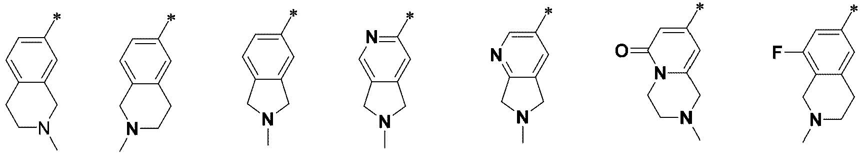 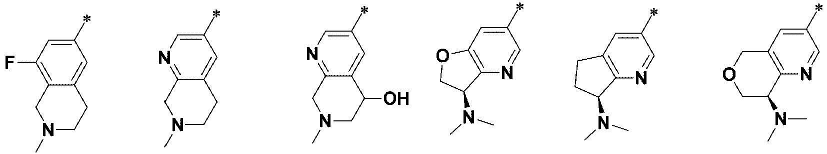 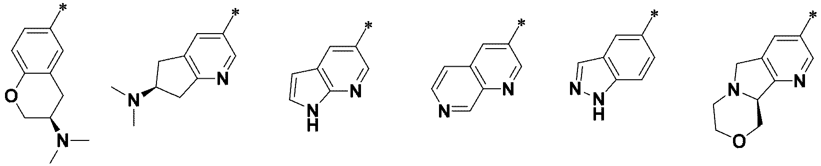 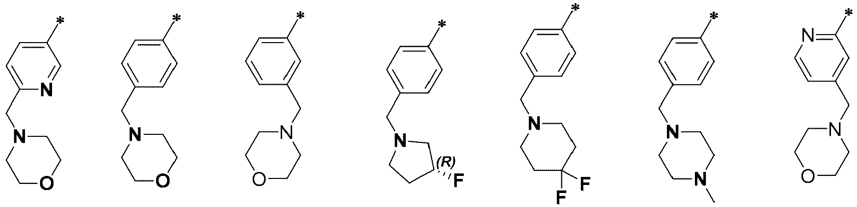 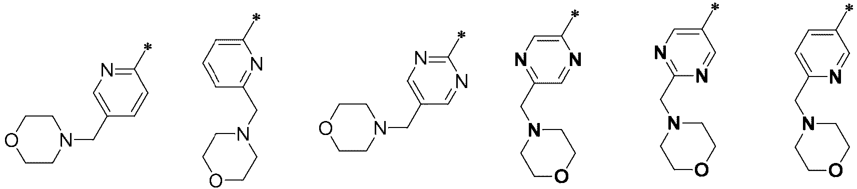 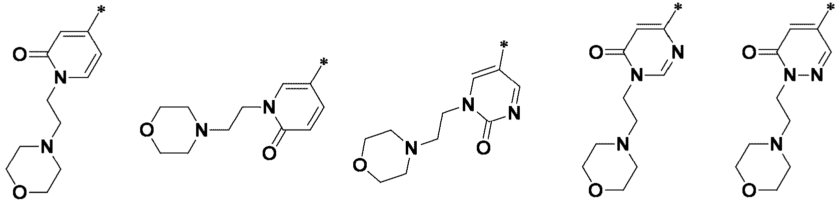 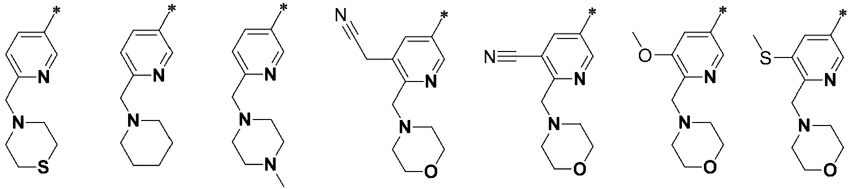 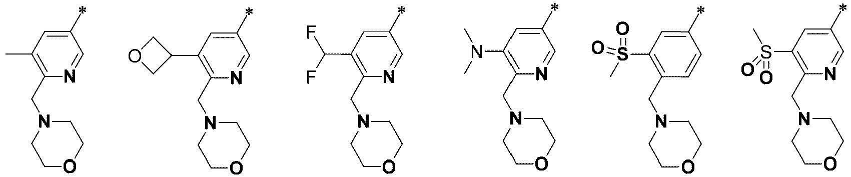 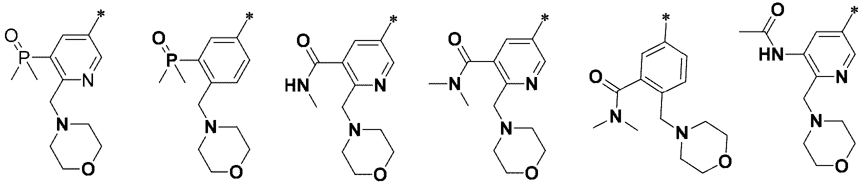 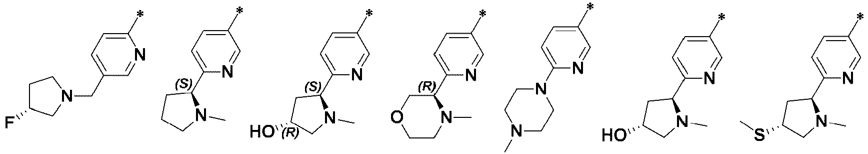 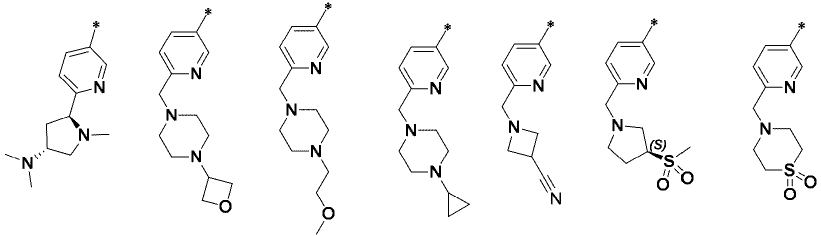 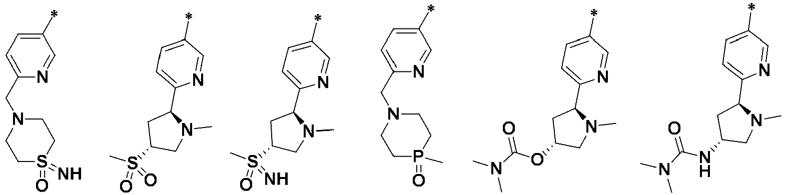 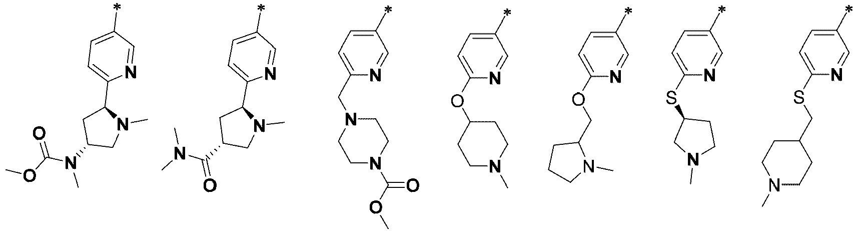 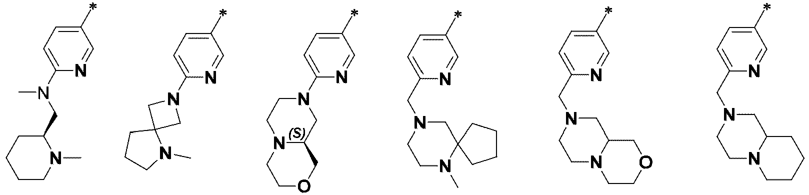 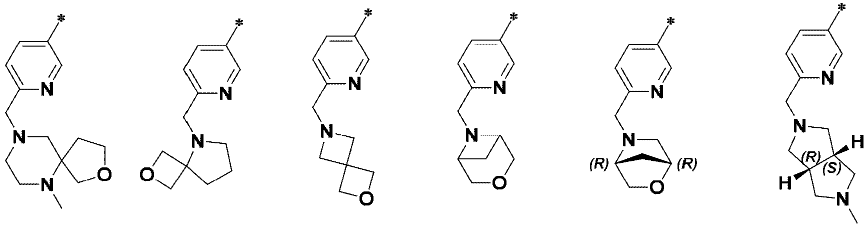 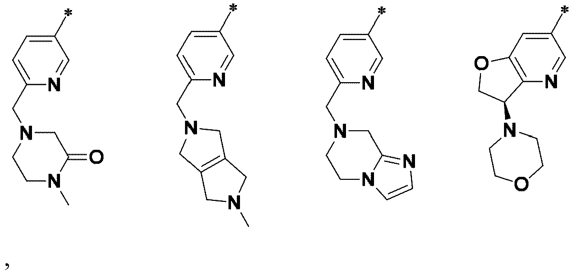 wherein * represents the site at which 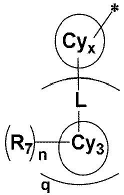 is connected to the corresponding position in formula (I).

In some embodiments, the structure in formula (I) 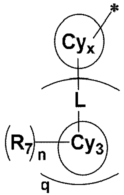 is selected from the following: 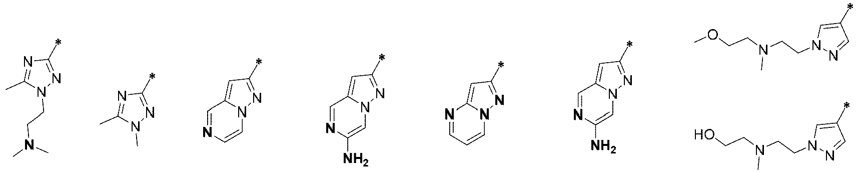 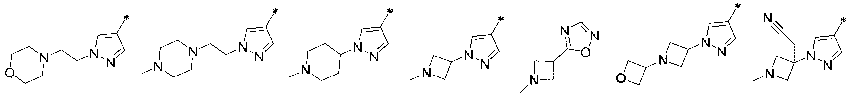 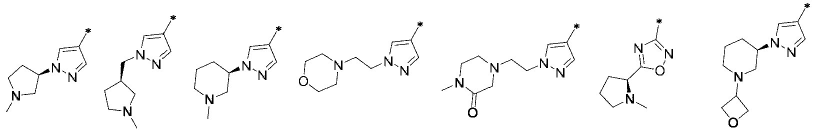 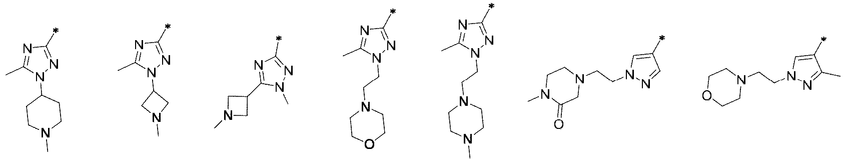 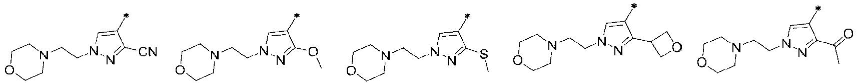 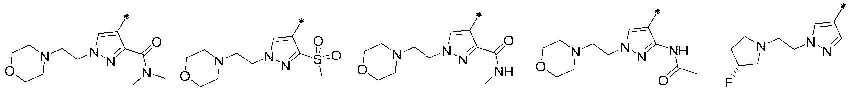 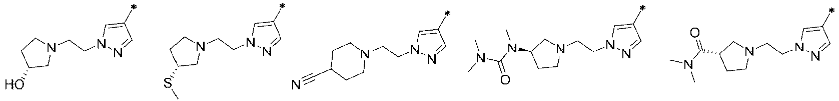 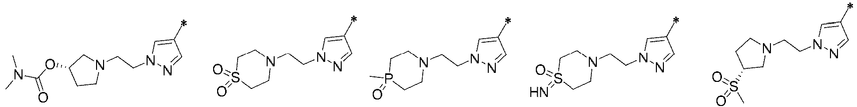 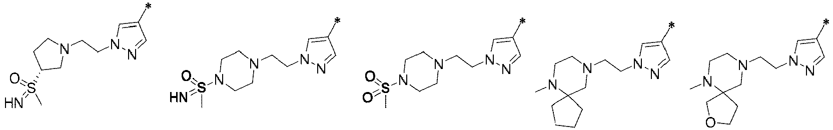 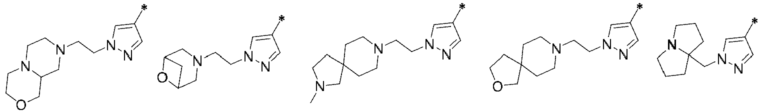 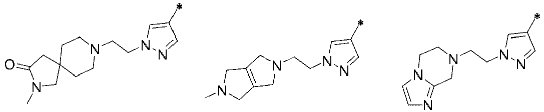 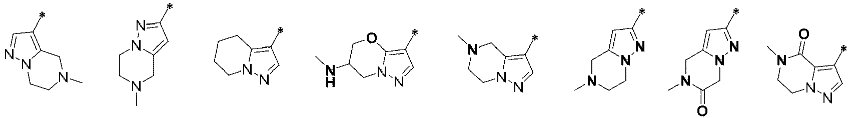 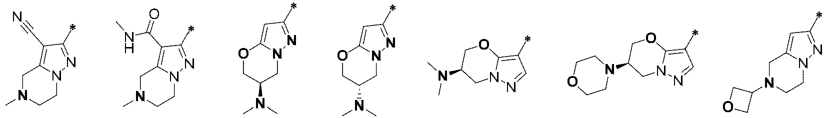 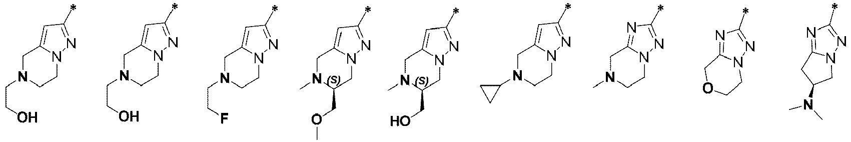 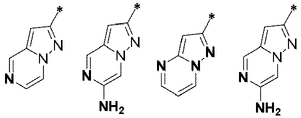 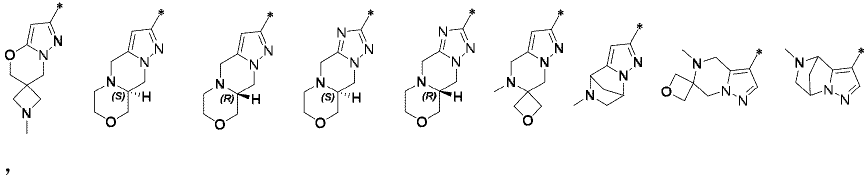 wherein * represents the site at which 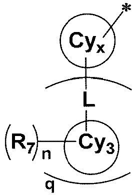 is connected to the corresponding position in formula (I).

In some embodiments, the compound of formula (I) has the structure of formula (V):

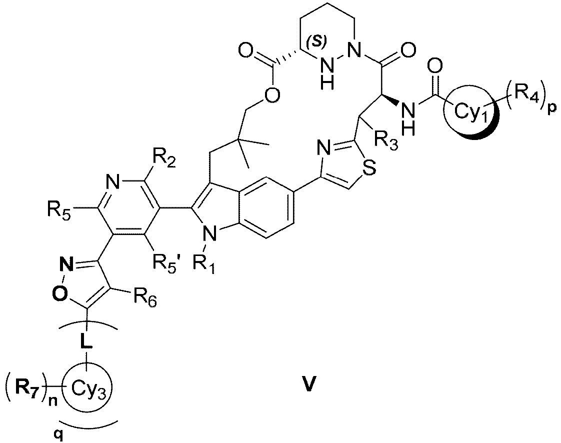

In some embodiments, the structure in formula (I) 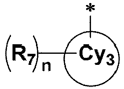 is selected from the following: 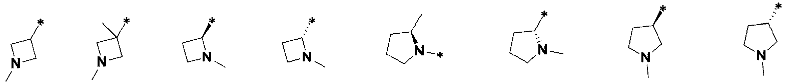 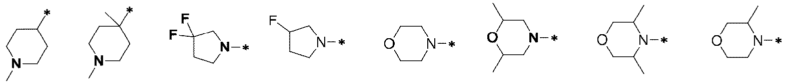 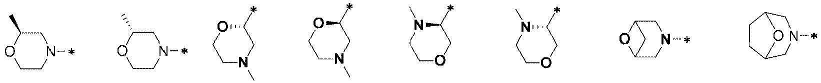 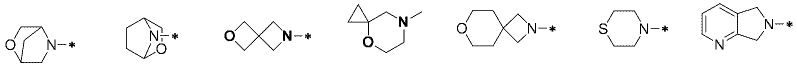 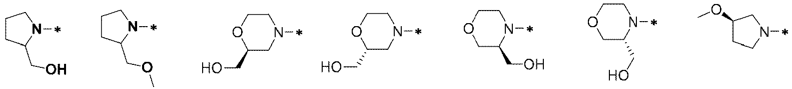 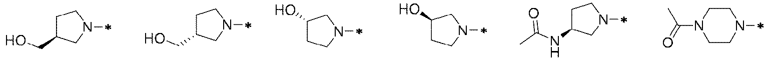 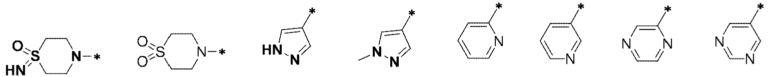 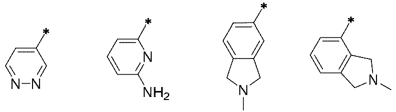 wherein * represents the site at which 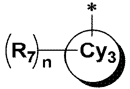 is connected to the corresponding position in formula (V).

In some embodiments, the compound of formula (I) has the structure of formula (II):

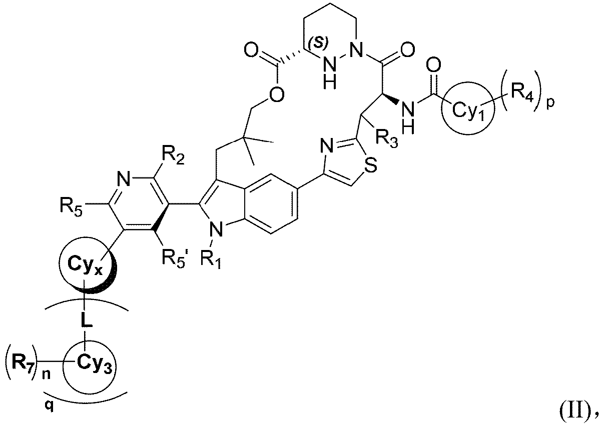

In another aspect, the present disclosure provides a compound having the following structure:

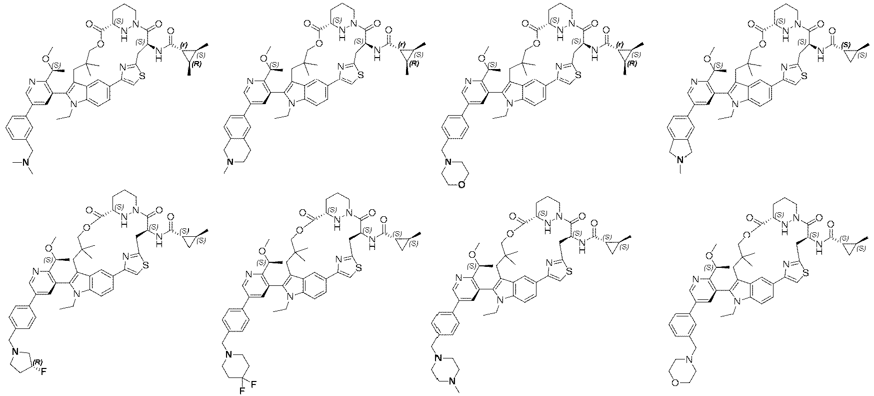

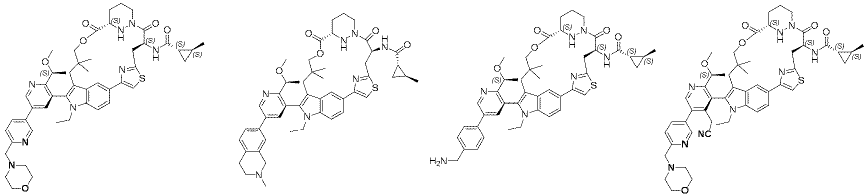

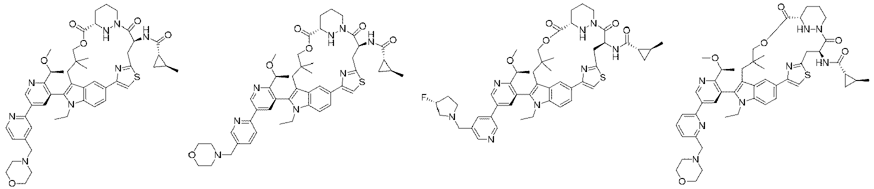

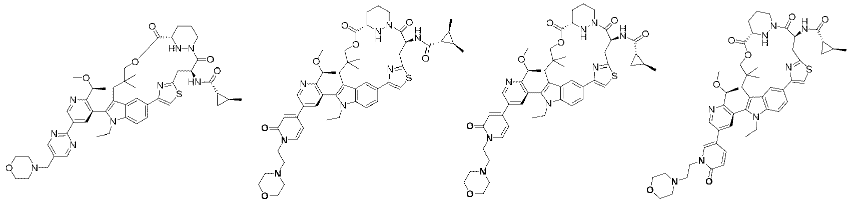

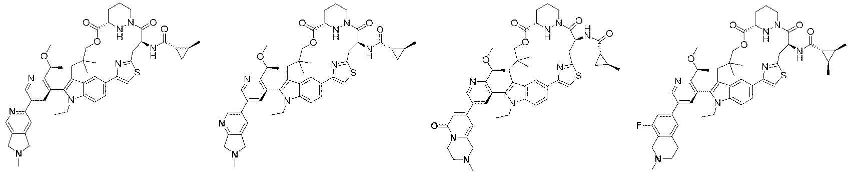

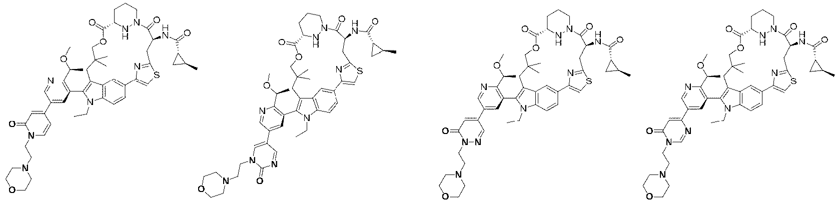

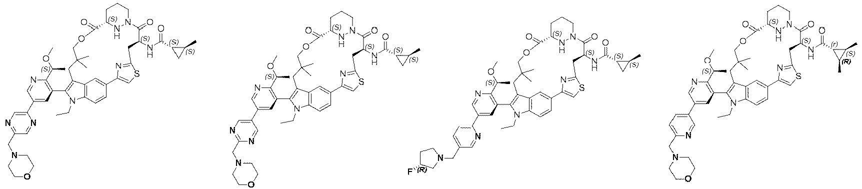

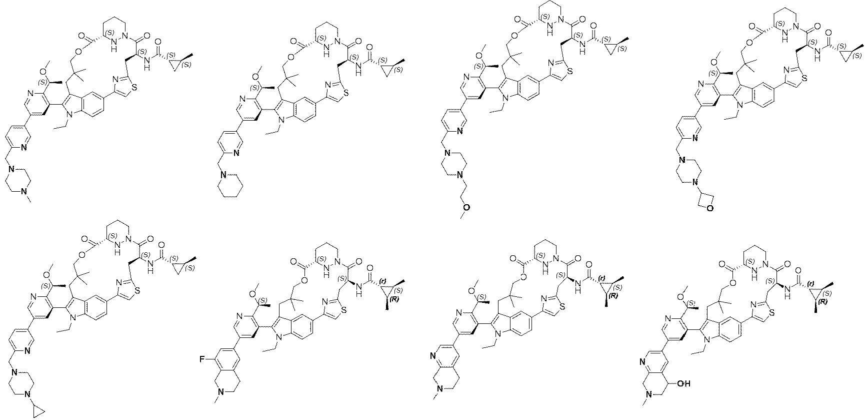

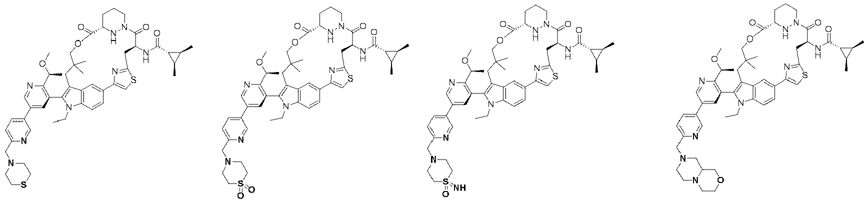

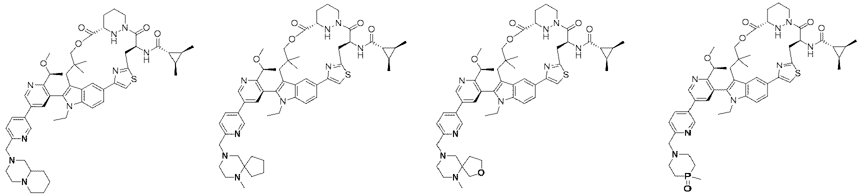

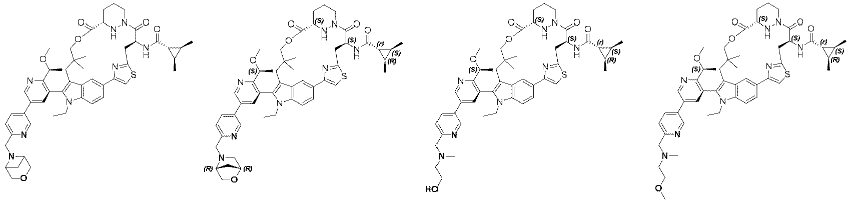

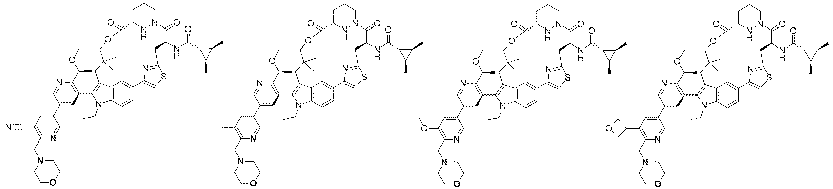

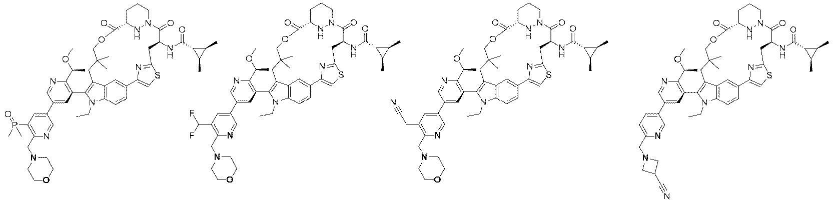

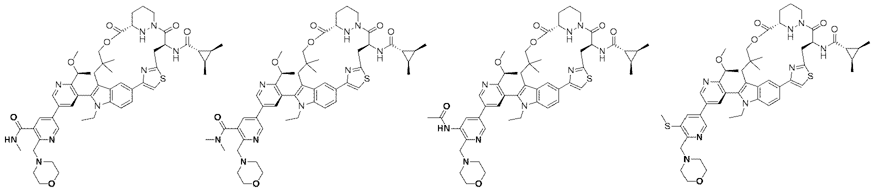

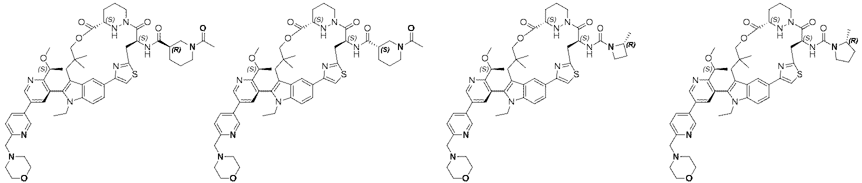

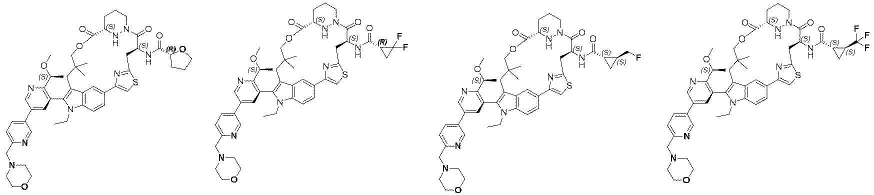

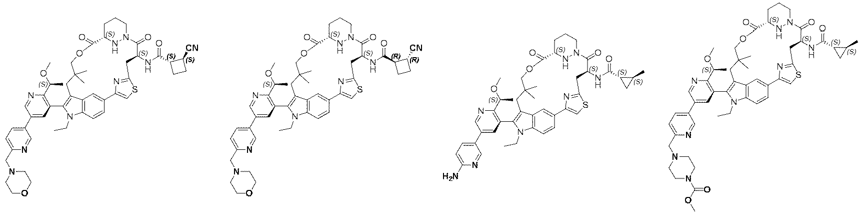

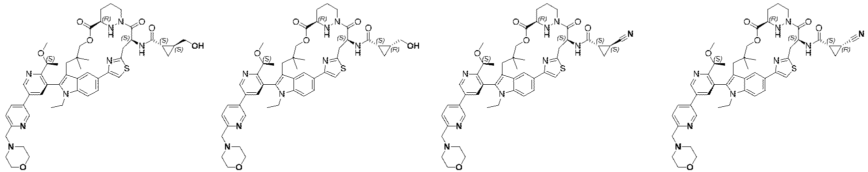

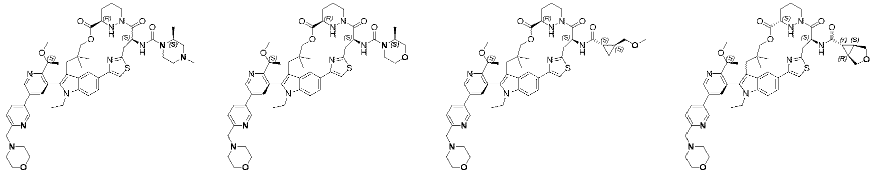

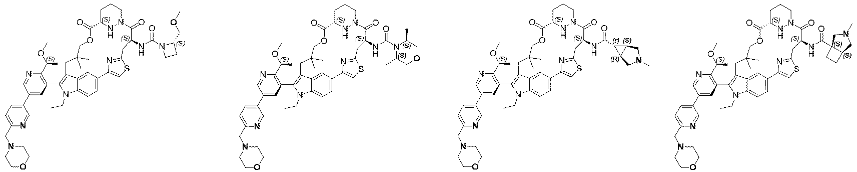

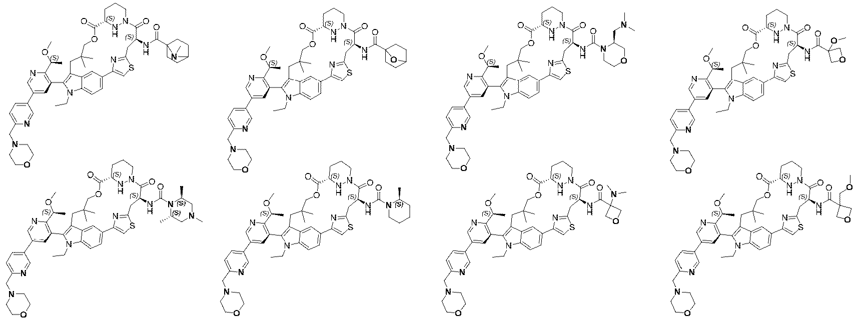

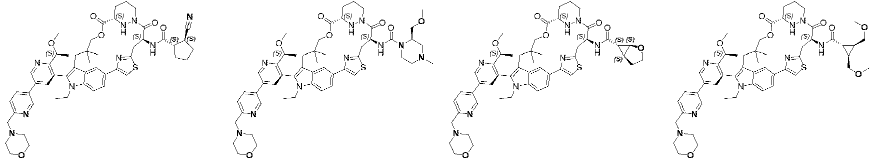

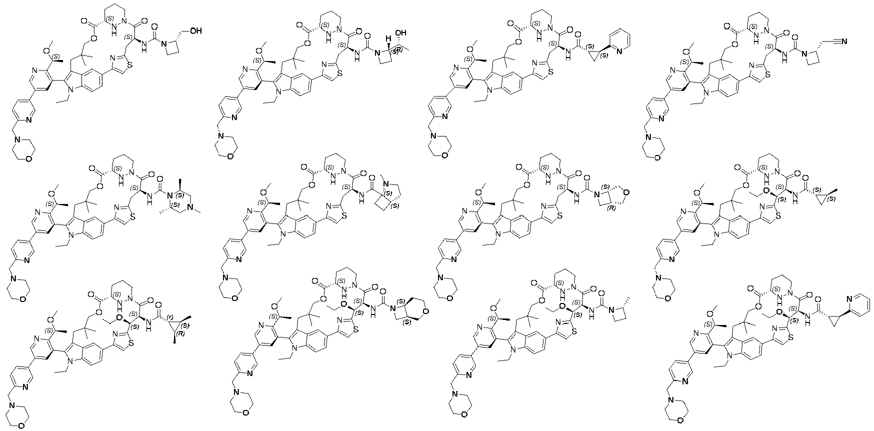

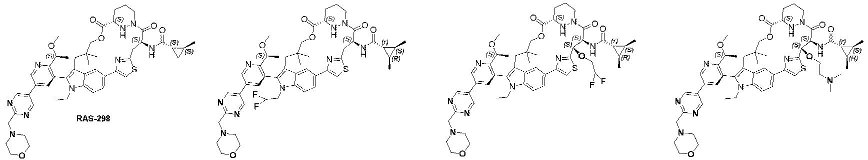

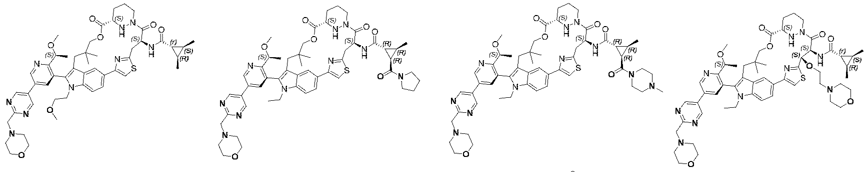

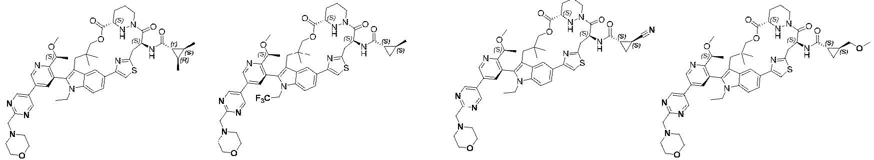

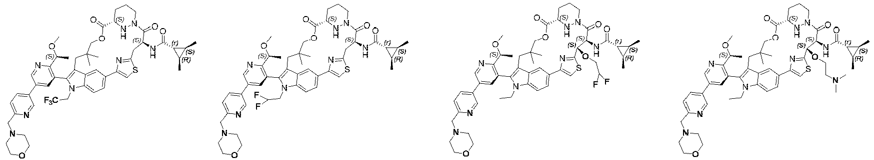

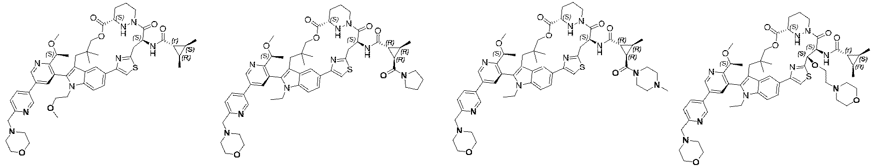

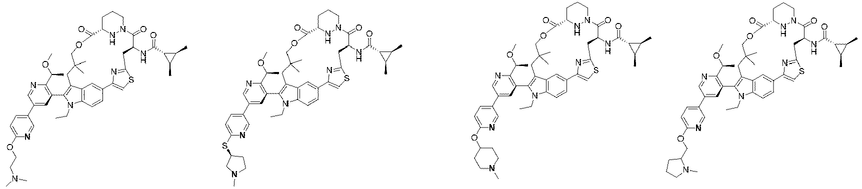

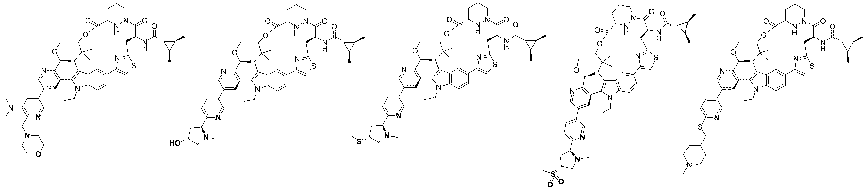

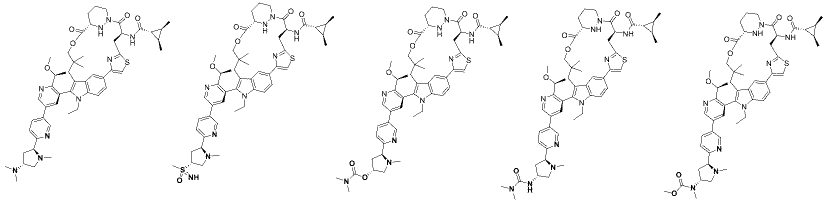

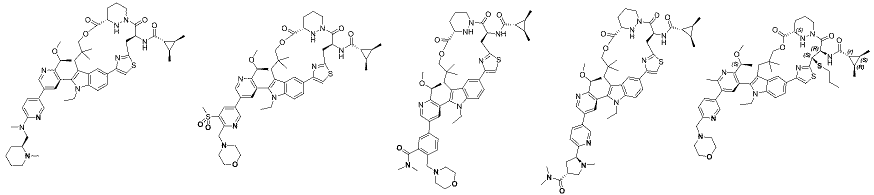

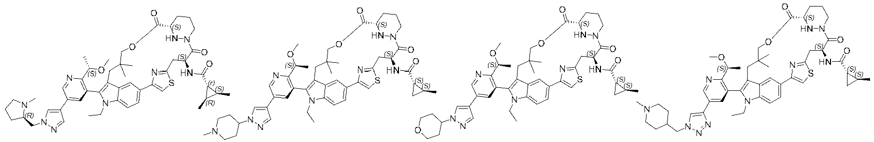

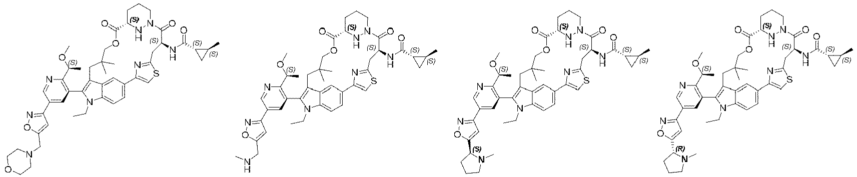

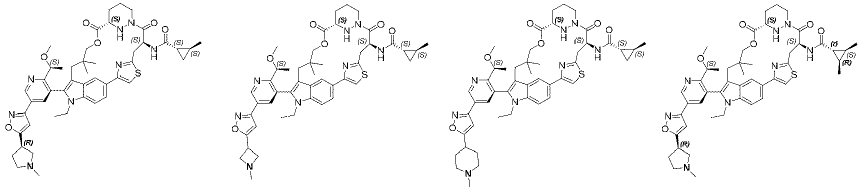

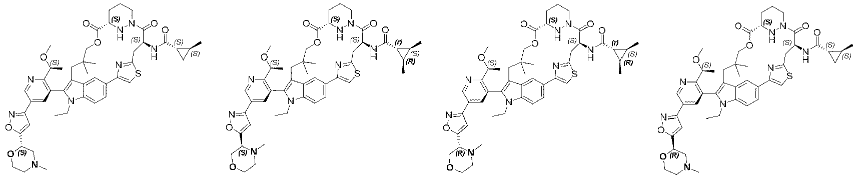

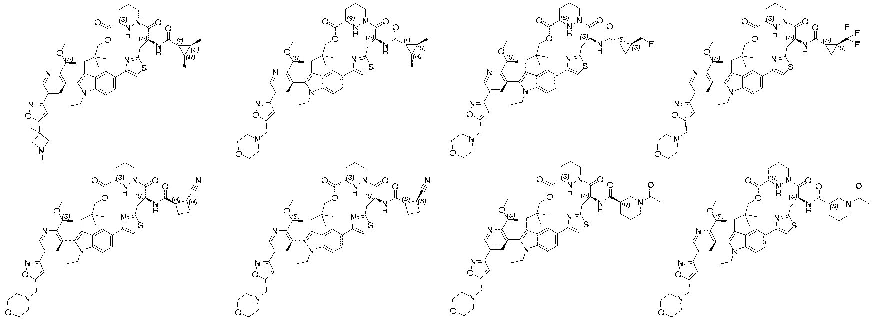

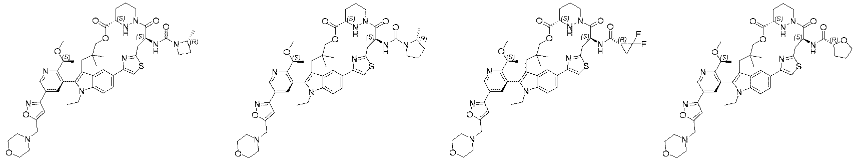

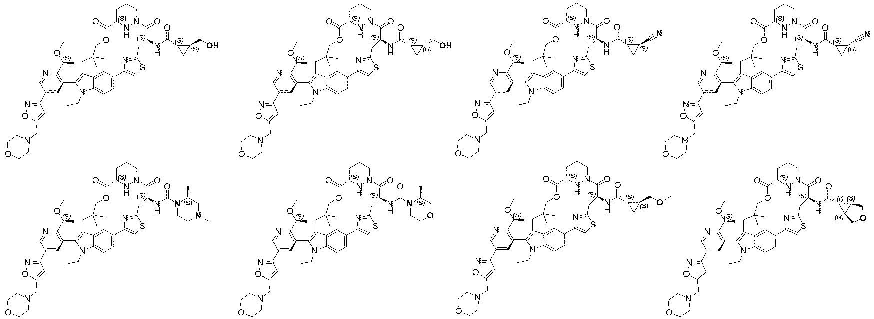

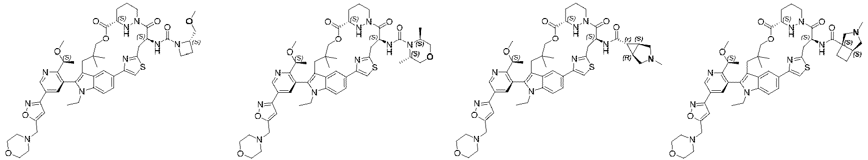

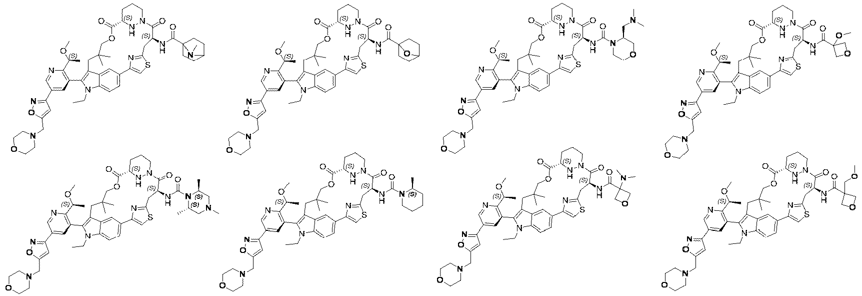

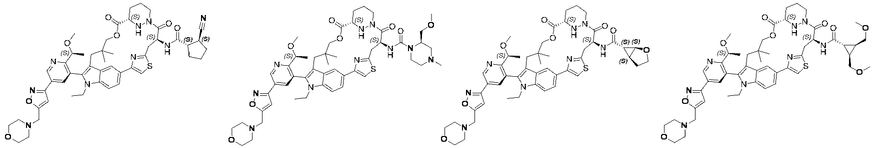

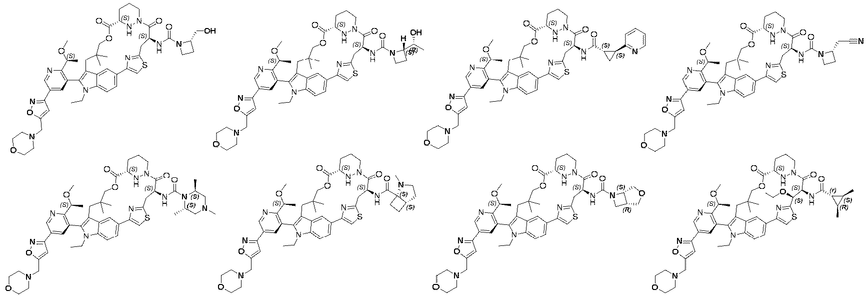

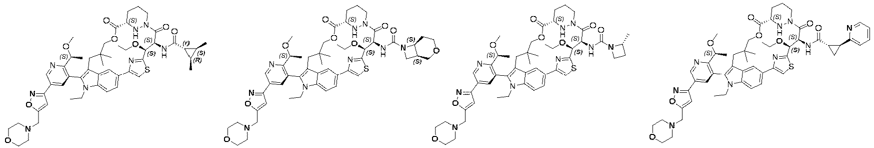

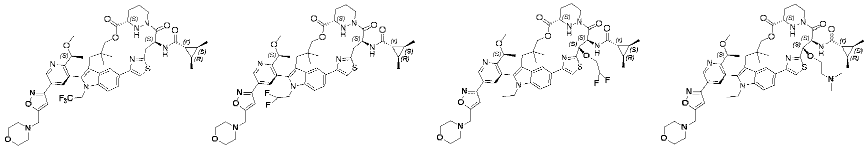

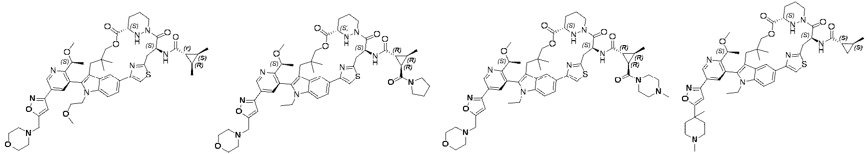

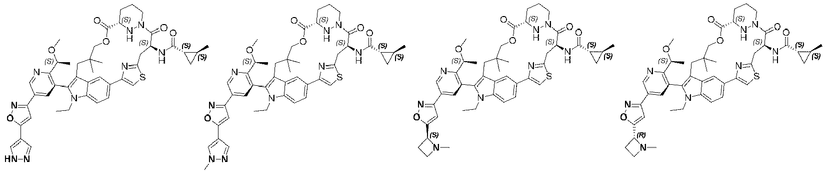

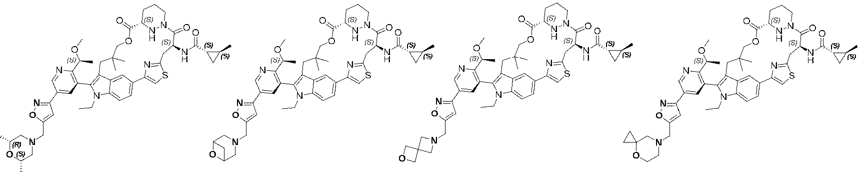

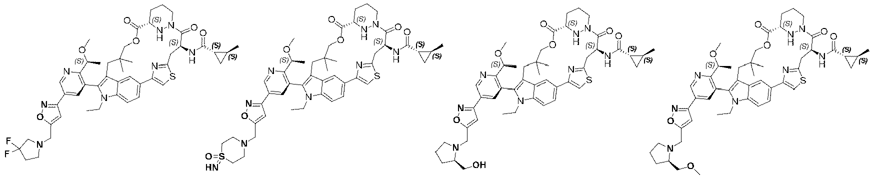

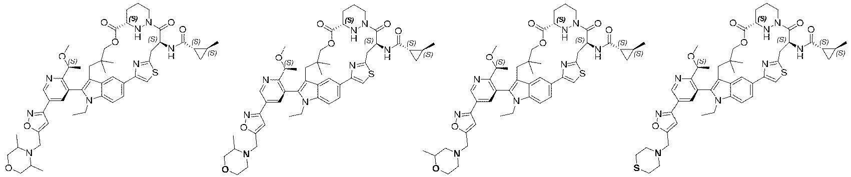

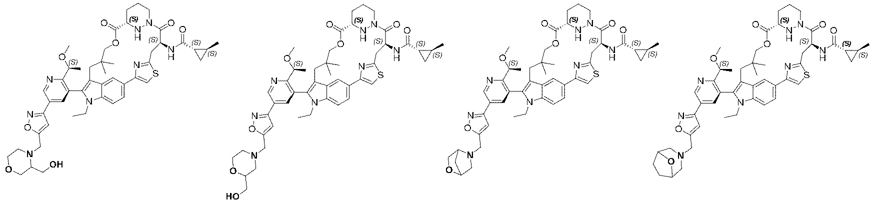

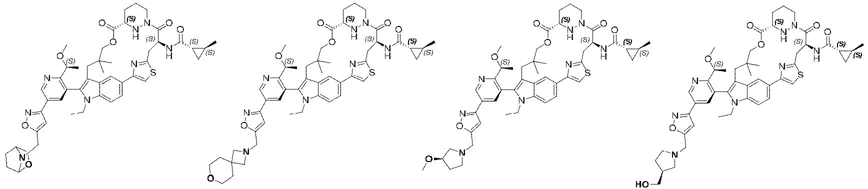

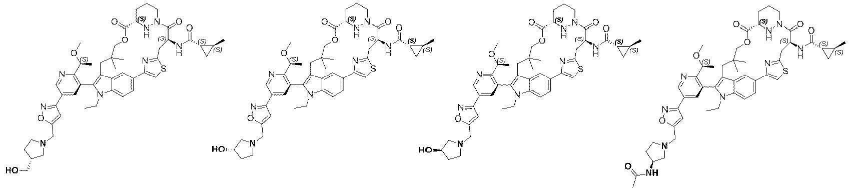

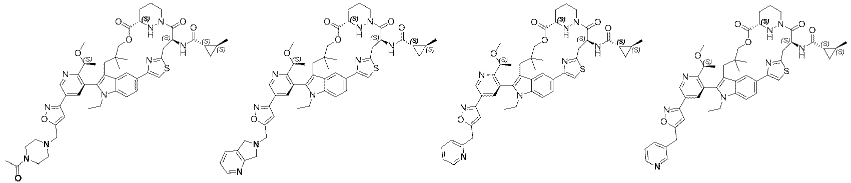

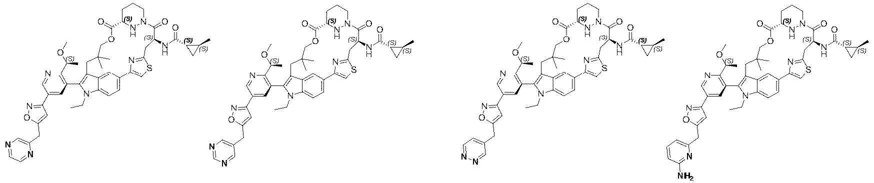

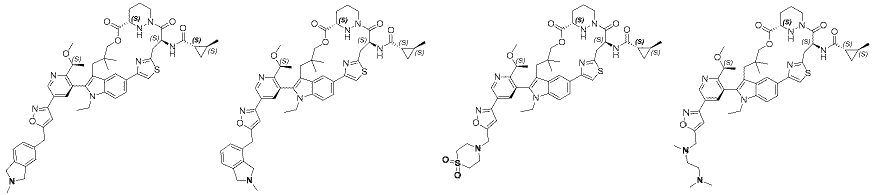

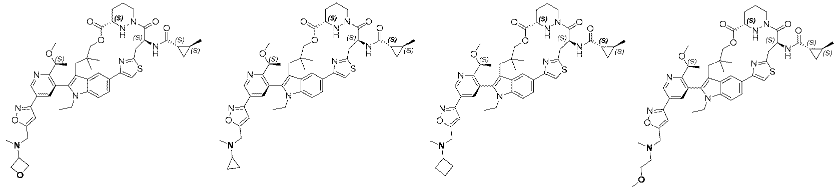

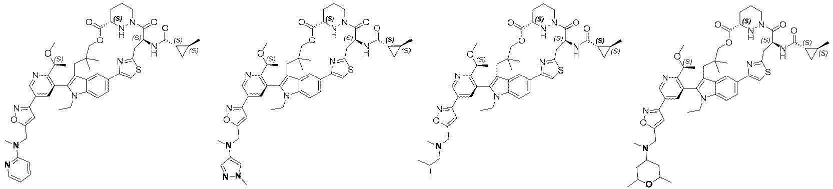

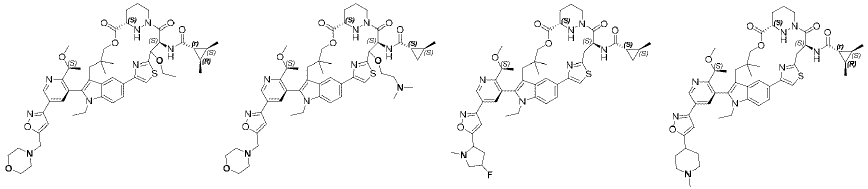

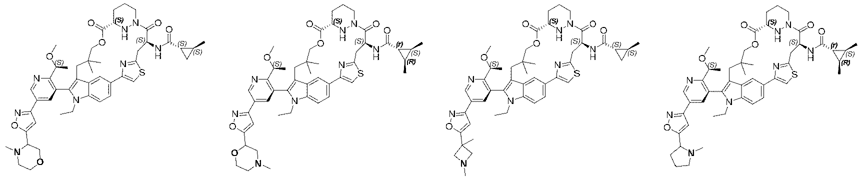

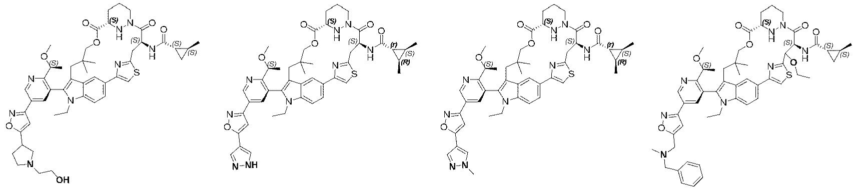

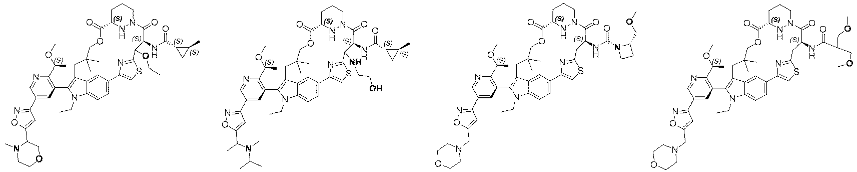

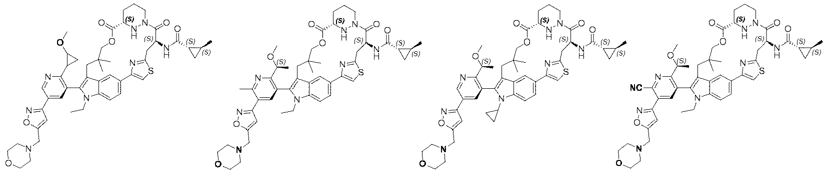

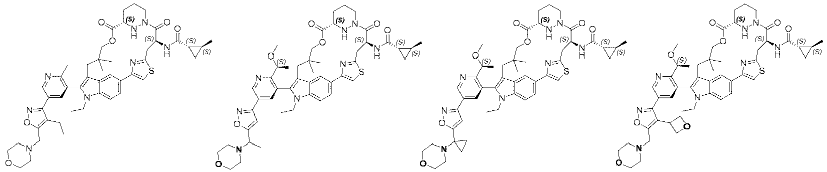

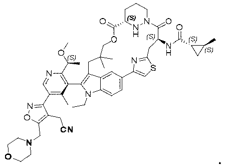

In another aspect, the present disclosure provides a pharmaceutical composition comprising the aforementioned compound or its pharmaceutically acceptable salt, isotope derivative, stereoisomer.

In another aspect, the present disclosure provides the use of the aforementioned compound or its pharmaceutically acceptable salt, isotope derivative, stereoisomer or pharmaceutical composition in the preparation of a drug for preventing and/or treating cancer, tumor, inflammatory disease, autoimmune disease or immune-mediated disease.

In another aspect, the present disclosure provides a method for preventing and/or treating cancer, tumor, inflammatory disease, autoimmune disease or immune-mediated disease, comprising administering a therapeutically effective amount of the aforementioned compound or its pharmaceutically acceptable salt, isotope derivative, stereoisomer and/or pharmaceutical composition to a patient in need.

It should be noted that, in this article, when referring to the "compound" of formula (I) and formula (II) and formula (V), it generally also covers its stereoisomers, diastere-omers, enantiomers, racemic mixtures and isotopic derivatives.

It is well known to those skilled in the art that a compound's salt, solvate, and hydrate are alternative forms of the compound, and they can all be converted into the compound under certain conditions. When referring to the compounds of formula (I) and formula (II) and formula (V), generally, their pharmaceutically acceptable salts are also included, and furthermore, their solvates and hydrates are also included.

Similarly, when referring to a compound herein, its prodrugs, metabolites and nitroxides are also generally included.

The pharmaceutically acceptable salts of the present disclosure may be formed using, for example, the following inorganic or organic acids. "Pharmaceutically acceptable salt" means a salt which, within the scope of reasonable medical judgment, is suitable for use in contact with tissues of humans and lower animals, without undue toxicity, irritation, allergic reaction, etc., can be called a reasonable benefit / risk ratio. The salts can be prepared in situ during the final isolation and purification of the compounds of the disclosure, or alone by reacting the free base or acid with a suitable reagent, as outlined below. For example, a free base function can be reacted with a suitable acid. Examples of pharmaceutically acceptable inorganic acid addition salts are amino acids with inorganic acids (e.g., hydrochloric, hydrobromic, phosphoric, sulfuric, and perchloric) or organic acids (e.g., acetic, oxalic, maleic, tartaric, lemon acid, succinic acid or malonic acid), or by using other methods known in the art such as ion exchange. Other pharmaceutically acceptable salts include adipate, sodium alginate, ascorbate, aspartate, benzenesulfonate, benzoate, bisulfate, borate, butyrate, camphorate, camphorsulfonate, citrate, cyclopentanepropionate, digluconate, lauryl sulfate, ethylate, formate, fumarate, glucoheptonate, glycerin phosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectate, persulfate, 3-phenylpropionate, phosphate, bitter salt, pivalate, propionate, stearate, succinate, sulfate, tartrate, thiocya-nate, p-toluene sulfonate, undecanoate, valerate, etc. Representative alkali or alkaline earth metal salts include those of sodium, lithium, potassium, calcium, magnesium, and the like. Other pharmaceutically acceptable salts include, where appropriate, nontoxic ammonium salts, quaternary ammonium salts, and amine cations formed with counteri-ons, for example, halides, hydroxides, carboxylates, sulfates, phosphates, nitrates, lower alkylsulfonates and arylsulfonates.

The pharmaceutically acceptable salts of the present disclosure can be prepared by conventional methods, for example, by dissolving the compound of the present invention in a water-miscible organic solvent (such as acetone, methanol, ethanol and acetonitrile), adding an excess of organic acid or inorganic acid aqueous solution, so that the salt is precipitated from the resulting mixture, the solvent and remaining free acid are removed therefrom, and the precipitated salt is isolated.

The precursors or metabolites described in the present disclosure may be precursors or metabolites known in the art, as long as the precursors or metabolites are transformed into compounds through in vivo metabolism. For example, "prodrugs" refer to those prodrugs of the compounds of the present disclosure which, within the scope of sound medical judgment, are suitable for use in contact with human and lower animal tissues without undue toxicity, irritation, allergic response, etc., qualified as having a reasonable benefit/risk ratio and valid for its intended use. The term "prodrug" refers to a com-pound that is rapidly transformed in vivo to yield the parent compound of the above formula, for example by in vivo metabolism, or N-demethylation of a compound of the disclosure.

"Solvate" as used herein means a physical association of a compound of the present disclosure with one or more solvent molecules, whether organic or inorganic. This physical association includes hydrogen bonding. In some cases, for example, when one or more solvent molecules are incorporated into the crystal lattice of a crystalline solid, solvates will be able to be isolated. Solvent molecules in solvates may exist in regular and/or disordered arrangements. Solvates may contain stoichiometric or non-stoichiometric amounts of solvent molecules. "Solvate" encompasses both solu-tion-phase and isolatable solvates. Exemplary solvates include, but are not limited to, hydrates, ethanolates, methanolates, and isopropanolates. Solvation methods are well known in the art.

The "stereoisomerism" described in the present disclosure is divided into conforma-tional isomerism and configurational isomerism, and configurational isomerism can also be divided into cis-trans isomerism and optical isomerism (that is, optical isomerism). Due to the rotation or twisting of carbon and carbon single bonds in organic molecules of a certain configuration, a stereoisomerism phenomenon in which each atom or atomic group of the molecule has a different arrangement in space, the common structures are alkanes and cycloalkanes. Such as the chair conformation and boat conformation that appear in the structure of cyclohexane. "Stereoisomer" means when a compound of the present disclosure contains one or more asymmetric centers and is thus available as racemates and racemic mixtures, single enantiomers, diastereomeric mixtures and single diastereomers. The compound of the present disclosure has an asymmetric center, and each asymmetric center can produce two optical isomers, and the scope of the present disclosure includes all possible optical isomers and diastereoisomer mixtures and pure or partially pure compounds. The compounds described herein may exist in tautomeric forms having different points of attachment of hydrogens by displacement of one or more double bonds. For example, a ketone and its enol form are keto-enol tautomers. Each tautomer and mixtures thereof are included in the compounds of the present in-vention. All enantiomers, diastereoisomers, racemates, mesoforms, cis-trans isomers, tautomers, geometric isomers, epimers of compounds of formula (I) to formula (IV) and mixtures thereof are included in the scope of the present disclosure.

An "isotopic derivative" of the present disclosure refers to a molecule in which a compound is isotopically labeled. Isotopes commonly used for isotopic labeling are: hydrogen isotopes, ²H and ³H; carbon isotopes: ¹¹C, ¹³C and ¹⁴C; chlorine isotopes: ³⁵Cl and ³⁷Cl; fluorine isotopes: ¹⁸F; iodine isotopes: ¹²³I and ¹²⁵I; nitrogen isotopes: ¹³N and ¹⁵N; Oxygen isotopes: ¹⁵O, ¹⁷O and ¹⁸O and sulfur isotope ³⁵S. These isotope-labeled compounds can be used to study the distribution of pharmaceutical molecules in tissues. Especially deuterium ³H and carbon ¹³C are more widely used due to their easy labeling and convenient detection. Substitution of certain heavy isotopes, such as deuterium (²H), can enhance metabolic stability, prolong half-life and thus achieve the purpose of reducing doses and provide therapeutic advantages. Isotopically labeled compounds are generally synthesized starting from labeled starting materials and carried out in the same way as non-isotopically labeled compounds using known synthetic techniques.

The present disclosure also provides the use of the compound of the present in-vention in the preparation of medicaments for preventing and/or treating cancer, tumor, inflammatory disease, autoimmune disease or immune-mediated disease.

In addition, the present disclosure provides a pharmaceutical composition for preventing and/or treating cancer, tumor, inflammatory disease, autoimmune disease, neurodegenerative disease, attention-related disease or immune-mediated disease, which comprises the present disclosure compounds as active ingredients. The pharmaceutical composition may optionally comprise a pharmaceutically acceptable carrier.

In addition, the present disclosure provides a method of preventing and/or treating cancer, tumor, inflammatory disease, autoimmune disease, neurodegenerative disease, attention-related disease or immune-mediated disease, which comprises administering the compound of the disclosure to the mammals in need thereof.

Representative examples of inflammatory, autoimmune, and immune-mediated diseases may include, but are not limited to, arthritis, rheumatoid arthritis, spondyloarthritis, gouty arthritis, osteoarthritis, juvenile arthritis , Other Arthritis Conditions, Lupus, Systemic Lupus Erythematosus (SLE), Skin Related Disorders, Psoriasis, Eczema, Dermatitis, Atopic Dermatitis, Pain, Pulmonary Disease, Lung Inflammation, Adult Respiratory Distress Syndrome (ARDS) , pulmonary sarcoidosis, chronic pulmonary inflammatory disease, chronic obstructive pulmonary disease (COPD), cardiovascular disease, atherosclerosis, myocardial infarction, congestive heart failure, myocardial is-chemia-reperfusion injury, inflammatory bowel disease, Crohn's disease, ulcerative coli-tis, irritable bowel syndrome, asthma, Sjogren's syndrome, autoimmune thyroid disease, urticaria (rubella), multiple sclerosis, scleroderma, organ transplant rejection, xenograft, idiopathic thrombocytopenic purpura (ITP), Parkinson's disease, Alzheimer's disease, diabetes-related diseases, inflammation, pelvic inflammatory disease, allergic rhinitis, allergic bronchitis, allergic sinusitis, leukemia, lymphoma, B Cell Lymphoma, T Cell Lymphoma, Myeloma, Acute Lymphocytic Leukemia (ALL), Chronic Lymphocytic Leukemia (CLL), Acute Myeloid Leukemia (AML), Chronic Myelogenous Leukemia (CML), Hairy Cell Leukemia, He Jie King's disease, non-Hodgkin's lymphoma, multi-ple myeloma, myelodysplastic syndrome (MDS), myeloproliferative neoplasm (MPN), diffuse large B-cell lymphoma, and follicular lymphoma.

Representative examples of cancer or tumor may include, but are not limited to, skin cancer, bladder cancer, ovarian cancer, breast cancer, stomach cancer, pancreatic cancer, prostate cancer, colon cancer, lung cancer, bone cancer, brain cancer, neuro-blastoma, rectal cancer , colon cancer, familial adenomatous polyposis carcinoma, hereditary nonpolyposis colorectal cancer, esophagus cancer, lip cancer, larynx cancer, hypopharyngeal cancer, tongue cancer, salivary gland cancer, stomach cancer, adenocarcinoma, medullary thyroid cancer, Papillary thyroid cancer, renal cancer, renal parenchymal cancer, ovarian cancer, cervical cancer, uterine body cancer, endometrial cancer, choriocarcinoma, pancreatic cancer, prostate cancer, testicular cancer, urinary cancer, melanoma, brain tumors such as Glioblastoma, astrocytoma, meningioma, me-dulloblastoma and peripheral neuroectodermal tumor, Hodgkin lymphoma, non-Hodgkin lymphoma, Burkitt lymphoma, acute lymphoblastic leukemia ( ALL), chronic lymphocytic leukemia (CLL), acute myeloid leukemia (AML), chronic mye-logenous leukemia (CML), adult T-cell leukemia lymphoma, diffuse large B-cell lym-phoma (DLBCL), hepatocellular carcinoma, gallbladder Carcinoma, bronchial carci-noma, small cell lung cancer, non-small cell lung cancer, multiple myeloma, basal cell tumor, teratoma, retinoblastoma, choroidal melanoma, seminoma, rhabdomyosarcoma, craniopharyngioma, Osteosarcoma, chondrosarcoma, sarcoma, liposarcoma, fibrosar-coma, Ewing sarcoma, or plasmacytoma.

When the compound of the present disclosure or a pharmaceutically acceptable salt thereof is administered in combination with another anticancer agent or immune checkpoint inhibitor for the treatment of cancer or tumors, the compound of the present disclosure or a pharmaceutically acceptable salt thereof can provide enhanced anticancer effects .

Representative examples of anticancer agents useful in the treatment of cancer or tumors may include, but are not limited to, cell signal transduction inhibitor, chlorambu-cil, melphalan, cyclophosphamide, ifosfamide, busulfan, carmustine, lomustine, strep-tozotocin, cisplatin, carboplatin, oxaliplatin, dacarbazine, temozolomide, procarbazine, methotrexate, fluorouracil, cytarabine, gemcitabine, Mercaptopurine, fludarabine, vin-blastine, vincristine, vinorelbine, paclitaxel, docetaxel, topotecan, irinotecan, etoposide, trabectedin, dactinomycin, doxorubicin , epirubicin, daunorubicin, mitoxantrone, bleo-mycin, mitomycin C, ixabepilone, tamoxifen, flutamide, gonadorelin analogs, metha-done progesterone, prednisone, dexamethasone, methylprednisolone, thalidomide, in-terferon alpha, leucovorin, sirolimus, sirolimus ester, everolimus, afatinib, alisertib, amuvatinib, apatinib, axitinib, bortezomib, bosutinib, britinib, cabozantinib, cediranib, crenolanib, kezhuotinib, dabrafenib, dabrafenib, Cotinib, danucitinib, dasatinib, dovit-inib, erlotinib, foretinib, ganetespib, gefitinib, ibrutinib, icotinib, imatinib, iniparib, la Patinib, lenvatinib, linifanib, linsitinib, masitinib, momelotinib, motisanib, neratinib, ni-lotinib, niraparib, oprozomib, olaparib, pazopanib, pictilisib, ponatinib, quizartinib, regorafenib, rigosertib, rucaparib, ruxolitinib, saracatinib, saridegib, sorafenib, sunitinib, tiratinib, tivantinib, tivozanib, tofacitinib, trametinib, vandetanib, veliparib, vemurafenib, vimodegib, volasertib, alemtuzumab, bevacizumab, berentuzumab vedotin, catumaxumab Antibodies, cetuximab, denosumab, gemtuzumab, ipilimumab, nimotuzumab, ofatumumab, panitumumab, rituximab, tositumomab, trastuzumab, PI3K inhibitor, CSF1R inhibitor, A2A and/or A2B receptor antagonist, IDO inhibitor, anti-PD-1 antibody, anti-PD-L₁ antibody, LAG3 antibody, TIM-3 antibody and an anti-CTLA-4 antibody, or any combination thereof.

When administered in combination with other therapeutic agents for the treatment of inflammatory disease, autoimmune disease and immune-mediated disease, the compounds of the present disclosure, or pharmaceutically acceptable salts thereof provide enhanced therapeutic effect.

Representative examples of therapeutic agents useful in the treatment of inflammatory, autoimmune, and immune-mediated diseases can include, but are not limited to, steroidal agents (e.g., prednisone, prednisone, prednisone, methylphenidate, cortisone, hydroxycortisone, betamethasone, dexamethasone, etc.), methotrexate, leflunomide, anti-TNF α agents (eg, etanercept, infliximab, adalib monoclonal antibody, etc.), cal-cineurin inhibitors (e.g., tacrolimus, pimecrolimus, etc.), and antihistamines (e.g., di-phenhydramine, hydroxyzine, loratadine, ebastine, ketotifen, cetirizine, levocetirizine, fexofenadine, etc.), and at least one or more therapeutic agents selected from them can be included in the pharmaceutical composition of the present disclosure.

Other features of the disclosure will become apparent in the course of the description of exemplary embodiments of the disclosure which are given to illustrate the disclosure and are not intended to be limiting thereof. In the following examples the methods disclosed in the disclosure were used for preparation, separation and characterization.

The compounds of the present disclosure can be prepared in a variety of methods known to those skilled in the art of organic synthesis, the methods described below as well as synthetic methods known in the art of synthetic organic chemistry or by variations thereof known to those skilled in the art may be used for the synthesis of the compounds of the present disclosure. Preferred methods include, but are not limited to, those described below. Reactions are performed in solvents or solvent mixtures appropriate to the kit materials used and to the transformations effected. Those skilled in the art of organic synthesis will appreciate that the functionality present on the molecule is consistent with the proposed transitions. This sometimes requires judgment to alter the order of synthetic steps or starting materials to obtain the desired compound of the disclosure.

### Embodiments

Unless otherwise stated, the terms used in the present application, including the specification and claims, are defined as follows. If not stated otherwise, conventional methods of mass spectrometry, NMR, HPLC, protein chemistry, biochemistry, recombinant DNA techniques and pharmacology are used. In this application, the use of "or" or "and" means "and/or" if not stated otherwise.

In the specification and claims, a given chemical formula or name shall encompass all stereoisomers and optical isomers thereof and racemates in which such isomers exist. Unless otherwise indicated, all chiral (enantiomers and diastereoisomers) and racemic forms are within the scope of the disclosure. Various geometric isomers of C=C double bonds, C=N double bonds, ring systems, etc. may also exist in the compounds, and all such stable isomers are encompassed by the present disclosure. The present disclosure describes cis- and trans- (or E- and Z-) geometric isomers of the compounds of the disclosure and which may be isolated as a mixture of isomers or as separated isomeric forms. The compounds of the disclosure may be isolated in optically active or racemic forms. All methods used to prepare the compounds of the disclosure and intermediates prepared therein are considered part of the disclosure. When preparing enantiomeric or diastereomeric products, they may be separated by customary methods, for example by chromatography or fractional crystallization. Depending on the process conditions, the end products of the disclosure are obtained in free (neutral) or salt form. The free forms and salts of these end products are within the scope of the present disclosure. A compound can be converted from one form to another, if desired. A free base or acid can be converted into a salt; a salt can be converted into the free compound or another salt; a mixture of isomeric compounds of the disclosure can be separated into the individual isomers. The compounds of the disclosure, their free forms and salts, may exist in various tautomeric forms in which the hydrogen atoms are transposed to other parts of the molecule and thus the chemical bonds between the atoms of the molecule are rearranged. It is to be understood that all tautomeric forms which may exist are included within the present disclosure.

In the present disclosure, when a listed linking group does not specify its direction of connectivity, the direction is considered to be arbitrary. For example, when L in 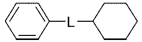 is -C(O)NH-, the group -C(O)NH- may connect a phenyl group and a cyclohexyl group in the left-to-right reading order to form 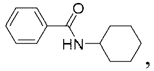 or in the opposite reading order to form 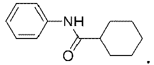 Such combinations of the linking group and the connected groups are permitted only when they result in a stable compound. In some preferred embodiments of the present disclosure, the connectivity follows the left-to-right reading order.

Unless otherwise defined, the definitions of the substituents in the present disclosure are independent and not interrelated, for example (listed but not exhaustive), in one aspect, for the substituents R^{a} (or R^{a}') in different definitions of the substituents, the definitions are independent of each other. Specifically, when one definition is selected for R^{a} (or R^{a}') in one substituent, it does not mean that R^{a} (or R^{a}') has the same definition in other substituents. More specifically, for example (but not exhaustively) for NR^{a}R^{a}', when R^{a} (or R^{a}') is defined from hydrogen, it does not mean that in -C(O)-NR^{a}R^{a}', R^{a} (or R^{a}') must be hydrogen. In another aspect, when there is more than one R^{a} (or R^{a}') in a certain substituent, these R^{a} (or R^{a}') are also independent. For example, in the substituent -(CR^{a}R^{a}')m-O-(CR^{a}R^{a}')n-, when m+n is greater than or equal to 2, the m+n pieces of R^{a} (or R^{a}') are independent, and they can be have the same or different meanings.

Unless otherwise defined, when a substituent is noted as "optionally substituted", the substituent is selected from, for example, the following substituents, such as alkyl, cycloalkyl, aryl, heterocyclyl, halogen, hydroxy, alkoxy, oxo, alkanoyl, aryloxy, alkanoyloxy, amino, alkylamino, arylamino, arylalkylamino, disubstituted amino (wherein 2 amino substituents are selected from alkyl radical, aryl or arylalkyl), alkanoylamino, aroylamino, aralkanoylamino, substituted alkanoylamino, substituted arylamino, substituted aralkanoylamino, thio, alkylthio, arylthio, arylalkylthio, arylthiocarbonyl, arylalkylthiocarbonyl, alkylsulfonyl, arylsulfonyl, arylalkylsulfonyl, aminosulfonyl such as -SO2NH2, substituted sulfonylamino, nitro, cyano, carboxyl, carbamoyl such as -CONH2, substituted carbamoyl such as -CONHalkyl, -CONHaryl, -CONHarylalkyl or two optional In the case of substituents from alkyl, aryl or arylalkyl, alkoxycarbonyl, aryl, substituted aryl, guanidino, heterocyclic, e.g. indolyl, imidazolyl, furyl, thienyl , Thiazolyl, pyrrolidinyl, pyridyl, pyrimidinyl, pyrrolidinyl, piperidinyl, morpholinyl, piperazinyl, homopiperazinyl, etc. and substituted heterocyclyl.

As used herein, the term "alkyl" is intended to include saturated aliphatic hydrocarbon groups having a specified number of carbon atoms, which may be either branched or straight-chained. For example, "C₁-C₆ alkyl" refers to an alkyl group containing from 1 to 6 carbon atoms. Examples of alkyl groups include, but are not limited to, methyl (Me), ethyl (Et), propyl (e.g., n-propyl and isopropyl), butyl (e.g., n-butyl, isobutyl, and tert-butyl), and pentyl (e.g., n-pentyl, isopentyl, and neopentyl). The alkyl group may be unsubstituted or substituted, and when substituted, may be substituted at any available position. The substituents are preferably selected from one or more of deuterium, halogen, hydroxyl, amino, cyano, alkyl, alkoxy, haloalkyl, cycloalkyl, heterocycloalkyl, aryl, and heteroaryl groups. In the present disclosure, the alkyl group is preferably an alkyl group having 1 to 6, more preferably 1 to 4 carbon atoms.

As used herein, the term "alkylene" is intended to refer to saturated aliphatic hydrocarbon bridges, either straight- or branched-chain, optionally containing or not containing a cycloalkyl group, derived by removal of two hydrogen atoms from the same or two different carbon atoms of a parent alkane. For example, "C₀-C₆ alkylene" refers to alkylene bridges having 0 (i.e., a bond), 1, 2, 3, 4, 5, or 6 carbon atoms. Examples of alkylene include, but are not limited to, methylene (-CH₂ - ), ethylene ( - CH₂CH₂ - ), propylene (e.g., -(CH₂)₃ - , - (CHCH₃)CH₂ - , - (CHCH₂CH) - ), butylene (e.g., - (CH₂)₄ - , - CH₂CH(CH₂CH₃) - , - CH₂(CHCH₂CH) - ), pentylene (e.g., - (CH₂)₅ - , - CH₂CH(CH(CH₃)₂) - , - CH₂(CHCH₂CH)CH₂ - ), and hexylene (e.g., - (CH₂)₆ - , - CH₂CH₂CH(CH(CH₃)₂) - , - CH₂(CHCH(CH₃)CH)CH₂ - ). Herein, the alkylene is preferably alkylene groups having 0 to 6, 0 to 4, 0 to 3, 1 to 6, 1 to 4, 1 to 3 carbon atoms , more preferably those not comprising cycloalkyl moieties.

The term "cycloalkyl" refers to a saturated hydrocarbon ring, which may be monocyclic, polycyclic, or branched. Exemplary C₃-C₁₂ cycloalkyl groups include, but are not limited to, cyclopropyl, cyclobutyl, cyclopentyl, cyclohexyl, and norbornyl. Branched cycloalkyls, such as 1-methylcyclopropyl and 2-methylcyclopropyl, are included. Polycyclic structures include bicyclic and tricyclic cycloalkyls, which may be bridged, spiro, or fused. Cycloalkyl groups may be unsubstituted or substituted at any available position, and the substituents are preferably selected from one or more of halogen, hydroxyl, amino, cyano, oxo, alkyl, alkoxy, haloalkyl, cycloalkyl, heterocycloalkyl, aryl, and heteroaryl. In the present disclosure, C₃-C₁₂ cycloalkyl groups are preferred, more preferably C₃-C₈.

Similarly, the term "heterocycloalkyl" refers to a saturated cyclic structure in which at least one carbon atom in a cycloalkyl ring is replaced by a heteroatom selected from N, O, S, or P. The nitrogen may optionally be quaternized, and N or S may be oxidized (e.g., to NO, SO, or SO₂). The term includes monocyclic, bicyclic, and tricyclic heterocyclic structures, including spiro, fused, and bridged heterocycles. The heterocycloalkyl may be unsubstituted or substituted, and when substituted, may be substituted at any available position. The substituents are preferably selected from one or more of halogen, hydroxyl, amino, cyano, oxo, alkyl, alkoxy, haloalkyl, cycloalkyl, heterocycloalkyl, aryl, and heteroaryl groups. In the present disclosure, 4- to 12-membered heterocycloalkyl groups are preferred, more preferably 4- to 8-membered ones.

In the present disclosure, the term "fused ring" refers to a polycyclic group formed by two or more cyclic structures sharing two adjacent atoms.

In the present disclosure, the term "bridged ring" refers to a polycyclic group in which two rings share more than two ring atoms.

In the present disclosure, the term "spiro ring" refers to a polycyclic group formed by two monocyclic structures sharing a single carbon atom, referred to as a spiro atom.

The term "alkenyl" denotes a straight or branched chain hydrocarbon group containing one or more double bonds and generally having a length of 2 to 20 carbon atoms. For example, "C2-C6 alkenyl" contains two to six carbon atoms. Alkenyl groups include, but are not limited to, e.g. vinyl, propenyl, butenyl, 1-methyl-2-buten-1-yl, and the like. Herein, alkenyl is preferably C₂-C₆ alkenyl.

The term "cycloalkenyl" refers to a monocyclic or bicyclic cyclic alkenyl group. Monocyclic cycloalkenyl refers to C₃-C₈ cyclic alkenyl, including but not limited to cyclopropenyl, cyclobutenyl, cyclopentenyl, cyclohexenyl and norbornenyl. Branched cycloalkenyl groups such as 1-methylcyclopropenyl and 2-methylcyclopropenyl are included within the definition of "cycloalkenyl". Bicyclic cycloalkenyl groups include bridged, spiro, or fused cycloalkenyl groups.

The term "alkynyl" denotes a straight or branched chain hydrocarbon group containing one or more triple bonds and generally having a length of 2 to 20 carbon atoms. For example, "C₂-C₆ alkynyl" contains two to six carbon atoms. Representative alkynyl groups include, but are not limited to, for example, ethynyl, 1-propynyl, 1-butynyl, and the like. Herein, the alkynyl group is preferably a C₂-C₆ alkynyl group.

The term "alkoxy" or "alkyloxy" refers to -O-alkyl. "C₁-C₆ alkoxy" (or alkyloxy) is intended to include C₁, C₂, C₃, C₄, C₅, C₆ alkoxy. Examples of alkoxy include, but are not limited to, methoxy, ethoxy, propoxy (e.g., n-propoxy and isopropoxy), and tert-butoxy. Herein, the alkoxy group is preferably an alkoxy group having 1 to 6, more preferably 1 to 4 carbon atoms. Similarly, "alkylthio" or "thiothio" denotes an alkyl group as defined above having the indicated number of carbon atoms attached through a sulfur bridge; e.g. methyl-S- and ethyl-S-. The alkoxy group may be unsubstituted or substituted, and when substituted, it may be substituted at any available position. Preferred substituents are selected from one or more of deuterium, halogen, hydroxyl, amino, cyano, alkyl, alkoxy, haloalkyl, cycloalkyl, heterocycloalkyl, aryl, and heteroaryl.

The term "carbonyl" refers to an organic functional group (C=O) consisting of two atoms, carbon and oxygen, linked by a double bond.

The term "aryl", alone or as part of a larger moiety such as "aralkyl", "arylalkoxy" or "aryloxyalkyl", refers to a monocyclic, bicyclic or tricyclic ring system having a total of 5 to 12 ring members, wherein at least one ring in the system is aromatic and wherein each ring in the system contains 3 to 7 ring members. In certain embodiments of the disclosure, "aryl" refers to an aromatic ring system which includes, but is not limited to, phenyl, biphenyl, indanyl, 1-naphthyl, 2-naphthyl, and tetrahydronaphthyl. The term "aralkyl" or "arylalkyl" refers to an alkyl residue attached to an aryl ring, non-limiting examples of which include benzyl, phenethyl, and the like. A fused aryl group can be attached to another group at a suitable position on the cycloalkyl ring or aromatic ring. Dashed lines drawn from ring systems indicate that bonds may be attached to any suitable ring atom. The aryl group may be unsubstituted or substituted, and when substituted, it may be substituted at any available position. The substituents are preferably selected from one or more of deuterium, halogen, hydroxyl, amino, cyano, alkyl, alkoxy, haloalkyl, cycloalkyl, heterocycloalkyl, aryl, and heteroaryl.

As used herein, the term "heterocycle" refers to a cyclic structure containing one or more heteroatoms, which may be fully saturated, partially saturated, or fully unsaturated, and includes heteroaryl rings.

The term "heteroaryl" means a stable 5-membered, 6-membered, or 7-membered aromatic monocyclic ring or aromatic bicyclic ring or a 7-membered, 8-membered, 9-membered, 10-membered, 11-membered, 12-membered aromatic polycyclic heterocycle, which is fully unsaturated or partially unsaturated, and contains carbon atoms and 1, 2, 3 or 4 heteroatoms independently selected from N, O and S; and includes cycloalkane or heterocycloalkane with a structure in which aromatic rings such as benzene rings or heteroaromatic rings such as pyridine are condensed. The position of the structure as a substituent may be located on a cycloalkane, a heterocycloalkane, an aromatic ring or a heteroaryl ring. Nitrogen and sulfur heteroatoms can be optionally oxidized. The nitrogen atom is substituted or unsubstituted (i.e. N or NR, where R is H or another substituent if defined). A heterocycle can be attached to its pendant group at any heteroatom or carbon atom that results in a stable structure. The heterocyclyl groups described herein may be substituted on carbon or nitrogen atoms if the resulting compound is stable. The nitrogen in the heterocycle can optionally be quaternized. Preferably, when the total number of S and O atoms in the heterocycle exceeds 1, then these heteroatoms are not adjacent to each other. Preferably, the total number of S and O atoms in the heterocycle is not greater than one. When the term "heterocycle" is used, it is intended to include heteroaryl. Examples of heteroaryl groups include, but are not limited to, acridinyl, azetidinyl, aziocinyl, benzimidazolyl, benzofuryl, benzothiofuranyl, benzothienyl, benzooxa azolyl, benzoxazolyl, benzothiazolyl, benzotriazolyl, benzotetrazolyl, benzisoxazolyl, benzisothiazolyl, benzimidazolyl, carbazolyl, 4aH-carbazolyl, carbolinyl, chromanyl, chromenyl, cinnolinyl, decahydroquinolyl, 2H,6H-1,5,2-dithiazinyl, dihydrofuro[2, 3-b] tetrahydrofuryl, furyl, furanyl, imidazolidinyl, imidazolinyl, imidazolyl, 1H-indazolyl, imidazopyridyl, indolenyl, indolinyl, Indolazinyl, indolyl, 3H-indolyl, isatinoyl, isobenzofuryl, isochromanyl, isoindazolyl, isoindolinyl, isoindolyl, isoindolyl quinolinyl, isothiazolyl, isothiazolopyridyl, isoxazolyl, isoxazolopyridyl, methylenedioxyphenyl, morpholinyl, diazanaphthyl, octahydroisoquinoline oxadiazolyl, 1,2,3-oxadiazolyl, 1,2,4-oxadiazolyl, 1,2,5-oxadiazolyl, 1,3,4-oxadiazolyl , oxazolidinyl, oxazolyl, oxazolopyridyl, oxazolidinyl, diazaphenyl, oxindolyl, pyrimidinyl, phenanthridinyl, phenanthrolinyl, phenazinyl , phenothiazinyl, phenoxathiyl, phenoxazinyl, phthalazinyl, piperazinyl, piperidinyl, piperidinyl, 4-piperidinyl, piperonyl, pteridinyl, purinyl, pyranyl, pyrazinyl, pyrazolidinyl, pyrazolinyl, pyrazolopyridyl, pyrazolyl, pyridazinyl, pyridoxazolyl, pyridimidazolyl, pyridothiazolyl, pyridyl , pyrimidinyl, pyrrolidinyl, pyrrolinyl, 2-pyrrolidonyl, 2H-pyrrolyl, pyrrolyl, quinazolinyl, quinolinyl, 4H-quinazinyl, quinoxalinyl, quinuclidinyl, tetrazolyl, tetrahydrofuryl, tetrahydroisoquinolyl, tetrahydroquinolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthryl, thiazolyl, thienyl, thiazolopyridyl, thienothiazolyl, thienooxa Azolyl, thienoimidazolyl, thienyl, triazinyl, 1,2,3-triazolyl, 1,2,4-triazolyl, 1,2,5-triazolyl, 1,3,4-triazolyl and xanthenyl, quinolinyl, isoquinolyl, phthalazinyl, quinazolinyl, indolyl, isoindolyl, indolinyl, 1H-indazolyl, benzo Imidazolyl, 1,2,3,4-tetrahydroquinolinyl, 1,2,3,4-tetrahydroisoquinolinyl, 5,6,7,8-tetrahydro-quinolinyl, 2,3 -Dihydro-benzofuryl, chromanyl, 1,2,3,4-tetrahydro-quinoxalinyl and 1,2,3,4-tetrahydro-quinazolinyl. The term "heteroaryl" may also include biaryl structures formed by the above-defined "aryl" and a monocyclic "heteroaryl", such as but not limited to "-phenyl bipyridyl-", "- "phenyl bipyrimidyl", "-pyridyl biphenyl", "-pyridyl bipyrimidyl-", "-pyrimidyl biphenyl-"; wherein the present disclosure also includes condensed ring and spiro compound containing, for example, the above-mentioned heterocycles.

The term "substituted" as used herein means that at least one hydrogen atom is replaced by a non-hydrogen group, provided that normal valences are maintained and that the substitution results in a stable compound. A cyclic double bond, as used herein, is a double bond formed between two adjacent ring atoms (e.g., C=C, C=N or N=N).

In the present disclosure, the one or more halogens may each be independently selected from fluorine, chlorine, bromine and iodine.

"Halo" or "halogen" includes fluoro, chloro, bromo and iodo. "Haloalkyl"/"haloalkylene" is intended to include branched and straight chain saturated alkyl/alkylene groups having the indicated number of carbon atoms substituted with one or more halogens. Examples of haloalkyl include, but are not limited to, fluoromethyl, difluoromethyl, trifluoromethyl, trichloromethyl, pentafluoroethyl, pentachloroethyl, 2,2,2-trifluoroethyl, heptafluoro propyl and heptachloropropyl. Examples of haloalkyl also include "fluoroalkyl" intended to include branched and straight-chain saturated aliphatic hydrocarbon groups having the specified number of carbon atoms and substituted with 1 or more fluorine atoms. "Halocycloalkyl"/"haloheterocycloalkyl" is intended to include cycloalkyl/heterocycloalkyl having the indicated number of carbon atoms substituted with one or more halogens. In the present disclosure, the halogen atom is preferably fluorine or chlorine, more preferably fluorine.

"Haloalkoxy" or "haloalkyloxy" means a haloalkyl group as defined above having the indicated number of carbon atoms attached through an oxygen bridge. For example, "haloC₁-C₆ alkoxy" is intended to include C₁, C₂, C₃, C₄, C₅, C₆ haloalkoxy. Examples of haloalkoxy include, but are not limited to, trifluoromethoxy, 2,2,2-trifluoroethoxy, and pentafluoroethoxy. Similarly, "haloalkylthio" or "thiohaloalkoxy" denotes a haloalkyl group as defined above having the indicated number of carbon atoms attached through a sulfur bridge; for example trifluoromethyl-S- and pentafluoroethyl -S-.

In the present disclosure, the expression Cₓ₁-Cₓ₂ is used when referring to some substituent groups, which means that the number of carbon atoms in the substituent groups may be x1 to x2. For example, C₀-C₈ means that the group contains 0, 1, 2, 3, 4, 5, 6, 7 or 8 carbon atoms, C₁-C₈ means that the group contains 1, 2, 3, 4, 5 , 6, 7 or 8 carbon atoms, C₂-C₈ means that the group contains 2, 3, 4, 5, 6, 7 or 8 carbon atoms, C₃-C₈ means that the group contains 3, 4, 5 , 6, 7 or 8 carbon atoms, C₄-C₈ means that the group contains 4, 5, 6, 7 or 8 carbon atoms, C₀-C₆ means that the group contains 0, 1, 2, 3, 4 , 5 or 6 carbon atoms, C₁-C₆ means that the group contains 1, 2, 3, 4, 5 or 6 carbon atoms, C₂-C₆ means that the group contains 2, 3, 4, 5 or 6 carbon atoms, C₃-C₆ means that the group contains 3, 4, 5 or 6 carbon atoms.

In this disclosure, the expression "x1-x2 membered ring" is used when referring to cyclic groups such as aryl, heteroaryl, cycloalkyl, and heterocycloalkyl, which means that the number of the ring atoms of the group can be x1 to x2. For example, the 3-12 membered cyclic group may be a 3, 4, 5, 6, 7, 8, 9, 10, 11 or 12 membered ring, and the number of ring atoms may be 3, 4, 5, 6, 7, 8, 9, 10, 11 or 12; 3-6-membered ring means that the cyclic group can be 3, 4, 5 or 6-membered ring, and the number of ring atoms can be 3, 4, 5 or 6 ; 3-8 membered ring means that the cyclic group can be 3, 4, 5, 6, 7 or 8-membered ring, and the number of ring atoms can be 3, 4, 5, 6, 7 or 8; 3-9 membered ring means that the cyclic group can be a 3, 4, 5, 6, 7, 8 or 9-membered ring, and the number of ring atoms can be 3, 4, 5, 6, 7, 8 or 9; 4-7 membered ring means that the cyclic group can be a 4, 5, 6 or 7-membered ring, and the number of ring atoms can be 4, 5, 6 or 7; 5-8-membered ring means that the cyclic group can be 5, 6, 7 or 8-membered ring, the number of ring atoms can be 5, 6, 7 or 8; 5-12 membered ring means that the ring group can be 5, 6, 7, 8, 9, 10, 11 or 12-membered ring, the number of ring atoms can be 5, 6, 7, 8, 9, 10, 11 or 12; 6-12 membered ring means that the ring group can be 6, 7, 8, 9, 10, 11- or 12-membered rings, the number of ring atoms may be 6, 7, 8, 9, 10, 11 or 12. The ring atoms may be carbon atoms or heteroatoms, for example selected from N, O and S. When the ring is heterocyclic, the heterocyclic ring may contain 1, 2, 3, 4, 5, 6, 7, 8, 9, 10 or more ring heteroatoms, for example selected from N, O and S of heteroatoms.

Where nitrogen atoms (e.g. amines) are present on compounds of the disclosure, these nitrogen atoms can be converted to N-oxides by treatment with oxidizing agents (e.g. mCPBA and/or hydrogen peroxide) to obtain other compounds of the disclosure. Accordingly, both shown and claimed nitrogen atoms are considered to cover both the shown nitrogen and its N-oxides to obtain the derivatives of the present disclosure.

When any variable occurs more than one time in any composition or formula of a compound, its definition on each occurrence is independent of its definition at every other occurrence. Thus, for example, if a group is shown to be substituted with 0-3 R, then said group may be optionally substituted with up to three R groups, and R at each occurrence is independently selected from the definition of R. Also, combinations of substituents and/or variables are permissible only if such combinations result in stable compounds.

The term "patient" as used herein refers to an organism being treated by the methods of the present disclosure. Such organisms preferably include, but are not limited to, mammals (e.g., murine, ape, monkey, equine, bovine, porcine, canine, feline, etc.) and most preferably refer to humans.

The term "effective amount" as used herein means an amount of a drug or agent (i.e., a compound of the disclosure) that will elicit a biological or medical response in a tissue, system, animal or human being sought, e.g., by a researcher or clinician. Furthermore, the term "therapeutically effective amount" means an amount which results in improved treatment, cure, prevention or alleviation of a disease, disorder or side effect, or a reduction in the rate of disease or disorder progression. An effective amount may be given in one or more administrations, applications or doses and is not intended to be limited by a particular formulation or route of administration. The term also includes within its scope amounts effective to enhance normal physiological function.

As used herein, the term "treating" includes any effect that results in amelioration of a condition, disease, disorder, etc., such as alleviation, reduction, regulation, amelioration or elimination, or amelioration of the symptoms thereof.

The term "pharmaceutically acceptable" is used herein to refer to those compounds, substances, compositions and/or dosage forms: within the scope of sound medical judgment, they are suitable for use in contact with human and animal tissues without excessive toxicity, irritation, allergic reactions, and/or other problems or complications, commensurate with a reasonable benefit/risk ratio.

The phrase "pharmaceutically acceptable carrier" as used herein means a pharmaceutical substance, composition or vehicle, such as a liquid or solid filler, diluent, excipient, manufacturing aid (e.g. lubricant, talc, magnesium stearate, calcium stearate, or zinc stearate, or stearic acid) or solvent-encapsulated substances involved in the carrying or transport of a subject compound from one organ or body part to another. Each carrier must be "acceptable" in the sense of being compatible with the other ingredients of the formulation and not injurious to the patient.

The term "pharmaceutical composition" means a composition comprising a compound of the present disclosure together with at least one other pharmaceutically acceptable carrier. "Pharmaceutically acceptable carrier" means a medium generally accepted in the art for delivering a biologically active agent to an animal, particularly a mammal, including (i.e.) an adjuvant, excipient or vehicle, such as a diluent , preservatives, fillers, flow regulators, disintegrants, wetting agents, emulsifiers, suspending agents, sweeteners, flavoring agents, fragrances, antibacterial agents, antifungal agents, lubricants and dispersants, which depends on the mode of administration and the nature of the dosage form.

### Certain pharmaceutical and medical terms

The term "acceptable", as used herein, means that a formulation ingredient or active ingredient does not have an undue adverse effect on health for the general purpose of treatment.

The term "cancer", as used herein, refers to an abnormal growth of cells that cannot be controlled and, under certain conditions, is capable of metastasizing (spreading). Cancers of this type include, but are not limited to, solid tumors (e.g., bladder, bowel, brain, chest, uterus, heart, kidney, lung, lymphoid tissue (lymphoma), ovary, pancreas or other endocrine organs (e.g., thyroid), prostate, skin (melanoma), or blood cancer (such as non-leukemic leukemia).

The term "administration in combination" or similar terms, as used herein, refers to the administration of several selected therapeutic agents to a patient, in the same or different modes of administration at the same or different times.

The term "enhancing" or "capable of enhancing", as used herein, means that a desired result is capable of being increased or prolonged, either in potency or duration. Thus, in relation to enhancing the therapeutic effect of a drug, the term "capable of potentiating" refers to the ability of the drug to increase or prolong its potency or duration in the system. As used herein, "potency value" refers to the ability to maximize the enhancement of another therapeutic drug in an ideal system.

The term "immune disease" refers to a disease or condition of an adverse or deleterious reaction to an endogenous or exogenous antigen. The result is usually dysfunction of the cells, or destruction thereof and dysfunction, or destruction of organs or tissues that may produce immune symptoms.

The term "subject" or "patient" includes mammals and non-mammals. Mammals include, but are not limited to, mammals: humans, non-human primates such as orangutans, apes, and monkeys; agricultural animals such as cattle, horses, goats, sheep, and pigs; domestic animals such as rabbits and dogs; experimental animals include rodents, such as rats, mice and guinea pigs. Non-mammalian animals include, but are not limited to, birds, fish, and the like. In a preferred aspect, the selected mammal is a human.

The term "treatment", "course of treatment" or "therapy" as used herein includes alleviating, suppressing or ameliorating the symptoms or conditions of a disease; inhibiting the development of complications; ameliorating or preventing the underlying metabolic syndrome; inhibiting the development of diseases or symptoms, such as controlling the development of a disease or condition; alleviating a disease or a symptom; causing a disease or a symptom to regress; alleviating a complication caused by a disease or a symptom, or preventing and/or treating a sign caused by a disease or a symptom.

As used herein, a certain compound or pharmaceutical composition, after administration, can improve a certain disease, symptom or condition, especially improve its severity, delay the onset, slow down the progression of the disease, or reduce the duration of the disease. Circumstances that may be attributable to or related to the administration, whether fixed or episodic, continuous or intermittent.

### Example

### General process

When the preparation route is not included, the raw materials and reagents used in the present disclosure are known products, which can be synthesized according to methods known in the art, or can be obtained by purchasing commercially available products. All commercially available reagents were used without further purification.

Room temperature means 20-30°C.

There is no special description in the reaction examples, and the reactions are all carried out under a nitrogen atmosphere. The nitrogen atmosphere refers to a nitrogen balloon of about 1 L connected to the reaction flask.

The hydrogenation reaction is usually vacuumized and filled with hydrogen, and the operation is repeated 3 times. The hydrogen atmosphere means that the reaction bottle is connected with a hydrogen balloon of about 1L.

Microwave reactions use the Biotage^{®} Initiator+ Microwave Reactor.

The structures of the compounds of the present disclosure were determined by nuclear magnetic resonance (NMR) and mass spectroscopy (MS). NMR shifts (δ) are given in units of 10⁻⁶ (ppm). The determination of NMR is to use (Bruker Ascend^{™} 500 type) nuclear magnetic analyzer, the measurement solvent is deuterated dimethyl sulfoxide (DMSO-d6), deuterated chloroform (CDCl₃), deuterated methanol (CD₃OD), and the internal standard is tetramethylsilane (TMS). The following abbreviations are used for the multiplicity of NMR signals: s = singlet, brs = broad singlet, d = doublet, t = triplet, m = multiplet. Coupling constants are listed as J values, measured in Hz.

As to reverse-phase preparative chromatography, a Thermo (UltiMate 3000) reverse-phase preparative chromatograph was used. The flash column chromatography uses Agela (FS-9200T) automatic column passing machine, and the silica gel prepacked column uses Santai SEPAFLASH^{®} prepacked column. Yantai Huanghai HSGF254 or Qingdao GF254 silica gel plates are used for thin-layer chromatography silica gel plates, and the specifications used for thin-layer chromatography separation and purification products are 0.4mm to 0.5mm.

LC-MS analysis method is as follows:
1) Mass spectrometry method: Thermo Fisher MSQ PLUS mass spectrometer, ESI source, positive ion mode. Ion source parameter settings: drying gas temperature is 350 °C; drying gas flow rate is 10 L/min; MS Range: 120-1000.
2) Liquid phase conditions: Chromatographic column: Waters XBridge (3.5µm, 50mm×4.6mm); mobile phase A is an aqueous solution containing 0.1% ammonium bicarbonate, mobile phase B is an acetonitrile solution, and perform linear gradient elution in the following table 1; Flow rate: 2 mL/min; column temperature: 30°C; UV detection wavelength: 214nm, 254nm, 280nm; injection volume 2µL.

**Table 1. Gradient elution conditions**

| Time/min | Mobile phase A/% | Mobile phase B/% |
|---|---|---|
| 0 | 95 | 5 |
| 1.4 | 5 | 95 |
| 2.8 | 5 | 95 |
| 2.820 | 95 | 5 |
| 3 | 95 | 5 |

HPLC analysis method is as follows:
Chromatographic column: Waters XBridge phenyl (3.5µm, 150mm×4.6mm); mobile phase A is aqueous solution containing 0.1% ammonium bicarbonate, mobile phase B is acetonitrile solution, and linear gradient elution is carried out in the following table 2; flow rate: 1 mL/ min; column temperature: 30°C; UV detection wavelength: 214nm, 254nm, 280nm; injection volume 2µL.

**Table 2. Gradient elution conditions**

| Time/min | Mobile phase A/% | Mobile phase B/% |
|---|---|---|
| 0 | 95 | 5 |
| 15 | 5 | 95 |
| 20 | 5 | 95 |
| 20.2 | 95 | 5 |
| 24 | 95 | 5 |

The synthesis methods of some intermediates in the disclosure are as follows:

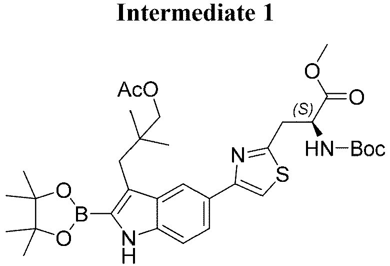

Intermediate 1 was prepared by the following steps:

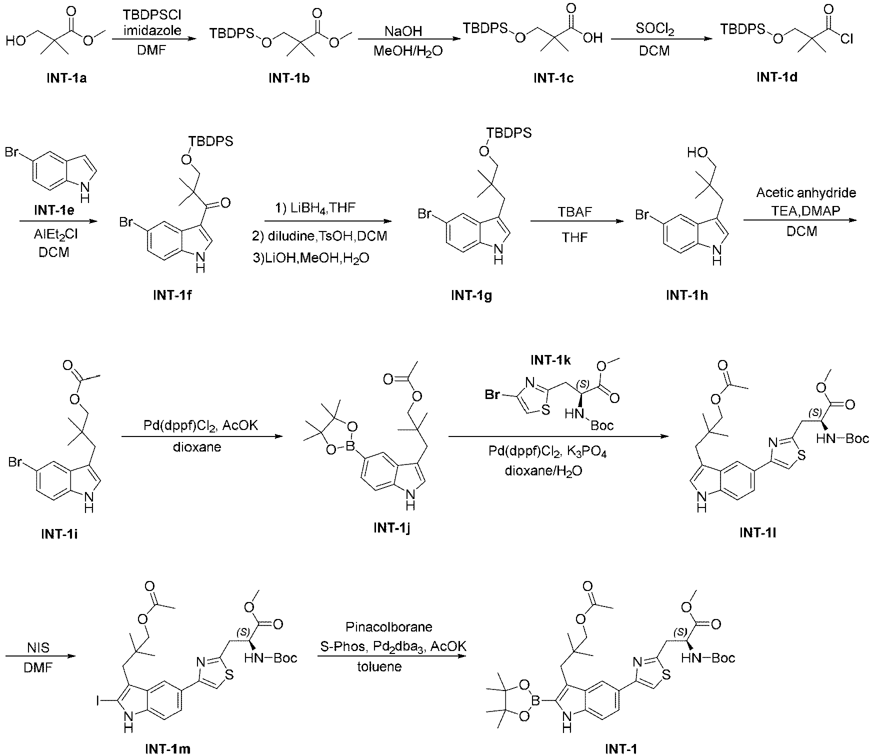

Step 1: Dissolve 2,2-dimethyl-3-hydroxypropionic acid methyl ester INT-1a (100 g, 756.7 mmol) in 1L N,N-dimethylformamide, add imidazole (128.8 g, 1.89 mol), stir to dissolve, add tert-butyldiphenylsilyl chloride (228.8 g, 832.3 mmol) dropwise at room temperature, and continue stirring for 4 hours after the addition is complete. After the reaction was complete, the reaction solution was poured into 3L of ice water, and the suspension was extracted with ethyl acetate (1L*2). The organic phase was washed with water three times and concentrated under reduced pressure to obtain a colorless oil INT-1b, which was directly used in the next step without purification. ESI-MS (m/z): 371.2 [M+H]⁺.

Step 2: Add the residual liquid INT-1b obtained in the previous step into 2 L of methanol, add 360 g of the prepared 33% sodium hydroxide aqueous solution, and stir at room temperature for 17 hours. After the reaction was completed, 1 L of water was added, and the methanol was removed under reduced pressure. The residual liquid was extracted with petroleum ether (1 L*5). After extraction, the pH value of the aqueous phase was adjusted to 4-5 with hydrochloric acid, and stirring was continued for 30 minutes. The mixture was filtered and dried to obtain a white solid INT-1c (269 g, yield 90%). ESI-MS (m/z): 357.8 [M+H]⁺.

Step 3: Dissolve INT-1c (130 g, 364.6 mmol) in 500 mL of dichloromethane, add thionyl chloride (130.1 g, 1.09 mol, 79.4 mL) at room temperature, stir at 60 °C for 3 hours, and when the reaction is complete, remove the dichloromethane and the remaining thionyl chloride under reduced pressure to obtain a light yellow oil INT-1d. Add 200 mL of dichloromethane for standby use without purification.

Step 4: Dissolve INT-1e (64.8 g, 331 mmol) in 400 mL of dichloromethane, add 198 mL of diethylaluminum chloride solution (2M in hexanes) dropwise at 0 °C. The temperature should not exceed 5 °C during the addition process. Stir for 30 minutes after the addition was completed. Add the obtained dichloromethane solution of INT-1d dropwise into the reaction flask. The temperature was controlled not to exceed 10 °C during the addition process. After the addition was complete, stirring was continued for 2 hours. After the reaction was completed, the reaction solution was poured into 1L of ice water, stirred for 30 minutes, and then concentrated under reduced pressure to remove dichloromethane. The residual liquid was extracted with ethyl acetate (1L*2), washed with water, and the organic phase was concentrated under reduced pressure to obtain a brown oil. The oil was added to 2L of a mixed solution of petroleum ether/ethyl acetate = 10/1, stirred to precipitate a solid, and filtered to obtain a yellow solid INT-1f (139 g, yield 78%). ESI-MS (m/z): 534.8 [M+H]⁺.

Step 5: INT-1f (100 g, 187.1 mmol) was dissolved in 500 mL of tetrahydrofuran, and lithium borohydride (12.2 g, 561.2 mmol) was added. The mixture was stirred at 60 °C overnight. After the raw material disappeared, the reaction solution was added to 200 mL of ice water to quench, and extracted with ethyl acetate (500 mL*3). The organic phase was washed with water, dried, and concentrated under reduced pressure. The residual liquid was dissolved in 500 mL of dichloromethane, and 2,6-dimethyl-1,4-dihydro-3,5-pyridinedicarboxylic acid diethyl ester (28.4 g, 112.2 mmol) and p-toluenesulfonic acid (21.4 g, 112.2 mmol) were added. The mixture was stirred at room temperature for 3 hours. After the reaction was completed, the mixture was concentrated under reduced pressure to remove dichloromethane. The residual liquid was dissolved in 500 mL of methanol, and a pre-prepared 14% lithium hydroxide aqueous solution (100 mL) was added, and the solution was stirred at room temperature for 3 hours, and filtered to obtain a yellow solid INT-1g (84 g, yield 86.3%). ESI-MS (m/z): 520.2 [M+H]⁺.

Step 6: Dissolve INT-1g (50 g, 96 mmol) in 250 mL of tetrahydrofuran, add tetrabutylammonium fluoride (1M in THF, 197 mL), stir overnight at 60°C, and after the reaction is completed, add the reaction solution into 300 mL of water, extract with ethyl acetate (200 mL*3), wash with water, and concentrate under reduced pressure to obtain a brown oil. The obtained residual liquid was dissolved in 40 mL of methanol, and 20 mL of water was added. The mixed solution was washed with petroleum ether (40 mL*5) and concentrated under reduced pressure to remove methanol. The residual liquid was extracted with ethyl acetate (50 mL*2), and the organic phase was washed with water and dried to obtain a light yellow oil INT-1h (25 g, yield 90.4%). ESI-MS (m/z): 282.8 [M+H]⁺.

Step 7: Dissolve compound INT-1h (22 g, 77 mmol) in 100 mL of dichloromethane. (467 mg, 3.82 mmol), triethylamine (23.2 g, 230 mmol), and acetic anhydride (7.9 g, 77 mmol) were added dropwise at 0 °C. After the addition was completed, the temperature was naturally raised and stirred overnight. After the reaction was completed, the reaction solution was washed with water, dried, and concentrated to obtain a brown oil, which was purified by silica gel column chromatography (petroleum ether/ethyl acetate = 4/1) to obtain a light yellow oil INT-li (22.5 g, yield 90.7%). ESI-MS (m/z): 324.2 [M+H]⁺.

Step 8: Compound INT-1i (40 g, 123.4 mmol) was dissolved in dioxane (400 mL), and potassium acetate (30.3 g, 308.4 mmol), [1,1'-bis(diphenylphosphino)ferrocene]palladium dichloride (10 g, 12.3 mmol), and diboronic acid pinacol ester (78.3 g, 308.4 mmol) were added. The reaction was carried out at 90 °C for 3 hours under nitrogen protection. LCMS monitored that the reaction of the raw materials was complete. The reaction solution was directly concentrated under reduced pressure. The residue was dissolved in ethyl acetate (300 mL), washed with water and brine, and the organic phase was purified by silica gel column chromatography to obtain a white solid compound INT-1J (35 g, yield 76.4%). ESI-MS (m/z): 372.5 [M+H]⁺.

Step 9: Compound INT-1J (35 g, 94.3 mmol) and compound INT-1k (37.9 g, 103.7 mmol) were dissolved in dioxane (300 mL) and water (30 mL), potassium phosphate (50 g, 235.7 mmol) and [1,1'-bis(diphenylphosphino)ferrocene]palladium dichloride (6.89 g, 9.43 mmol) were added, and the reaction was carried out at 90 °C overnight under nitrogen protection. LCMS monitored that the raw material reaction was complete. The reaction solution was directly concentrated under reduced pressure, and the residue was dissolved in ethyl acetate (300 mL), washed with water and brine, and the organic phase was purified by silica gel column chromatography to obtain a yellow oily compound INT-1l (28 g, yield 56.08%). ESI-MS (m/z): 530.7 [M+H]⁺.

Step 10: Dissolve compound INT-1l (28 g, 52.9 mmol) in N,N-dimethylformamide (280 mL), add N-iodosuccinimide (11.9 g, 52.9 mmol), react at 50°C for 2 hours, and monitor the complete reaction of the raw materials by LCMS. Pour the reaction solution into water (800 mL), extract with ethyl acetate (200 mL*2), wash the organic phase with saturated brine, dry, filter, and purify by silica gel column chromatography to obtain a yellow solid compound INT-1m (22 g, yield 63.5%). ESI-MS (m/z): 656.6 [M+H]⁺.

Step 11: Dissolve compound INT-1m (5 g, 7.63 mmol), 2-dicyclohexylphosphine-2',6'-dimethoxy-biphenyl (939.4 mg, 2.29 mmol), tri(dibenzylideneacetone)dipalladium (838.1 mg, 0.915 mmol), and potassium acetate (2.6 g, 26.7 mmol) in toluene (100 mL), add pinacol borane (4.9 g, 38.1 mmol) under nitrogen protection, add dropwise, react at 50 °C under nitrogen protection for 5 hours, monitor the reaction of the raw materials, filter the reaction solution, and purify it by silica gel column chromatography to obtain a yellow oily compound INT-1 (4.5 g, yield 90%). ESI-MS (m/z): 656.5 [M+H]⁺.

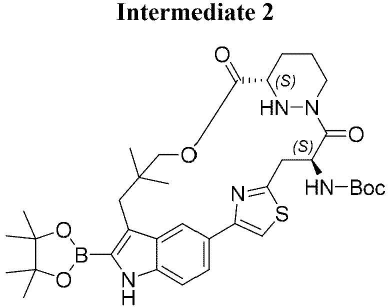

Intermediate 2 was prepared by the following steps:

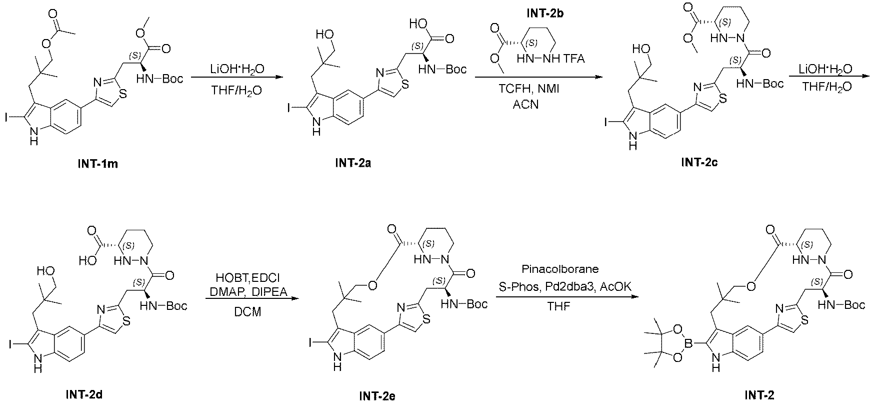

Step 1: Dissolve compound INT-1m (12 g, 18.3 mmol) in tetrahydrofuran (120 mL) and water (20 mL), add lithium hydroxide monohydrate (3.84 g, 91.5 mmol), react at room temperature overnight, monitor the reaction of the raw materials, concentrate the reaction solution under reduced pressure, dissolve the residue in water (100 mL), adjust the pH to 4-5 with 4M hydrochloric acid, extract with dichloromethane (100 mL*3), wash the organic phase with water, wash with brine, dry with anhydrous sodium sulfate, filter, and concentrate to obtain a white solid compound INT-2a (10.6 g, yield 96.6%). ESI-MS (m/z): 600.7 [M+H]⁺.

Step 2: Dissolve compound INT-2a (9.5 g, 15.9 mmol) and compound INT-2b (11.7 g, 31.7 mmol) in acetonitrile (190 mL), add N,N,N',N'-tetramethylchloroformamidine hexafluorophosphate (6.67 g, 23.8 mmol) and 1-methylimidazole (6.51 g, 79.2 mmol) at 0 °C, react at 0 °C for 1 hour, and LCMS monitored the complete reaction of the raw materials. Pour the reaction solution into water (200 mL), extract with dichloromethane (100 mL*3), wash the organic phase with water, and purify by silica gel column chromatography to obtain a yellow solid compound INT-2c (9.6 g, yield 83.5%). ESI-MS (m/z): 726.3 [M+H]⁺.

Step 3: Dissolve compound INT-2c (9.6 g, 13.2 mmol) in tetrahydrofuran (100 mL) and water (10 mL), add lithium hydroxide monohydrate (1.39 g, 33.1 mmol), react at room temperature for 4 hours, and monitor the complete reaction of the raw materials by LCMS. The reaction solution was directly concentrated under reduced pressure, and the residue was dissolved in water (100 mL). 4M hydrochloric acid was used to adjust the pH to 4-5. A white solid was precipitated. Filter, wash the solid with water, and dry to obtain a white solid compound INT-2d (8.3 g, yield 88.2%). ESI-MS (m/z): 712.6 [M+H]⁺.

Step 4: Compound INT-2d (3.5 g, 4.9 mmol), 1-hydroxybenzotriazole (1.99 g, 14.8 mmol), and 4-dimethylaminopyridine (1.8 g, 14. mmol) were dissolved in dichloromethane (170 mL). N,N-diisopropylethylamine (6 mL, 34.4 mmol) was added at 0°C, followed by 1-(3-dimethylaminopropyl)-3-ethylcarbodiimide hydrochloride (4.71 g, 24.6 mmol). The reaction was allowed to proceed overnight at room temperature. LCMS monitored the complete reaction of the raw materials. The reaction solution was washed with saturated aqueous ammonium chloride solution, dried with sodium sulfate, and purified by silica gel column chromatography to obtain a yellow solid compound INT-2e (2 g, yield 58.6%). ESI-MS (m/z): 694.6 [M+H]⁺.

Step 5: Dissolve compound INT-2e (500 mg, 0.721 mmol), 2-dicyclohexylphosphine-2',6'-dimethyl-biphenyl (88.8 mg, 0.216 mmol), tri(dibenzylideneacetone)dipalladium (79 mg, 0.086 mmol), and potassium acetate (247 mg, 2.52 mmol) in tetrahydrofuran (20 mL), add pinacol borane (461 mg, 3.6 mmol) under nitrogen protection, add dropwise, react at 50 °C under nitrogen protection for 3 hours, monitor the reaction of the raw materials, filter the reaction solution, and purify it by silica gel column chromatography to obtain a yellow solid compound INT-2 (400 mg, yield 80%). ESI-MS (m/z): 694.6 [M+H]⁺.

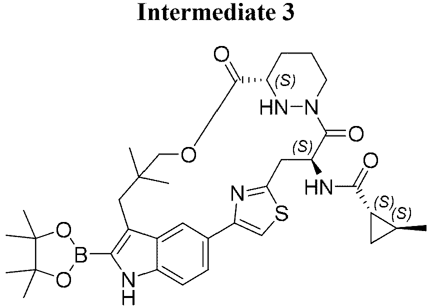

Intermediate 3 was prepared by the following steps:

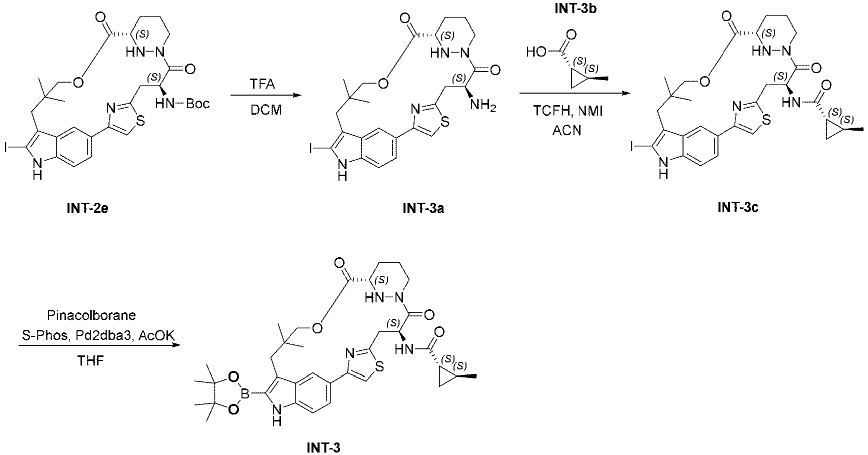

Step 1: Dissolve compound INT-2e (1.7 g, 2.45 mmol) in dichloromethane (20 mL), add trifluoroacetic acid (5 mL), react at room temperature for 2 hours, and monitor the complete reaction of the raw material by LCMS. The reaction solution was directly concentrated under reduced pressure, and the residue was dissolved in DCM (50 mL), washed twice with saturated NaHCO3 aqueous solution, and the organic phase was washed with water, dried with sodium sulfate, filtered, and concentrated to obtain a yellow solid compound INT-3a (1.3 g, yield 89.4%). ESI-MS (m/z): 594.7 [M+H]⁺.

Step 2: Dissolve compound INT-3a (1.3 g, 2.19 mmol) and compound INT-3b (0.24 g, 2.41 mmol) in acetonitrile (30 mL), add N,N,N',N'-tetramethylchloroformamidine hexafluorophosphate (921.9 mg, 3.29 mmol) and 1-methylimidazole (414 mg, 5.04 mmol) at 0 °C, react at 0 °C for 1 hour, LCMS monitored the complete reaction of the raw materials, pour the reaction solution into water (50 mL), extract with dichloromethane (50 mL*3), wash the organic phase with water, mix the sample and purify it through a column to obtain a white solid compound INT-3c (1.3 g, yield 87.9%). ESI-MS (m/z): 675.7 [M+H]⁺.

Step 3: Dissolve compound INT-3c (1.1 g, 1.63 mmol), 2-dicyclohexylphosphine-2',6'-dimethyl-biphenyl (200.5 mg, 0.188 mmol), tri(dibenzylideneacetone)dipalladium (179 mg, 0.195 mmol), and potassium acetate (559 mg, 5.7 mmol) in toluene (30 mL), and add pinacol borane (1.04 g, 8.14 mmol) under nitrogen protection. After the addition was complete, react at 50 °C under nitrogen protection for 3 hours. LCMS monitored the complete reaction of the raw materials. The reaction solution was filtered and purified by silica gel column chromatography to obtain a yellow solid compound INT-3 (990 mg, yield 90%). ESI-MS (m/z): 676.9 [M+H]⁺.

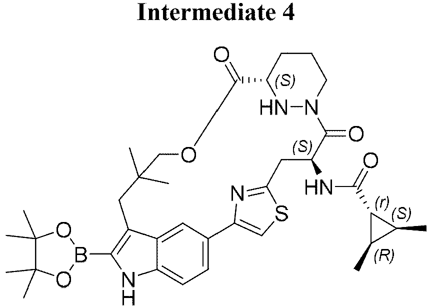

Intermediate 4 was prepared by the following steps:

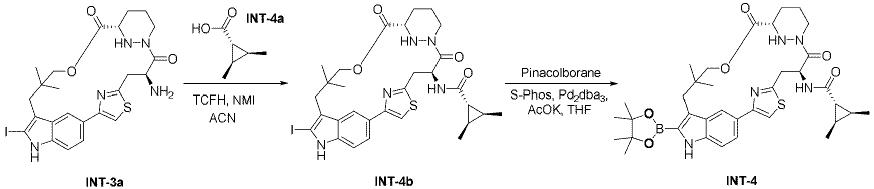

Step 1: Dissolve compound INT-3a (2.2 g, 3.71 mmol) and compound INT-4a (0.47 g, 4.08 mmol) in dichloromethane (50 mL), add N,N,N',N'-tetramethylchloroformamidine hexafluorophosphate (1.56 g, 5.56 mmol) and 1-methylimidazole (0.70 g, 8.53 mmol) at 0 °C, react at 0 °C for 1 hour, and LCMS monitored the complete reaction of the raw materials, pour the reaction solution into water (50 mL), extract with dichloromethane (50 mL*3), wash the organic phase with water, mix the sample and purify it through a column to obtain a white solid compound INT-4b (2.3 g, yield 90.0%). ESI-MS (m/z): 690.2 [M+H]⁺.

Step 2: Dissolve compound INT-4b (2.1 g, 3.05 mmol), 2-dicyclohexylphosphine-2',6'-dimethyl-biphenyl (375.0 mg, 0.91 mmol), tri(dibenzylideneacetone)dipalladium (334.6 mg, 0.365 mmol), and potassium acetate (1.05 g, 10.7 mmol) in toluene (30 mL). Add pinacol borane (1.95 g, 15.2 mmol) under nitrogen protection. After the addition was complete, react at 50 °C under nitrogen protection for 3 hours. LCMS monitored the complete reaction of the raw materials. Filter the reaction solution and purify it by silica gel column chromatography to obtain a yellow solid compound INT-4 (1.8 g, yield 85.7%). ESI-MS (m/z): 690.3 [M+H]⁺.

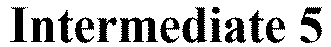

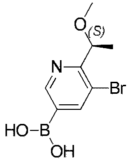

Intermediate 5 was prepared by the following steps:

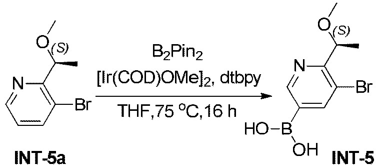

Step 1: Dissolve compound INT-5a (20.0 g, 92.56 mmol) in tetrahydrofuran (200 mL), then add methoxy(cyclooctadiene)iridium dimer (606 mg, 0.925 mmol), 4,4'-di-tert-butyl-2,2'-bipyridine, and bis(pinacolato)diboron (35.3 g, 138.8 mmol), and react the system at 75 oC for 16 hours under nitrogen protection. The reaction of the raw material was completed as monitored by LCMS. The reaction solution was filtered through celite and the filtrate was concentrated. The residue was dissolved in ethyl acetate (200 mL), and the pH was adjusted to 10 with aqueous sodium hydroxide solution. The organic phase was discarded, and the aqueous phase was adjusted to pH 6 with hydrochloric acid. A white solid precipitated and was filtered and dried to obtain a white solid compound INT-5 (20 g, yield 83.1%). ESI-MS (m/z): 260.3 [M+H]⁺.

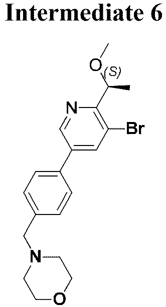

Intermediate 6 was prepared by the following steps:

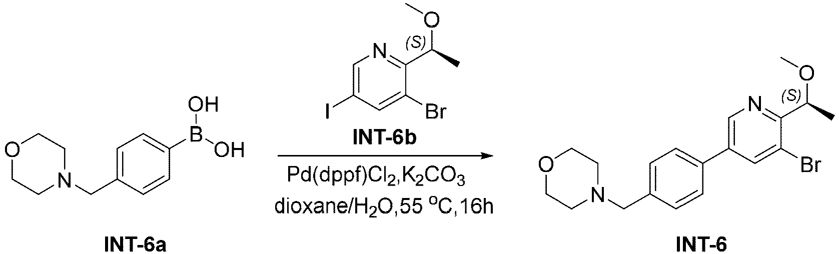

Step 1: Dissolve compound INT-6a (156.4 mg, 0.71 mmol) in a mixed solution of 1,4-dioxane (5 mL) and water (0.5 mL), and add INT-6b (220 mg, 0.64 mmol), [1,1'-bis(diphenylphosphino)ferrocene]palladium dichloride (47.1 mg, 0.064 mmol) and potassium carbonate (266.7 mg, 1.92 mmol) in sequence. The reaction mixture was stirred at 55 °C for 16 hours under nitrogen protection. After the reaction was complete, water (20 mL) was added to the reaction system, and the mixture was extracted with ethyl acetate (30 mL*2). The organic phases were combined and washed with saturated brine, dried over anhydrous sodium sulfate, filtered and concentrated. The residue was purified by silica gel column chromatography (petroleum ether/ethyl acetate = 1:1) to obtain a light yellow solid compound INT-6 (200 mg, yield 79.5%). ESI-MS (m/z): 391.2 [M+H]⁺.

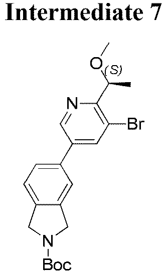

Intermediate 7 was prepared by the following steps:

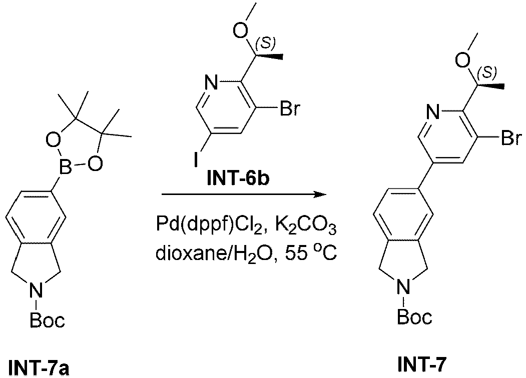

By replacing INT-6a in the synthesis step of intermediate INT-6 with INT-7a, compound INT-7 can be obtained using similar methods and reaction steps. ESI-MS (m/z): 433.4 [M+H]⁺.

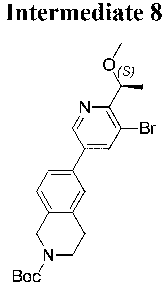

Intermediate 8 was prepared by the following steps:

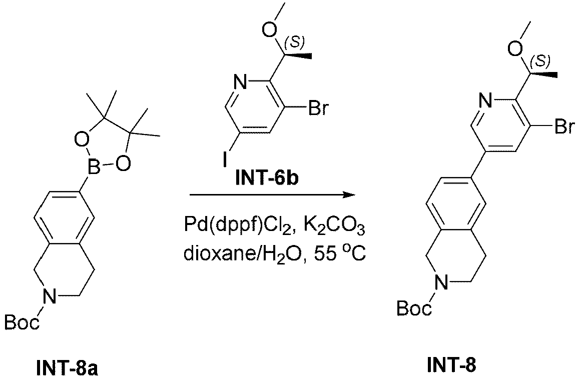

By replacing INT-6a in the synthesis step of intermediate INT-6 with INT-8a, compound INT-8 can be obtained using similar methods and reaction steps. ESI-MS (m/z): 447.6 [M+H]⁺.

### Intermediate 9

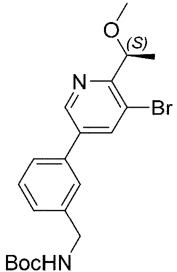

Intermediate 9 was prepared by the following steps:

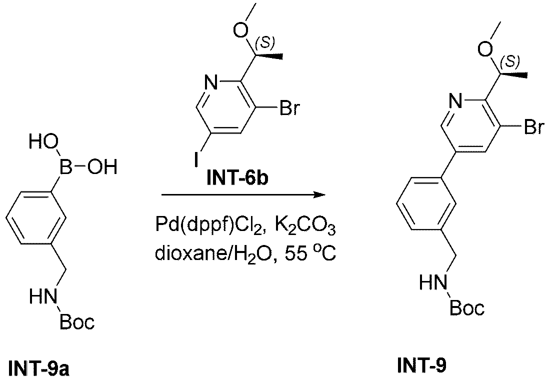

By replacing INT-6a in the synthesis step of intermediate INT-6 with INT-9a, compound INT-9 can be obtained using similar methods and reaction steps. ESI-MS (m/z): 421.5 [M+H]⁺.

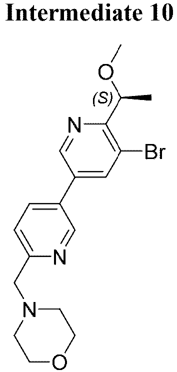

Intermediate 10 was prepared by the following steps:

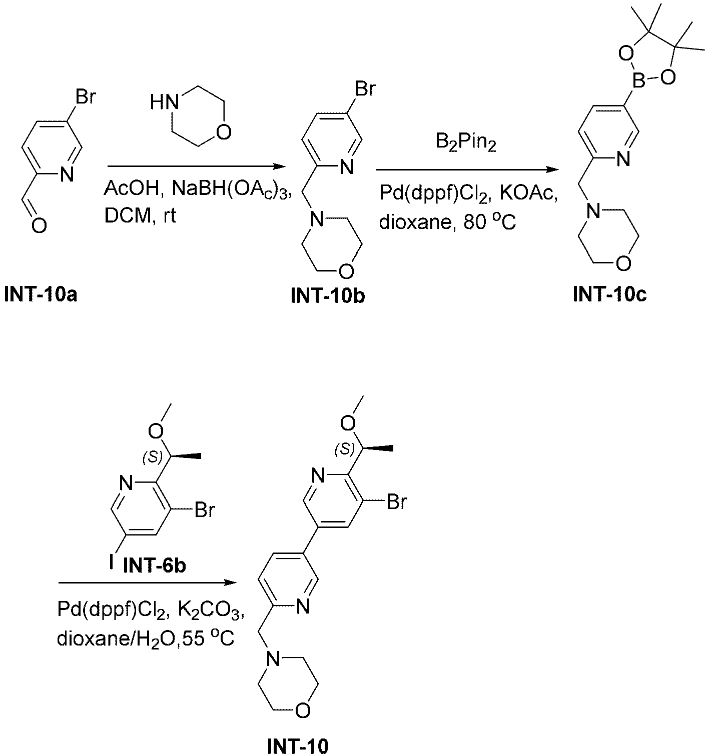

Step 1: Dissolve INT-10a (363 mg, 1.95 mmol) in dichloromethane (20 mL), add morpholine (170 mg, 1.95 mmol) and glacial acetic acid (12 mg, 0.195 mmol), stir at room temperature for 30 minutes, and then add sodium triacetoxyborohydride (1.24 g, 5.85 mmol). The reaction solution was stirred at room temperature for 4 hours, and the reaction was completed as monitored by LCMS. Saturated aqueous sodium bicarbonate solution was added to quench the reaction, and the mixture was extracted with dichloromethane. The organic phases were combined and washed with saturated brine and dried over anhydrous sodium sulfate. The organic phase was concentrated and purified by silica gel column chromatography (petroleum ether/ethyl acetate = 2/1) to obtain a colorless oily liquid INT-10b (453 mg, yield 90.3%). ESI-MS (m/z): 257.4 [M+H]⁺.

Step 2: Dissolve compound INT-10b (400 mg, 1.56 mmol) in 1,4-dioxane (5 mL), and add bis(pinacolato)diboron (415 mg, 1.63 mmol), [1,1'-bis(diphenylphosphino)ferrocene]palladium dichloride (114 mg, 0.156 mmol) and potassium acetate (305.4 mg, 3.12 mmol) in sequence. The reaction mixture was stirred at 100 °C for 16 h under nitrogen protection. After the reaction was complete, the mixture was filtered through celite and the filtrate was concentrated. The residue was dissolved in ethyl acetate, washed with saturated brine, dried over anhydrous sodium sulfate, filtered and concentrated to obtain a crude product of compound INT-10c, which was directly used in the next reaction without purification. ESI-MS (m/z): 305.4 [M+H]⁺.

Step 3: The crude product of the above compound INT-10c was dissolved in a mixed solution of 1,4-dioxane (5 mL) and water (0.5 mL), and INT-6b (400 mg, 1.17 mmol), [1,1'-bis(diphenylphosphino)ferrocene]palladium dichloride (85.5 mg, 0.117 mmol) and potassium carbonate (485 mg, 3.51 mmol) were added in sequence. The reaction mixture was stirred at 55 °C for 16 hours under nitrogen protection. After the reaction was complete, water (20 mL) was added to the reaction system, and the mixture was extracted with ethyl acetate (30 mL*2). The organic phases were combined and washed with saturated brine, dried over anhydrous sodium sulfate, filtered and concentrated. The residue was purified by silica gel column chromatography (dichloromethane/methanol = 30:1) to obtain a light yellow solid compound INT-10 (406 mg, yield 88.5%). ESI-MS (m/z): 392.5 [M+H]⁺.

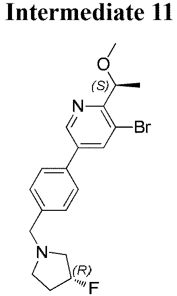

Intermediate 11 was prepared by the following steps:

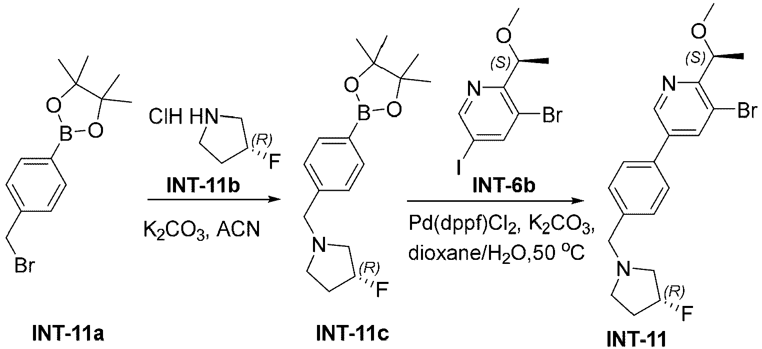

Step 1: Intermediate INT-11a (100 mg, 0.337 mmol) and potassium carbonate (186 mg, 1.35 mmol) were dissolved in acetonitrile (3 mL), and then intermediate TNT11b (42.3 mg, 0.337 mmol) was added thereto. The reaction solution was stirred at room temperature for 4 hours. After the reaction was completed as monitored by LCMS, water was added to the reaction system, and the mixture was extracted with ethyl acetate. The organic phases were combined, washed with saturated brine, dried over anhydrous sodium sulfate, filtered, and concentrated. The residue was purified by silica gel column chromatography (petroleum ether: ethyl acetate = 3:1) to obtain a light yellow solid INT-11c (49 mg, yield 47.7%). ESI-MS (m/z): 306.6 [M+H]⁺.

Step 2: INT-11c (49 mg, 0.161 mmol) was dissolved in a mixed solution of 1,4-dioxane (2 mL) and water (0.2 mL), and INT-6b (57.7 mg, 0.169 mmol), [1,1'-bis(diphenylphosphino)ferrocene]palladium dichloride (11.8 mg, 0.016 mmol) and potassium carbonate (44.4 mg, 0.322 mmol) were added in sequence. The reaction mixture was stirred at 50 °C for 16 h under nitrogen protection. After the reaction was complete, water (20 mL) was added to the reaction system, and the mixture was extracted with ethyl acetate (30 mL*2). The organic phases were combined and washed with saturated brine, dried over anhydrous sodium sulfate, filtered and concentrated. The residue was purified by silica gel column chromatography (petroleum ether/ethyl acetate = 1:1) to obtain a light yellow solid compound INT-11 (33 mg, yield 52.2%). ESI-MS (m/z): 393.5 [M+H]⁺.

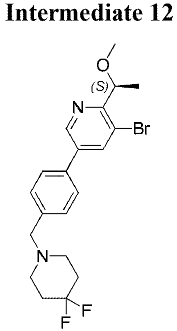

Intermediate 12 was prepared by the following steps:

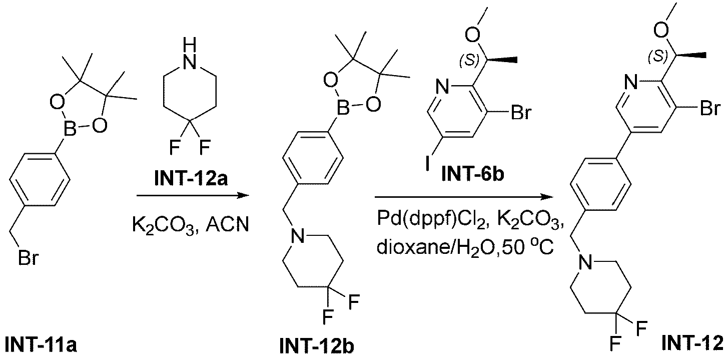

By replacing INT-11b in the synthesis step of intermediate INT-11 with INT-12a, compound INT-12 can be obtained using similar methods and reaction steps. ESI-MS (m/z): 425.5 [M+H]⁺.

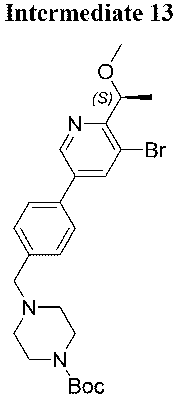

Intermediate 13 was prepared by the following steps:

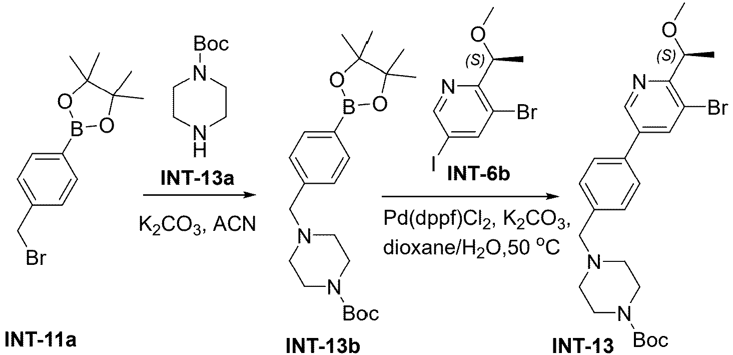

By replacing INT-11b in the synthesis step of intermediate INT-11 with INT-13a, compound INT-13 can be obtained using similar methods and reaction steps. ESI-MS (m/z): 490.7 [M+H]⁺.

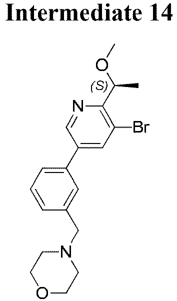

Intermediate 14 was prepared by the following steps:

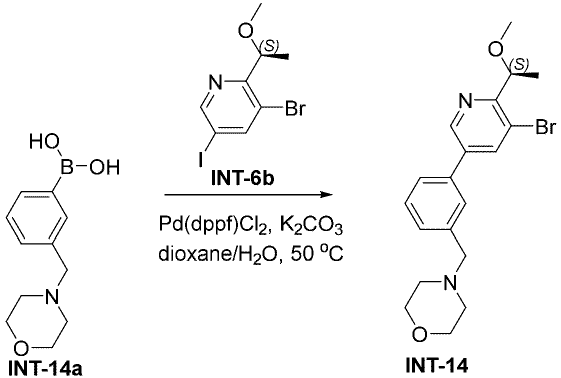

By replacing INT-6a in the synthesis step of intermediate INT-6 with INT-14a, compound INT-14 can be obtained using similar methods and reaction steps. ESI-MS (m/z): 391.3 [M+H]⁺.

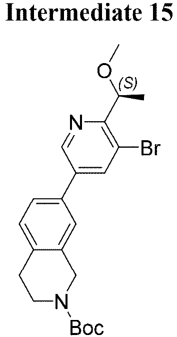

Intermediate 15 was prepared by the following steps:

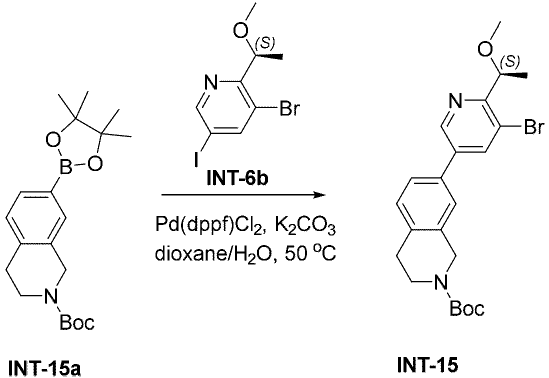

By replacing INT-6a in the synthesis step of intermediate INT-6 with INT-15a, compound INT-15 can be obtained using similar methods and reaction steps. ESI-MS (m/z): 447.6 [M+H]⁺.

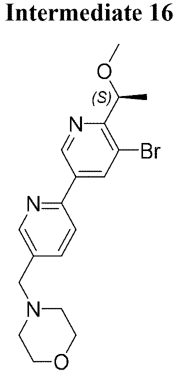

Intermediate 16 was prepared by the following steps:

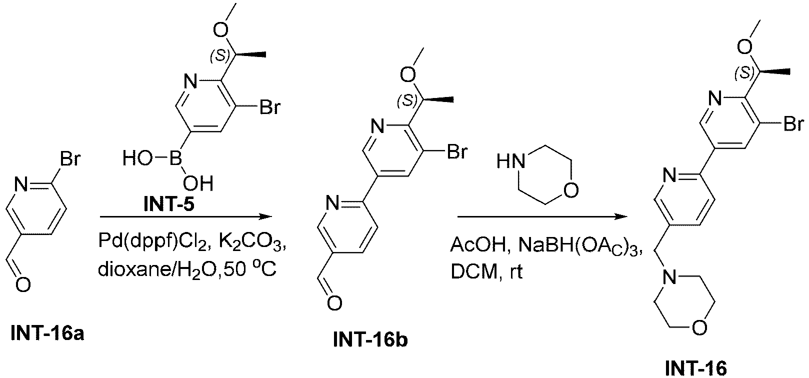

Step 1: INT-16a (322 mg, 1.73 mmol) was dissolved in a mixed solution of 1,4-dioxane (5 mL) and water (0.5 mL), and INT-5 (300 mg, 1.15 mmol), [1,1'-bis(diphenylphosphino)ferrocene]palladium dichloride (84.5 mg, 0.115 mmol) and potassium carbonate (478.6 mg, 3.46 mmol) were added in sequence. The reaction mixture was stirred at 50 °C for 16 h under nitrogen protection. After the reaction was complete, water (20 mL) was added to the reaction system, and the mixture was extracted with ethyl acetate (30 mL*2). The organic phases were combined, washed with saturated brine, dried over anhydrous sodium sulfate, filtered and concentrated, and the residue was purified by silica gel column chromatography (petroleum ether/ethyl acetate = 2:1) to obtain a light yellow solid compound INT-16b (280 mg, yield 75.5%). ESI-MS (m/z): 321.2 [M+H]⁺.

Step 2: Dissolve INT-16b (280 mg, 0.872 mmol) in dichloromethane (5 mL), add morpholine (152 mg, 1.74 mmol) and glacial acetic acid (5 mg, 0.087 mmol), stir at room temperature for 30 minutes, and then add sodium triacetoxyborohydride (554.3 mg, 2.62 mmol). The reaction solution was stirred at room temperature for 16 h, and the reaction was completed as monitored by LCMS. Saturated aqueous sodium bicarbonate solution was added to quench the reaction, and the mixture was extracted with dichloromethane. The organic phases were combined and washed with saturated brine and dried over anhydrous sodium sulfate. The organic phase was concentrated and purified by silica gel column chromatography (dichloromethane/methanol = 20/1) to obtain a light yellow oily liquid INT-16 (290 mg, yield 84.8%). ESI-MS (m/z): 392.4 [M+H]⁺.

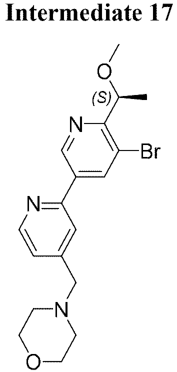

Intermediate 17 was prepared by the following steps:

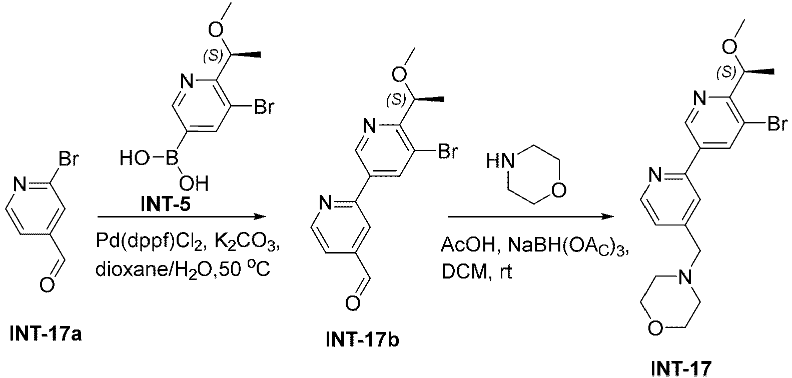

By replacing INT-16a in the synthesis step of intermediate INT-16 with INT-17a, compound INT-17 can be obtained using similar methods and reaction steps. ESI-MS (m/z): 392.4 [M+H]⁺.

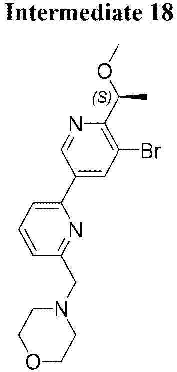

Intermediate 18 was prepared by the following steps:

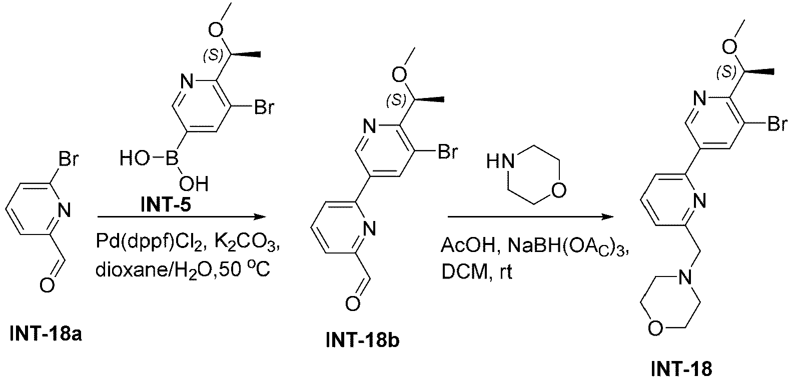

By replacing INT-16a in the synthesis step of intermediate INT-16 with INT-18a, compound INT-18 can be obtained using similar methods and reaction steps. ESI-MS (m/z): 392.4 [M+H]⁺.

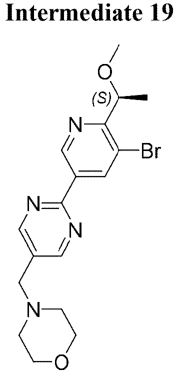

Intermediate 19 was prepared by the following steps:

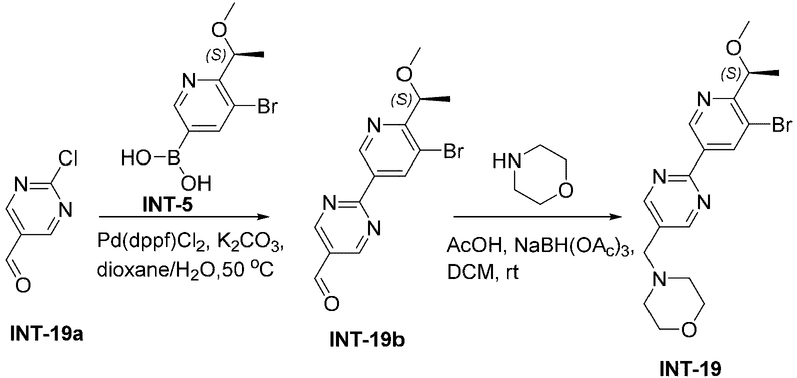

By replacing INT-16a in the synthesis step of intermediate INT-16 with INT-19a, compound INT-19 can be obtained using similar methods and reaction steps. ESI-MS (m/z): 393.4 [M+H]⁺.

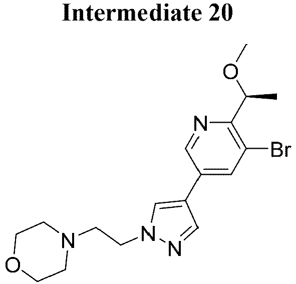

Intermediate 5 was prepared by the following steps:

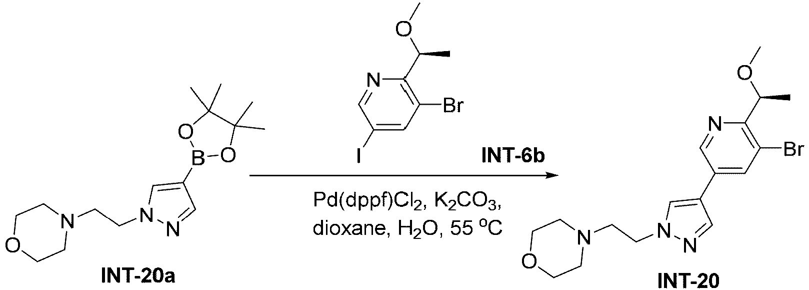

By replacing INT-6a in the synthesis step of intermediate INT-6 with INT-20a, compound INT-20 can be obtained using similar methods and reaction steps. ESI-MS (m/z): 447.6 [M+H]⁺.

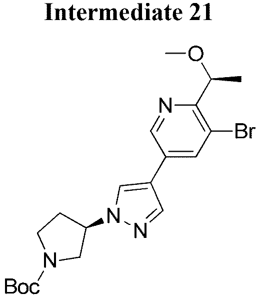

Intermediate 21 was prepared by the following steps:

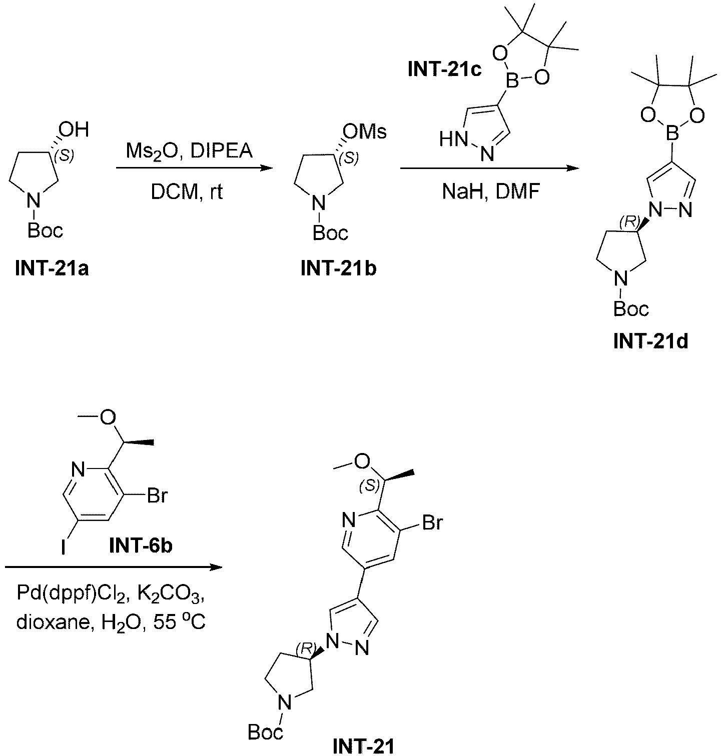

Step 1: Dissolve compound INT-21a (500 mg, 2.67 mmol) in dichloromethane (10 mL), add methanesulfonic anhydride (558 mg, 3.20 mmol) and N, N-diisopropylethylamine (518 mg, 4.0 mmol) under ice bath, and stir at room temperature for 1 hour. LCMS monitoring shows that the reaction of the raw material is complete. Water was added to the system, the system was extracted with ethyl acetate, and the organic phases were combined, washed with saturated brine, dried over anhydrous sodium sulfate, filtered, and concentrated to obtain a light yellow oily compound INT-21b (700 mg, yield 98.8%). ESI-MS (m/z): 266.4 [M+H]⁺.

Step 2: Dissolve compound 4-pyrazole boric acid pinacol ester INT-21c (460.7 mg, 2.37 mmol) in N, N-dimethylformamide (5 mL), add sodium hydride (116 mg, 2.90 mmol) under ice bath, and stir under ice bath for 30 minutes. Then INT-21b (700 mg, 2.64 mmol) was added, and the reaction mixture was heated to room temperature and stirred for 4 hours. LCMS monitored the complete reaction of the starting material. Water was added to the system to quench the reaction, and the system was extracted with ethyl acetate. The organic phases were combined, washed with saturated brine, dried over anhydrous sodium sulfate, filtered, and concentrated to obtain a light yellow oily compound INT-21d (950 mg, yield 99.1%). ESI-MS (m/z): 364.4 [M+H]⁺.

Step 3: Dissolve the intermediate INT-6b (300 mg, 0.88 mmol) and the intermediate INT-21d (382.4 mg, 1.05 mmol) in a mixed solvent of 1,4-dioxane (5 mL) and water (0.5 mL), and add [1,1'-bis(diphenylphosphino)ferrocene]palladium dichloride (64.1 mg, 0.088 mmol) and potassium carbonate (363.7 mg, 2.64 mmol). The system was replaced with nitrogen and heated to 55 °C with stirring for 16 hours. After the reaction solution was cooled to room temperature, it was filtered through celite and the filtrate was concentrated. The residue was purified by silica gel column chromatography (petroleum ether/ethyl acetate = 1/1) to obtain a light yellow solid compound INT-21 (110 mg, yield 27.8%). ESI-MS (m/z): 451.5 [M+H]⁺.

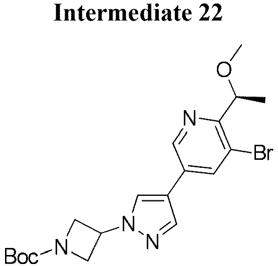

Intermediate 22 was prepared by the following steps:

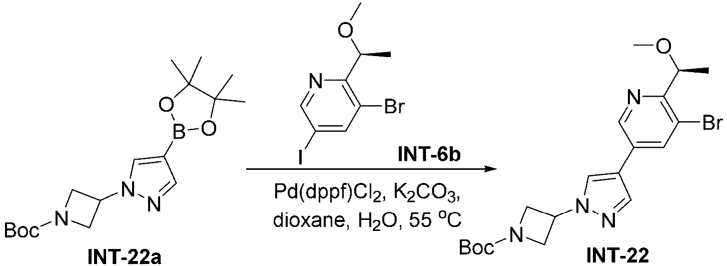

By replacing INT-6a in the synthesis step of intermediate INT-6 with INT-22a, compound INT-22 can be obtained using similar methods and reaction steps. ESI-MS (m/z): 437.5 [M+H]⁺.

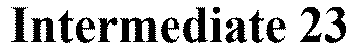

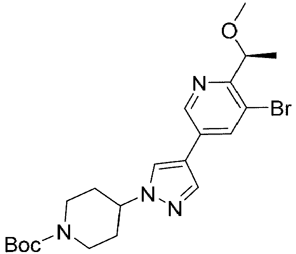

Intermediate 23 was prepared by the following steps:

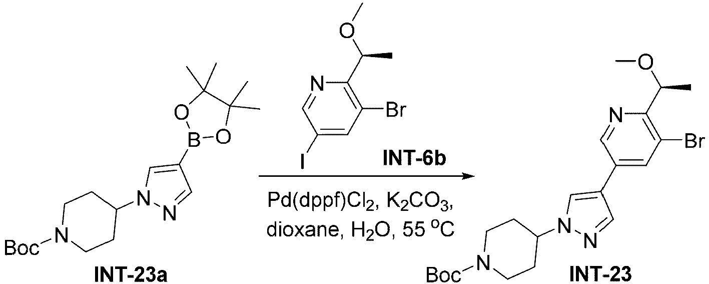

By replacing INT-6a in the synthesis step of intermediate INT-6 with INT-23a, compound INT-23 can be obtained using similar methods and reaction steps. ESI-MS (m/z): 465.6 [M+H]⁺.

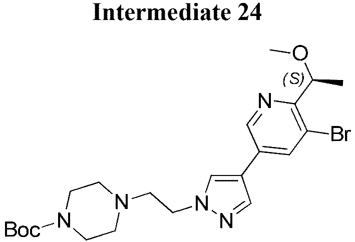

Intermediate 24 was prepared by the following steps:

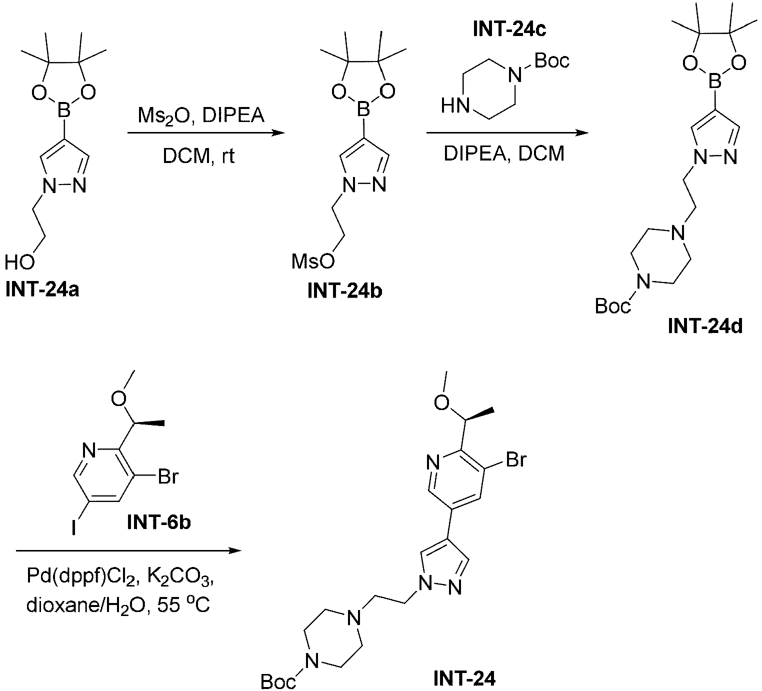

By replacing INT-21a in the synthesis step of intermediate INT-21 with INT-24a, compound INT-24 can be obtained using similar methods and reaction steps. ESI-MS (m/z): 494.5 [M+H]⁺.

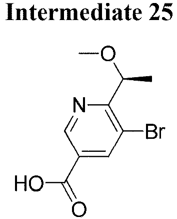

Intermediate 25 was prepared by the following steps:

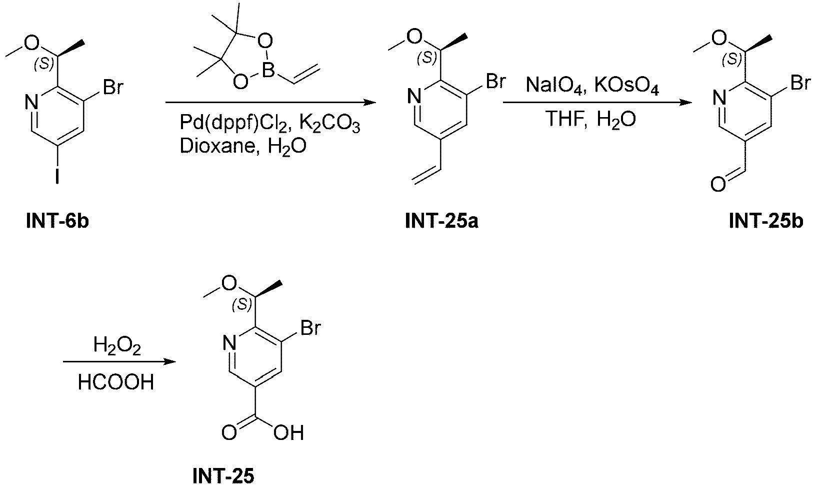

Step 1: Dissolve compound INT-6b (20 g, 58.5 mmol) and vinylboronic acid pinacol ester (10.8 g, 70.2 mmol) in 1,4-dioxane (120 mL) and water (12 mL), add potassium carbonate (16.2 g, 117.0 mmol) and [1,1'-bis(diphenylphosphino)ferrocene]dichloropalladium (2.12 g, 2.92 mmol). The mixture was stirred at 60 °C overnight under N₂ protection. The reaction of the raw material was complete as monitored by LCMS. The excess solvent was removed by distillation under reduced pressure. The residue was added to water (100 mL) and extracted with ethyl acetate (100 mL*3). The organic phase was filtered, washed with water, and purified by column to obtain a white solid compound INT-25a (13 g, yield 92.0%). ESI-MS (m/z): 242.2 [M+H]⁺.

Step 2: Dissolve compound INT-25a (13 g, 53.69 mmol) in 300 mL of tetrahydrofuran, add 100 mL of water, stir, add sodium periodate (28.7 g, 134.2 mmol) and potassium osmate (157.50 mg, 536.9 umol), stir at 40°C for 3 hours, monitor the reaction of the raw materials by LCMS, separate the liquids, extract the aqueous phase with ethyl acetate (50 mL*3), combine the organic phases, wash with water, mix the sample and purify by column to obtain white solid compound INT-25b (11.2 g, yield 85.5%). ESI-MS (m/z): 244.5 [M+H]⁺.

Step 3: Dissolve compound INT-25b (500 mg, 2.05 mmol) in formic acid (2 mL), add hydrogen peroxide (697 mg, 20.5 mmol) in an ice bath, continue stirring in an ice bath for 6 hours, and monitor the complete reaction of the raw materials by LCMS. Add water to the system, extract with dichloromethane, dry the organic phase, dry over anhydrous sodium sulfate, filter and concentrate to obtain a white solid compound INT-25 (500 mg, yield 93.9%). ESI-MS (m/z): 260.4 [M+H]⁺.

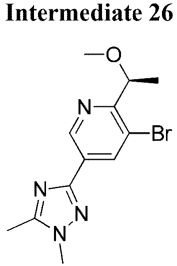

Intermediate 26 was prepared by the following steps

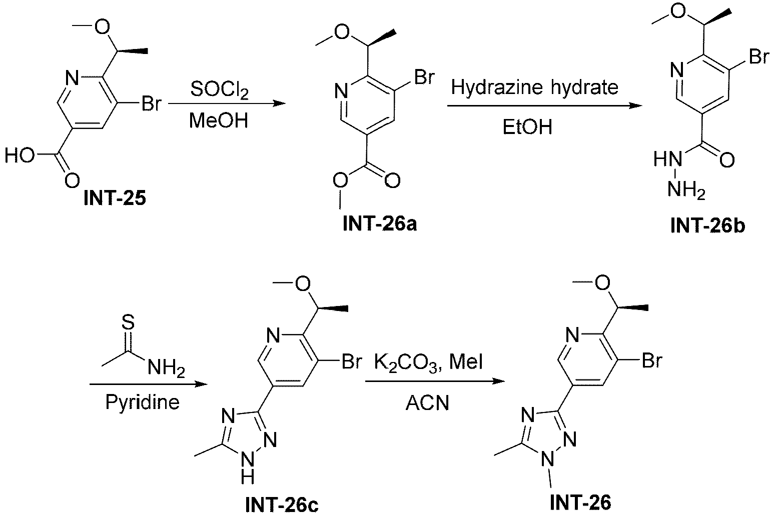

Step 1: Dissolve INT-25 (200 mg, 0.77 mmol) in methanol (4 mL), add thionyl chloride (275 mg, 0.17 mol, 2.31 mL) at room temperature, stir at 60 °C for 3 hours, and when the reaction was completed, remove dichloromethane and remaining thionyl chloride under reduced pressure to obtain a light yellow oily compound INT-26a (200 mg, yield 94.9%). ESI-MS (m/z): 274.4 [M+H]⁺.

Step 2: Dissolve INT-26a (200 mg, 0.73 mmol) in ethanol (2 mL), add hydrazine hydrate (110 mg, 2.19 mmol) at room temperature, and stir the reaction solution at 90 °C for 16 hours. After the reaction, concentrate under reduced pressure to obtain a light yellow oil INT-26b (180 mg, yield 90.0%). ESI-MS (m/z): 274.3[M+H]⁺.

Step 3: Dissolve INT-26b (180 mg, 0.66 mmol) in pyridine (2 mL), add thioacetamide (82 mg, 0.98 mmol) at room temperature, stir the reaction solution at 150 °C under microwave conditions for 1 hour, and concentrate under reduced pressure after the reaction was completed. The residue was purified by silica gel column chromatography (dichloromethane/methanol = 20:1) to obtain a light yellow solid compound INT-26c (90 mg, yield 45.5%). ESI-MS (m/z): 297.3[M+H]⁺.

Step 4: Dissolve INT-26c (90 mg, 0.30 mmol) in acetonitrile (2 mL), add potassium carbonate (125 mg, 0.90 mmol) and iodomethane (64 mg, 0.45 mmol) at room temperature, and stir the reaction solution at room temperature for 16 hours. After the reaction, add water, extract with dichloromethane, dry the organic phase with anhydrous sodium sulfate, filter and concentrate to obtain a light yellow oily compound INT-26 (70 mg, yield 74.3%). ESI-MS (m/z): 311.3 [M+H]⁺.

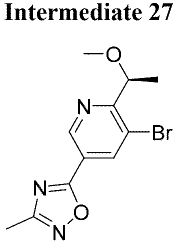

Intermediate 27 was prepared by the following steps

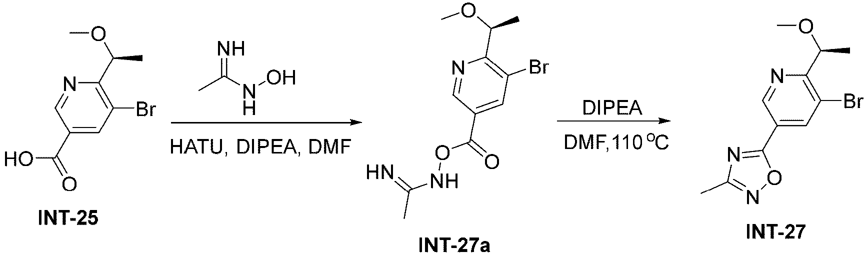

Step 1: Dissolve compound INT-25 (300 mg, 1.15 mmol) in N,N-dimethylformamide (4 mL), then add N-hydroxyacetamidine (128 mg, 1.73 mmol), N,N-diisopropylethylamine (447 mg, 3.45 mmol), and 2-(7-azobenzotriazole)-N,N,N',N'-tetramethyluronium hexafluorophosphate (658 mg, 1.73 mmol), and react the system at 60 °C for 16 hours. The reaction of the starting material was complete as monitored by LCMS. The reaction solution was directly used in the next reaction without purification. ESI-MS (m/z): 316.3[M+H]⁺.

Step 2: Heat the reaction solution to 110 °C and continue the reaction for 16 hours. The reaction of the starting material was complete as monitored by LCMS. The reaction system was added with water, extracted with ethyl acetate, dried over anhydrous sodium sulfate, filtered and concentrated. The residue was purified by silica gel column chromatography (petroleum ether/ethyl acetate = 5:1) to obtain a light yellow oily compound INT-27 (60 mg, two-step yield 31.8%). ESI-MS (m/z): 298.3 [M+H]⁺.

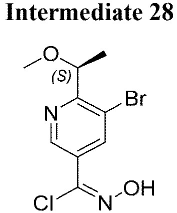

Intermediate 28 was prepared by the following steps:

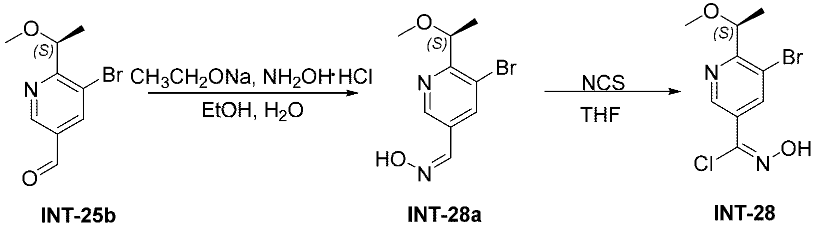

Step 1: Dissolve compound INT-25b (1 g, 4.10 mmol) in 15 mL of ethanol and 10 mL of water, then add hydroxylamine hydrochloride (313.17 mg, 4.51 mmol) and sodium acetate (613.26 mg, 4.51 mmol), stir at room temperature for 1 hour, monitor the reaction of the raw materials by LCMS, and filter to obtain a light yellow solid compound INT-28a (1.06 g, yield 94.2%). ESI-MS (m/z): 259.4 [M+H]⁺.

Step 2: Dissolve compound INT-28a (1.06 g, 3.86 mmol) in 15 mL of tetrahydrofuran, add N-chlorosuccinimide (1.20 g, 9.01 mmol), stir at room temperature for 3 hours, monitor the reaction of the raw material by LCMS, add water (30 mL), extract with ethyl acetate (20 mL*3), and concentrate the organic phase to obtain a light yellow solid compound INT-28 (1.1 g, yield 85.8%). ESI-MS (m/z): 293.0 [M+H]⁺.

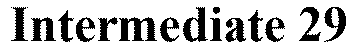

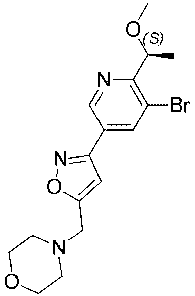

Intermediate 29 was prepared by the following steps:

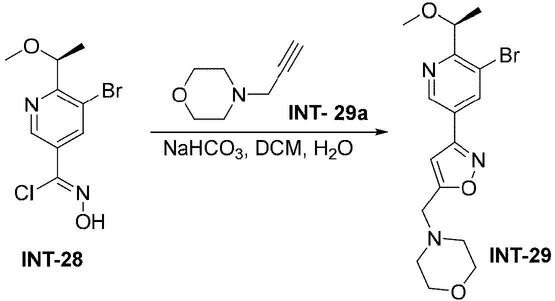

Step 1: Dissolve intermediate INT-28 (150 mg, 0.51 mmol) and intermediate INT-29a (95 mg, 0.77 mmol) in a mixed solvent of dichloromethane (2 mL) and water (2 mL), and add sodium bicarbonate (129 mg, 1.53 mmol). The reaction solution was stirred at room temperature for 4 hours. The reaction was complete as monitored by LCMS. The reaction system was added with saturated brine, extracted with dichloromethane, and the organic phases were combined, washed with saturated brine, dried over anhydrous sodium sulfate, filtered and concentrated, and the residue was purified by silica gel column chromatography (dichloromethane/methanol = 10/1) to obtain colorless oily liquid INT-29 (160 mg, yield 81%). ESI-MS (m/z): 382.7 [M+H]⁺.

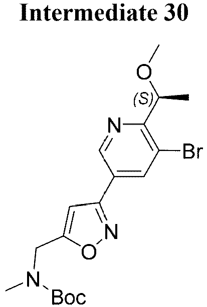

Intermediate 30 was prepared by the following steps:

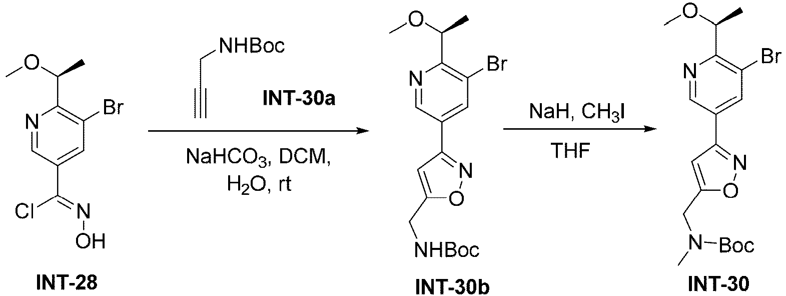

Step 1: Dissolve intermediate INT-28 (200 mg, 0.68 mmol) and intermediate INT-30a (158.6 mg, 1.02 mmol) in a mixed solvent of dichloromethane (2 mL) and water (2 mL), and add sodium bicarbonate (171.7 mg, 2.04 mmol). The reaction solution was stirred at room temperature for 4 hours. The reaction was complete as monitored by LCMS. **250409** Saturated brine was added to the reaction system, extracted with dichloromethane, the organic phases were combined, washed with saturated brine, dried over anhydrous sodium sulfate, filtered and concentrated, and the residue was purified by silica gel column chromatography (petroleum ether/ethyl acetate = 5/2) to obtain colorless oily liquid INT-30b (228 mg, yield 81.2%). ESI-MS (m/z): 412.4 [M+H]⁺.

Step 2: Dissolve the intermediate INT-30b (228 mg, 0.55 mmol) in anhydrous tetrahydrofuran (5 mL), add sodium hydride (33 mg, 0.825 mmol) under ice bath, and stir for 30 minutes. Iodomethane (117 mg, 0.825 mmol) was then added and stirring was continued for 2 hours. The reaction was completed as monitored by LC-MS. Saturated ammonium chloride solution was added to the reaction system, and the reaction system was extracted with dichloromethane, and the organic phases were combined, washed with saturated brine, dried over anhydrous sodium sulfate, filtered and concentrated, and the residue was purified by silica gel column chromatography (petroleum ether/ethyl acetate = 3/1) to obtain colorless oily liquid INT-30 (210 mg, yield 90%). ESI-MS (m/z): 426.4 [M+H]⁺.

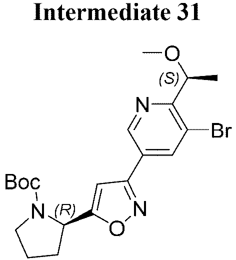

Intermediate 31 was prepared by the following steps:

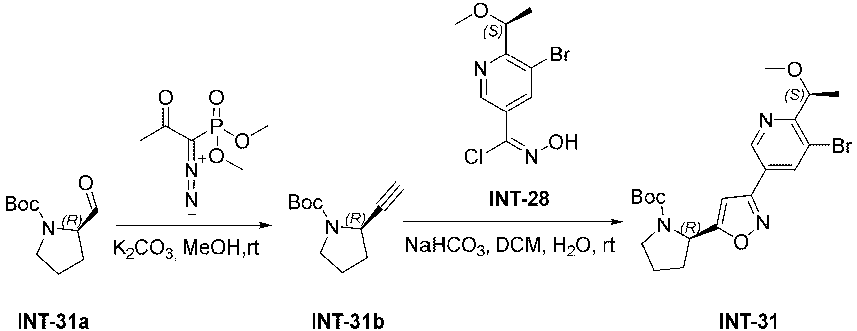

Step 1: Dissolve compound INT-31a (600 mg, 3 mmol) in methanol (5 mL), add potassium carbonate (1.25 g, 9 mmol) and (1-diazo-2-oxopropyl) dimethyl phosphate (1.16 g, 6 mmol) at room temperature. The reaction solution was stirred at room temperature for 12 h. The reaction was completed as monitored by TLC. Saturated brine was added to the reaction system, and the reaction system was extracted with dichloromethane, and the organic phases were combined, washed with saturated brine, dried over anhydrous sodium sulfate, filtered and concentrated to obtain a crude product of compound INT-31b (587 mg, yield 100%).

Step 2: Dissolve intermediate INT-28 (100 mg, 0.34 mmol) and intermediate INT-31b (100 mg, 0.51 mmol) in a mixed solvent of dichloromethane (2 mL) and water (2 mL), and add sodium bicarbonate (86 mg, 1.02 mmol). The reaction solution was stirred at room temperature for 4 hours. The reaction was complete as monitored by LCMS. Saturated brine was added to the reaction system, and the reaction system was extracted with dichloromethane, the organic phases were combined, washed with saturated brine, dried over anhydrous sodium sulfate, filtered and concentrated, and the residual liquid was purified by silica gel column chromatography (petroleum ether/ethyl acetate = 1/1) to obtain colorless oily liquid INT-31 (109 mg, yield 71%). ESI-MS (m/z): 452.6 [M+H]⁺.

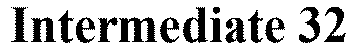

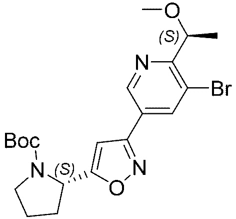

Intermediate 32 was prepared by the following steps:

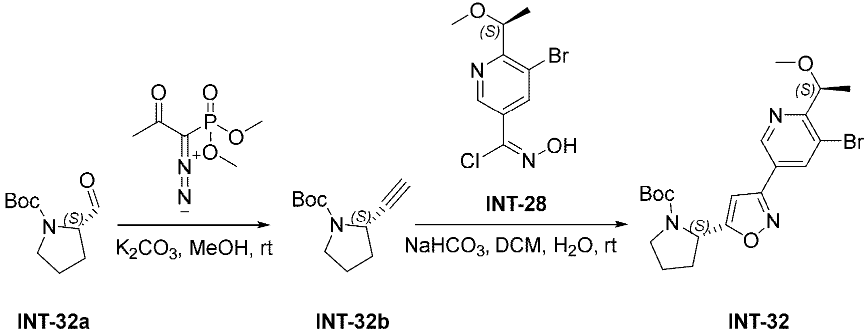

By replacing INT31a in the synthesis step of intermediate INT-31 with INT-32a, compound INT-32 can be obtained using similar methods and reaction steps. ESI-MS (m/z): 452.3 [M+H]⁺.

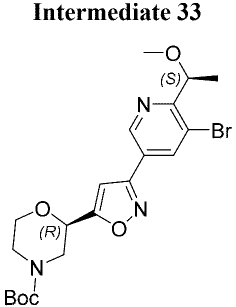

Intermediate 33 was prepared by the following steps:

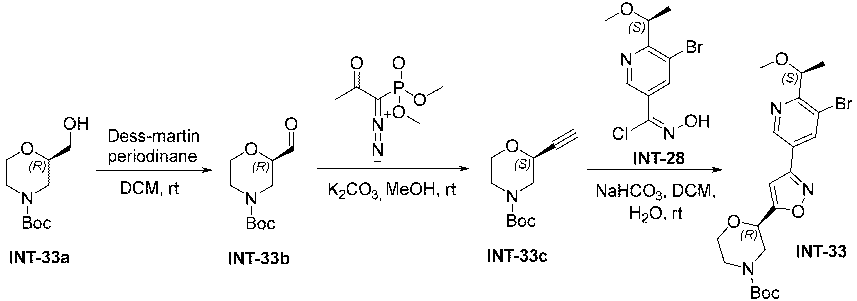

Step 1: Add Dess-Martin reagent (5.08 g, 11.97 mmol) to a solution of INT-33a (2.0 g, 9.21 mmol) in dichloromethane (40 mL) at 0 °C. The reaction mixture was warmed to room temperature and stirred for 5 hours. After the reaction, saturated sodium bicarbonate and sodium thiosulfate aqueous solutions were added to quench the reaction, and the reaction system was extracted with dichloromethane, and the organic phases were combined and washed with saturated brine, dried over anhydrous sodium sulfate, and the organic phase was concentrated to obtain the crude product INT-33b.

Step 2: The crude product INT-33b and potassium carbonate (3.81 g, 27.60 mmol) were added to methanol (40 mL), and dimethyl (1-diazo-2-oxopropyl) phosphate (3.53 g, 18.40 mmol) was added dropwise at room temperature. The reaction solution was stirred at room temperature for 4 hours and the reaction was completed under TLC monitoring. Water was added to quench the reaction, and the mixture was extracted with ethyl acetate. The organic phases were combined and washed with saturated brine, dried over anhydrous sodium sulfate, and concentrated to obtain a crude intermediate INT-33c (1.20 g).

Step 3: Intermediate INT-28 (200 mg, 0.68 mmol) and intermediate INT-33c (144 mg, 0.68 mmol) were dissolved in a mixed solvent of dichloromethane (2 mL) and water (2 mL), and sodium bicarbonate (172 g, 2.04 mmol) was added. The reaction was stirred at room temperature for 4 hours. The reaction was complete as monitored by LCMS. Saturated brine was added to the reaction system, and the reaction system was extracted with dichloromethane, the organic phases were combined, washed with saturated brine, dried over anhydrous sodium sulfate, filtered and concentrated, and the residual liquid was purified by silica gel column chromatography (petroleum ether/ethyl acetate = 1/1) to obtain colorless oily liquid INT-33 (224 mg, yield 70%). ESI-MS (m/z): 469.9 [M+H]⁺.

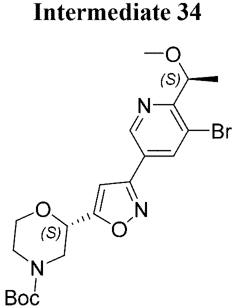

Intermediate 34 was prepared by the following steps:

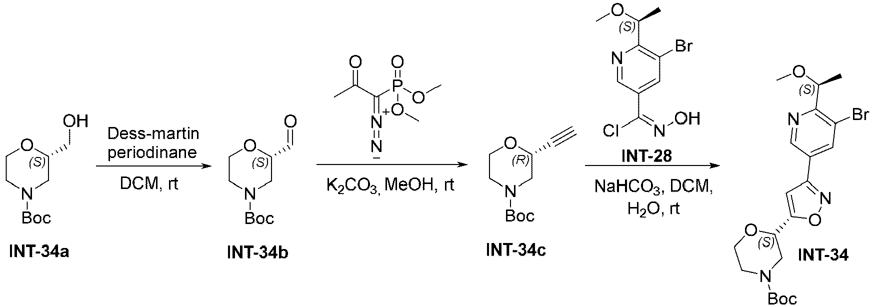

By replacing INT-33a in the synthesis step of intermediate INT-33 with INT-34a, compound INT-34 can be obtained using similar methods and reaction steps. ESI-MS (m/z): 469.9 [M+H]⁺.

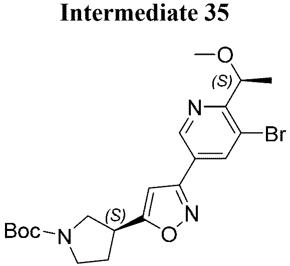

Intermediate 35 was prepared by the following steps:

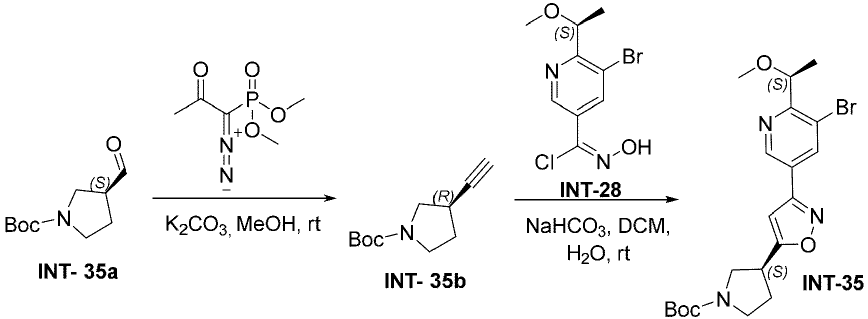

By replacing INT-31a in the synthesis step of intermediate INT-31 with INT-35a, compound INT-35 can be obtained using similar methods and reaction steps. ESI-MS (m/z): 452.6 [M+H]⁺.

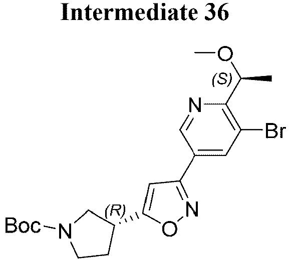

Intermediate 36 was prepared by the following steps:

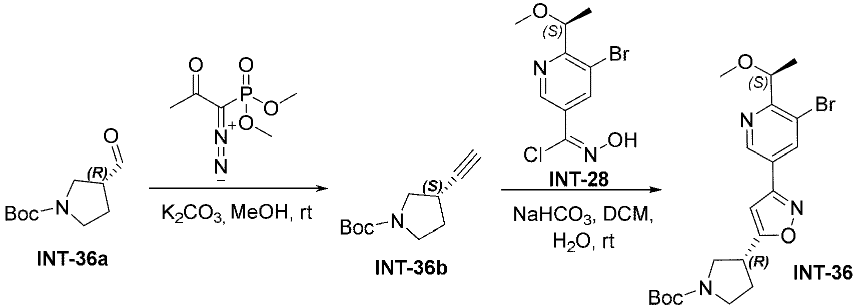

By replacing INT-31a in the synthesis step of intermediate INT-31 with INT-36a, compound INT-36 can be obtained using similar methods and reaction steps. ESI-MS (m/z): 452.6 [M+H]⁺.

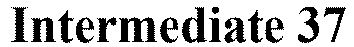

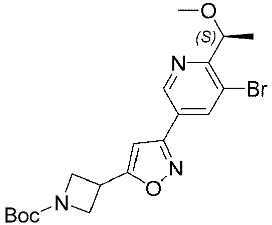

Intermediate 37 was prepared by the following steps:

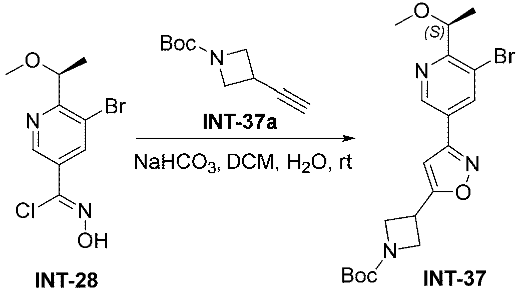

By replacing INT-29a in the synthesis step of intermediate INT-29 with INT-37a, compound INT-37 can be obtained using similar methods and reaction steps. ESI-MS (m/z): 381.1 [M+H]⁺.

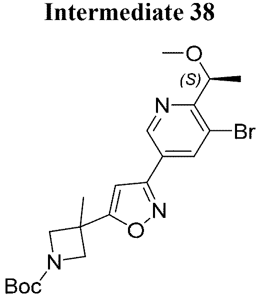

Intermediate 38 was prepared by the following steps:

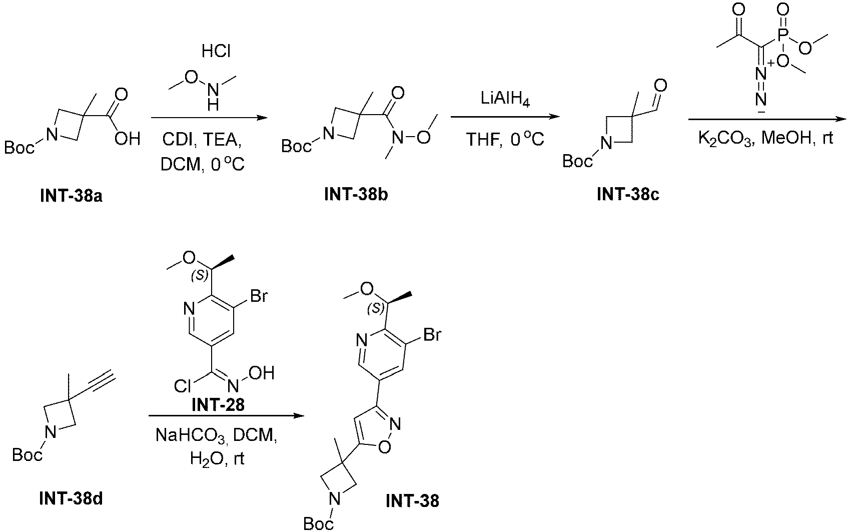

Step 1: Dissolve the intermediate INT-38a (200 mg, 0.93 mmol) and N,N'-carbonyldiimidazole (211 mg, 1.3 mmol) in dichloromethane (5 mL), and stir the reaction solution at 0 °C for 30 minutes. Then, methoxymethylamine hydrochloride (127 mg, 1.3 mmol) and triethylamine (132 mg, 1.3 mmol) were added to the reaction solution. The reaction solution was slowly warmed to room temperature and stirred for 12 hours. The reaction was completed as monitored by TLC. After the reaction solution was concentrated, water was added to quench the reaction, and the mixture was extracted with ethyl acetate. The organic phases were combined and washed with saturated sodium bicarbonate solution and dried over anhydrous sodium sulfate. The organic phase was concentrated to obtain a crude intermediate INT-38b (240 mg, yield 100%).

Step 2: The crude INT-38b (240 mg, 0.93 mmol) was dissolved in tetrahydrofuran (5 mL), the reaction liquid was replaced with nitrogen, and then stirred at 0 °C and lithium aluminum tetrahydride (0.4 mL, 2.5 M in THF) was added. The reaction was stirred at this temperature for an additional hour. The reaction was completed as monitored by TLC. The reaction solution was quenched with water, extracted with ethyl acetate, and the organic phases were combined and washed with saturated brine, dried over anhydrous sodium sulfate, and the organic phase was concentrated to obtain a crude intermediate INT-38c (183 mg, yield 98.9%).

Step 3: Add the crude INT-38c (183 mg, 0.92 mmol) and potassium carbonate (381 mg, 2.76 mmol) to methanol (3 mL), and add dimethyl (1-diazo-2-oxopropyl) phosphate (353 mg, 1.84 mmol) dropwise at room temperature. The reaction solution was stirred at room temperature for 4 hours and the reaction was completed under TLC monitoring. Water was added to quench the reaction, and the mixture was extracted with ethyl acetate. The organic phases were combined and washed with saturated brine, dried over anhydrous sodium sulfate, and concentrated to obtain a crude intermediate INT-38d (179 mg, yield 100%).

Step 4: Intermediate INT-28 (200 mg, 0.68 mmol) and intermediate INT-38d (133 mg, 0.68 mmol) were dissolved in a mixed solvent of dichloromethane (2 mL) and water (2 mL), and sodium bicarbonate (172 g, 2.04 mmol) was added. The reaction was stirred at room temperature for 4 hours. The reaction was complete as monitored by LCMS. Saturated brine was added to the reaction system, and the reaction system was extracted with dichloromethane, and the organic phases were combined, washed with saturated brine, dried over anhydrous sodium sulfate, filtered and concentrated, and the residue was purified by silica gel column chromatography (petroleum ether/ethyl acetate = 1/1) to obtain colorless oily liquid INT-38 (203 mg, yield 66%). ESI-MS (m/z): 452.6 [M+H]⁺.

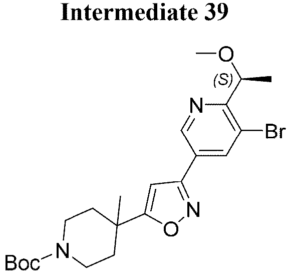

Intermediate 39 was prepared by the following steps:

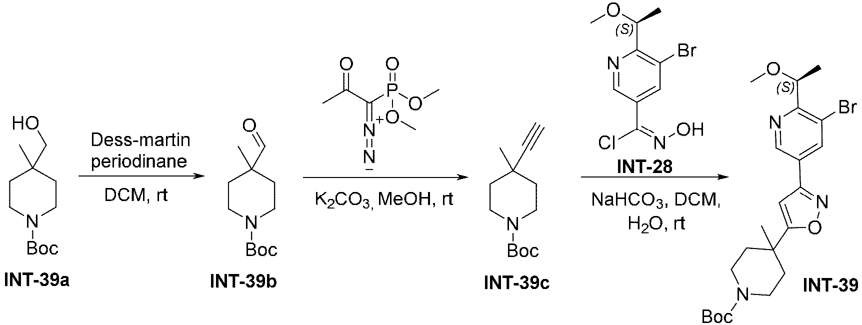

By replacing INT-33a in the synthesis step of intermediate INT-33 with INT-39a, compound INT-39 can be obtained using similar methods and reaction steps. ESI-MS (m/z): 480.4 [M+H]⁺.

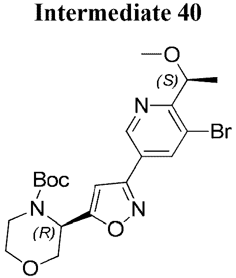

Intermediate 40 was prepared by the following steps:

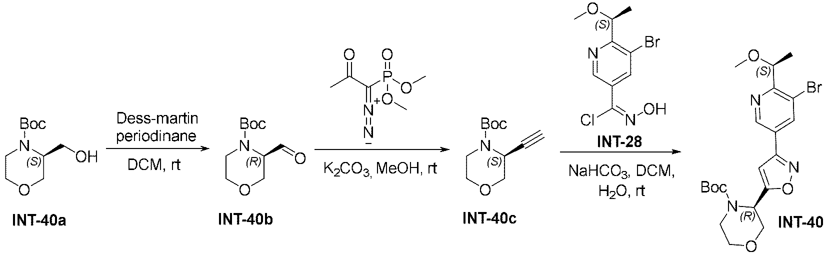

By replacing INT-33a in the synthesis step of intermediate INT-33 with INT-40a, compound INT-40 can be obtained using similar methods and reaction steps. ESI-MS (m/z): 468.4 [M+H]⁺.

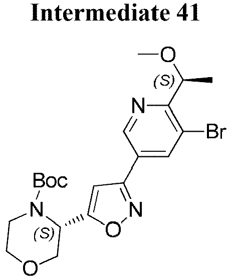

Intermediate 41 was prepared by the following steps:

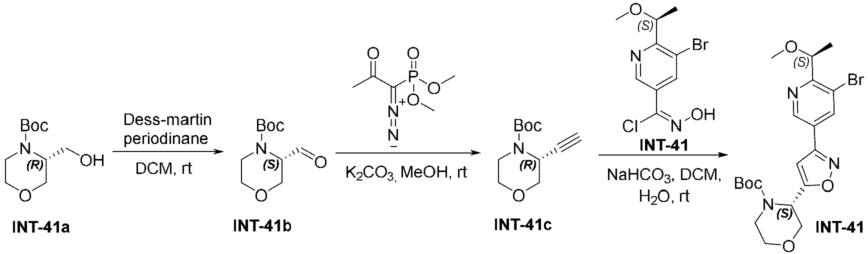

By replacing INT-33a in the synthesis step of intermediate INT-33 with INT-41a, compound INT-41 can be obtained using similar methods and reaction steps. ESI-MS (m/z): 468.4 [M+H]⁺.

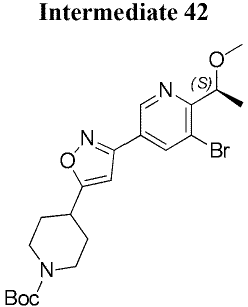

Intermediate 42 was prepared by the following steps:

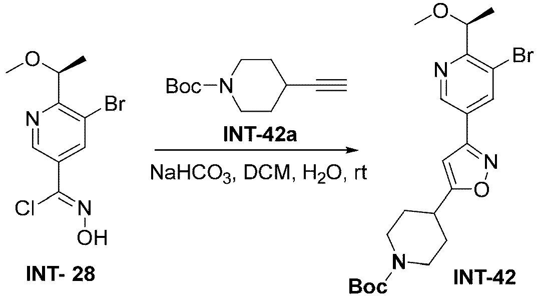

By replacing INT-29a in the synthesis step of intermediate INT-29 with INT-42a, compound INT-42 can be obtained using similar methods and reaction steps. ESI-MS (m/z): 480.4 [M+H]⁺.

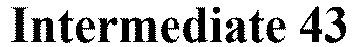

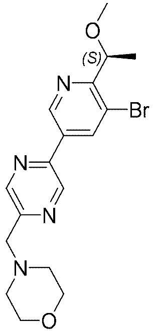

By replacing INT-16a in the synthesis step of intermediate INT-16 with 5-chloropyrazine-2-carboxaldehyde, compound INT-43 can be obtained using similar methods and reaction steps. ESI-MS (m/z): 393.4 [M+H]⁺.

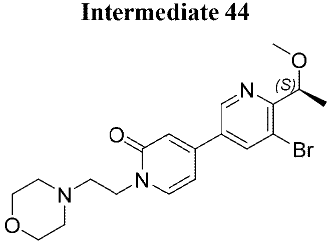

Intermediate 44 was prepared by the following steps:

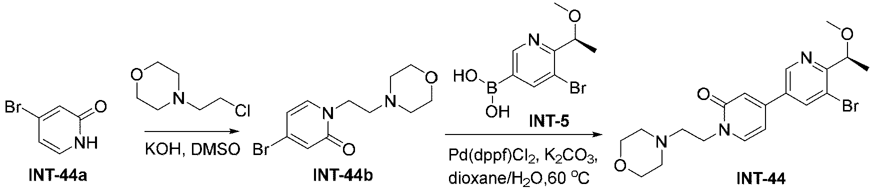

Step 1: Dissolve the intermediate INT-44a (174 mg, 1.0 mmol) in dimethyl sulfoxide (2 mL) and add potassium hydroxide (224 mg, 4.0 mmol). After the reaction was stirred at room temperature for 10 minutes, 4-(2-chloroethyl)morpholine (180 mg, 1.2 mmol) was added. The temperature was raised to 30 °C and the reaction was continued with stirring for 1 hour. The reaction was complete as monitored by LCMS. Saturated brine was added to the reaction system, the reaction system was extracted with ethyl acetate, and the organic phases were combined, washed with saturated brine, dried over anhydrous sodium sulfate, filtered and concentrated, and the residue was purified by silica gel column chromatography (dichloromethane/methanol = 20/1) to obtain colorless oily liquid INT-44b (120 mg, yield 41.8%). ESI-MS (m/z): 287.2 [M+H]⁺.

Step 2: INT-44b (106 mg, 0.37 mmol) was dissolved in a mixed solution of 1,4-dioxane (5 mL) and water (0.5 mL), and INT-5 (80 mg, 0.31 mmol), [1,1'-bis(diphenylphosphino)ferrocene]palladium dichloride (22.5 mg, 0.031 mmol) and potassium carbonate (106.4 mg, 0.77 mmol) were added in sequence. The reaction mixture was stirred at 50 °C for 16 h under nitrogen protection. After the reaction was complete, water (20 mL) was added to the reaction system, the reaction system was extracted with ethyl acetate (30 mL*2), the organic phases were combined, washed with saturated brine, dried over anhydrous sodium sulfate, filtered and concentrated, and the residue was purified by silica gel column chromatography (dichloromethane/methanol = 20/1) to obtain a light yellow solid compound INT-44 (75 mg, yield 57.7%). ESI-MS (m/z):422.3 [M+H]⁺.

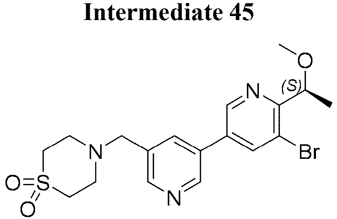

Intermediate 45 was prepared by the following steps:

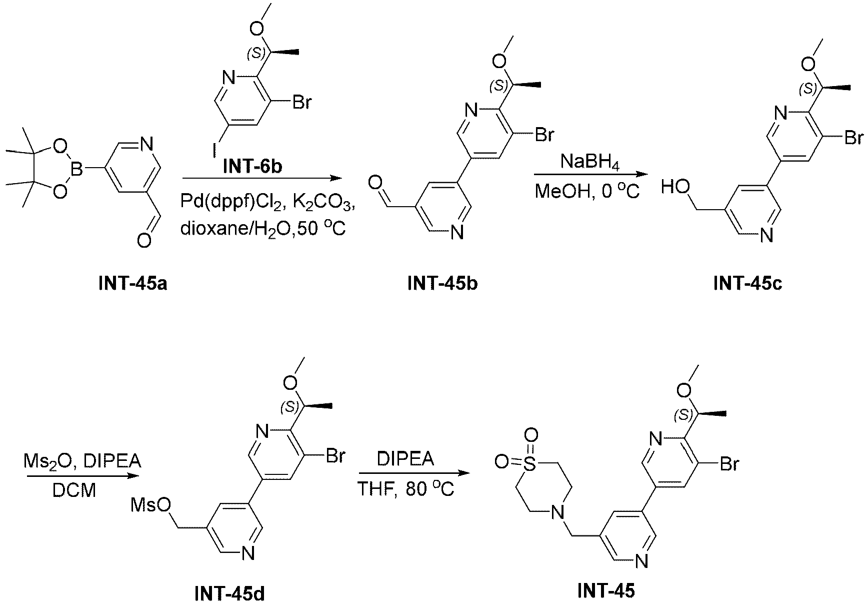

Step 1: INT-45a (238 mg, 1.02 mmol) was dissolved in a mixed solution of 1,4-dioxane (5 mL) and water (0.5 mL), and INT-6b (350 mg, 1.02 mmol), [1,1'-bis(diphenylphosphino)ferrocene]palladium dichloride (75 mg, 0.10 mmol) and potassium carbonate (283 mg, 2.04 mmol) were added in sequence. The reaction mixture was stirred at 50 °C for 16 h under nitrogen protection. After the reaction was complete, water (20 mL) was added to the reaction system, and the mixture was extracted with ethyl acetate (30 mL*2). The organic phases were combined, washed with saturated brine, dried over anhydrous sodium sulfate, filtered and concentrated, and the residue was purified by silica gel column chromatography (petroleum ether/ethyl acetate = 1:1) to obtain a light yellow solid compound INT-45b (286 mg, yield 87.0%). ESI-MS (m/z): 321.2 [M+H]⁺.

Step 2: Dissolve INT-45b (286 mg, 0.89 mmol) in methanol (5 mL), add sodium borohydride (40 mg, 1.07 mmol) under ice bath, and continue stirring for 1 hour. The reaction was complete as monitored by LCMS. Saturated ammonium chloride solution was added to quench the reaction, and the mixture was extracted with dichloromethane. The organic phases were combined and washed with saturated brine and dried over anhydrous sodium sulfate. The organic phase was concentrated and purified by silica gel column chromatography (dichloromethane/methanol = 20/1) to obtain a colorless oily liquid INT-45c (240 mg, yield 83.4%). ESI-MS (m/z): 323.4 [M+H]⁺.

Step 3: Dissolve compound INT-45c (240 mg, 0.74 mmol) in dichloromethane (3 mL), and add methanesulfonic anhydride (256 mg, 1.48 mmol) and N,N-diisopropylethylamine (384 mg, 2.96 mmol) in sequence. The reaction mixture was stirred at room temperature for 3 hours. The reaction of the starting material was complete as monitored by LCMS. Water (20 mL) was added to the reaction mixture to quench the reaction, and the mixture was extracted with ethyl acetate (20 mL*2). The organic phases were combined, dried over anhydrous sodium sulfate, filtered, and the filtrate was concentrated to obtain a crude product of compound INT-45d (298 mg, yield 99%). ESI-MS (m/z) : 401.3 [M+H]⁺.

Step 4: Dissolve compound INT-45d (130 mg, 0.32 mmol) and thiomorpholine-1,1-dioxide (66 mg, 0.49 mmol) in tetrahydrofuran (5 mL), and add N,N-diisopropylethylamine (83 mg, 0.64 mmol). The reaction mixture was stirred at 80°C for 16 hours. The reaction of the starting material was complete as monitored by LCMS. Water (20 mL) was added to the reaction mixture to quench the reaction, and the mixture was extracted with ethyl acetate (20 mL*2). The organic phases were combined and concentrated under reduced pressure. The residue was purified by column chromatography (dichloromethane/methanol = 20/1) to obtain compound INT-45 (105 mg, yield 73.6%). ESI-MS (m/z) : 440.4 [M+H]⁺.

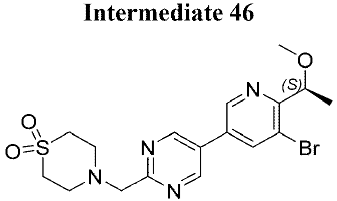

Intermediate 46 was prepared by the following steps:

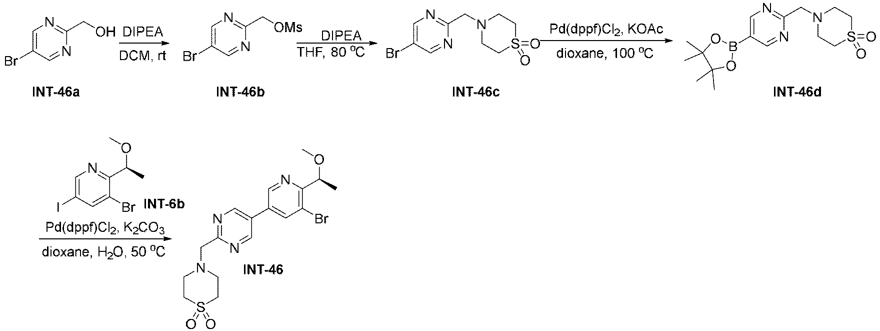

Step 1: Dissolve compound INT-46a (500 mg, 2.65 mmol) in dichloromethane (5 mL), and add N,N-diisopropylethylamine (513 mg, 3.97 mmol) and methanesulfonic anhydride (553 mg, 3.17 mmol) in turn under ice bath. The reaction mixture was stirred at room temperature for 1 hour. After the reaction was complete, water (20 mL) was added to the reaction system, and the mixture was extracted with dichloromethane (20 mL*2). The organic phases were combined and washed with saturated brine, dried over anhydrous sodium sulfate, filtered and concentrated to obtain a brown solid compound INT-46b (687 mg, yield 97.2%). ESI-MS (m/z): 267.2 [M+H]⁺.

Step 2: Dissolve compound INT-46b (687 mg, 2.57 mmol) in tetrahydrofuran (10 mL), and add N,N-diisopropylethylamine (997 mg, 7.72 mmol) and thiomorpholine 1,1-dioxide (521 mg, 3.86 mmol) under ice bath. The reaction mixture was stirred at 80°C for 8 hours. After the reaction was complete, water (20 mL) was added to the reaction system, and the mixture was extracted with dichloromethane (20 mL*2). The organic phases were combined and washed with saturated brine, dried over anhydrous sodium sulfate, filtered and concentrated to obtain a brown solid compound INT-46c (600 mg, yield 76.2%). ESI-MS (m/z): 306.2 [M+H]⁺.

Step 3: Dissolve compound INT-46c (300 mg, 0.98 mmol) in 1,4-dioxane (5 mL), and add bis(pinacolato)diboron (299 mg, 1.18 mmol), [1,1'-bis(diphenylphosphino)ferrocene]palladium dichloride (72 mg, 0.098 mmol) and potassium acetate (240 mg, 2.45 mmol) in sequence. The mixture was stirred at 100 °C overnight under N2 protection. The reaction was complete after monitoring by LCMS. The mixture was filtered through celite and the filtrate was concentrated to obtain a crude black solid compound INT-46d, which was used directly in the next step without purification. ESI-MS (m/z): 354.3 [M+H]⁺.

Step 4: The crude compound INT-46d obtained above was dissolved in 1,4-dioxane (5 mL) and water (0.5 mL), and INT-6b (320 mg, 0.91 mmol), potassium carbonate (314 mg, 2.45 mmol) and [1,1'-bis(diphenylphosphino)ferrocene]palladium dichloride (72 mg, 0.098 mmol) were added in sequence. The mixture was stirred at 50 °C overnight under N2 protection. The reaction of the raw material was completed after LCMS monitoring. The product was filtered through celite, the filtrate was concentrated, and the residue was purified by column chromatography (dichloromethane/methanol = 30:1) to obtain a light yellow solid compound INT-46 (350 mg, yield 81.1%). ESI-MS (m/z): 441.3 [M+H]⁺.

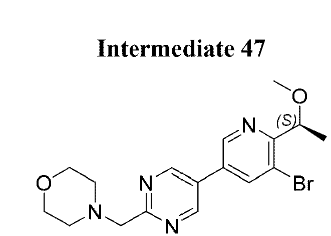

By replacing thiomorpholine 1,1-dioxide in the synthesis step of intermediate INT-46 with morpholine, compound INT-47 can be obtained using similar methods and reaction steps. ESI-MS (m/z): 393.4 [M+H]⁺.

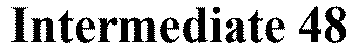

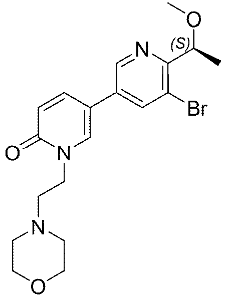

By replacing INT-44a in the synthesis step of intermediate INT-44 with 2-hydroxy-5-bromopyridine, compound INT-48 can be obtained using similar methods and reaction steps. ESI-MS (m/z): 422.4 [M+H]⁺.

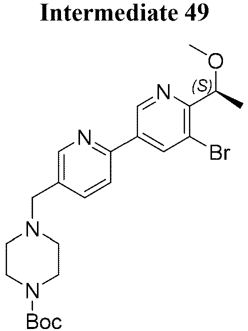

By replacing morpholine in the synthesis step of intermediate INT-16 with 1-tert-butyloxycarbonylpiperazine, compound INT-49 can be obtained using similar methods and reaction steps. ESI-MS (m/z): 491.4 [M+H]⁺.

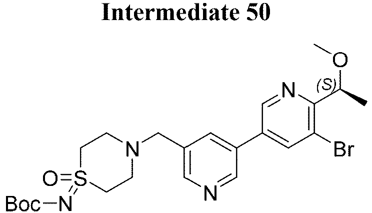

Intermediate 50 was prepared by the following steps:

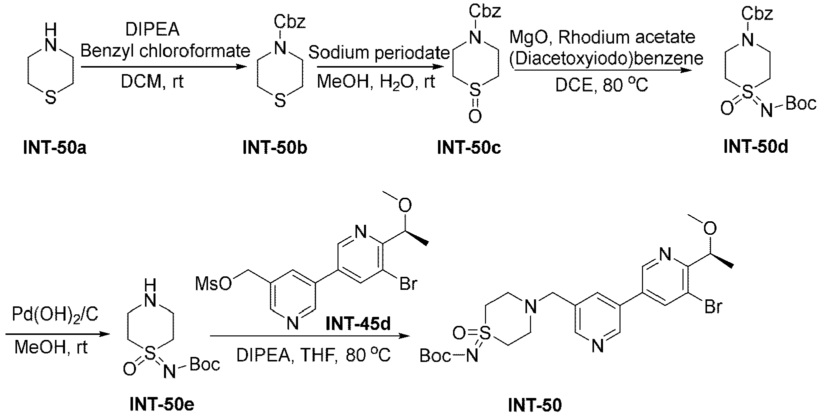

Step 1: Dissolve thiomorpholine INT-50a (4.0 g, 38.8 mmol), benzyl chloroformate (7.94 g, 46.5 mmol), and N,N-diisopropylethylamine (15.0 g, 116 mmol) in dichloromethane (40 mL), and stir the reaction solution at room temperature overnight. After the reaction was completed, the reaction solution was extracted with dichloromethane, and the organic phase was dried and concentrated to obtain a crude product. The crude product was purified by column chromatography (petroleum ether/ethyl acetate = 1/1) to obtain a light yellow oily compound INT-50b (9.0 g, yield 97.8%). ESI-MS (m/z): 238.3 [M+H]⁺.

Step 2: Compound INT-50b (1.0 g, 38.8 mmol) was dissolved in a mixed solution of methanol (10 mL) and water (5 mL), sodium periodate (1.17 g, 5.5 mmol) was added under ice bath, and the reaction solution was stirred at room temperature overnight. After the reaction was completed, the reaction solution was extracted with ethyl acetate, and the organic phase was dried and concentrated to obtain a crude product INT-50c (900 mg, yield 84.3%). The crude product was used directly in the next reaction without purification. ESI-MS (m/z): 254.3 [M+H]⁺.

Step 3: Dissolve compound INT-50c (850 mg, 3.36 mmol) in 1,2-dichloroethane (15 mL), add tert-butyl carbamate (1.97 g, 16.8 mmol), rhodium acetate (94 mg, 0.34 mmol), magnesium oxide (541 mg, 13.4 mmol) and iodophenyl diacetic acid (3.24 g, 10.1 mmol) at room temperature, and stir the reaction solution at 80 °C overnight. After the reaction was completed, the reaction solution was concentrated. The crude product was purified by column chromatography (dichloromethane/methanol = 10/1) to obtain a light yellow solid compound INT-50d (1.0 g, yield 80.9%). ESI-MS (m/z): 369.3 [M+H]⁺.

Step 4: Dissolve compound INT-50d (850 mg, 2.31 mmol) in methanol (10 mL) and add palladium hydroxide/carbon (85 mg, 10% w/w). The reaction solution was stirred at room temperature overnight under a hydrogen atmosphere. After the reaction was completed, the mixture was filtered through celite and the filtrate was concentrated to obtain a light yellow oily compound INT-50e (410 mg, yield 75.9%). ESI-MS (m/z): 235.3 [M+H]⁺.

By replacing thiomorpholine-1,1-dioxide in the synthesis step of intermediate INT-45 with INT-50e, compound INT-50 can be obtained using similar methods and reaction steps. ESI-MS (m/z): 539.4 [M+H]⁺.

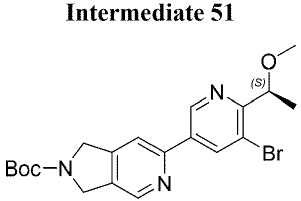

Intermediate 51 was prepared by the following steps:

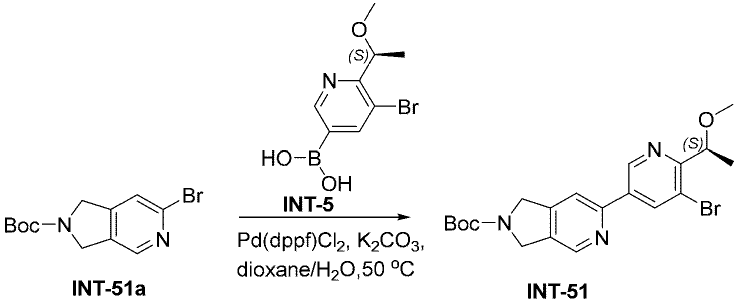

Step 1: INT-51a (200 mg, 0.67 mmol) was dissolved in a mixed solution of 1,4-dioxane (5 mL) and water (0.5 mL), and INT-5 (174 mg, 0.67 mmol), [1,1'-bis(diphenylphosphino)ferrocene]palladium dichloride (49 mg, 0.067 mmol) and potassium carbonate (185 mg, 1.34 mmol) were added in sequence. The reaction mixture was stirred at 50 °C for 16 h under nitrogen protection. After the reaction was complete, water (20 mL) was added to the reaction system, and the mixture was extracted with ethyl acetate (30 mL*2). The organic phases were combined and washed with saturated brine, dried over anhydrous sodium sulfate, filtered and concentrated. The residue was purified by silica gel column chromatography (petroleum ether/ethyl acetate = 1/1) to obtain a light yellow solid compound INT-51 (130 mg, yield 44.8%). ESI-MS (m/z): 434.2 [M+H]⁺.

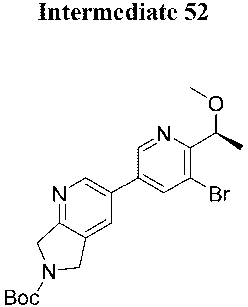

By replacing INT-51a in the synthesis step of intermediate INT-51 with 3-bromo-6-(tert-butoxycarbonyl)-6,7-dihydro-5H-pyrrolo[3,4-b]pyridine, compound INT-52 can be obtained using similar methods and reaction steps. ESI-MS (m/z): 434.4 [M+H]⁺.

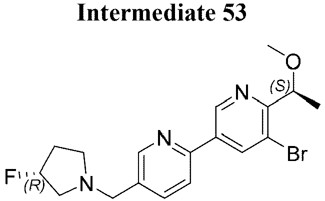

By replacing morpholine in the synthesis step of intermediate INT-16 with (R)-3-fluoropyrrolidine hydrochloride, compound INT-53 can be obtained using similar methods and reaction steps. ESI-MS (m/z): 394.4 [M+H]⁺.

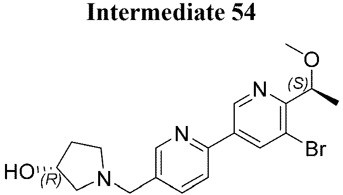

By replacing morpholine in the synthesis step of intermediate INT-16 with (R)-3-fluoropyrrolidine hydrochloride, compound INT-54 can be obtained using similar methods and reaction steps. ESI-MS (m/z): 392.4 [M+H]⁺.

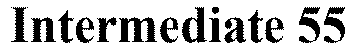

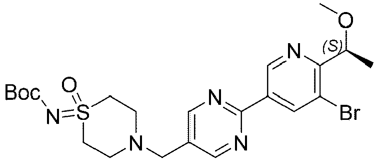

By replacing INT-45b in the synthesis step of intermediate INT-45 with INT-19b and replacing thiomorpholine-1,1-dioxide with INT-50e, compound INT-55 can be obtained using similar methods and reaction steps. ESI-MS (m/z): 540.4 [M+H]⁺.

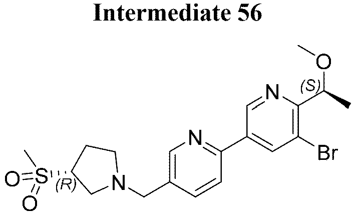

By replacing morpholine in the synthesis step of intermediate INT-16 with (R)-3-(methylsulfonyl)pyrrolidine, compound INT-56 can be obtained using similar methods and reaction steps. ESI-MS (m/z): 454.4 [M+H]⁺.

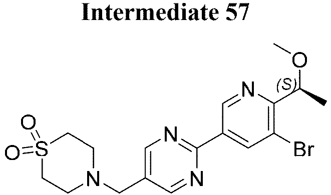

By replacing INT-45b in the synthesis step of intermediate INT-45 with INT-19b, compound INT-57 can be obtained using similar methods and reaction steps. ESI-MS (m/z): 441.3 [M+H]⁺.

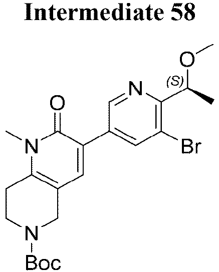

Intermediate 58 was prepared by the following steps:

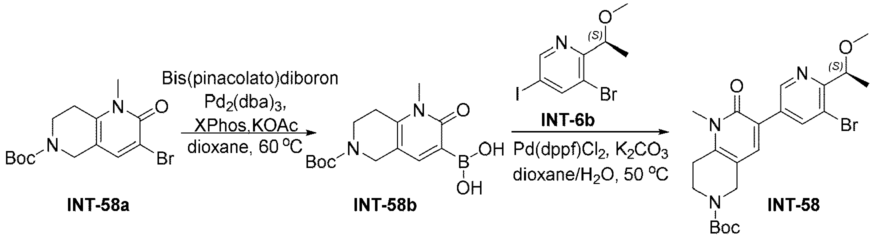

By replacing INT-10b in the synthesis step of intermediate INT-10 with INT-58a, compound INT-58 can be obtained using similar methods and reaction steps. ESI-MS (m/z): 478.3 [M+H]⁺.

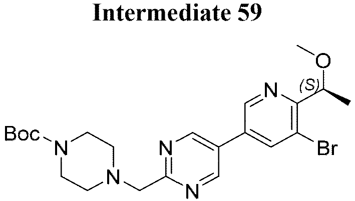

By replacing 1,1-thiomorpholine dioxide in the synthesis step of intermediate INT-46 with 1-tert-butyloxycarbonylpiperazine, compound INT-59 can be obtained using similar methods and reaction steps. ESI-MS (m/z): 492.3 [M+H]⁺.

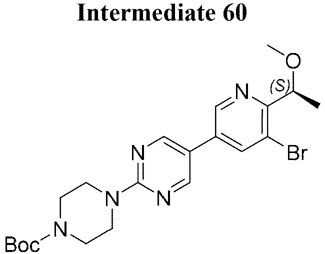

By replacing INT-6a in the synthesis step of intermediate INT-6 with tert-butyl 4-(5-(4,4,5,5-tetramethyl-1,3,2-dioxaborolan-2-yl)pyrimidin-2-yl)piperazine-1-carboxylate, compound INT-60 can be obtained using similar methods and reaction steps. ESI-MS (m/z): 478.3 [M+H]⁺.

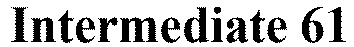

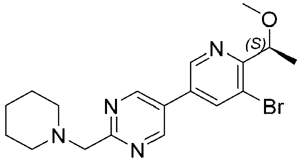

By replacing thiomorpholine-1,1-dioxide in the synthesis step of intermediate INT-46 with piperidine, compound INT-61 can be obtained using similar methods and reaction steps. ESI-MS (m/z): 391.3 [M+H]⁺.

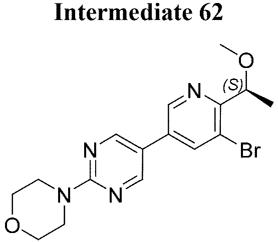

By replacing INT-6a in the synthesis step of intermediate INT-6 with 2-(4-morpholinyl)pyrimidine-5-boronic acid naphthalene ester, compound INT-62 can be obtained using similar methods and reaction steps. ESI-MS (m/z): 379.3 [M+H]⁺.

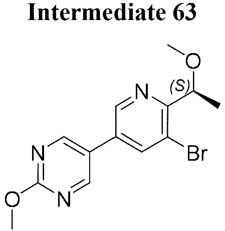

By replacing INT-6a in the synthesis step of intermediate INT-6 with 2-methoxy-5-pyrimidineboronic acid, compound INT-63 can be obtained using similar methods and reaction steps. ESI-MS (m/z): 324.3 [M+H]⁺.

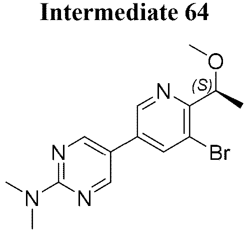

By replacing INT-6a in the synthesis step of intermediate INT-6 with (2-(dimethylamino)pyrimidin-5-yl)boronic acid, compound INT-64 can be obtained using similar methods and reaction steps. ESI-MS (m/z): 337.3 [M+H]⁺.

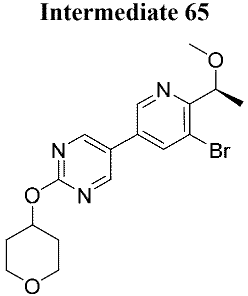

Intermediate 65 was prepared by the following steps:

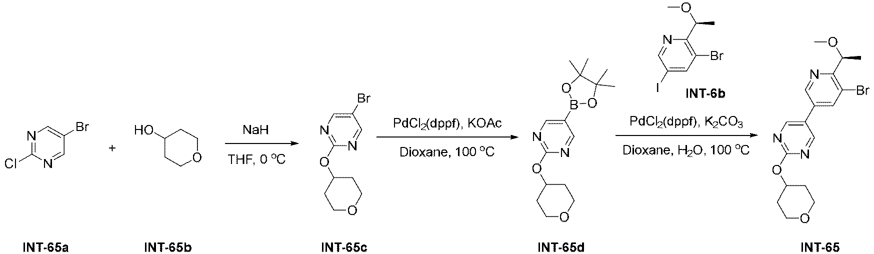

Step 1: Dissolve sodium hydride (134 mg, 3.35 mmol, 60% purity) in tetrahydrofuran (5 mL) and replace with nitrogen. INT-65b (264 mg, 2.58 mmol) was added under ice-cooling and the reaction was stirred for 30 minutes. INT-65a (500 mg, 2.58 mmol) was then added, and the reaction solution was stirred in an ice bath for 2 hours. After the reaction was completed as monitored by LCMS, saturated aqueous ammonium chloride solution (20 mL) was added to the reaction system, and the mixture was extracted with ethyl acetate (20 mL*2). The organic phases were combined, washed with saturated brine, dried over anhydrous sodium sulfate, filtered and concentrated, and the residue was purified by silica gel column chromatography (dichloromethane) to obtain a brown solid compound INT-65c (288 mg, yield 43.0%). ESI-MS (m/z) : 259.3 [M+H]⁺.

Step 2: Dissolve compound INT-65c (200 mg, 0.77 mmol) in 1,4-dioxane (3 mL), and add bis(pinacolato)diboron (196 mg, 0.77 mmol), [1,1'-bis(diphenylphosphino)ferrocene]palladium dichloride (56 mg, 0.08 mmol) and potassium acetate (227 mg, 2.32 mmol) in sequence. The reaction system was replaced with nitrogen and heated to 100 °C and stirred for 12 h. LCMS monitored that the reaction of the raw materials was complete. After the reaction solution was cooled to room temperature, the reaction solution was filtered through diatomaceous earth and the filtrate was concentrated to obtain a crude product of compound INT-65d (236 mg, yield 99.9%). ESI-MS (m/z): 307.6 [M+H]⁺.

Step 3: Dissolve compound INT-65d (236 mg, 0.77 mmol) and compound INT-6b (220 mg, 0.64 mmol) in 1,4-dioxane (3 mL) and water (0.3 mL), and add [1,1'-bis(diphenylphosphino)ferrocene]palladium dichloride (47 mg, 0.06 mmol) and potassium carbonate (266 mg, 1.93 mmol) in sequence. The reaction system was replaced with nitrogen and heated to 50 °C and stirred for 12 h. LCMS monitored the complete reaction of the raw materials. After the reaction solution was cooled to room temperature, the reaction solution was filtered through diatomaceous earth, the filtrate was concentrated, and the residue was purified by silica gel column chromatography (ethyl acetate) to obtain a yellow oily liquid INT-65 (182 mg, yield 71.9%). ESI-MS (m/z): 394.4 [M+H]⁺.

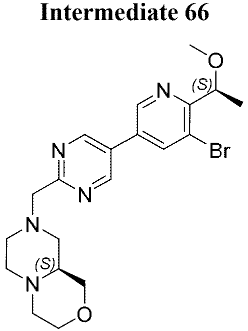

By replacing the thiomorpholine-1,1-dioxide in the synthesis step of the intermediate INT-46 with (S)-octahydropyrazine[2,1-c][1,4]oxazine dihydrochloride, the compound INT-66 can be obtained using a similar method and reaction steps. ESI-MS (m/z): 448.3 [M+H]⁺.

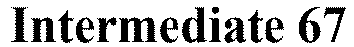

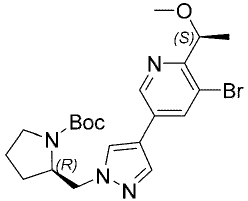

By replacing INT-21a in the synthesis step of intermediate INT-21 with (R)-2-(hydroxymethyl)pyrrolidine-1-carboxylic acid tert-butyl ester, compound INT-67 can be obtained using similar methods and reaction steps. ESI-MS (m/z): 465.3 [M+H]⁺.

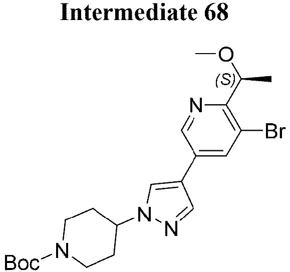

By replacing INT-6a in the synthesis step of intermediate INT-6 with tert-butyl 4-[4-(4,4,5,5-tetramethyl-1,3,2-dioxaborolan-2-yl)-1H-pyrazol-1-yl]piperidine-1-carboxylate, compound INT-68 can be obtained using similar methods and reaction steps. ESI-MS (m/z): 465.3 [M+H]⁺.

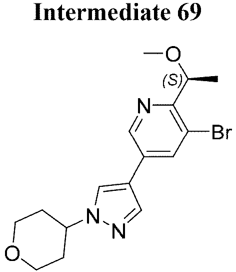

By replacing INT-6a in the synthesis step of intermediate INT-6 with 1-(tetrahydropyran-4-yl)-1H-pyrazole-4-boronic acid pinacol ester, compound INT-69 can be obtained using similar methods and reaction steps. ESI-MS (m/z): 366.3 [M+H]⁺.

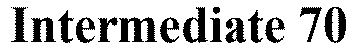

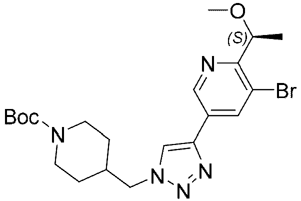

Intermediate 70 was prepared by the following steps:

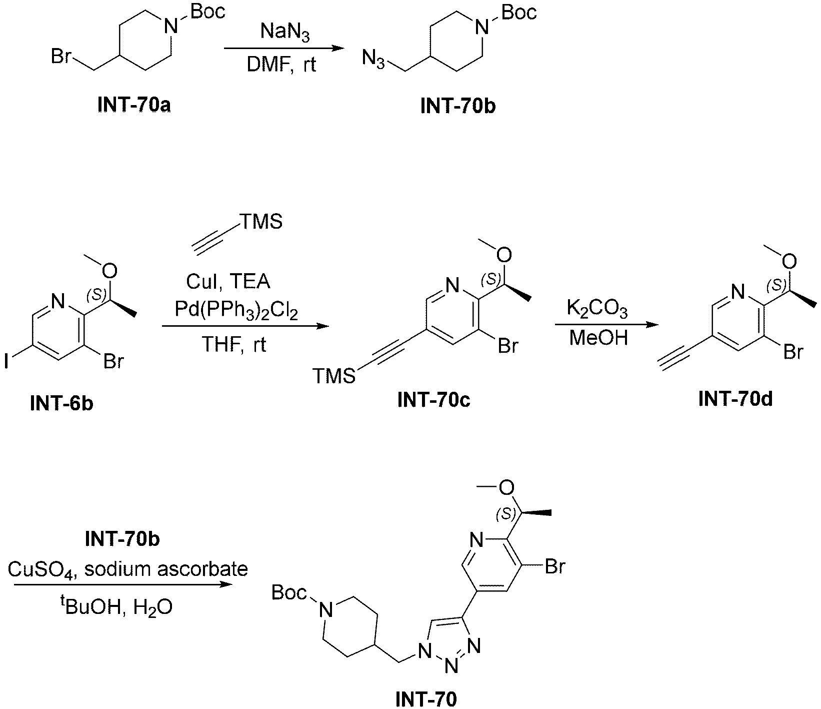

Step 1: Dissolve INT-70a (520 mg, 1.87 mmol) in N,N-dimethylformamide (3 mL), add sodium azide (182 mg, 2.81 mmol) under ice bath, and stir the reaction solution at room temperature for 16 hours. After the reaction was completed as monitored by LCMS, water (20 mL) was added to the reaction system, and the mixture was extracted with ethyl acetate (20 mL*2). The organic phases were combined and washed with saturated brine, dried over anhydrous sodium sulfate, filtered and concentrated to obtain a brown oily compound INT-70b (430 mg, yield 95.7%). ESI-MS (m/z) : 241.3 [M+H]⁺.

Step 2: INT-6b (1.0 g, 2.92 mmol), trimethylsilyl acetylene (345 mg, 3.51 mmol), cuprous iodide (56 mg, 0.29 mmol), bistriphenylpalladium dichloride (205 mg, 0.29 mmol) and triethylamine (592 mg, 5.84 mmol) were dissolved in 1, 4-dioxane (10 mL), and the reaction solution was stirred at room temperature overnight. After the reaction is completed, the reaction solution is filtered, and the filtrate is concentrated and spin-dried to obtain a crude product. The crude product was purified by column chromatography (petroleum ether/ethyl acetate = 10/1) to obtain a light yellow oily liquid compound INT-70c (880 mg, yield 96.4%). ESI-MS (m/z): 312.3 [M+H]⁺.

Step 3: INT-70c (880 mg, 2.82 mmol) was dissolved in methanol (10 mL), potassium carbonate (584 mg, 4.23 mmol) was added at room temperature, and the reaction solution was stirred at room temperature for 4 h. After the reaction was completed, water (30 mL) was added, extracted with ethyl acetate (30 mL*2), the organic phases were combined, dried over anhydrous sodium sulfate, filtered, and the filtrate was concentrated and spin-dried to obtain a crude product. The crude product was purified by column chromatography (petroleum ether/ethyl acetate = 3/1) to obtain a light yellow oily compound INT-70d (450 mg, yield 66.5%). ESI-MS (m/z): 240.3 [M+H]⁺.

Step 4: Dissolve compound INT-70d (300 mg, 1.25 mmol) in tert-butyl alcohol (2 mL) and water (2 mL), and add INT-70b (450 mg, 1.87 mmol), sodium ascorbate (1.24 g, 6.25 mmol), and copper sulfate (997 mg, 6.25 mmol) in sequence. The reaction mixture was stirred at room temperature for 16 hour. After the reaction was complete, water (20 mL) was added to the reaction system, and the mixture was extracted with ethyl acetate (20 mL*2). The organic phases were combined and washed with saturated brine, dried over anhydrous sodium sulfate, filtered and concentrated. The residue was purified by column chromatography (petroleum ether/ethyl acetate = 1:1) to obtain a light yellow oily compound INT-70 (460 mg, yield 76.6%). ESI-MS (m/z): 480.3 [M+H]⁺.

The synthesis method of the embodiment compounds in the examples of the present disclosure are as follows:

### Example 1

### (1r,2R,3S)-N-((6³S,4S,Z)-1¹-ethyl-1²-(2-((S)-1-methoxyethyl)-5-(4-(morpholinomethyl)phenyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

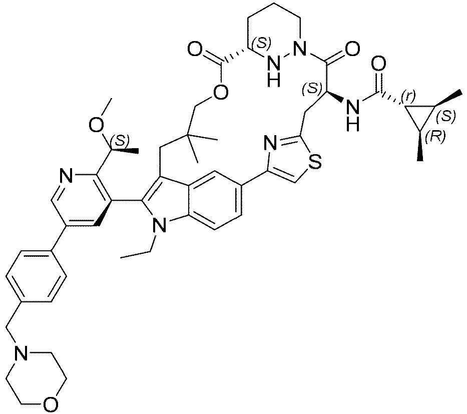

Example 1 was prepared by the following steps:

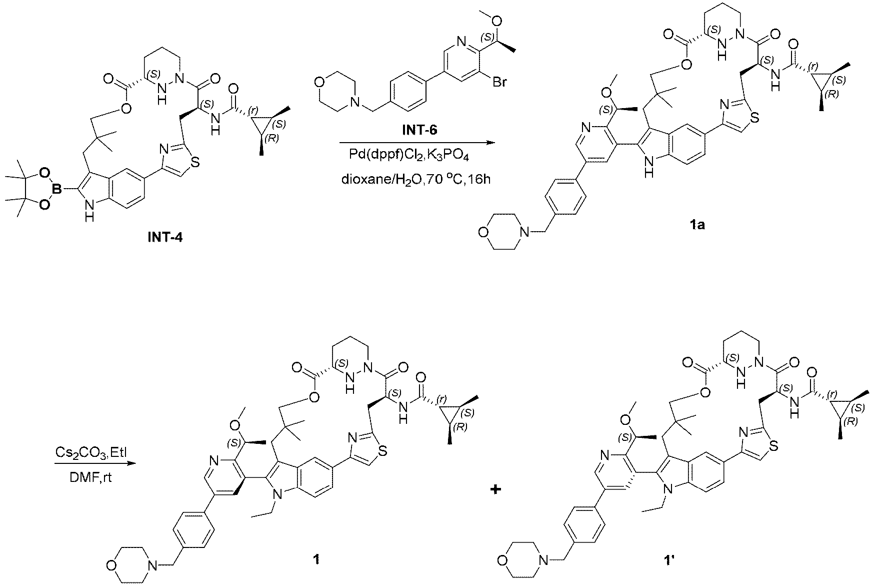

Step 1: Dissolve compound INT-4 (84.6 mg, 0.12 mmol) in a mixed solution of 1,4-dioxane (3 mL) and water (0.3 mL), and add INT-6 (40 mg, 0.10 mmol), [1,1'-bis(diphenylphosphino)ferrocene]palladium dichloride (7.5 mg, 0.010 mmol) and potassium phosphate (65.1 mg, 0.30 mmol) in sequence. The reaction mixture was stirred at 70 °C for 16 h under nitrogen protection. After the reaction was complete, water (20 mL) was added to the reaction system, and the mixture was extracted with ethyl acetate (30 mL*2). The organic phases were combined, washed with saturated brine, dried over anhydrous sodium sulfate, filtered and concentrated. The residue was purified by preparative thin layer chromatography (dichloromethane/methanol = 20:1) to obtain a light yellow solid compound 1a (55 mg, yield 62.9%). ESI-MS (m/z): 874.5 [M+H]⁺.

Step 2: Compound 1a (55 mg, 0.063 mmol) was dissolved in N,N-dimethylformamide (2 mL), and cesium carbonate (41 mg, 0.126 mmol) and iodoethane (14.7 mg, 0.094 mmol) were added thereto. The reaction mixture was stirred at room temperature for 16 hours. After the reaction was complete, water (30 mL) was added to the reaction system, and the mixture was extracted with ethyl acetate (30 mL*2). The organic phases were combined and washed with saturated brine, dried over anhydrous sodium sulfate, filtered and concentrated. The residue was purified by preparative liquid chromatography to obtain white solid compound 1 (6 mg, yield 10.6%) and diastereomer 1' (12 mg, yield 21.1%). The absolute configurations drawn for the two compounds are assumptions made based on experience. Compound 1 is an isomer with a longer retention time in LCMS or HPLC, while 1' is an isomer with a shorter retention time in LCMS or HPLC.

### Compound 1

ESI-MS (m/z): 902.5 [M+H]⁺; LC-MS retention time RT=1.96 min. HPLC retention time RT= 15.10 min.

¹H NMR (500 MHz, DMSO-d6) δ 9.09 (d, *J* = 2.5 Hz, 1H), 8.55 - 8.49 (m, 1H), 8.41 - 8.35 (m, 1H), 8.06 (d, *J=* 2.5 Hz, 1H), 7.82 (s, 1H), 7.81 - 7.78 (m, 2H), 7.77 - 7.74 (m, 1H), 7.61 - 7.58 (m, 1H), 7.47 - 7.43 (m, 2H), 5.56 (t, *J* = 9.0 Hz, 1H), 5.10 - 5.04 (m, 1H), 4.38 - 4.28 (m, 2H), 4.26 - 4.13 (m, 3H), 3.62 - 3.56 (m, 6H), 3.52 (s, 2H), 3.31 - 3.30 (m, 1H), 3.27 (s, 3H), 3.17 - 3.12 (m, 1H), 3.01 - 2.93 (m, 1H), 2.79 - 2.72 (m, 1H), 2.49 - 2.43 (m, 1H), 2.41 - 2.34 (m, 4H), 2.10 - 2.03 (m, 1H), 1.83 - 1.71 (m, 2H), 1.57 - 1.48 (m, 1H), 1.41 (d, *J =* 6.0 Hz, 3H), 1.21 - 1.13 (m, 3H), 1.11 - 1.03 (m, 6H), 0.95 - 0.86 (m, 6H), 0.38 (s, 3H).

### Compound 1'

ESI-MS (m/z): 902.5 [M+H]⁺; LC-MS retention time RT=1.94 min. HPLC retention time RT= 14.90 min.

¹H NMR (500 MHz, DMSO-d6) δ 9.09 (d, *J =* 2.5 Hz, 1H), 8.57 - 8.53 (m, 1H), 8.43 - 8.38 (m, 1H), 8.21 (d, *J =* 2.5 Hz, 1H), 7.82 (s, 1H), 7.78 - 7.73 (m, 3H), 7.58 - 7.53 (m, 1H), 7.47 - 7.43 (m, 2H), 5.54 (t, *J* = 9.0 Hz, 1H), 5.07 - 5.02 (m, 1H), 4.27 - 4.17 (m, 2H), 4.05 - 3.95 (m, 2H), 3.92 - 3.84 (m, 1H), 3.70 - 3.65 (m, 1H), 3.62 - 3.55 (m, 5H), 3.52 (s, 2H), 3.37 - 3.31 (m, 1H), 3.20 - 3.14 (m, 1H), 3.13 (s, 3H), 3.10 - 3.04 (m, 1H), 2.80 - 2.73 (m, 1H), 2.44 - 2.35 (m, 5H), 2.16 - 2.08 (m, 1H), 1.84 - 1.74 (m, 2H), 1.57 - 1.49 (m, 1H), 1.28 (d, *J* = 6.0 Hz, 3H), 1.22 - 1.05 (m, 12H), 0.93 (s, 3H), 0.54 (s, 3H).

### Example 2

### (1S,2S)-N-((6³S,4S,Z)-11-ethyl-1²-(2-((S)-1-methoxyethyl)-5-(2-methylisoindolin-5-yl)pyridin-3-yl)-10,10-dimethyl-5,7-dioxo-6¹,6²,6³ 6⁴ 6⁵ 6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

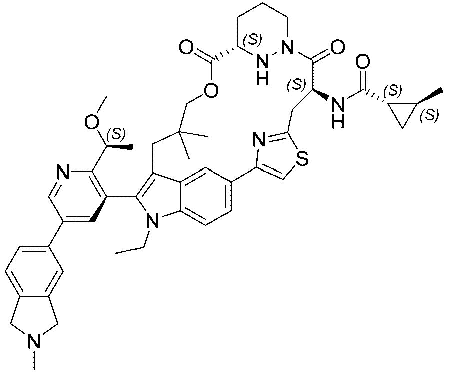

Example 2 was prepared by the following steps:

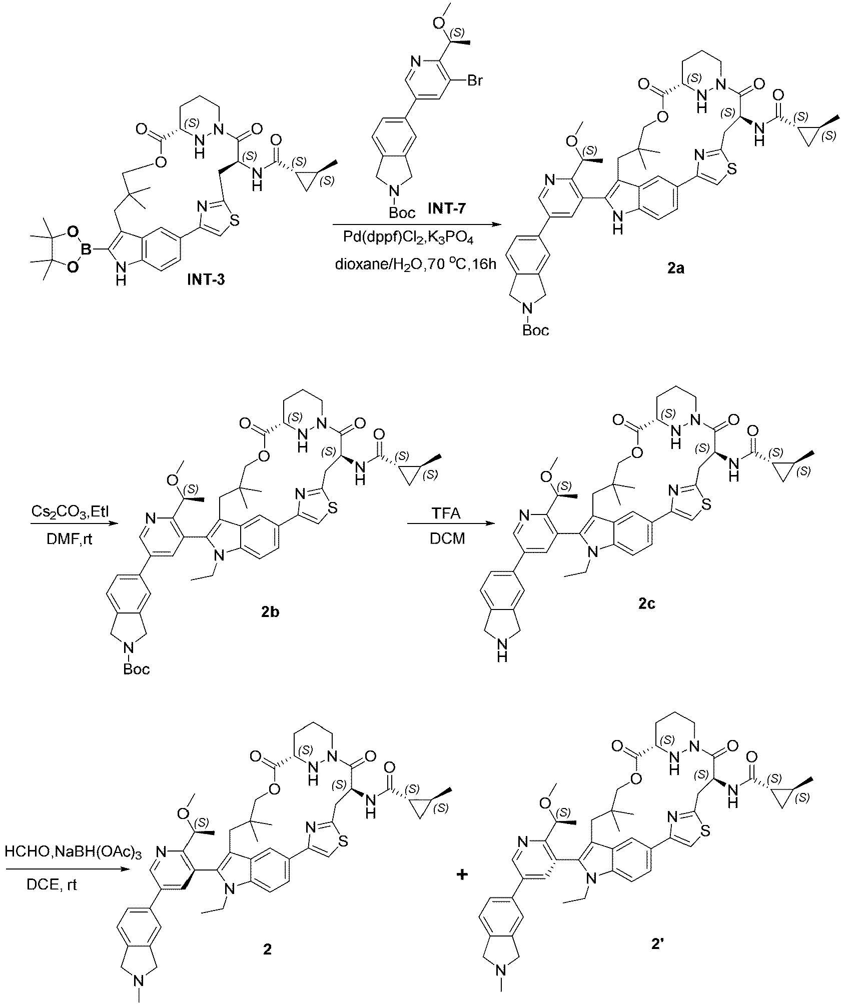

Step 1: Dissolve compound INT-3 (85.8 mg, 0.127 mmol) in a mixed solution of 1,4-dioxane (3 mL) and water (0.3 mL), and add INT-7 (50 mg, 0.115 mmol), [1,1'-bis(diphenylphosphino)ferrocene]palladium dichloride (8.4 mg, 0.012 mmol) and potassium phosphate (98.0 mg, 0.461 mmol) in sequence. The reaction mixture was stirred at 70 °C for 16 h under nitrogen protection. After the reaction was complete, water (20 mL) was added to the reaction system, and the mixture was extracted with ethyl acetate (30 mL*2). The organic phases were combined, washed with saturated brine, dried over anhydrous sodium sulfate, filtered and concentrated. The residue was purified by preparative thin layer chromatography (dichloromethane/methanol = 20:1) to obtain a light yellow solid compound 2a (80 mg, yield 76.9%). ESI-MS (m/z): 902.7 [M+H]⁺.

Step 2: Compound 2a (80 mg, 0.089 mmol) was dissolved in DMF (2 mL), cesium carbonate (58 mg, 0.178 mmol) was added thereto, and then iodoethane (21 mg, 0.134 mmol) was added dropwise to the reaction solution, and the reaction solution was stirred at room temperature for 16 hours. After the reaction was completed by LCMS detection, saturated brine was added to the reaction solution, and the mixture was extracted with ethyl acetate. The organic phases were combined and washed with saturated brine, dried over anhydrous sodium sulfate, and the organic phase was concentrated to obtain compound 2b (78.6 mg, yield 95%). ESI-MS (m/z): 930.7 [M+H]⁺.

Step 3: Trifluoroacetic acid (0.5 mL) was added dropwise to a solution of 2b (78.6 mg, 0.085 mmol) in dichloromethane (2 mL). The reaction solution was stirred at room temperature for 30 minutes and the reaction was completed as monitored by LCMS. The reaction solution was concentrated by distillation under reduced pressure, and saturated aqueous sodium carbonate solution was added, extracted with ethyl acetate, and the organic phases were combined and washed with saturated brine, dried over anhydrous sodium sulfate, and concentrated to give compound 2c (67 mg, yield 95%). ESI-MS (m/z): 830.5 [M +H]⁺.

Step 4: Formaldehyde aqueous solution (20 mg, 0.243 mmol, 37%) was added dropwise to a solution of compound 3c (67 mg, 0.081 mmol) in 1,2-dichloroethane (2 mL). After the reaction solution was stirred at room temperature for 20 minutes, sodium triacetoxyborohydride (51.5 mg, 0.243 mmol) was added thereto, and the reaction solution was further stirred at room temperature for 30 minutes. After the reaction was completed as monitored by LCMS, saturated aqueous ammonium chloride was added to quench the reaction, the reaction solution was extracted with ethyl acetate, and the organic phases were combined and washed with saturated brine, dried over anhydrous sodium sulfate, and the organic phase was concentrated and purified by preparative liquid chromatography to obtain white solid compound 2 (2 mg, yield 2.9%) and diastereomer compound 2' (4 mg, yield 5.8%). The absolute configurations of the two compounds are drawn based on the empirical assumption that compound 2 is the isomer with a longer retention time in LCMS or HPLC, while 2' is the isomer with a shorter retention time in LCMS or HPLC.

### Compound 2

ESI-MS (m/z): 844.6 [M+H]⁺; LC-MS retention time RT = 1.87 min. HPLC retention time; Retention time RT = 13.87 min.

### Compound 2'

ESI-MS (m/z): 844.6 [M+H]⁺. LC-MS retention time RT = 1.82 min. HPLC retention time Retention time RT = 13.33 min.

### Example 3

### (1r,2R,3S)-N-((6³S,4S,Z)-1'-ethyl-1²-(2-((S)-1-methoxyethyl)-5-(2-methyl-1,2,3,4-tetrahydroisoquinolin-6-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

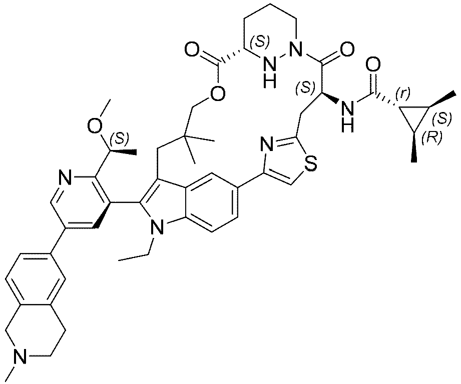

Example 3 was prepared by the following steps:

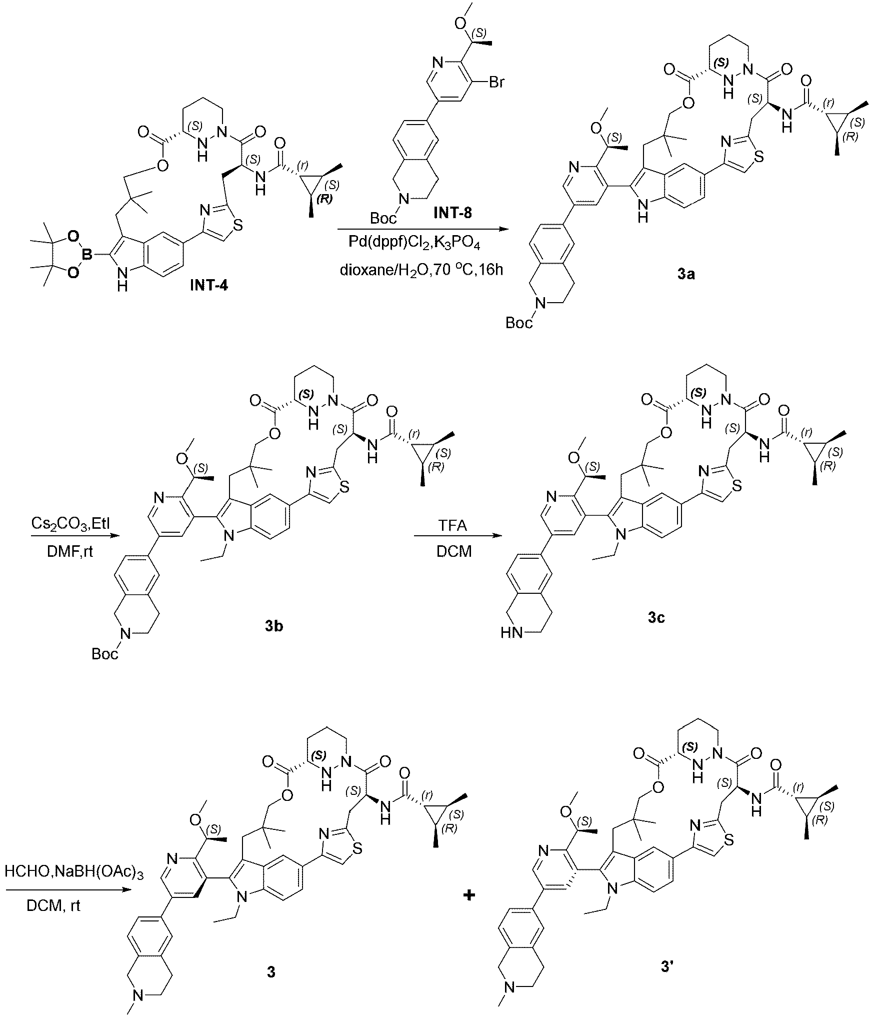

By replacing INT-7 in the synthesis step of compound 2 with INT-8 and replacing INT-3 with INT-4, compound 3 and its diastereoisomer compound 3' can be obtained using similar methods and reaction steps. The absolute configurations of the two compounds are drawn based on the empirical assumption that compound 3 is the isomer with a longer retention time in LCMS or HPLC, while 3' is the isomer with a shorter retention time in LCMS or HPLC.

### Compound 3

ESI-MS (m/z): 872.6 [M+H]⁺; LC-MS retention time RT = 1.83 min. HPLC retention time RT = 14.98 min.

¹H NMR (500 MHz, DMSO-*d*₆) δ 8.99 (d, *J =* 2.5 Hz, 1H), 8.44 (d, *J =* 1.5 Hz, 1H), 8.32 (d, *J* = 9.0 Hz, 1H), 7.95 (d, *J* = 2.5 Hz, 1H), 7.74 (s, 1H), 7.68 (dd, *J* = 8.5, 1.5 Hz, 1H), 7.56 - 7.48 (m, 3H), 7.12 (d, *J =* 8.5 Hz, 1H), 5.49 (t, *J* = 9.0 Hz, 1H), 5.02 - 4.96 (m, 1H), 4.29 - 4.24 (m, 2H), 4.19 - 4.06 (m, 3H), 3.52 (s, 2H), 3.45 (s, 3H), 3.25 - 3.20 (m, 1H), 3.20 (s, 3H), 3.11 - 3.06 (m, 1H), 2.94 - 2.86 (m, 1H), 2.85 - 2.80 (m, 2H), 2.72 - 2.64 (m, 1H), 2.57 - 2.50 (m, 2H), 2.40 - 2.36 (m, 1H), 2.28 (s, 3H), 2.03-1.96 (m, 1H), 1.75 - 1.70 (m, 2H), 1.47 - 1.42 (m, 1H), 1.34 (d, *J =* 6.0 Hz, 3H), 1.18 - 1.13 (m, 1H), 1.11 - 1.08 (m, 2H), 1.03-0.99 (m, 6H), 0.85 (t, *J* = 7.0 Hz, 3H), 0.84 - 0.79 (m, 2H), 0.31 (s, 3H).

### Compound 3'

ESI-MS (m/z): 872.6 [M+H]⁺. LC-MS retention time RT = 1.94 min. HPLC retention time RT = 14.48 min.

¹H NMR (500 MHz, DMSO-*d*₆) δ 9.06 (d, *J =* 2.5 Hz, 1H), 8.55 (d, *J =* 2.0 Hz, 1H), 8.41 (d, *J =* 9.0 Hz, 1H), 8.16 (t, *J* = 2.5 Hz, 1H), 7.82 (d, *J =* 2.5 Hz, 1H), 7.74 (dd, *J* = 8.5, 1.5 Hz, 1H), 7.58 - 7.48 (m, 3H), 7.19 (d, *J* = 8.0 Hz, 1H), 5.54 (t, *J =* 9.0 Hz, 1H), 5.05-5.00 (m, 1H), 4.25- 4.19 (m, 2H), 4.05-3.96 (m, 2H), 3.91 - 3.85 (m, 1H), 3.69 - 3.66 (m, 1H), 3.59 - 3.56 (m, 1H), 3.53 (s, 3H), 3.33 - 3.31 (m, 1H), 3.20 - 3.15 (m, 1H), 3.13 (s, 3H), 3.09 - 3.06 (m, 1H), 2.93 - 2.88 (m, 2H), 2.77 - 2.72 (m, 1H), 2.67 - 2.62 (m, 2H), 2.45 - 2.40 (m, 1H), 2.36 (s, 3H), 2.15 - 2.08 (m, 1H), 1.84 - 1.79 (m, 2H), 1.59 - 1.48 (m, 1H), 1.28 (d, *J =* 6.0 Hz, 3H), 1.20 - 1.17 (m, 2H), 1.13 (d, *J =* 7.0 Hz, 3H), 1.13 - 1.08 (m, 4H), 1.08 - 1.05 (m, 3H), 0.92 (s, 3H), 0.52 (s, 3H).

### Example 4

### (1r,2R,3S)-N-((6³S,4S,Z)-1²-(5-(3-((dimethylamino)methyl)phenyl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

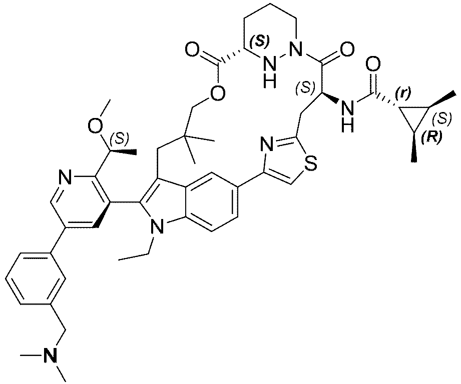

Example 4 was prepared by the following steps:

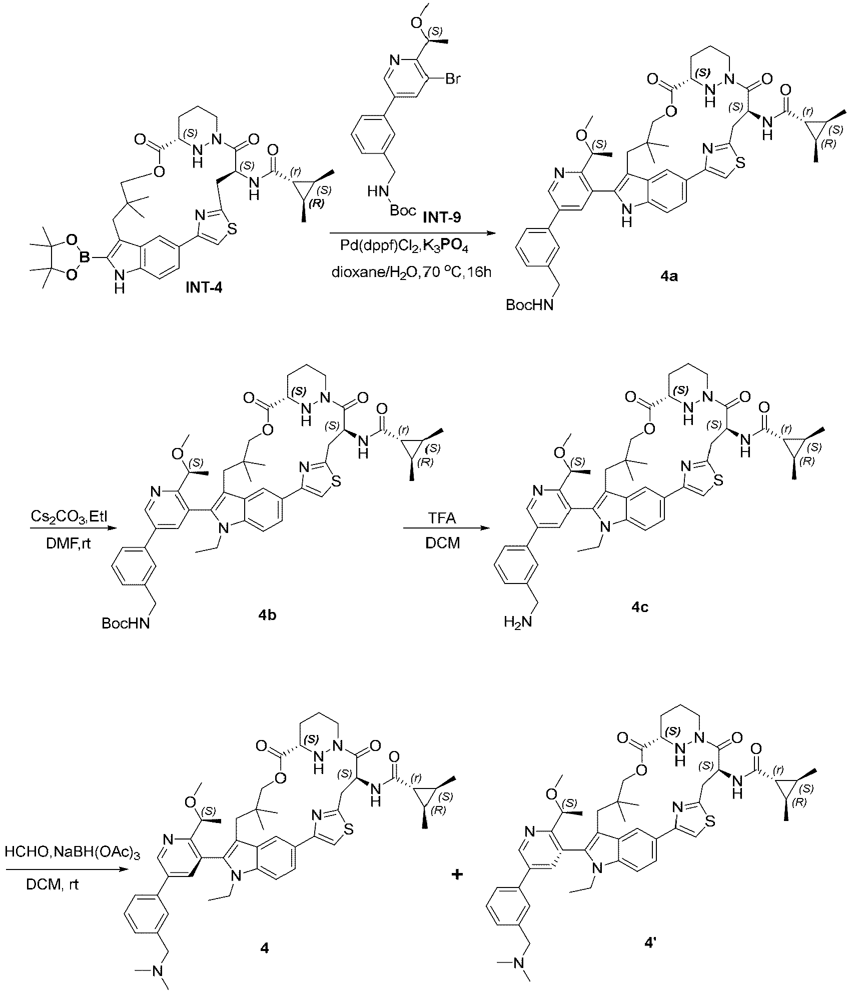

By replacing INT-7 in the synthesis step of compound 2 with INT-9 and replacing INT-3 with INT-4, compound 4 and its diastereoisomer compound 4' can be obtained using similar methods and reaction steps. The absolute configurations of the two compounds are drawn based on the empirical assumption that compound 4 is the isomer with a longer retention time in LCMS or HPLC, while 4' is the isomer with a shorter retention time in LCMS or HPLC.

### Compound 4

ESI-MS (m/z): 860.6 [M+H]⁺; LC-MS retention time RT = 2.01 min. HPLC retention time RT = 15.29 min.

¹H NMR (500 MHz, DMSO-*d*₆) δ 9.08 (d, *J =* 2.5 Hz, 1H), 8.52 (d, *J =* 1.5 Hz, 1H), 8.39 (d, *J* = 9.0 Hz, 1H), 8.06 (d, *J =* 2.5 Hz, 1H), 7.82 (s, 1H), 7.76 (dd, *J* = 8.5, 1.5 Hz, 1H), 7.72 - 7.70 (m, 2H), 7.60 (d, *J* = 8.5 Hz, 1H), 7.50 - 7.45 (m, 1H), 7.39 - 7.37 (m, 1H), 5.57 (t, *J* = 9.0 Hz, 1H), 5.09 - 5.02 (m, 1H), 4.37 - 4.32 (m, 2H), 4.25 - 4.13 (m, 3H), 3.62 - 3.57 (m, 2H), 3.47 (s, 2H), 3.28 (s, 3H), 3.17 - 3.13 (m, 1H), 3.00 - 2.95 (m, 1H), 2.78 - 2.72 (m, 1H), 2.47 - 2.45 (m, 1H), 2.17 (s, 6H), 2.11 - 2.03 (m, 1H), 1.82 - 1.74 (m, 2H), 1.57 - 1.48 (m, 1H), 1.42 (d, *J* = 6.0 Hz, 3H), 1.25 -1.14 (m, 4H), 1.09 - 1.06 (m, 6H), 0.93 (t, *J* = 7.0 Hz, 3H), 0.89 (s, 3H), 0.39 (s, 3H).

### Compound 4'

ESI-MS (m/z): 860.6 [M+H]⁺. LC-MS retention time RT = 1.95 min. HPLC retention time RT = 14.74 min.

¹H NMR (500 MHz, DMSO-*d*6) δ 9.08 (d, *J* = 2.5 Hz, 1H), 8.55 (d, *J* = 1.5 Hz, 1H), 8.40 (d, *J* = 9.0 Hz, 1H), 8.21 (d, *J* = 2.5 Hz, 1H), 7.82 (s, 1H), 7.75 (dd, *J* = 8.5, 1.5 Hz, 1H), 7.68 - 7.66 (m, 2H), 7.56 (d, *J* = 8.5 Hz, 1H), 7.50 - 7.46 (m, 1H), 7.39 - 7.37 (m, 1H), 5.54 (t, *J =* 9.0 Hz, 1H), 5.07 - 5.01 (m, 1H), 4.25 - 4.20 (m, 2H), 4.06 - 3.95 (m, 2H), 3.93 - 3.86 (m, 1H), 3.70 - 3.66 (m, 1H), 3.60 - 3.56 (m, 1H), 3.47 (s, 2H), 3.18 - 3.15 (m, 1H), 3.13 (s, 3H), 3.10 - 3.06 (m, 1H), 2.80 - 2.74 (m, 1H), 2.45 - 2.40 (m, 1H), 2.17 (s, 6H), 2.13 - 2.11 (m, 1H), 1.82 - 1.80 (m, 2H), 1.56 - 1.47 (m, 1H), 1.29 (d, *J* = 6.0 Hz, 3H), 1.24 - 1.20 (m, 1H), 1.18 - 1.16 (m, 2H), 1.15 - 1.04 (m, 10H), 0.93 (s, 3H), 0.54 (s, 3H).

### Example 5

### (1S,2S)-N-((6³S,4S,Z)-1¹-ethyl-1²-(6-((S)-1-methoxyethyl)-6'-(morpholinomethyl)-[3,3'-bipyridinl-5-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

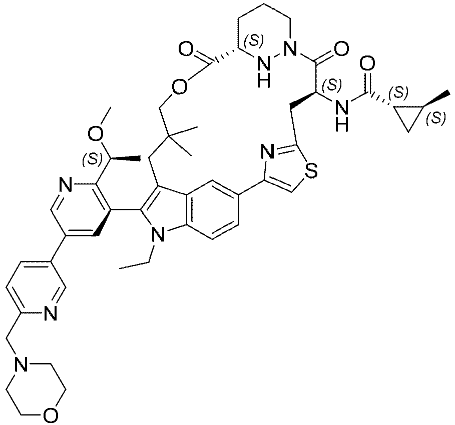

Example 5 was prepared by the following steps:

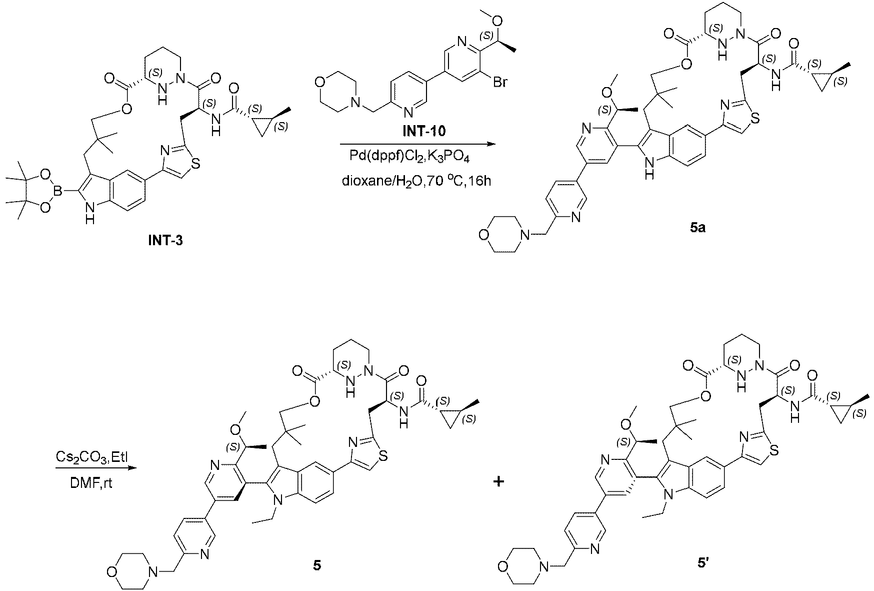

By replacing INT-6 in the synthesis step of compound 1 with INT-10 and INT-4 with INT-3, compound 5 and its diastereoisomer compound 5' can be obtained using similar methods and reaction steps. The absolute configurations drawn for the two compounds are assumptions made based on experience. Compound 5 is an isomer with a longer retention time in LCMS or HPLC, while 5' is an isomer with a shorter retention time in LCMS or HPLC.

### Compound 5

ESI-MS (m/z): 889.6 [M+H]⁺; LC-MS retention time RT = 1.70 min. HPLC retention time RT = 12.36 min.

¹H NMR (500 MHz, DMSO) δ 9.14 (d, *J =* 2.5 Hz, 1H), 8.97 (d, *J* = 2.5 Hz, 1H), 8.52 - 8.49 (m, 2H), 8.26 - 8.23 (m, 1H), 8.18 (d, *J =* 2.5 Hz, 1H), 7.81 (s, 1H), 7.77 - 7.74 (m, 1H), 7.59 (d, *J* = 8.5 Hz, 1H), 7.57 (d, *J* = 8.5 Hz, 1H), 5.58 - 5.55 (m, 1H), 5.10 - 5.06 (m, 1H), 4.37 - 4.31 (m, 3H), 4.26 - 4.21 (m, 2H), 4.19 - 4.13 (m, 3H), 3.65 (s, 2H), 3.62 - 3.58 (m, 3H), 3.28 (s, 3H), 3.00 - 2.95 (m, 1H), 2.65 - 2.62 (m, 1H), 2.38 -2.35 (m, 2H), 2.09 - 2.04 (m, 2H), 1.80 - 1.76 (m, 2H), 1.51 - 1.47 (m, 2H), 1.41 (d, *J* = 6.0 Hz, 3H), 1.23 (t, *J =* 7.0 Hz, 2H), 1.15 - 1.11 (m, 2H), 1.10 - 1.05 (m, 4H), 0.92 (t, *J =* 7.0 Hz, 3H), 0.89 (s, 3H), 0.56 - 0.53 (m, 2H), 0.37 (s, 3H).

### Compound 5'

ESI-MS (m/z): 889.6 [M+H]⁺. LC-MS retention time RT = 1.69 min. HPLC retention time RT = 12.24 min.

¹H NMR (500 MHz, DMSO) δ 9.15 (d, *J =* 2.0 Hz, 1H), 8.92 (d, *J =* 1.0 Hz, 1H), 8.55 (s, 1H), 8.53 *(d, J=* 9.0 Hz, 1H), 8.33 (d, *J =* 2.0 Hz, 1H), 8.21 - 8.17 (m, 1H), 7.81 (s, 1H), 7.76 - 7.72 (m, 1H), 7.61 - 7.54 (m, 2H), 5.55 (t, *J =* 9.0 Hz, 1H), 5.07 - 5.02 (m, 1H), 4.27 - 4.20 (m, 2H), 4.04 -3.97 (m, 2H), 3.92 - 3.86 (m, 1H), 3.71 - 3.65 (m, 1H), 3.65 (s, 2H), 3.63 - 3.58 (m, 3H), 3.59 - 3.55 (m, 2H), 3.31 - 3.27 (m, 2H), 3.13 (s, 3H), 3.09 - 3.02 (m, 1H), 2.81 - 2.73 (m, 2H), 2.47 - 2.41 (m, 4H), 2.14 - 2.10 (m, 1H), 1.84 - 1.77 (m, 2H), 1.53 - 1.48 (m, 2H), 1.28 (d, *J =* 6.0 Hz, 3H), 1.13 (t, *J* = 7.0 Hz, 3H), 1.10 - 1.04 (m, 4H), 0.92 (s, 3H), 0.90 - 0.84 (m, 2H), 0.53 (s, 3H).

### Example 6

### (1S,2S)-N-((6³S,4S,Z)-1¹-ethyl-1²-(5-(4-(((R)-3-fluoropyrrolidin-1-yl)methyl)phenyl)-2-((S)-1-methoxyethyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

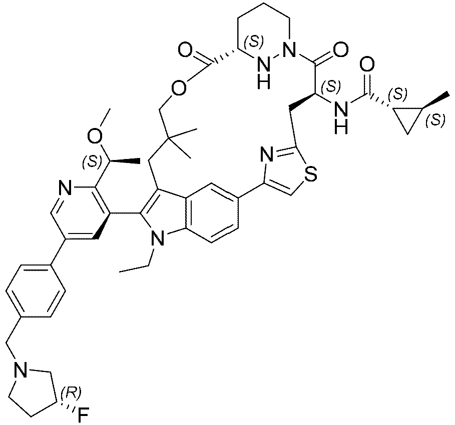

Example 6 was prepared by the following steps:

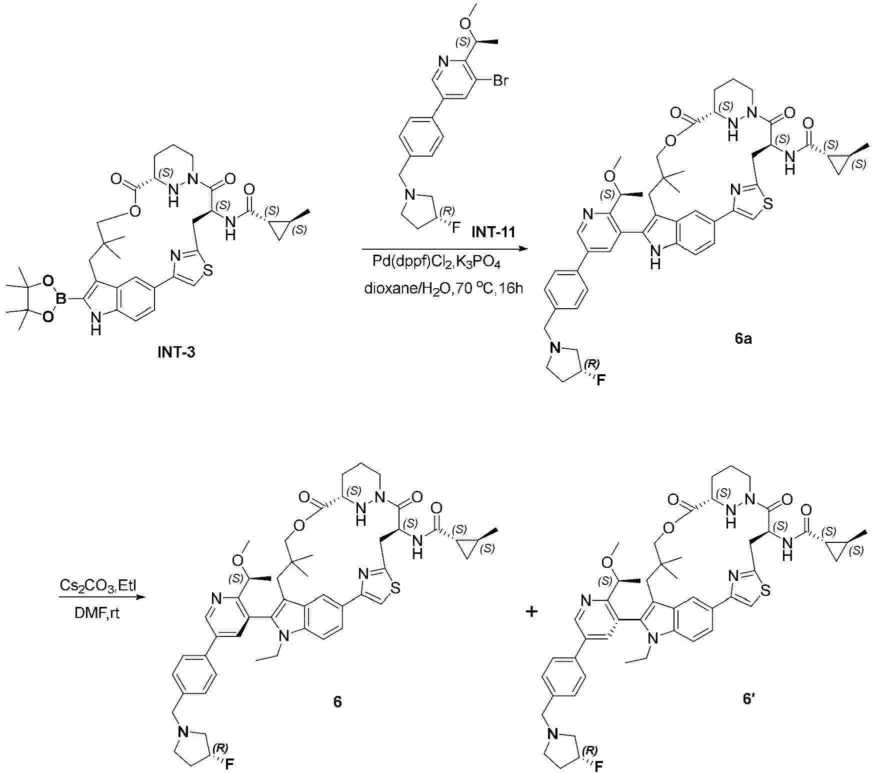

By replacing INT-6 in the synthesis step of compound 1 with INT-11 and INT-4 with INT-3, compound 6 and its diastereoisomer compound 6' can be obtained using similar methods and reaction steps. The absolute configurations drawn for the two compounds are assumptions made based on experience. Compound 6 is an isomer with a longer retention time in LCMS or HPLC, while 6' is an isomer with a shorter retention time in LCMS or HPLC.

### Compound 6

ESI-MS (m/z): 890.6 [M+H]⁺; LC-MS retention time RT = 1.99 min. HPLC retention time RT = 14.98 min.

¹H NMR (500 MHz, DMSO-*d*₆) δ 9.02 (d, *J* = 2.5 Hz, 1H), 8.49 - 8.42 (m, 2H), 7.99 (d, *J* = 2.5 Hz, 1H), 7.74 (s, 1H), 7.72 (s, 1H), 7.69 (dd, *J* = 8.5, 1.5 Hz, 1H), 7.52 (d, *J* = 8.5 Hz, 1H), 7.39 - 7.33 (m, 2H), 5.50 (t, *J* = 9.0 Hz, 1H), 5.20 - 5.05 (m, 1H), 5.03 - 4.98 (m, 1H), 4.32 - 4.24 (m, 2H), 4.20 - 4.05 (m, 3H), 3.59 (s, 2H), 3.52 (s, 2H), 3.27 - 3.22 (m, 1H), 3.21 (s, 3H), 3.11 - 3.06 (m, 1H), 2.94 - 2.90 (m, 1H), 2.77 - 2.68 (m, 3H), 2.56 - 2.51 (m, 1H), 2.43 - 2.3 8 (m, 1H), 2.29 - 2.22 (m, 1H), 2.12 - 2.08 (m, 1H), 2.05 - 2.01 (m, 1H), 1.79 - 1.75 (m, 1H), 1.74 - 1.70 (m, 2H), 1.45 - 1.42 (m, 2H), 1.34 (d, *J =* 6.0 Hz, 3H), 1.22 - 1.13 (m, 1H), 1.05 - 0.98 (m, 4H), 0.86 - 0.80 (m, 7H), 0.50 - 0.42 (m, 1H), 0.31 (s, 3H).

### Compound 6'

ESI-MS (m/z): 890.6 [M+H]⁺. LC-MS retention time RT = 1.95 min. HPLC retention time RT = 14.63 min.

¹H NMR (500 MHz, DMSO-*d*₆) δ 9.03 (d, *J* = 2.5 Hz, 1H), 8.50 - 8.44 (m, 2H), 8.14 (d, *J* = 2.5 Hz, 1H), 7.74 (s, 1H), 7.72 - 7.66 (m, 3H), 7.49 (d, *J* = 8.5 Hz, 1H), 7.41 - 7.37 (m, 2H), 5.48 (t, *J =* 9.0 Hz, 1H), 5.22 - 5.05 (m, 1H), 5.00 - 4.95 (m, 1H), 4.20 - 4.15 (m, 2H), 3.95 - 3.90 (m, 2H), 3.83 - 3.79 (m, 1H), 3.64 - 3.60 (m, 3H), 3.54 - 3.49 (m, 1H), 3.29 - 3.25 (m,1H), 3.12 - 3.07 (m, 1H), 3.06 (s, 3H), 3.05 - 2.98 (m, 1H), 2.79 - 2.67 (m, 3H), 2.59 - 2.50 (m, 1H), 2.38 - 2.32 (m, 1H), 2.29 - 2.22 (m, 1H), 2.13 - 2.08 (m, 1H), 2.07 - 2.02 (m, 1H), 1.87 - 1.79 (m, 1H), 1.76 - 1.72 (m, 2H), 1.50 - 1.40 (m, 2H), 1.21 (d, *J =* 6.0 Hz, 3H), 1.06 (t, *J =* 7.0 Hz, 3H), 1.04 - 0.98 (m, 4H), 0.86 (s, 3H), 0.83 - 0.75 (m, 1H), 0.50 - 0.45 (m, 4H).

### Example 7

### (1S,2S)-N-((6³S,4S,Z)-1²-(5-(4-((4,4-difluoropiperidin-1-yl)methyl)phenyl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

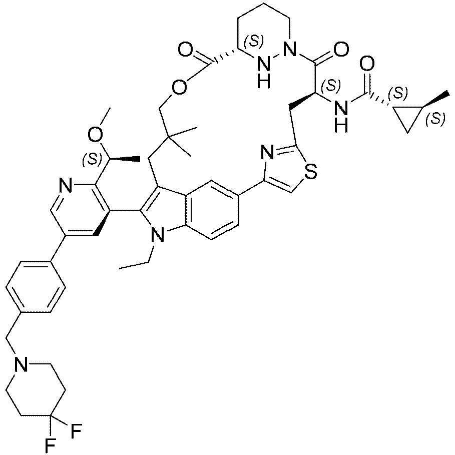

Example 7 was prepared by the following steps:

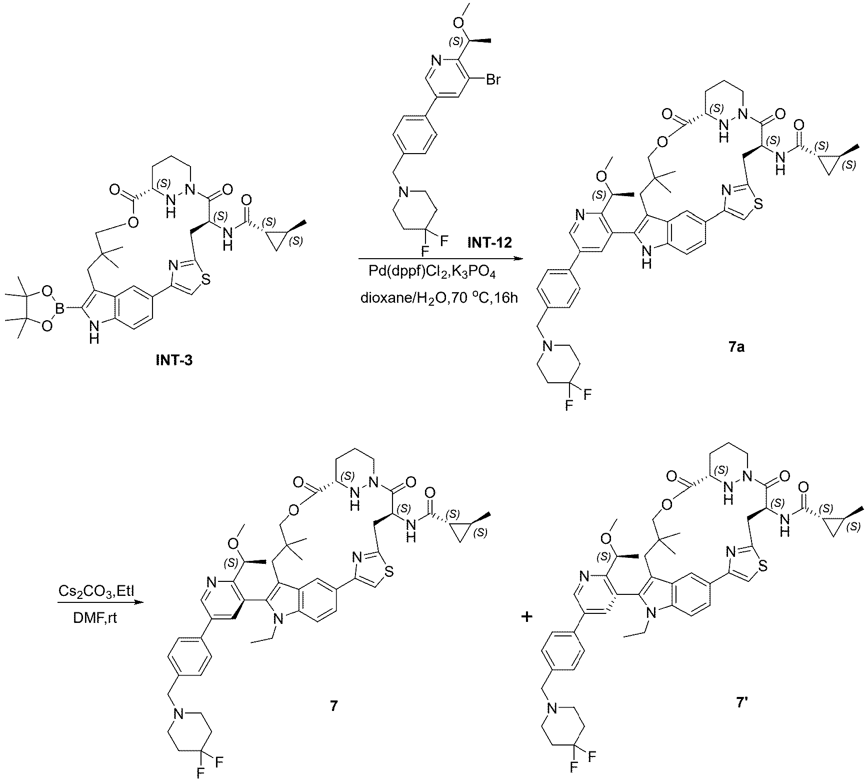

By replacing INT-6 in the synthesis step of compound 1 with INT-12 and INT-4 with INT-3, compound 7 and its diastereoisomer compound 7' can be obtained using similar methods and reaction steps. The absolute configurations drawn for the two compounds are assumptions made based on experience. Compound 7 is an isomer with a longer retention time in LCMS or HPLC, while 7' is an isomer with a shorter retention time in LCMS or HPLC.

### Compound 7

ESI-MS (m/z): 922.6 [M+H]⁺; LC-MS retention time RT = 2.10 min. HPLC retention time RT = 15.98 min.

¹H NMR (500 MHz, DMSO-*d*₆) δ 9.02 (d, *J* = 2.5 Hz, 1H), 8.50 - 8.40 (m, 2H), 7.99 (d, *J* = 2.5 Hz, 1H), 7.76 - 7.72 (m, 3H), 7.69 (dd, *J =* 8.5, 2.0 Hz, 1H), 7.52 (d, *J* = 8.5 Hz, 1H), 7.42 - 7.37 (m, 2H), 5.50 (t, *J* = 9.0 Hz, 1H), 5.03 - 4.98 (m, 1H), 4.29 - 4.25 (m, 2H), 4.20 - 4.06 (m, 4H), 3.56 - 3.50 (m, 5H), 8.56 - 8.47 (m, 2H), 3.26 - 3.21 (m, 1H), 3.21 (s, 3H), 3.11 - 3.05 (m, 1H), 2.93 - 2.88 (m, 1H), 2.71 - 2.65 (m, 1H), 2.43 - 2.38 (m, 1H), 2.03 - 1.99 (m, 1H), 1.95 -1.83 (m, 5H), 1.76 -1.71 (m, 2H), 1.50 - 1.41 (m, 3H), 1.34 (d, *J =* 6.0 Hz, 3H), 1.20 - 1.15 (m, 1H), 1.04 - 0.96 (m, 5H), 0.88 - 0.76 (m, 7H), 0.50 - 0.47 (m, 1H), 0.31 (s, 3H).

### Compound 7'

ESI-MS (m/z): 922.6 [M+H]⁺. LC-MS retention time RT = 2.06 min. HPLC retention time RT = 15.63 min.

¹H NMR (500 MHz, DMSO-*d*₆) δ 9.03 (d, *J* = 2.5 Hz, 1H), 8.50 - 8.44 (m, 2H), 8.14 (d, *J =* 2.5 Hz, 1H), 7.74 (s, 1H), 7.70 (d, *J =* 2.0 Hz, 1H), 7.69 - 7.66 (m, 2H), 7.48 (d, *J =* 8.5 Hz, 1H), 7.42 - 7.37 (m, 2H), 5.49 (t, *J =* 9.0 Hz, 1H), 5.00 - 4.95 (m, 1H), 4.20 - 4.15 (m, 2H), 3.95 - 3.90 (m, 2H), 3.84 - 3.78 (m, 1H), 3.64 - 3.60 (m, 1H), 3.56 - 3.50 (m, 3H), 3.26 - 3.22 (m, 1H), 3.12 - 3.07 (m, 1H), 3.06 (s, 3H), 3.03 - 2.97 (m, 1H), 2.72 - 2.67 (m, 1H), 2.47 - 2.42 (m, 2H), 2.38 - 2.33 (m, 1H), 2.08 - 2.03 (m, 1H), 1.95 - 1.89 (m, 5H), 1.76 - 1.71 (m, 2H), 1.49 - 1.41 (m, 3H), 1.21 (d, *J =* 6.0 Hz, 3H), 1.06 (t, *J* = 7.0 Hz, 3H), 1.02 - 0.97 (m, 4H), 0.86 (s, 3H), 0.84 - 0.80 (m, 1H), 0.51 - 0.48 (m, 1H), 0.47 (s, 3H).

### Example 8

### (1S,2S)-N-((6³S,4S,Z)-1¹-ethyl-1²-(2-((S)-1-methoxyethyl)-5-(4-((4-methylpiperazin-1-yl)methyl)phenyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

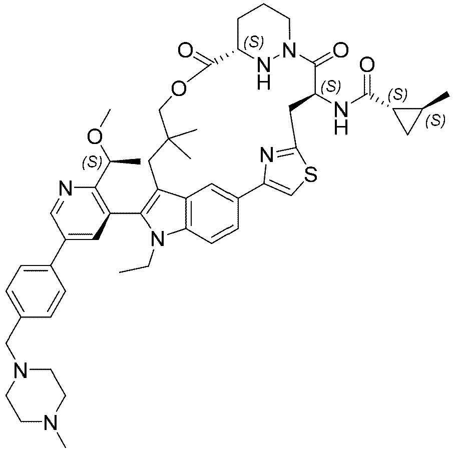

Example 8 was prepared by the following steps:

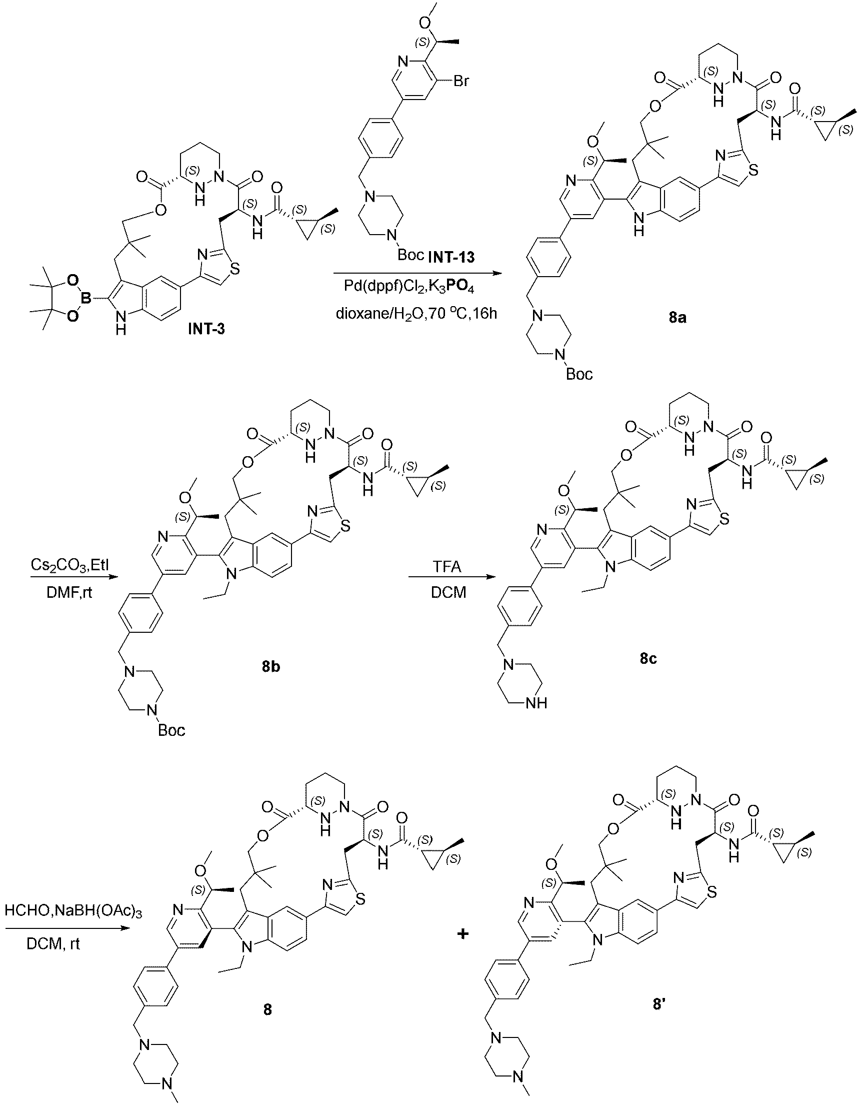

By replacing INT-7 in the synthesis step of compound 2 with INT-13, compound 8 and its diastereoisomer compound 8' can be obtained using similar methods and reaction steps. The absolute configurations of the two compounds are drawn based on the empirical assumption that compound 8 is the isomer with a longer retention time in LCMS or HPLC, while 8' is the isomer with a shorter retention time in LCMS or HPLC.

### Compound 8

ESI-MS (m/z): 901.7 [M+H]⁺; LC-MS retention time RT = 1.84 min. HPLC retention time RT = 13.67 min.

¹H NMR (500 MHz, DMSO-*d*₆) δ 9.08 (d, *J* = 2.5 Hz, 1H), 8.56 - 8.47 (m, 2H), 8.05 (d, *J* = 2.5 Hz, 1H), 7.84 - 7.73 (m, 4H), 7.59 (d, *J* = 8.5 Hz, 1H), 7.46 - 7.41 (m, 2H), 5.57 (t, *J =* 9.0 Hz, 1H), 5.10 - 5.05 (m, 1H), 4.40 - 4.28 (m, 2H), 4.28 - 4.12 (m, 3H), 3.63 - 3.56 (m, 2H), 3.50 (s, 2H), 3.28 (s, 3H), 3.16 - 3.12 (m, 1H), 3.00 - 2.94 (m, 1H), 2.80 - 2.72 (m, 1H), 2.50 - 2.44 (m, 1H), 2.43 - 2.22 (m, 7H), 2.15 (s, 3H), 2.10 - 2.04 (m, 1H), 1.82 - 1.74 (m, 2H), 1.53 - 1.47 (m, 2H), 1.41 (d, *J =* 6.0 Hz, 3H), 1.25 - 1.22 (m, 2H), 1.09 - 1.03 (m, 4H), 0.93 - 0.85 (m, 7H), 0.57 - 0.52 (m, 1H), 0.37 (s, 3H).

### Compound 8'

ESI-MS (m/z): 901.7 [M+H]⁺. LC-MS retention time RT = 1.79 min. HPLC retention time RT = 13.10 min.

¹H NMR (500 MHz, DMSO-*d*₆) δ 9.09 (d, *J* = 2.5 Hz, 1H), 8.56 - 8.47 (m, 2H), 8.20 (d, *J* = 2.5 Hz, 1H), 7.81 (s, 1H), 7.80 - 7.73 (m, 3H), 7.55 (d, *J* = 8.5 Hz, 1H), 7.46 - 7.41 (m, 2H), 5.56 (t, *J* = 9.0 Hz, 1H), 5.07 - 5.02 (m, 1H), 4.26 - 4.22 (m, 2H), 4.07 - 3.95 (m, 2H), 3.93 - 3.83 (m, 1H), 3.70 - 3.65 (m, 1H), 3.60 - 3.55 (m, 1H), 3.51 (s, 2H), 3.20 - 3.11 (m, 4H), 3.10 - 3.06 (m, 1H), 2.81 - 2.73 (m, 1H), 2.47 - 2.25 (m, 8H), 2.18 - 2.08 (m, 4H), 1.86 - 1.76 (m, 2H), 1.55 - 1.48 (m, 2H), 1.28 (d, *J=* 6.0 Hz, 3H), 1.25 -1.21 (m, 2H), 1.13 (t, *J* = 7.0 Hz, 3H), 1.07 (s, 3H), 0.93 (s, 3H), 0.90 - 0.86 (m, 1H), 0.60 - 0.51 (m, 4H).

### Example 9

### (1S,2S)-N-((6³S,4S,Z)-1¹-ethyl-1²-(2-((S)-1-methoxyethyl)-5-(3-(morpholinomethyl)phenyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

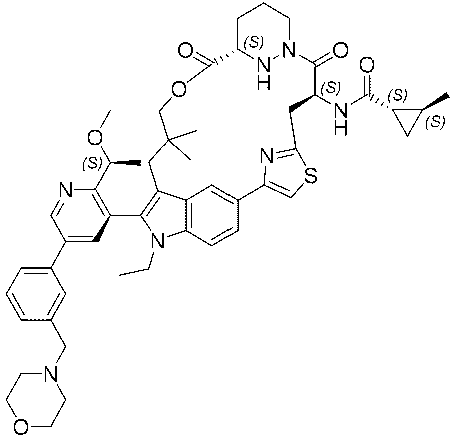

Example 9 was prepared by the following steps:

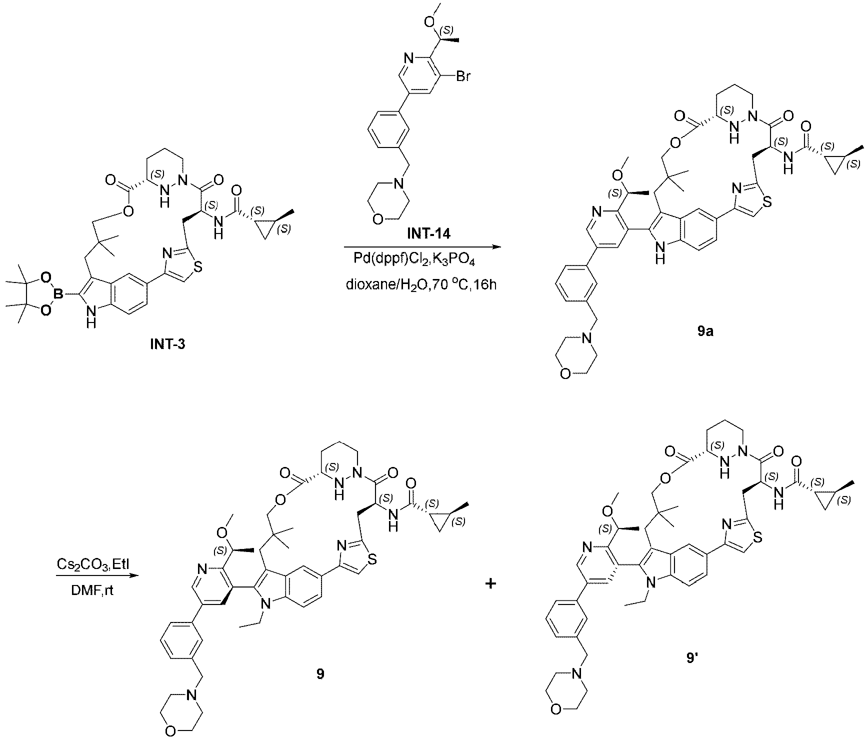

By replacing INT-6 in the synthesis step of compound 1 with INT-14 and INT-4 with INT-3, compound 9 and its diastereoisomer compound 9' can be obtained using similar methods and reaction steps. The absolute configurations drawn for the two compounds are assumptions made based on experience. Compound 9 is an isomer with a longer retention time in LCMS or HPLC, while 9' is an isomer with a shorter retention time in LCMS or HPLC.

### Compound 9

ESI-MS (m/z): 888.6 [M+H]⁺; LC-MS retention time RT = 1.94 min. HPLC retention time RT = 14.52 min.

¹H NMR (500 MHz, DMSO-*d*₆) δ 9.07 (d, *J* = 2.5 Hz, 1H), 8.52 - 8.50 (m, 2H), 8.06 (d, *J* = 2.5 Hz, 1H), 7.81 (s, 1H), 7.77 - 7.71 (m, 3H), 7.60 (d, *J =* 9.0 Hz, 1H), 7.48 (t*, J =* 8.0 Hz, 1H), 7.40 (d, *J =* 8.0 Hz, 1H), 5.57 (t, *J* = 9.0 Hz, 1H), 5.10 - 5.05 (m, 1H), 4.39 - 4.32 (m, 2H), 4.25 - 4.14 (m, 3H), 3.60 - 3.54 (m, 8H), 3.28 (s, 3H), 3.17 - 3.12 (m, 1H), 3.00 - 2.97 (m, 1H), 2.81 - 2.73 (m, 1H), 2.48 - 2.45 (m, 1H), 2.43 - 2.38 (m, 4H), 2.08 - 2.06 (m, 1H), 1.78 - 1.75 (m, 2H), 1.53 - 1.48 (m, 2H), 1.41 (d, *J =* 6.0 Hz, 3H), 1.27 - 1.22 (m, 2H), 1.07 (s, 3H), 0.93 (t*, J =* 7.0 Hz, 3H), 0.89 (s, 3H), 0.87 - 0.84 (m, 1H), 0.56 - 0.54 (m, 1H), 0.38 (s, 3H).

### Compound 9'

ESI-MS (m/z): 888.6 [M+H]⁺. LC-MS retention time RT = 1.90 min. HPLC retention time RT = 14.10 min.

¹H NMR (500 MHz, DMSO-*d*₆) δ 9.08 (d, *J =* 2.5 Hz, 1H), 8.55 (d, *J =* 1.5 Hz, 1H), 8.53 (d, *J =* 9.0 Hz, 1H), 8.20 (d, *J* = 2.5 Hz, 1H), 7.81 (s, 1H), 7.75 (dd, *J* = 9.0, 2.0 Hz, 1H), 7.71 - 7.70 (m, 1H), 7.69 - 7.67 (m, 1H), 7.56 (d, *J* = 8.5 Hz, 1H), 7.48 (t, *J* = 8.0 Hz, 1H), 7.41 - 7.39 (m, 1H), 5.56 (t, *J=* 9.0 Hz, 1H), 5.07 - 5.03 (m, 1H), 4.26 - 4.20 (m, 2H), 4.04 - 3.96 (m, 2H), 3.92 - 3.85 (m, 1H), 3.70 - 3.68 (m, 1H), 3.60 - 3.55 (m, 8H), 3.19 - 3.14 (m, 1H), 3.13 (s, 3H), 3.09 - 3.06 (m, 1H), 2.81 - 2.72 (m, 1H), 2.44 - 2.36 (m, 6H), 2.14 - 2.12 (m, 1H), 1.80 - 1.77 (s, 2H), 1.59 - 1.40 (m, 3H), 1.29 (d, *J* = 6.0 Hz, 3H), 1.27 - 1.22 (m, 1H), 1.13 (t, *J =* 7.0 Hz, 3H), 1.08-1.06 (m, 4H), 0.93 (s, 3H), 0.55 (s, 3H).

### Example 10

### (1S,2S)-N-((6³S,4S,Z)-1¹-ethyl-1²-(2-((S)-1-methoxyethyl)-5-(2-methyl-1,2,3,4-tetrahydroisoquinolin-7-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

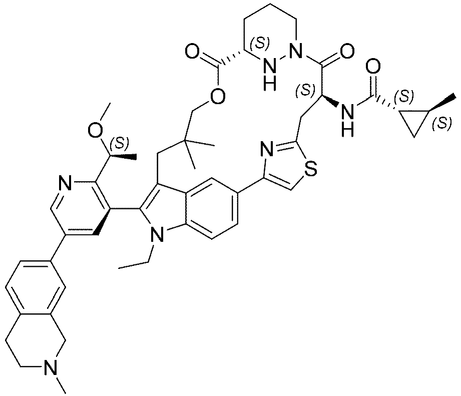

Example 10 was prepared by the following steps:

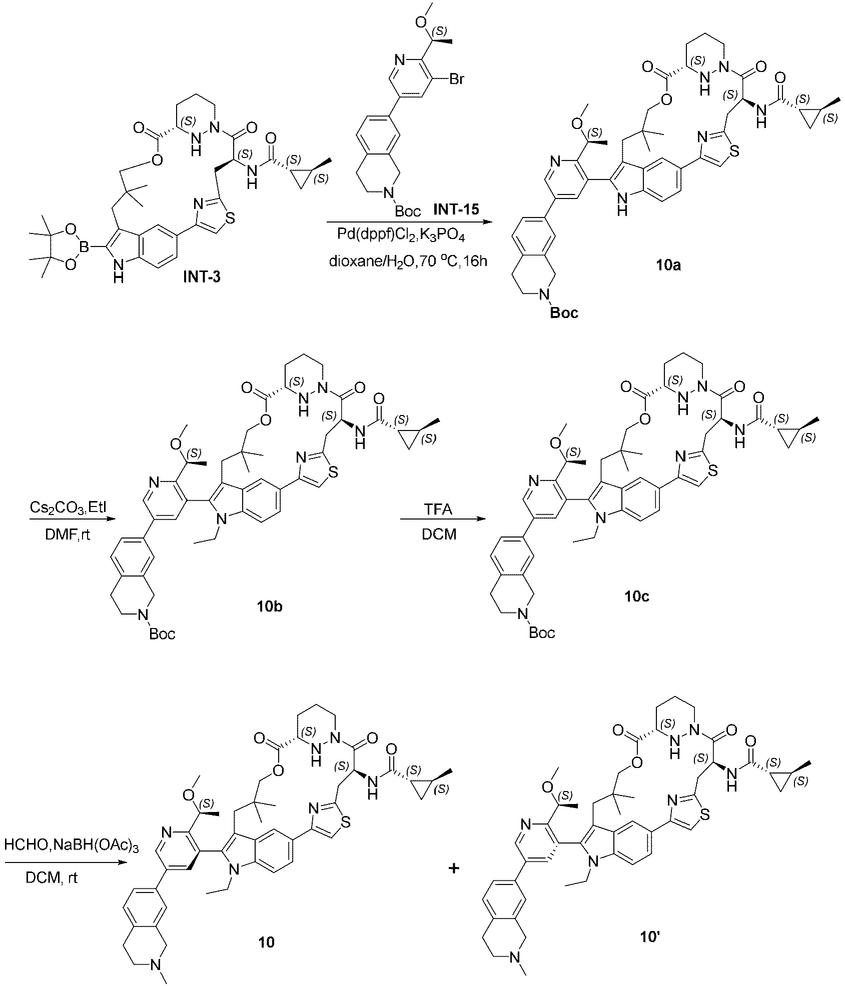

By replacing INT-7 in the synthesis step of compound 2 with INT-15, compound 10 and its diastereoisomer compound 10' can be obtained using similar methods and reaction steps. The absolute configurations of the two compounds are drawn based on the empirical assumption that compound 10 is the isomer with a longer retention time in LCMS or HPLC, while 10' is the isomer with a shorter retention time in LCMS or HPLC.

### Compound 10

ESI-MS (m/z): 858.6 [M+H]⁺; LC-MS retention time RT = 1.94 min. HPLC retention time RT = 14.69 min.

¹H NMR (500 MHz, DMSO-*d*₆) δ 9.05 (d, *J* = 2.5 Hz, 1H), 8.55 - 8.47 (m, 2H), 8.01 (d, *J =* 2.5 Hz, 1H), 7.81 (s, 1H), 7.78 - 7.72 (m, 1H), 7.63 - 7.55 (m, 2H), 7.53 (d, *J* = 2.0 Hz, 1H), 7.24 (d, *J =* 8.0 Hz, 1H), 5.64 - 5.51 (m, 1H), 5.14 - 5.02 (m, 1H), 4.42 - 4.29 (m, 2H), 4.29 - 4.10 (m, 3H), 3.60 - 3.54 (m, 4H), 3.28 (s, 3H), 3.17 - 3.11 (m, 2H), 2.99 - 2.95 (m, 1H), 2.88 - 2.84 (m, 2H), 2.64 - 2.60 (m, 2H), 2.47 - 2.42 (m, 1H), 2.35 (s, 3H), 2.11 - 2.04 (m, 1H), 1.91 - 1.85 (m, 1H), 1.83 - 1.73 (m, 2H), 1.51 - 1.48 (m, 1H), 1.44 -1.39 (m, 3H), 1.32 -1.22 (m, 3H), 1.10 - 1.04 (m, 4H), 0.90 - 0.86 (m, 4H), 0.58 - 0.53 (m, 1H), 0.37 (s, 3H).

### Compound 10'

ESI-MS (m/z): 858.6 [M+H]⁺. LC-MS retention time RT = 1.88 min. HPLC retention time RT = 14.08 min.

¹H NMR (500 MHz, DMSO-*d*₆) δ 9.05 (d, *J =* 2.5 Hz, 1H), 8.58 - 8.48 (m, 2H), 8.16 (d, *J* = 2.5 Hz, 1H), 7.81 (s, 1H), 7.74 (dd, *J* = 8.5, 1.5 Hz, 1H), 7.60 - 7.51 (m, 2H), 7.48 (d, *J* = 1.5 Hz, 1H), 7.24 (d, *J* = 8.5 Hz, 1H), 5.63 - 5.50 (m, 1H), 5.11 - 4.96 (m, 1H), 4.32 - 4.18 (m, 2H), 4.07 - 3.94 (m, 2H), 3.95 - 3.82 (m, 1H), 3.72 - 3.62 (m, 1H), 3.60 - 3.50 (m, 3H), 3.19 - 3.14 (m, 1H), 3.13 (s, 3H), 3.09 - 3.03 (m, 1H), 2.89 - 2.83 (m, 2H), 2.81 - 2.73 (m, 1H), 2.64 - 2.60 (m, 2H), 2.46 - 2.39 (m, 1H), 2.35 (s, 3H), 2.18 - 2.07 (m, 1H), 1.87 - 1.73 (m, 2H), 1.58 - 1.46 (m, 2H), 1.28 (d, *J =* 6.0 Hz, 3H), 1.13 (t, *J =* 7.0 Hz, 3H), 1.09 - 1.03 (m, 4H), 0.92 (s, 3H), 0.90 - 0.86 (m, 1H), 0.58 - 0.54 (m, 1H), 0.51 (s, 3H).

### Example 11

### (1S,2S)-N-((6³S,4S,Z)-1¹-ethyl-1²-(6'-((S)-1-methoxyethyl)-4-(morpholinomethyl)-[2,3'-bipyridin]-5'-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

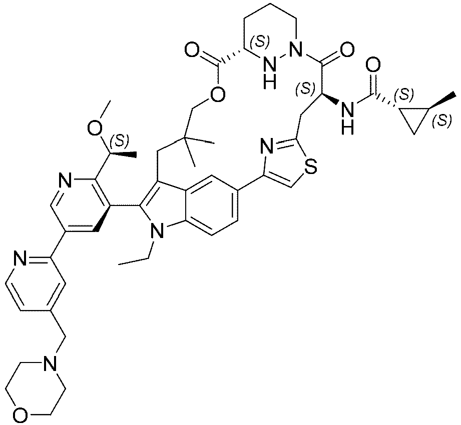

Example 11 was prepared by the following steps:

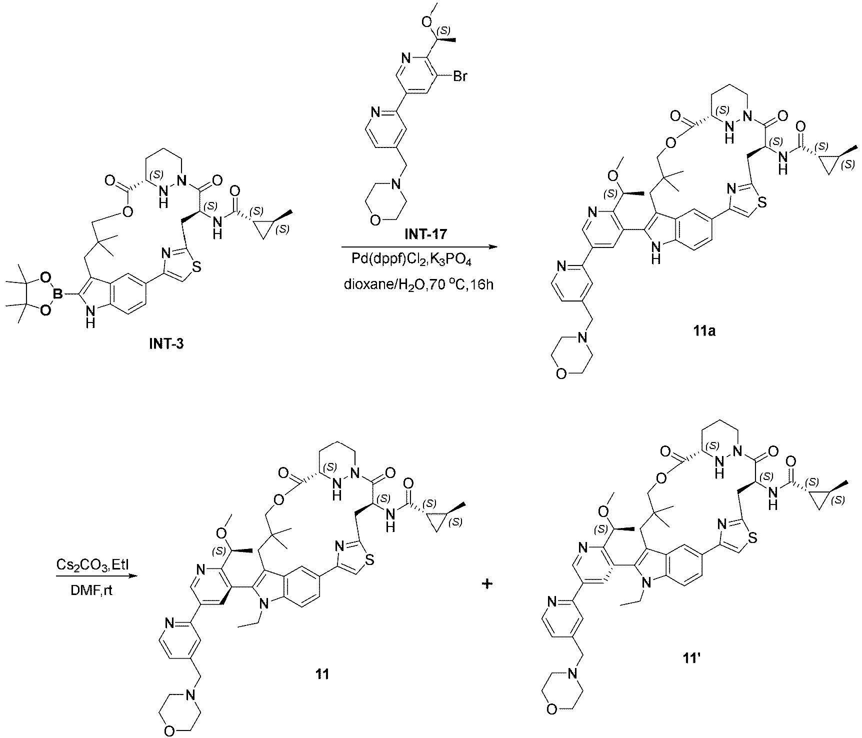

By replacing INT-6 in the synthesis step of compound 1 with INT-17 and INT-4 with INT-3, compound 11 and its diastereoisomer compound 11' can be obtained using similar methods and reaction steps. The absolute configurations drawn for the two compounds are assumptions made based on experience. Compound 11 is an isomer with a longer retention time in LCMS or HPLC, while 11' is an isomer with a shorter retention time in LCMS or HPLC.

### Compound 11

ESI-MS (m/z): 889.9 [M+H]⁺; LC-MS retention time RT = 1.81 min. HPLC retention time RT = 13.17 min.

¹H NMR (500 MHz, DMSO-*d*₆) δ 9.42 (d, *J* = 2.0 Hz, 1H), 8.68 - 8.64 (m, 1H), 8.56-8.49 (m, 2H), 8.40 (d, *J =* 2.0 Hz, 1H), 8.08 (s, 1H), 7.81 (s, 1H), 7.76 (dd, *J=* 8.5, 1.5 Hz, 1H), 7.60 (d, *J =* 8.5 Hz, 1H), 7.45 - 7.39 (m, 1H), 5.63 - 5.51 (m, 1H), 5.14 - 5.00 (m, 1H), 4.43 - 4.29 (m, 2H), 4.28 - 4.12 (m, 3H), 3.67 - 3.58 (m, 8H), 3.29 (s, 3H), 3.17 - 3.12 (m, 1H), 3.03 -2.97 (m, 1H), 2.78 -2.71 (m, 1H), 2.45 - 2.38 (m, 5H), 2.10 - 2.03 (m, 1H), 1.83 - 1.75 (m, 2H), 1.54 - 1.47 (m, 2H), 1.45 -1.39 (m, 3H), 1.29 - 1.22 (m, 1H), 1.07 (s, 3H), 0.96 - 0.85 (m, 7H), 0.58 - 0.52 (m, 1H), 0.38 (s, 3H).

### Compound 11'

ESI-MS (m/z): 889.9 [M+H]⁺. LC-MS retention time RT = 1.78 min. HPLC retention time RT = 12.89 min.

¹H NMR (500 MHz, DMSO-*d*₆) δ 9.44 (d, *J* = 2.0 Hz, 1H), 8.68 - 8.64 (m, 1H), 8.58-8.49 (m, 3H), 8.08 (s, 1H), 7.82 (s, 1H), 7.75 (dd, *J* = 8.5, 1.5 Hz, 1H), 7.56 (d, *J* = 8.5 Hz, 1H), 7.44 - 7.39 (m, 1H), 5.62 - 5.49 (m, 1H), 5.10 - 5.01 (m, 1H), 4.30 - 4.17 (m, 2H), 4.09 - 3.96 (m, 2H), 3.95 - 3.83 (m, 1H), 3.73 - 3.65 (m, 1H), 3.65 - 3.56 (m, 7H), 3.20 - 3.06 (m, 5H), 2.81 - 2.74 (m, 1H), 2.46 - 2.38 (m, 5H), 2.16 - 2.09 (m, 1H), 1.85 - 1.75 (m, 2H), 1.55 - 1.46 (m, 2H), 1.28 (d, *J =* 6.0 Hz, 3H), 1.13 (t, *J* = 7.0 Hz, 3H), 1.07 (s, 3H), 0.96 - 0.91 (m, 4H), 0.90 - 0.86 (m, 1H), 0.58 - 0.54 (m, 1H), 0.52 (s, 3H).

### Example 12

### (1S,2S)-N-((6³S,4S,Z)-1¹-ethyl-1²-(6'-((S)-1-methoxyethyl)-5-(morpholinomethyl)-[2,3'-bipyridin]-5'-yl)-10,10-dimethyl-5,7-dioxo-6¹,6²,6³ 6⁴ 6⁵ 6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

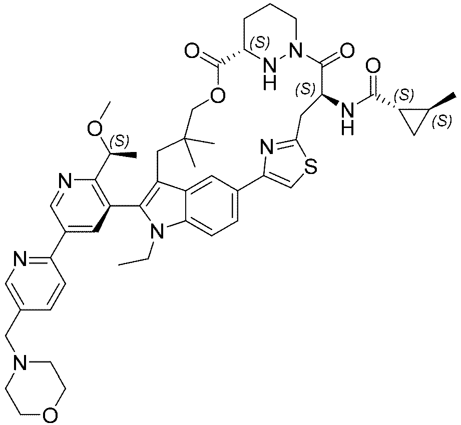

Example 12 was prepared by the following steps:

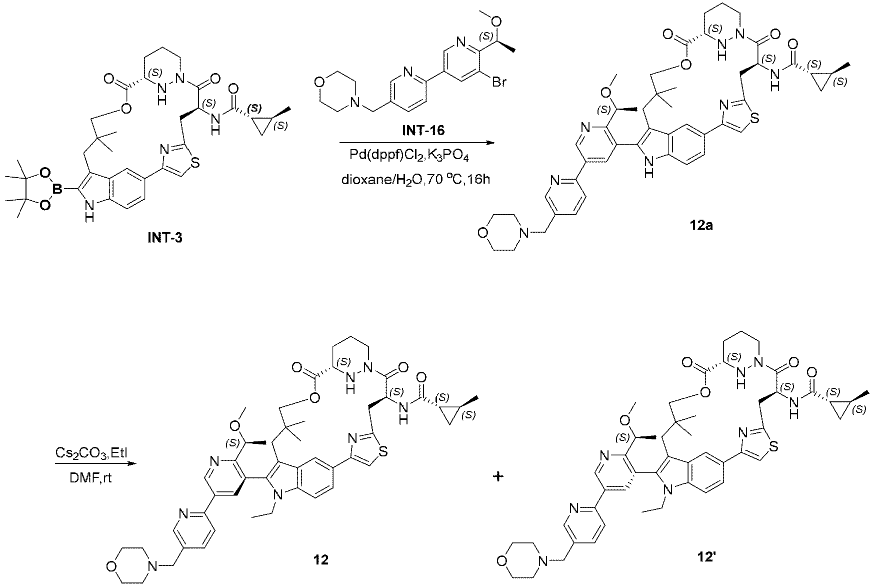

By replacing INT-6 in the synthesis step of compound 1 with INT-16 and INT-4 with INT-3, compound 12 and its diastereoisomer compound 12' can be obtained using similar methods and reaction steps. The absolute configurations drawn for the two compounds are assumptions made based on experience. Compound 12 is an isomer with a longer retention time in LCMS or HPLC, while 12' is an isomer with a shorter retention time in LCMS or HPLC.

### Compound 12

ESI-MS (m/z): 889.9 [M+H]⁺; LC-MS retention time RT = 1.78 min. HPLC retention time RT = 12.89 min.

¹H NMR (500 MHz, DMSO-*d*₆) δ 9.44 (d, *J* = 2.0 Hz, 1H), 8.64 (d, *J* = 2.0 Hz, 1H), 8.56 - 8.48 (m, 2H), 8.41 (d, *J* = 2.0 Hz, 1H), 8.16 (d, *J* = 8.0 Hz, 1H), 7.86 (dd, *J* = 8.0, 2.0 Hz, 1H), 7.82 (s, 1H), 7.76 (dd, *J* = 8.5, 2.0 Hz, 1H), 7.60 (d, *J =* 8.5 Hz, 1H), 5.64 - 5.50 (m, 1H), 5.12 - 5.04 (m, 1H), 4.42 - 4.31 (m, 2H), 4.29 - 4.10 (m, 3H), 3.61 - 3.54 (m, 8H), 3.29 (s, 3H), 3.18 - 3.13 (m, 1H), 3.02 - 2.97 (m, 1H), 2.79 - 2.72 (m, 1H), 2.45 -2.36 (m, 5H), 2.13 -2.01 (m, 1H), 1.83 - 1.74 (m, 2H), 1.55 -1.47 (m, 2H), 1.41 (d, *J* = 6.0 Hz, 3H), 1.10 - 1.05 (m, 4H), 0.91 - 0.83 (m, 7H), 0.58 - 0.50 (m, 1H), 0.37 (s, 3H).

### Compound 12'

ESI-MS (m/z): 889.9 [M+H]⁺. LC-MS retention time RT = 1.76 min. HPLC retention time RT = 12.66 min.

¹H NMR (500 MHz, DMSO-*d*₆) δ 9.46 (d, *J =* 2.5 Hz, 1H), 8.65 (d, *J=* 2.0 Hz, 1H), 8.58-8.50 (m, 3H), 8.11 (d, *J* = 8.0 Hz, 1H), 7.86 (dd, *J* = 8.0, 2.5 Hz, 1H), 7.82 (s, 1H), 7.75 (dd, *J* = 8.5, 2.0 Hz, 1H), 7.56 (d, *J* = 8.5 Hz, 1H), 5.63 - 5.50 (m, 1H), 5.13 - 5.01 (m, 1H), 4.31 - 4.18 (m, 2H), 4.08 - 3.96 (m, 2H), 3.93 - 3.79 (m, 1H), 3.72 - 3.64 (m, 1H), 3.63 - 3.52 (m, 7H), 3.20 - 3.15 (m, 1H), 3.13 (s, 3H), 3.10 - 3.04 (m, 1H), 2.81 - 2.73 (m, 1H), 2.44 - 2.36 (m, 5H), 2.17 - 2.09 (m, 1H), 1.84 - 1.75 (m, 2H), 1.56 - 1.47 (m, 2H), 1.28 (d, *J =* 6.0 Hz, 3H), 1.13 (t, *J =* 7.0 Hz, 3H), 1.10 - 1.04 (m, 4H), 0.92 (s, 3H), 0.90 - 0.86 (m, 1H), 0.58 - 0.54 (m, 1H), 0.51 (s, 3H).

### Example 13

### (1S,2S)-N-((6³S,4S,Z)-1¹-ethyl-1²-(6'-((S)-1-methoxyethyl)-6-(morpholinomethyl)-[2,3'-bipyridin]-5'-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

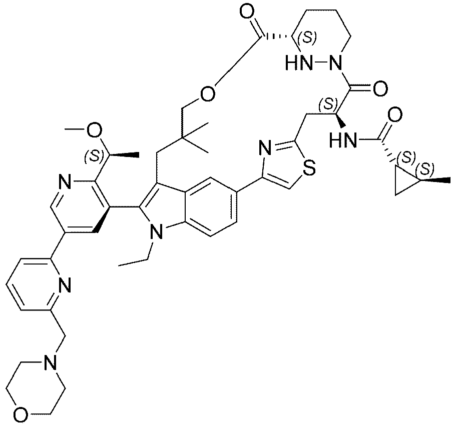

Example 13 was prepared by the following steps:

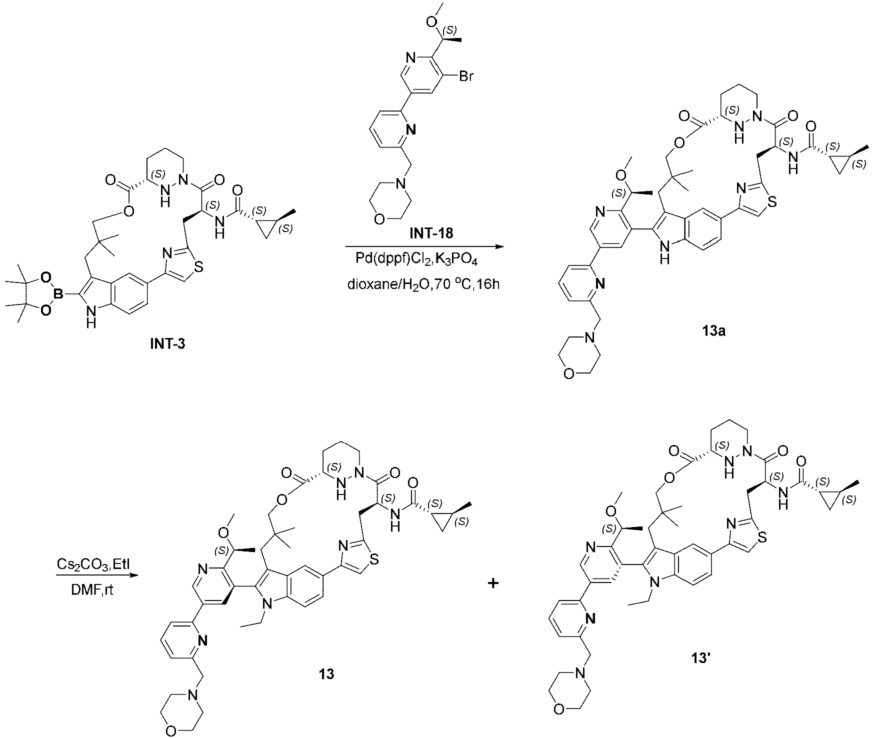

By replacing INT-6 in the synthesis step of compound 1 with INT-18 and INT-4 with INT-3, compound 13 and its diastereoisomer compound 13' can be obtained using similar methods and reaction steps. The absolute configurations drawn for the two compounds are assumptions made based on experience. Compound 13 is an isomer with a longer retention time in LCMS or HPLC, while 13' is an isomer with a shorter retention time in LCMS or HPLC.

### Compound 13

ESI-MS (m/z): 889.9 [M+H]⁺; LC-MS retention time RT = 1.83 min. HPLC retention time RT = 13.43 min.

¹H NMR (500 MHz, DMSO-*d*₆) δ 9.45 - 9.39 (m, 1H), 8.56 - 8.49 (m, 2H), 8.44 - 8.37 (m, 1H), 8.09 - 8.04 (m, 1H), 7.92 (t, *J =* 8.0 Hz, 1H), 7.82 (s, 1H), 7.79 - 7.74 (m, 1H), 7.60 (d, *J* = 8.5 Hz, 1H), 7.49 (d, *J* = 8.0 Hz, 1H), 5.57 (t, *J* = 9.0 Hz, 1H), 5.13 - 5.05 (m, 1H), 4.42 - 4.32 (m, 2H), 4.28 - 4.14 (m, 3H), 3.74 - 3.65 (m, 2H), 3.62 - 3.54 (m, 6H), 3.33 - 3.26 (m, 5H), 3.19 - 3.09 (m, 1H), 3.05 - 2.94 (m, 1H), 2.79 - 2.73 (m, 1H), 2.49 - 2.42 (m, 5H), 2.10 - 2.03 (m, 1H), 1.80 - 1.76 (m, 2H), 1.56 - 1.47 (m, 2H), 1.41 (d, *J =* 6.0 Hz, 3H), 1.11 - 1.02 (m, 4H), 0.94 - 0.85 (m, 6H), 0.60 - 0.50 (m, 1H), 0.38 (s, 3H).

### Compound 13'

ESI-MS (m/z): 889.9 [M+H]⁺. LC-MS retention time RT = 1.80 min. HPLC retention time RT = 13.13 min.

¹H NMR (500 MHz, DMSO-*d*₆) δ 9.47 - 9.43 (m, 1H), 8.58 - 8.51 (m, 3H), 8.05 - 7.99 (m, 1H), 7.92 (t, *J =* 7.5 Hz, 1H), 7.82 (s, 1H), 7.77 - 7.73 (m, 1H), 7.56 (d, *J* = 8.5 Hz, 1H), 7.49 (d, *J* = 7.5 Hz, 1H), 5.56 (t, *J* = 9.0 Hz, 1H), 5.10 - 5.03 (m, 1H), 4.29 - 4.19 (m, 2H), 4.07 - 3.97 (m, 2H), 3.91 - 3.81 (m, 1H), 3.74 - 3.65 (m, 3H), 3.62 - 3.55 (m, 5H), 3.37 - 3.32 (m, 1H), 3.20 - 3.05 (m, 5H), 2.81 - 2.74 (m, 1H), 2.49 - 2.44 (m, 4H), 2.42 - 2.36 (m, 1H), 2.15 - 2.09 (m, 1H), 1.85 - 1.76 (m, 2H), 1.57 - 1.48 (m, 2H), 1.28 (d, *J= 6.0* Hz, 3H), 1.14 (t, *J* = 7.0 Hz, 3H), 1.11 - 1.05 (m, 4H), 0.96 - 0.85 (m, 4H), 0.59 - 0.54 (m, 1H), 0.52 (s, 3H).

### Example 14

### (1S,2S)-N-((6³S,4S,Z)-1¹-ethyl-1²-(2-((S)-1-methoxyethyl)-5-(5-(morpholinomethyl)pyrimidin-2-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

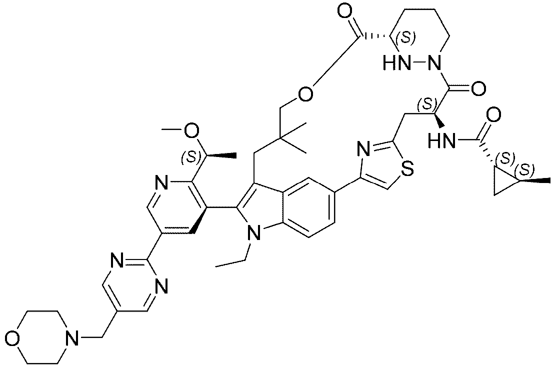

Example 14 was prepared by the following steps:

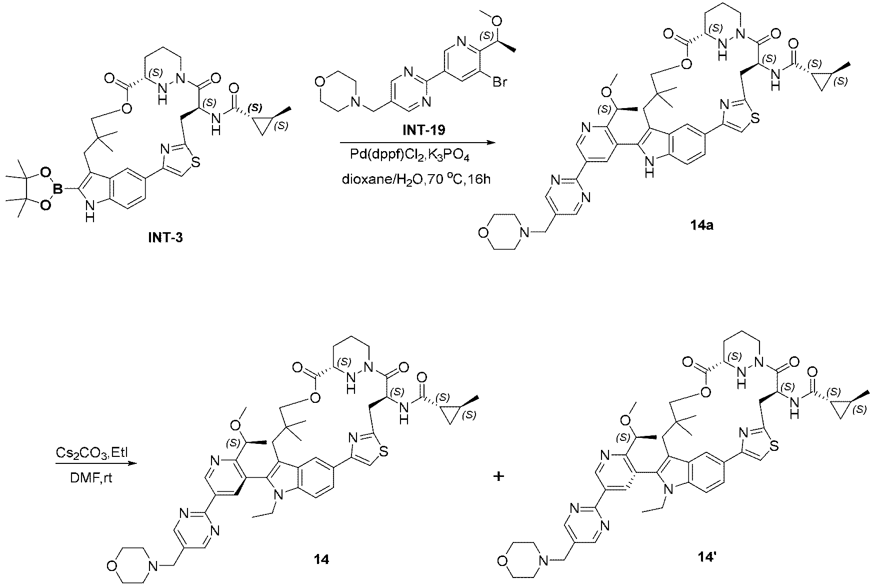

By replacing INT-6 in the synthesis step of compound 1 with INT-19 and INT-4 with INT-3, compound 14 and its diastereoisomer compound 14' can be obtained using similar methods and reaction steps. The absolute configurations drawn for the two compounds are assumptions made based on experience. Compound 14 is an isomer with a longer retention time in LCMS or HPLC, while 14' is an isomer with a shorter retention time in LCMS or HPLC.

### Compound 14

ESI-MS (m/z): 890.9 [M+H]⁺; LC-MS retention time RT = 1.83 min. HPLC retention time RT = 13.23 min.

### Compound 14'

ESI-MS (m/z): 889.9 [M+H]⁺. LC-MS retention time RT = 1.80 min. HPLC retention time RT = 12.94 min.

¹H NMR (500 MHz, DMSO-*d*₆) δ 9.70 - 9.66 (m, 1H), 8.93 - 8.87 (m, 2H), 8.73 - 8.69 (m, 1H), 8.57 - 8.51 (m, 2H), 7.82 (s, 1H), 7.78 - 7.73 (m, 1H), 7.59 - 7.54 (m, 1H), 5.55 (t, *J* = 9.0 Hz, 1H), 5.08 - 5.03 (m, 1H), 4.27 - 4.18 (m, 2H), 4.06 - 4.00 (m, 2H), 3.85 - 3.78 (m, 1H), 3.70 - 3.65 (m, 1H), 3.62 - 3.55 (m, 6H), 3.19 - 3.06 (m, 5H), 2.80 - 2.72 (m, 1H), 2.45 - 2.40 (m, 3H), 2.38 - 2.33 (m, 1H), 2.15 - 2.10 (m, 1H), 1.84 - 1.76 (m, 2H), 1.55 - 1.47 (m, 2H), 1.27 (d, *J=* 6.0 Hz, 3H), 1.13 (t, *J* = 7.0 Hz, 3H), 1.11 - 1.05 (m, 4H), 0.94 - 0.87 (m, 4H), 0.60 - 0.54 (m, 1H), 0.51 (s, 3H).

### Example 15

### (1S,2S)-N-((6³S,4S,Z)-1¹-ethyl-1²-(2-((S)-1-methoxyethyl)-5-(1-(2-morpholinoethyl)-1H-pyrazol-4-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

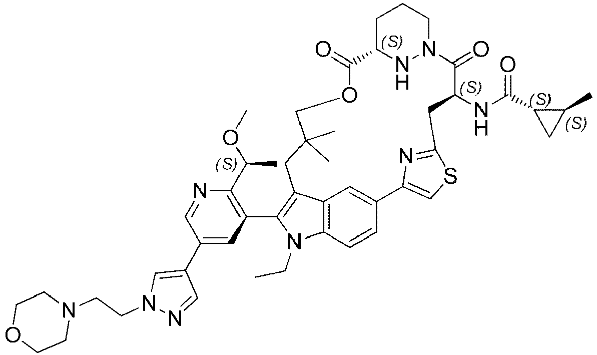

Example 15 was prepared by the following steps:

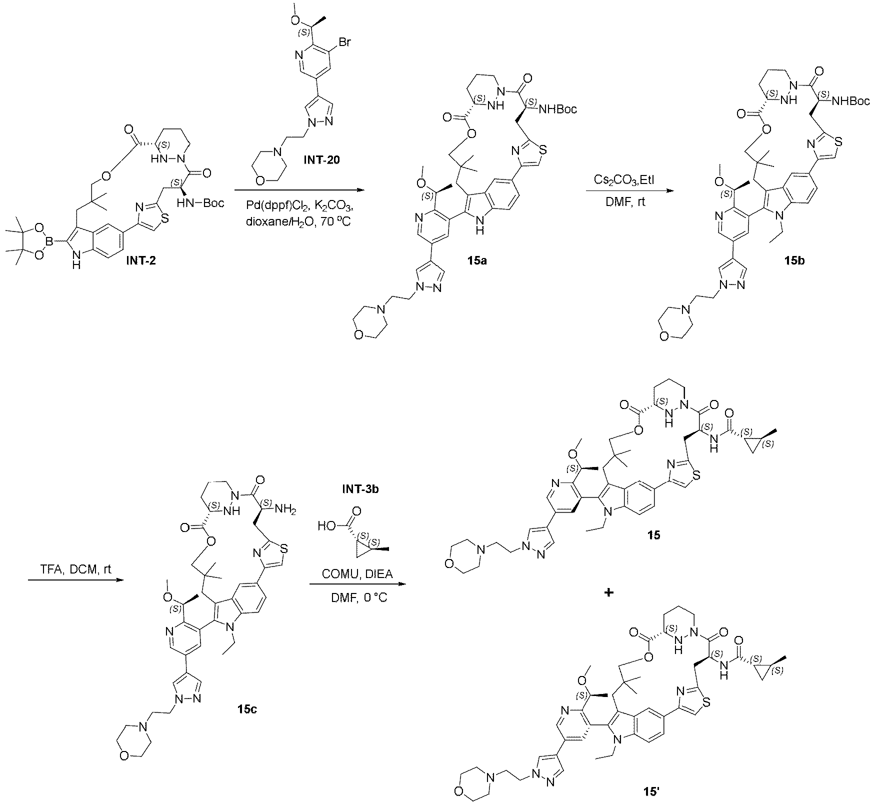

Step 1: Dissolve intermediate INT-2 (105.3 mg, 0.152 mmol) and intermediate INT-20 (60 mg, 0.152 mmol) in a mixed solvent of 1,4-dioxane (5 mL) and water (0.5 mL), add [1,1'-bis(diphenylphosphino)ferrocene]palladium dichloride (11 mg, 0.015 mmol) and potassium carbonate (96.7 mg, 0.456 mmol). The system was replaced with nitrogen and heated to 70 °C with stirring for 16 hours. After the reaction solution was cooled to room temperature, it was filtered through celite and the filtrate was concentrated. The residue was purified by silica gel column chromatography (dichloromethane/methanol = 20/1) to give a light yellow solid compound 15a (100 mg, yield 74.7%). ESI-MS (m/z): 883.6 [M+H]⁺.

Step 2: Compound 15a (100 mg, 0.113 mmol) was dissolved in DMF (2 mL), cesium carbonate (73.9 mg, 0.226 mmol) was added thereto, and then iodoethane (26.5 mg, 0.170 mmol) was added dropwise to the reaction solution, and the reaction solution was stirred at room temperature for 16 hours. After the reaction was completed by LCMS, saturated brine was added to the reaction solution, extracted with ethyl acetate, the organic phases were combined and washed with saturated brine, dried over anhydrous sodium sulfate, and the organic phase was concentrated to obtain a light yellow solid compound 15b (100 mg, yield 96.9%). ESI-MS (m/z): 911.5 [M+H]⁺.

Step 3: Compound 15b (100 mg, 0.110 mmol) was dissolved in dichloromethane (2 mL). Trifluoroacetic acid (1 mL) was added dropwise thereto under ice cooling. The reaction solution was stirred at room temperature for 2 hours, and the reaction was completed after monitoring by LCMS. Saturated aqueous sodium carbonate solution was added, the reaction solution was extracted with ethyl acetate, the organic phases were combined and washed with saturated brine, dried over anhydrous sodium sulfate, and the organic phase was concentrated to obtain a light yellow solid compound 15c (80 mg, yield 89.9%). ESI-MS (m/z): 811.5 [M+H]⁺.

Step 4: Compound 15c (80 mg, 0.099 mmol) was dissolved in N,N-dimethylformamide (2 mL), and (1S,2S)-2-methylcyclopropanecarboxylic acid INT-3b (19.8 mg, 0.198 mmol), N,N-diisopropylethylamine (38.3 mg, 0.296 mmol) and (2-hydroxyimino-ethyl cyanoacetate)-N,N-dimethyl-morpholinyl uronium hexafluorophosphate (105.7 mg, 0.198 mmol) were added thereto. The reaction mixture was stirred in an ice bath for 1 hour. After the reaction was complete, water was added to the reaction system, and the reaction system was extracted with ethyl acetate, the organic phases were combined, washed with saturated brine, dried over anhydrous sodium sulfate, filtered and concentrated, and the residue was purified by preparative liquid chromatography to obtain white solid compound 15 (6 mg, yield 6.8%) and diastereoisomer compound 15' (8 mg, yield 9.1%). The absolute configurations of the two compounds are drawn based on the empirical assumption that compound 15 is the isomer with a longer retention time in LCMS or HPLC, while 15' is the isomer with a shorter retention time in LCMS or HPLC.

### Compound 15

ESI-MS (m/z): 892.9 [M+H]⁺; LC-MS retention time RT = 1.66 min. HPLC retention time; Retention time RT = 12.06 min.

¹H NMR (500 MHz, DMSO-d6) δ 8.99 (d, *J* = 2.0 Hz, 1H), 8.53 - 8.49 (m, 2H), 8.42 (s, 1H), 8.08 (s, 1H), 7.99 (d, *J* = 2.0 Hz, 1H), 7.81 (s, 1H), 7.76 - 7.73 (m, 1H), 7.61 - 7.53 (m, 1H), 5.56 (t, *J= 9.0* Hz, 1H), 5.10 - 5.04 (m, 1H), 4.36 - 4.15 (m, 7H), 3.61 - 3.52 (m, 6H), 3.35 - 3.29 (m, 2H), 3.25 (s, 3H), 3.18 - 3.11 (m, 1H), 3.02 - 2.93 (m, 1H), 2.79 - 2.70 (m, 3H), 2.47 - 2.38 (m, 5H), 2.11 - 2.05 (m, 1H), 1.85 - 1.73 (m, 2H), 1.54 - 1.46 (m, 2H), 1.38 (d, *J=* 6.0 Hz, 3H), 1.10 - 1.01 (m, 4H), 0.94 - 0.85 (m, 6H), 0.61 - 0.50 (m, 1H), 0.37 (s, 3H).

### Compound 15'

ESI-MS (m/z): 892.9 [M+H]⁺. LC-MS retention time RT = 1.65 min. HPLC retention time Retention time RT = 12.02 min.

¹H NMR (500 MHz, DMSO) δ 9.06 - 8.93 (m, 1H), 8.59 - 8.47 (m, 2H), 8.42 - 8.32 (m, 1H), 8.15 - 8.02 (m, 2H), 7.85 - 7.70 (m, 2H), 7.59 - 7.49 (m, 1H), 5.64 - 5.47 (m, 1H), 5.16 - 4.95 (m, 1H), 4.30 - 4.13 (m, 4H), 4.04 - 3.83 (m, 3H), 3.71 - 3.48 (m, 7H), 3.18 - 3.03 (m, 6H), 2.83 - 2.67 (m, 3H), 2.45 - 2.34 (m, 4H), 2.17 - 2.05 (m, 1H), 1.88 - 1.71 (m, 2H), 1.58 - 1.44 (m, 2H), 1.28 - 1.20 (m, 3H), 1.17 - 1.02 (m, 7H), 0.98 - 0.84 (m, 4H), 0.62 - 0.38 (m, 4H).

### Example 16

### (1S,2S)-N-((6³S,4S,Z)-1¹-ethyl-1²-(2-((S)-1-methoxyethyl)-5-(1-((R)-1-methylpyrrolidin-3-yl)-1H-pyrazol-4-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

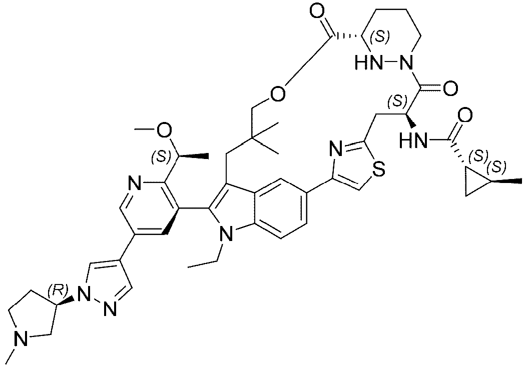

By replacing INT-7 in the synthesis step of compound 2 with INT-21, compound 16 and its diastereoisomer compound 16' can be obtained using similar methods and reaction steps. The absolute configurations of the two compounds are drawn based on the empirical assumption that compound 16 is the isomer with a longer retention time in LCMS or HPLC, while 16' is the isomer with a shorter retention time in LCMS or HPLC.

### Compound 16

ESI-MS (m/z): 862.9 [M+H]⁺; LC-MS retention time RT = 1.69 min. HPLC retention time RT = 12.26 min.

¹H NMR (500 MHz, DMSO) δ 9.01 (d, *J* = 2.0 Hz, 1H), 8.53 - 8.48 (m, 2H), 8.48 (s, 1H), 8.09 (s, 1H), 8.02 (d, *J=* 2.0 Hz, 1H), 7.80 (s, 1H), 7.76 - 7.73 (m, 1H), 7.58 (d, *J* = 8.5 Hz, 1H), 5.56 (t, *J* = 9.0 Hz, 1H), 5.10 - 5.05 (m, 1H), 4.93 - 4.87 (m, 1H), 4.36 - 4.29 (m, 1H), 4.28 - 4.23 (m, 1H), 4.24 - 4.20 (m, 1H), 4.20 - 4.12 (m, 2H), 3.61 - 3.55 (m, 2H), 3.25 (s, 3H), 3.17 - 3.12 (m, 1H), 3.00 - 2.94 (m, 1H), 2.90 - 2.85 (m, 1H), 2.77 - 2.71 (m, 2H), 2.46 - 2.41 (m, 1H), 2.40 - 2.33 (m, 2H), 2.28 (s, 3H), 2.17 - 2.11 (m, 1H), 2.10 - 2.05 (m, 1H), 2.03 - 1.96 (m, 1H), 1.82 - 1.76 (m, 2H), 1.53 - 1.47 (m, 2H), 1.38 (d, *J* = 6.0 Hz, 3H), 1.25 - 1.20 (m, 3H), 1.06 (s, 3H), 0.93 - 0.85 (m, 6H), 0.57 - 0.52 (m, 1H), 0.36 (s, 3H).

### Compound 16'

ESI-MS (m/z): 862.9 [M+H]⁺; LC-MS retention time RT = 1.68 min. HPLC retention time RT = 11.27 min.

¹H NMR (500 MHz, DMSO) δ 9.01 (d, *J* = 2.0 Hz, 1H), 8.57 - 8.50 (m, 2H), 8.45 (s, 1H), 8.15 (d, *J =* 2.0 Hz, 1H), 8.06 (s, 1H), 7.81 (s, 1H), 7.76 - 7.71 (m, 1H), 7.55 (d, *J* = 8.5 Hz, 1H), 5.56 (t, *J* = 9.0 Hz, 1H), 5.07 - 5.01 (m, 1H), 4.94 - 4.88 (m, 1H), 4.27 - 4.18 (m, 2H), 4.01 - 3.95 (m, 2H), 3.94 - 3.86 (m, 1H), 3.73 - 3.67 (m, 1H), 3.57 - 3.52 (m, 1H), 3.18 - 3.14 (m, 1H), 3.12 (s, 3H), 3.08 - 3.01 (m, 1H), 2.91 - 2.86 (m, 1H), 2.79 - 2.72 (m, 3H), 2.56 - 2.52 (m, 1H), 2.47 - 2.40 (m, 1H), 2.40 - 2.35 (m, 1H), 2.29 (s, 3H), 2.18 - 2.09 (m, 2H), 1.83 - 1.77 (m, 2H), 1.55 - 1.46 (m, 2H), 1.29 - 1.21 (m, 4H), 1.10 (t, *J* = 7.0 Hz, 3H), 1.08 - 1.03 (m, 4H), 0.91 (s, 3H), 0.89 - 0.85 (m, 1H), 0.58 - 0.53 (m, 1H), 0.49 (s, 3H).

### Example 17

### (1r,2R,3S)-N-((6³S,4S,Z)-1¹-ethyl-1²-(2-((S)-1-methoxyethyl)-5-(1-(1-methylazetidin-3-yl)-1H-pyrazol-4-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

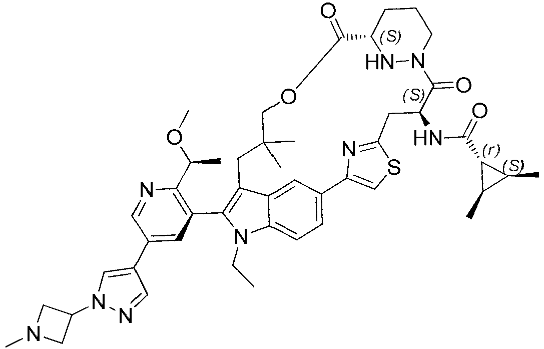

Example 17 was prepared by the following steps:

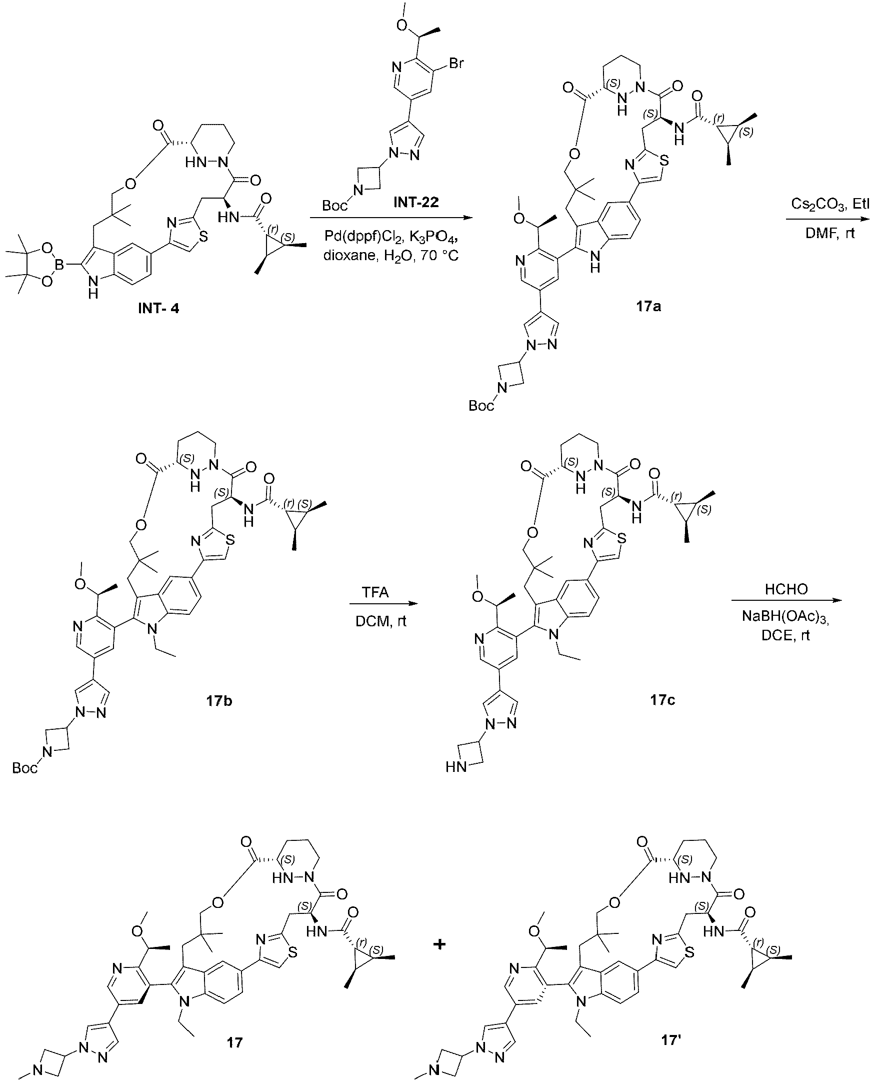

By replacing INT-7 in the synthesis step of compound 2 with INT-22 and replacing INT-3 with INT-4, compound 17 and its diastereoisomer compound 17' can be obtained using similar methods and reaction steps. The absolute configurations drawn for the two compounds are assumptions made based on experience. Compound 17 is an isomer with a longer retention time in LCMS or HPLC, while 17' is an isomer with a shorter retention time in LCMS or HPLC.

### Compound 17

ESI-MS (m/z): 862.9 [M+H]⁺; LC-MS retention time RT = 1.69 min. HPLC retention time RT = 12.52 min.

¹H NMR (500 MHz, DMSO-*d*₆) δ 8.96 (d, *J* = 2.5 Hz, 1H), 8.52 (s, 1H), 8.44 (d, *J* = 1.5 Hz, 1H), 8.33 (d, *J* = 9.0 Hz, 1H), 8.11 (s, 1H), 7.97 (d, *J* = 2.5 Hz, 1H), 7.75 (s, 1H), 7.68 (dd, *J* = 9.0, 1.5 Hz, 1H), 7.52 (d, *J* = 8.5 Hz, 1H), 5.48 (t, *J* = 9.0 Hz, 1H), 5.02 - 4.96 (m, 1H), 4.93 - 4.88 (m, 1H), 4.30 - 4.24 (m, 1H), 4.23 - 4.18 (m, 1H), 4.18 - 4.13 (m, 1H), 4.11 - 4.05 (m, 1H), 3.73 - 3.68 (m, 2H), 3.53 - 3.48 (m, 2H), 3.43 - 3.38 (m, 2H), 3.25 - 3.21 (m, 1H), 3.19 (s, 3H), 3.13 - 3.08 (m, 1H), 3.07 - 3.01 (m, 1H), 2.93 - 2.87 (m, 1H), 2.71 - 2.67 (m, 1H), 2.41 - 2.36 (m, 1H), 2.29 (s, 3H), 2.03 - 1.98 (m, 1H), 1.75 - 1.70 (m, 2H), 1.47 - 1.41 (m, 1H), 1.31 (d, *J =* 6.0 Hz, 3H), 1.20 - 1.15 (m, 2H) , 1.13 - 1.08 (m, 1H), 1.02 (d, *J* = 5.5 Hz, 3H), 1.00 (d, *J* = 5.5 Hz, 3H), 0.84 (d, *J* = 7.0 Hz, 3H), 0.82 (s, 3H), 0.30 (s, 3H).

### Compound 17'

ESI-MS (m/z): 862.9 [M+H]⁺; LC-MS retention time RT = 1.68 min. HPLC retention time RT = 12.28 min.

¹H NMR (500 MHz, DMSO-*d*₆) δ 9.03 (d, *J* = 2.5 Hz, 1H), 8.57 - 8.52 (m, 2H), 8.41 (d, *J =* 9.0 Hz, 1H), 8.17 (d, *J* = 2.5 Hz, 1H), 8.15 (s, 1H), 7.82 (s, 1H), 7.74 (dd, *J* = 9.0, 1.5 Hz, 1H), 7.56 (d, *J* = 8.5 Hz, 1H), 5.55 (t, *J* = 9.0 Hz, 1H), 5.06 - 5.00 (m, 1H), 5.00 - 4.95 (m, 1H), 4.26 - 4.20 (m, 2H), 4.03 - 3.88 (m, 2H), 3.92 - 3.86 (m, 1H), 3.78 - 3.72 (m, 2H), 3.70 - 3.65 (m, 1H), 3.59 - 3.55 (m, 1H), 3.47 - 3.41 (m, 2H), 3.33 - 3.30 (m, 1H), 3.20 - 3.15 (m, 1H), 3.12 (s, 3H), 3.08 - 3.02 (m, 1H), 2.80 - 2.75 (m, 2H), 2.47 - 2.41 (m, 1H), 2.36 (s, 3H), 2.15 - 2.09 (m, 1H), 1.83 - 1.78 (m, 2H), 1.56 - 1.48 (m, 1H), 1.27 - 1.22 (m, 6H), 1.13 - 1.08 (m, 6H), 1.07 (d, *J =* 5.5 Hz, 3H), 0.92 (s, 3H), 0.49 (s, 3H).

### Example 18

### (1r,2R,3S)-N-((6³S,4S,Z)-1¹-ethyl-1²-(2-((S)-1-methoxyethyl)-5-(1-(1-methylpiperidin-4-yl)-1H-pyrazol-4-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

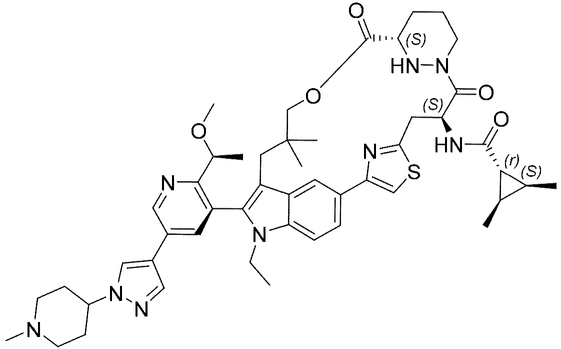

Example 18 was prepared by the following steps:

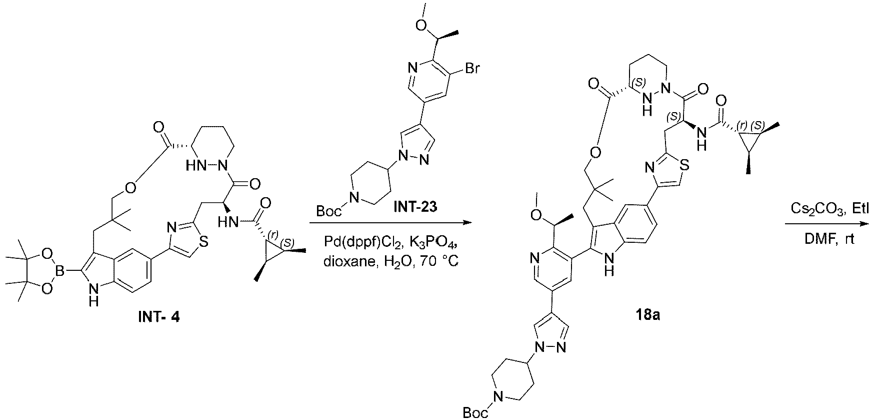

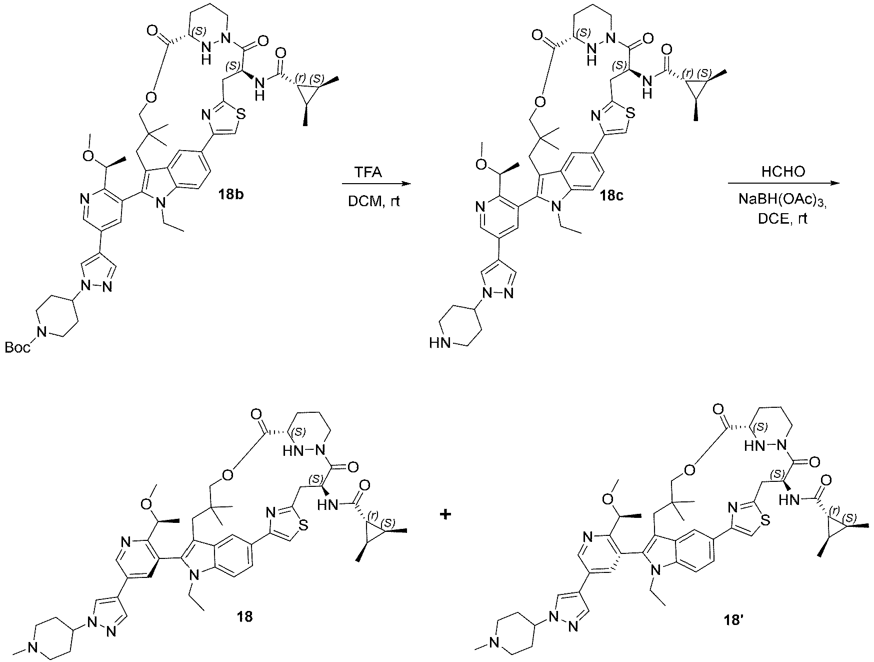

By replacing INT-7 in the synthesis step of compound 2 with INT-23 and INT-3 with INT-4, compound 18 and its diastereoisomer compound 18' can be obtained using similar methods and reaction steps. The absolute configurations drawn for the two compounds are assumptions made based on experience. Compound 18 is an isomer with a longer retention time in LCMS or HPLC, while 18' is an isomer with a shorter retention time in LCMS or HPLC.

### Compound 18

ESI-MS (m/z): 890.9 [M+H]⁺; LC-MS retention time RT = 1.71 min. HPLC retention time RT = 12.54 min.

### Compound 18'

ESI-MS (m/z): 890.9 [M+H]⁺. LC-MS retention time RT = 1.70 min. HPLC retention time RT = 12.52 min.

¹H NMR (500 MHz, DMSO-*d*₆) δ 9.01 (d, *J* = 2.5 Hz, 1H), 8.53 - 8.48 (m, 3H), 8.09 (s, 1H), 8.02 (d, *J* = 2.5 Hz, 1H), 7.81 (s, 1H), 7.75 (dd, *J* = 8.5, 1.5 Hz, 1H), 7.58 (d, *J =* 8.5 Hz, 1H), 5.57 - 5.51 (m, 1H), 5.10-5.03 (m, 1H), 4.92-4.85 (m, 1H), 4.36-4.29 (m,1H), 4.26-4.20 (m, 2H), 4.20-4.12 (m,2H), 3.61 - 3.56 (m, 2H), 3.26 (s, 3H), 3.20 - 3.15 (m, 2H), 3.03-2.96 (m, 1H), 2.90 - 2.85 (m, 1H), 2.79-2.73 (m, 3H), 2.47 - 2.41 (m, 1H), 2.39-2.33 (m, 2H), 2.29 (s, 3H), 2.18-2.12 (m,2H), 2.11-2.05 (m, 2H), 1.83 - 1.78 (m, 2H), 1.50 - 1.45 (m, 2H), 1.38 (d, *J* = 6.0 Hz, 3H), 1.33 - 1.26 (m, 1H), 1.09 - 1.05 (m, 4H), 0.93 - 0.88 (m, 8H), 0.57 - 0.51 (m, 1H), 0.37 (s, 3H).

### Example 19

### (1r,2R,3S)-N-((6³S,4S,Z)-1¹-ethyl-1²-(2-((S)-1-methoxyethyl)-5-(1-(2-(4-methylpiperazin-1-yl)ethyl)-1H-pyrazol-4-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

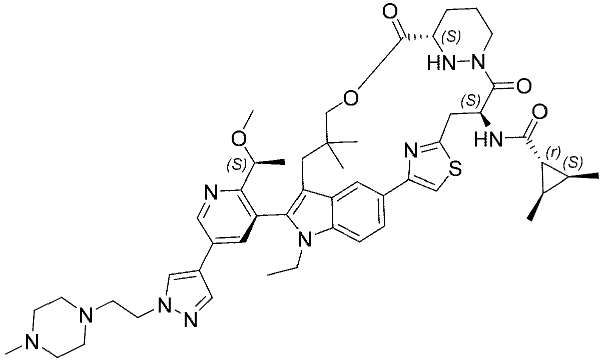

Example 19 was prepared by the following steps:

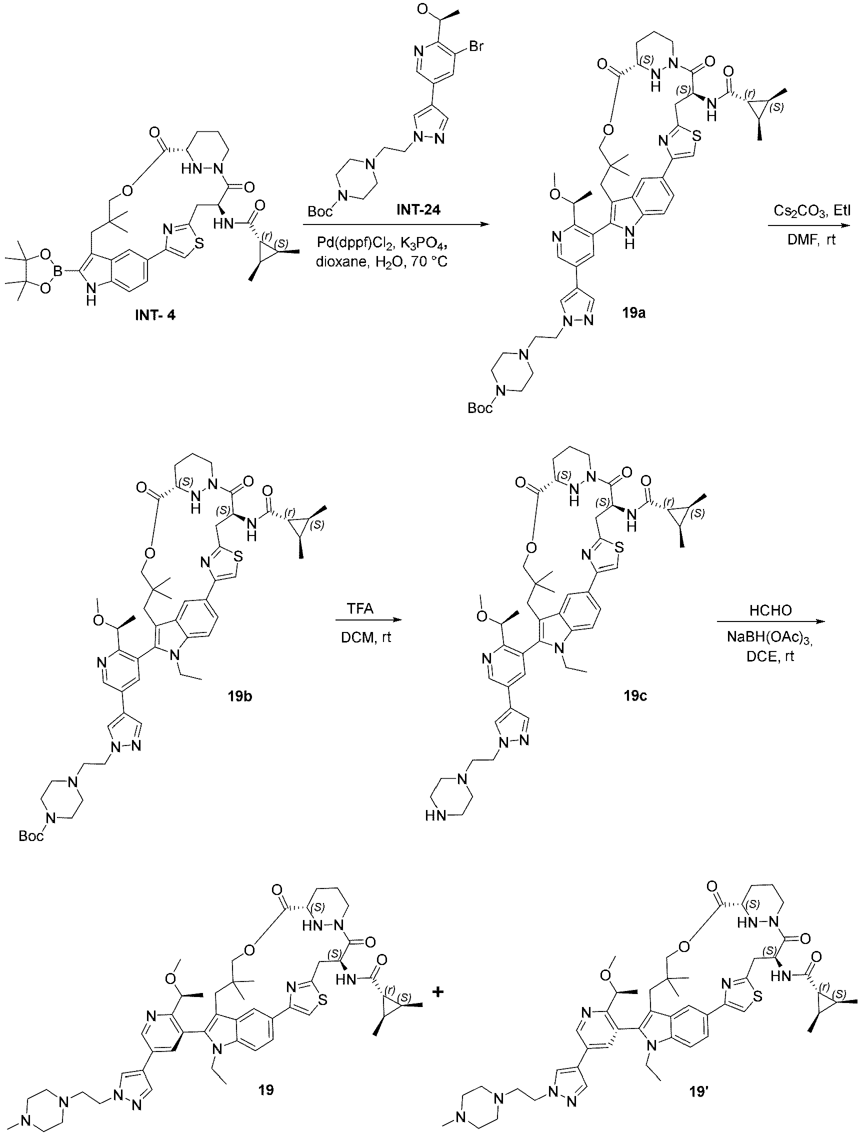

By replacing INT-7 in the synthesis step of compound 2 with INT-24 and INT-3 with INT-4, compound 19 and its diastereoisomer compound 19' can be obtained using similar methods and reaction steps. The absolute configurations drawn for the two compounds are assumptions made based on experience. Compound 19 is an isomer with a longer retention time in LCMS or HPLC, while 19' is an isomer with a shorter retention time in LCMS or HPLC.

### Compound 19

ESI-MS (m/z): 919.9 [M+H]⁺; LC-MS retention time RT = 1.63 min. HPLC retention time RT = 11.86 min.

1H NMR (500 MHz, DMSO-*d*6) δ 9.00 (d, *J* = 2.0 Hz, 1H), 8.50 (s, 1H), 8.43 - 8.38 (m, 2H), 8.09 (s, 1H), 7.99 (d, *J* = 2.0 Hz, 1H), 7.82 (s, 1H), 7.75 (d, *J* = 8.5 Hz, 1H), 7.58 (d, *J* = 8.5 Hz, 1H), 5.56 (t, *J =* 9.0 Hz, 1H), 5.10 - 5.03 (m, 1H), 4.39 - 4.30 (m, 1H), 4.28 - 4.22 (m, 4H), 4.18 - 4.12 (m, 2H), 3.63 - 3.55 (m, 2H), 3.33 - 3.29 (m, 1H), 3.25 (s, 3H), 3.18 - 3.13 (m, 1H), 2.99-2.93 (m, 2H), 2.84 - 2.78 (m, 3H), 2.77-2.71 (m, 2H), 2.60 - 2.56 (m, 2H), 2.45 - 2.40 (m, 2H), 2.10 - 2.03 (m, 1H), 1.79-1.73 (m, 3H), 1.53-1.48 (m, 2H), 1.38 (d, *J=* 6.0 Hz, 3H), 1.25 - 1.20 (m, 1H), 1.19 - 1.13 (m, 3H), 1.10-1.03 (m, 7H), 0.95 - 0.85 (m, 7H), 0.37 (s, 3H).

### Compound 19'

ESI-MS (m/z): 919.9 [M+H]⁺. LC-MS retention time RT = 1.62 min. HPLC retention time RT = 11.33 min.

¹H NMR (500 MHz, DMSO-*d*₆) δ 9.02 (d, *J* = 2.0 Hz, 1H), 8.54 (s, 1H), 8.45 - 8.40 (m, 2H), 8.13 (d, *J =* 2.0 Hz, 1H), 8.07 (s, 1H), 7.82 (s, 1H), 7.74 (d, *J =* 8.5 Hz, 1H), 7.56 (d, *J* = 8.5 Hz, 1H), 5.55 (t, *J* = 9.0 Hz, 1H), 5.07-5.01 (m, 1H), 4.33 - 4.28 (m, 2H), 4.25 - 4.20 (m, 2H), 4.00 - 3.93 (m, 2H), 3.91 - 3.83 (m, 1H), 3.72 - 3.68 (m, 1H), 3.58 - 3.55 (m, 1H), 3.33 - 3.31 (m, 1H), 3.28 - 3.23 (m, 1H), 3.18 - 3.14 (m, 1H), 3.12 (s, 3H), 3.10 - 3.03 (m, 2H), 3.00 - 2.95 (m, 2H), 2.93 - 2.88 (m, 2H), 2.77 - 2.70 (m, 4H), 2.47 - 2.40 (m, 2H), 2.14 - 2.09 (m, 1H), 1.83 - 1.78 (m, 2H), 1.57 - 1.47 (m, 1H), 1.25 (d, *J* = 6.0 Hz, 3H), 1.23 - 1.17 (m, 3H), 1.13 (d, *J* = 7.0 Hz, 3H), 1.13 - 1.88 (m, 4H), 1.07 (d, *J* = 6.0 Hz, 3H), 0.92 (s, 3H), 0.51 (s, 3H).

### Example 20

### (1S,2S)-N-((6³S,4S,Z)-1²-(5-(4,5-dimethyl-4H-1,2,4-triazol-3-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

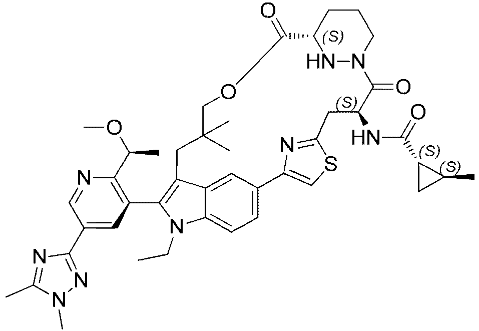

Example 20 was prepared by the following steps:

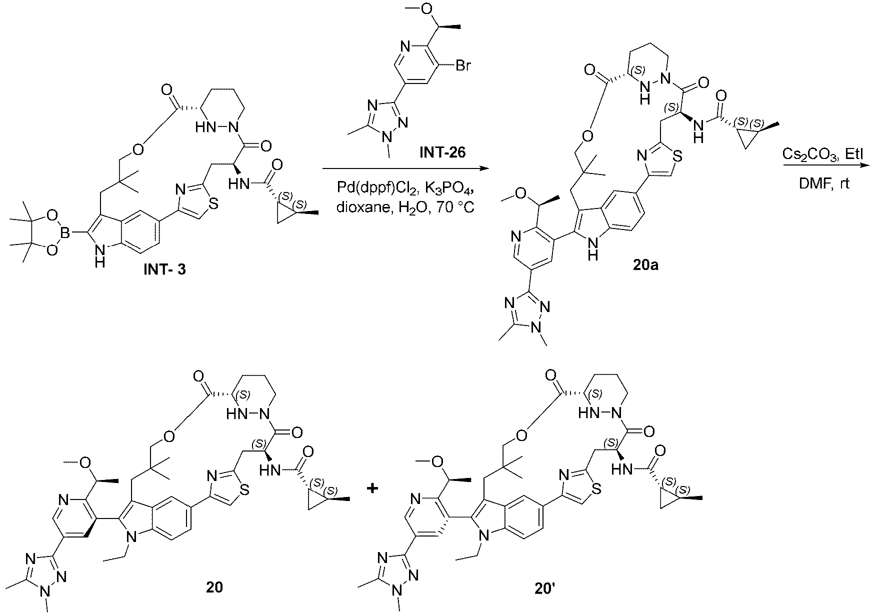

By replacing INT-6 in the synthesis step of compound 1 with INT-26 and INT-4 with INT-3, compound 20 and its diastereoisomer compound 20' can be obtained using similar methods and reaction steps. The absolute configurations drawn for the two compounds are assumptions made based on experience. Compound 20 is an isomer with a longer retention time in LCMS or HPLC, while 20' is an isomer with a shorter retention time in LCMS or HPLC.

### Compound 20

ESI-MS (m/z): 808.5 [M+H]⁺; LC-MS retention time RT = 1.66 min. HPLC retention time RT = 12.15 min.

¹H NMR (500 MHz, DMSO-*d*₆) δ 9.26 (d, *J* = 2.0 Hz, 1H), 8.54 - 8.50 (m, 2H), 8.12 (d, *J =* 2.0 Hz, 1H), 7.81 (s, 1H), 7.76 (dd, *J =* 8.5, 1.5 Hz, 1H), 7.60 (d, *J =* 8.5 Hz, 1H), 5.62 - 5.52 (m, 1H), 5.12 - 5.04 (m, 1H), 4.39 - 4.30 (m, 2H), 4.24 - 4.19 (m, 1H), 4.17 - 4.09 (m, 2H), 3.85 (s, 3H), 3.61 - 3.55 (m, 2H), 3.28 (s, 3H), 3.16 - 3.13 (m, 2H), 3.03 - 2.98 (m, 1H), 2.79 - 2.73 (m, 1H), 2.46 (s, 3H), 2.43 - 2.37 (m, 1H), 2.12 - 2.04 (m, 1H), 1.83 - 1.75 (m, 2H), 1.54 - 1.48 (m, 2H), 1.44 - 1.38 (m, 4H), 1.35 - 1.31 (m, 1H), 1.09 - 1.05 (m, 4H), 0.90 - 0.84 (m, 8H), 0.58 - 0.53 (m, 1H), 0.36 (s, 3H).

### Compound 20'

ESI-MS (m/z): 808.5 [M+H]⁺. LC-MS retention time RT = 1.65 min. HPLC retention time RT = 11.96 min.

¹H NMR (500 MHz, DMSO-*d*₆) δ 9.28 (d, *J* = 2.0 Hz, 1H), 8.56 - 8.51 (m, 2H), 8.26 (d, *J* = 2.0 Hz, 1H), 7.82 (s, 1H), 7.75 (dd, *J* = 8.5, 1.5 Hz, 1H), 7.55 (d, *J* = 8.5 Hz, 1H), 5.60 - 5.52 (m, 1H), 5.09 - 5.01 (m, 1H), 4.28 - 4.20 (m, 2H), 4.02 - 3.94 (m, 2H), 3.86 (s, 3H), 3.84 - 3.75 (m, 1H), 3.72 - 3.63 (m, 1H), 3.60 - 3.53 (m, 1H), 3.21 - 3.13 (m, 1H), 3.10 (s, 3H), 3.09 - 3.04 (m, 1H), 2.79 - 2.74 (m, 1H), 2.47 (s, 3H), 2.37 - 2.32 (m, 1H), 2.16 - 2.07 (m, 1H), 1.83 - 1.77 (m, 2H), 1.53 - 1.48 (m, 2H), 1.25 (d, *J* = 6.0 Hz, 3H), 1.12 (t, *J* = 7.0 Hz, 3H), 1.08 - 1.04 (m, 4H), 0.92 (s, 3H), 0.89 - 0.87 (m, 1H), 0.58 - 0.53 (m, 1H), 0.49 (s, 3H).

### Example 21

### (1S,2S)-N-((6³S,4S,Z)-1¹-ethyl-1²-(2-((S)-1-methoxyethyl)-5-(3-methyl-1,2,4-oxadiazol-5-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

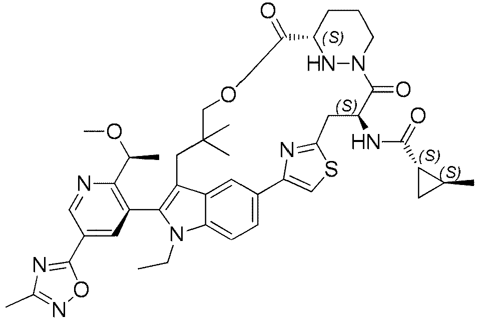

Example 21 was prepared by the following steps:

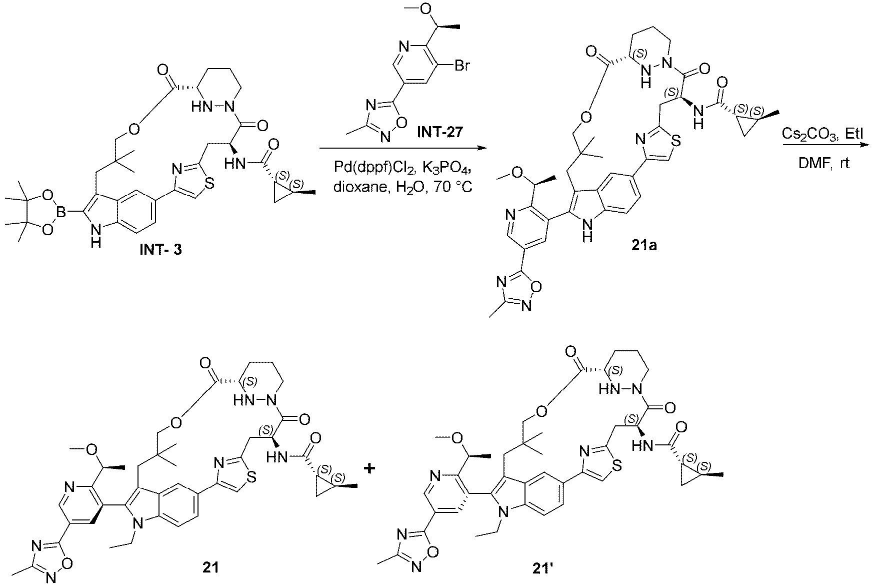

By replacing INT-6 in the synthesis step of compound 1 with INT-27 and INT-4 with INT-3, compound 21 and its diastereoisomer compound 21' can be obtained using similar methods and reaction steps. The absolute configurations drawn for the two compounds are assumptions made based on experience. Compound 21 is an isomer with a longer retention time in LCMS or HPLC, while 21' is an isomer with a shorter retention time in LCMS or HPLC.

### Compound 21

ESI-MS (m/z): 795.5 [M+H]⁺; LC-MS retention time RT = 1.92 min. HPLC retention time RT = 14.84 min.

¹H NMR (500 MHz, DMSO-*d*₆) δ 9.41 (d, *J* = 2.0 Hz, 1H), 8.56 - 8.49 (m, 2H), 8.34 (d, *J =* 2.0 Hz, 1H), 7.82 (s, 1H), 7.78 (dd, *J* = 8.5, 1.5 Hz, 1H), 7.61 (d, *J* = 8.5 Hz, 1H), 5.63 - 5.53 (m, 1H), 5.11 - 5.02 (m, 1H), 4.43 - 4.34 (m, 2H), 4.29 - 4.17 (m, 2H), 4.14 - 4.05 (m, 1H), 3.60 - 3.55 (m, 2H), 3.30 (s, 3H), 3.19 - 3.12 (m, 1H), 3.02 - 2.97 (m, 1H), 2.81 - 2.73 (m, 1H), 2.46 (s, 3H), 2.46 - 2.42 (m, 1H), 2.12 - 2.05 (m, 1H), 1.85 - 1.75 (m, 2H), 1.54 - 1.46 (m, 2H), 1.41 (d, *J =* 6.0 Hz, 3H), 1.25 - 1.23 (m, 1H), 1.09 - 1.06 (m, 3H), 0.92 - 0.84 (m, 7H), 0.59 - 0.51 (m, 1H), 0.35 (s, 3H).

### Compound 21'

ESI-MS (m/z): 795.5 [M+H]⁺. LC-MS retention time RT = 1.86 min. HPLC retention time RT = 13.81 min.

¹H NMR (500 MHz, DMSO-*d*₆) δ 9.43 (d, *J* = 2.0 Hz, 1H), 8.57 - 8.51 (m, 2H), 8.50 (d, *J* = 2.0 Hz, 1H), 7.82 (s, 1H), 7.76 (dd, *J* = 8.5, 1.5 Hz, 1H), 7.57 (d, *J* = 8.5 Hz, 1H), 5.62 - 5.50 (m, 1H), 5.12 - 5.02 (m, 1H), 4.30 - 4.18 (m, 2H), 4.07 - 3.97 (m, 2H), 3.90 - 3.79 (m, 1H), 3.72 - 3.64 (m, 1H), 3.61 - 3.54 (m, 1H), 3.20 - 3.05 (m, 6H), 2.83 - 2.73 (m, 1H), 2.47 (s, 3H), 2.36 - 2.27 (m, 1H), 2.17 - 2.09 (m, 1H), 1.86 - 1.75 (m, 2H), 1.56 - 1.48 (m, 2H), 1.29 - 1.24 (m, 3H), 1.12 (t, *J=* 7.0 Hz, 3H), 1.09 - 1.04 (m, 4H), 0.92 (s, 3H), 0.90 - 0.86 (m, 1H), 0.59 - 0.54 (m, 1H), 0.52 (s, 3H).

### Example 22

### (1S,2S)-N-((6³S,4S,Z)-1¹-ethyl-1²-(2-((S)-1-methoxyethyl)-5-(5-(morpholinomethyl)isoxazol-3-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

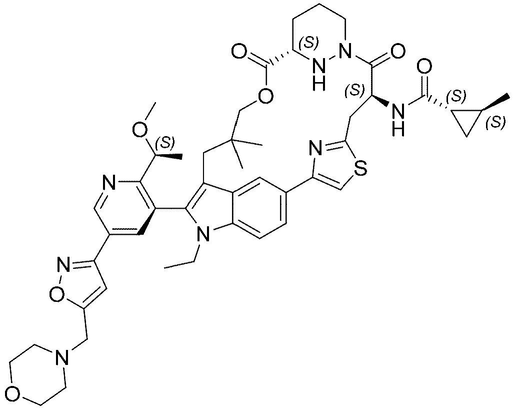

Example 22 was prepared by the following steps:

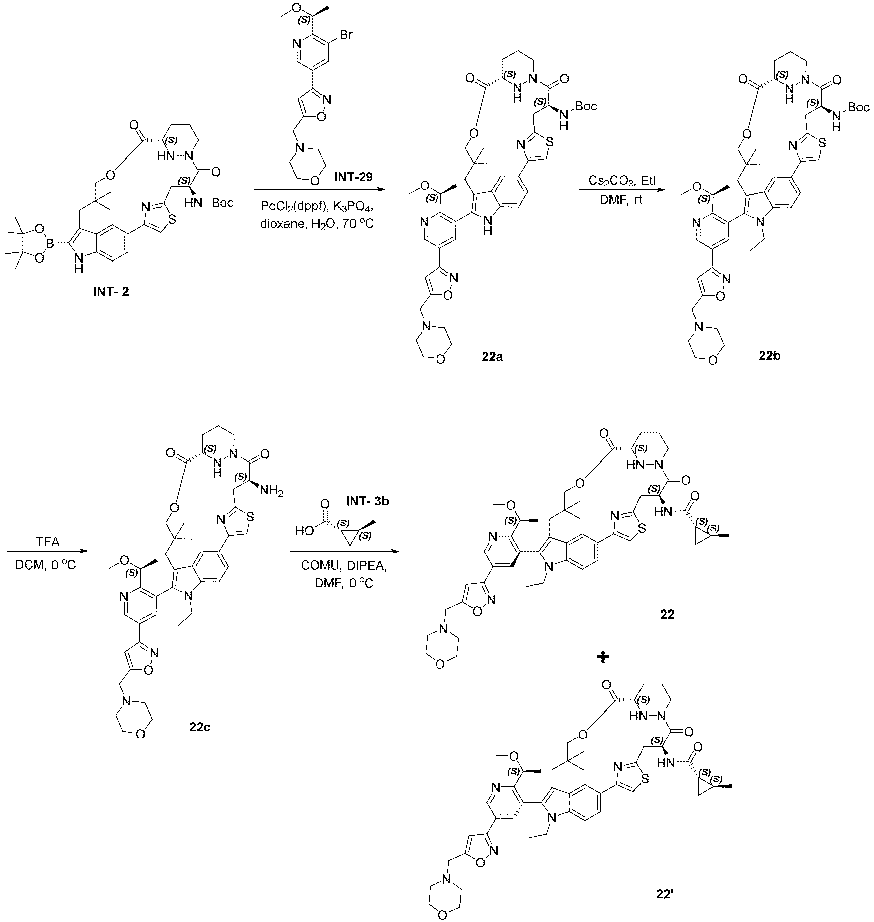

By replacing INT-20 in the synthesis step of compound 15 with INT-29, compound 22 and its diastereoisomer compound 22' can be obtained using similar methods and reaction steps. The absolute configurations drawn for the two compounds are assumptions made based on experience. Compound 22 is an isomer with a longer retention time in LCMS or HPLC, while 22' is an isomer with a shorter retention time in LCMS or HPLC.

### Compound 22

ESI-MS (m/z): 879.5 [M+H]⁺; LC-MS retention time RT = 1.68 min. HPLC retention time; Retention time RT = 12.78 min.

¹H NMR (500 MHz, DMSO-*d6*) δ 9.25 (d, *J* = 2.0 Hz, 1H), 8.55 - 8.50 (m, 2H), 8.26 (d, *J* = 2.0 Hz, 1H), 7.82 (s, 1H), 7.77 (dd, *J =* 8.5, 1.5 Hz, 1H), 7.60 (d, *J* = 8.5 Hz, 1H), 7.22 (s, 1H), 5.56 (t, *J =* 9.3 Hz, 1H), 5.10 - 5.05 (m, 1H), 4.39 - 4.32 (m, 2H), 4.28 - 4.08 (m, 4H), 3.76 (s, 2H), 3.62 - 3.57 (m, 6H), 3.29 (s, 3H), 3.19 - 3.10 (m, 2H), 3.02 - 2.97 (m, 1H), 2.78 - 2.72 (m, 1H), 2.48 (d, *J=* 4.1 Hz, 3H), 2.08 (t, *J* = 5.0 Hz, 1H), 1.83 - 1.76 (m, 2H), 1.54 - 1.47 (m, 2H), 1.40 (d, *J=* 6.1 Hz, 3H), 1.27 - 1.23 (m, 1H), 1.16 - 1.10 (m, 1H), 1.07 (s, 3H), 0.92 - 0.85 (m, 7H), 0.58 - 0.53 (m, 1H), 0.36 (s, 3H).

### Compound 22'

ESI-MS (m/z): 879.5 [M+H]⁺. LC-MS retention time RT = 1.65 min. HPLC retention time Retention time RT = 12.59 min.

¹H NMR (500 MHz, DMSO-d6) δ 9.26 (d, *J* = 2.1 Hz, 1H), 8.53 (d, *J =* 9.0 Hz, 2H), 8.40 (d, *J =* 2.2 Hz, 1H), 7.82 (s, 1H), 7.75 (dd, *J =* 8.7, 1.5 Hz, 1H), 7.56 (d, *J =* 8.7 Hz, 1H), 7.17 (s, 1H), 5.55 (t, *J* = 9.4 Hz, 1H), 5.10 - 5.05 (m, 1H), 4.27 - 4.21 (m, 2H), 4.07 - 4.02 (m, 1H), 4.02 - 3.95 (m, 1H), 3.89 - 3.82 (m, 1H), 3.76 (s, 2H), 3.67 (d, *J =* 10.8 Hz, 1H), 3.63 - 3.54 (m, 6H), 3.18 - 3.14 (m, 1H), 3.12 (s, 3H), 3.10 - 3.05 (m, 1H), 2.80 - 2.73 (m, 1H), 2.49 - 2.47 (m, 3H), 2.40 - 2.35 (m, 1H), 2.15 - 2.10 (m, 1H), 1.80 (s, 2H), 1.55 - 1.47 (m, 2H), 1.27 (d, *J* = 6.3 Hz, 3H), 1.12 (t, *J* = 7.1 Hz, 3H), 1.07 (s, 4H), 0.92 (s, 3H), 0.88 (dd, *J=* 7.1, 6.1 Hz, 2H), 0.56 (dd, *J* = 7.6, 5.4 Hz, 1H), 0.49 (s, 3H).

### Example 23

### (1S,2S)-N-((6³S,4S,Z)-1¹-ethyl-1²-(2-((S)-1-methoxyethyl)-5-(5-((methylamino)methyl)isoxazol-3-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

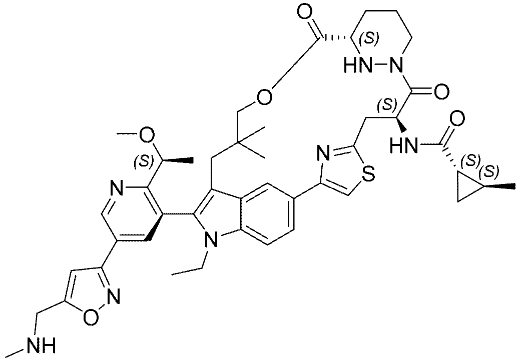

Example 23 was prepared by the following steps:

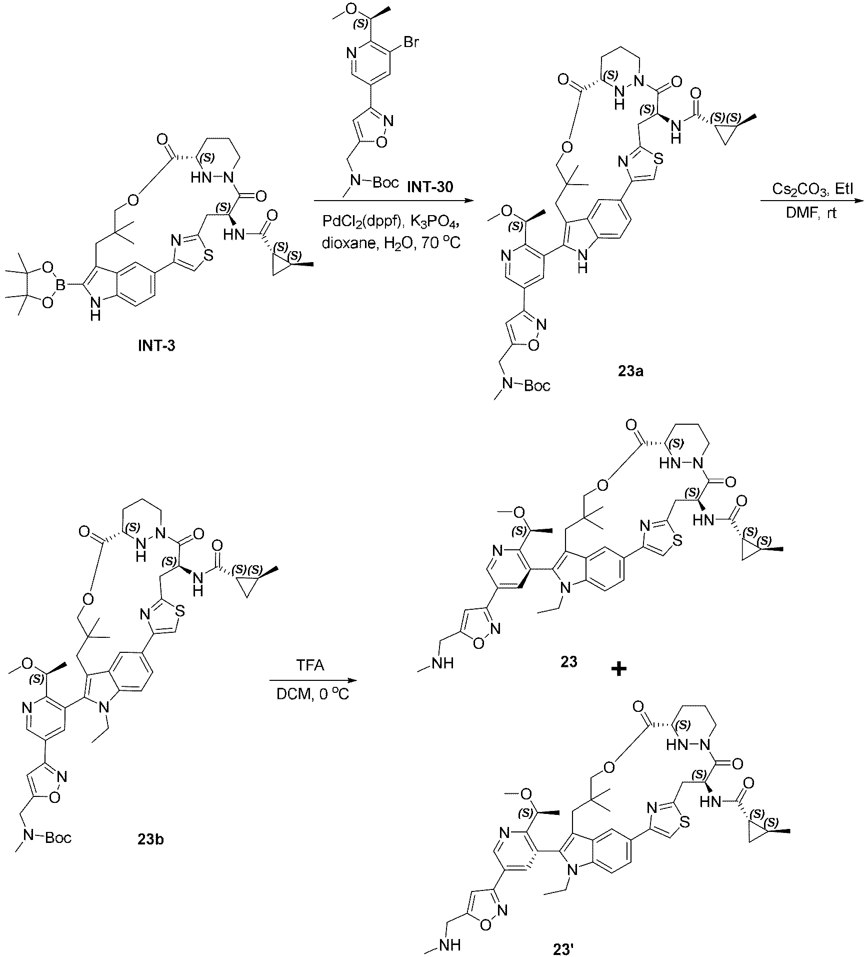

By replacing INT-7 in the synthesis step of compound 2 with INT-30, compound 23 and its diastereoisomer compound 23' can be obtained using similar methods and reaction steps. The absolute configurations of the two compounds are drawn based on the empirical assumption that compound 23 is the isomer with a longer retention time in LCMS or HPLC, while 23' is the isomer with a shorter retention time in LCMS or HPLC.

### Compound 23

ESI-MS (m/z): 823.2 [M+H]⁺; LC-MS retention time RT = 1.68 min. HPLC retention time RT= 12.07 min.

¹H NMR (500 MHz, DMSO-*d6*) δ 9.24 (d, *J* = 2.0 Hz, 1H), 8.55 - 8.50 (m, 2H), 8.24 (d, *J* = 2.0 Hz, 1H), 7.81 (s, 1H), 7.77 (dd, *J* = 8.5, 1.5 Hz, 1H), 7.60 (d, *J* = 8.5 Hz, 1H), 7.13 (s, 1H), 5.56 (t, *J =* 9.0 Hz, 1H), 5.10 - 5.05 (m, 1H), 4.42 - 4.31 (m, 2H), 4.28 - 4.08 (m, 3H), 3.84 (s, 2H), 3.58 (s, 2H), 3.32 (s, 2H), 3.28 (s, 3H), 3.18 - 3.12 (m, 1H), 3.00 - 2.95 (m, 1H), 2.81- 2.82 (m, 1H), 2.45 - 2.40 (m, 1H), 2.33 (s, 3H), 2.10 - 2.05 (m, 1H), 1.80 - 1.75 (m, 2H), 1.57 - 1.46 (m, 2H), 1.40 (d, *J* = 6.0 Hz, 3H), 1.25 - 1.20 (m, 1H), 1.12 - 1.01 (m, 4H), 0.93 - 0.83 (m, 6H), 0.59 - 0.52 (m, 1H), 0.36 (s, 3H).

### Compound 23'

ESI-MS (m/z): 823.3 [M+H]⁺; LC-MS retention time RT=1.67 min. HPLC retention time RT = 11.92 min.

¹H NMR (500 MHz, DMSO-d6) δ 9.25 (d, *J =* 2.0 Hz, 1H), 8.55 - 8.50 (m, 2H), 8.38 (d, *J* = 2.0 Hz, 1H), 7.82 (s, 1H), 7.75 (dd, *J* = 8.5, 1.5 Hz, 1H), 7.56 (d, *J* = 8.5 Hz, 1H), 7.07 (s, 1H), 5.55 (t, *J =* 9.0 Hz, 1H), 5.10 - 5.05 (m, 1H), 4.27 - 4.18 (m, 2H), 4.05 - 3.96 (m, 2H), 3.89 - 3.81 (m, 3H), 3.70 - 3.65 (m, 1H), 3.58 - 3.55 (m, 1H), 3.31 (s, 2H), 3.19 - 3.14 (m, 1H), 3.12 (s, 3H), 3.10 - 3.05 (m, 1H), 2.82 - 2.73 (m, 1H), 2.40 - 2.35 (m, 1H), 2.33 (s, 3H), 2.15 - 2.10 (m, 1H), 1.83 - 1.77 (m, 2H), 1.55 - 1.47 (m, 2H), 1.26 (d, *J* = 6.0 Hz, 3H), 1.25 - 1.20 (m, 1H), 1.12 (t, *J* = 7.0 Hz, 3H), 1.07 (s, 3H), 0.92 (s, 3H), 0.90 - 0.84 (m, 1H), 0.58 - 0.53 (m, 1H), 0.49 (s, 3H).

### Example 24

### (1S,2S)-N-((6³S,4S,Z)-1¹-ethyl-1²-(2-((S)-1-methoxyethyl)-5-(5-((R)-1-methylpyrrolidin-2-yl)isoxazol-3-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

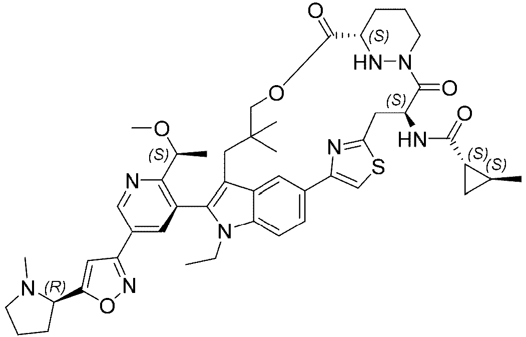

Example 24 was prepared by the following steps:

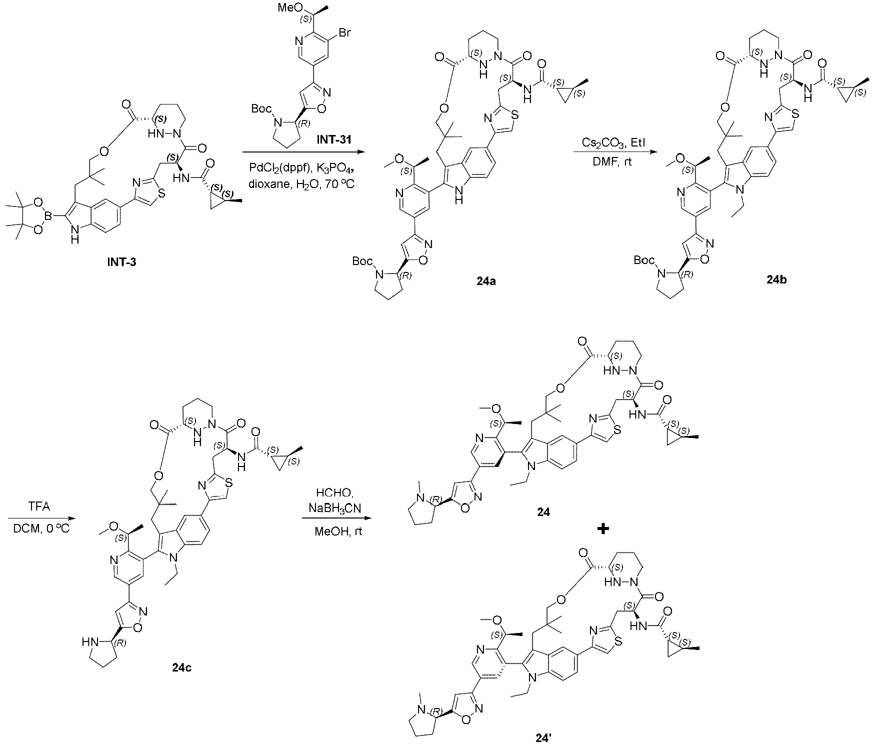

By replacing INT-7 in the synthesis step of compound 2 with INT-31, compound 24 and its diastereoisomer compound 24' can be obtained using similar methods and reaction steps. The absolute configurations of the two compounds are drawn based on the empirical assumption that compound 24 is the isomer with a longer retention time in LCMS or HPLC, while 24' is the isomer with a shorter retention time in LCMS or HPLC.

### Compound 24

ESI-MS (m/z): 863.4 [M+H]⁺; LC-MS retention time RT = 1.94 min. HPLC retention time RT = 14.35 min.

¹H NMR (500 MHz, DMSO-d6) δ 9.24 (d, *J =* 2.0 Hz, 1H), 8.55 - 8.50 (m, 2H), 8.25 (d, *J* = 2.0 Hz, 1H), 7.82 (s, 1H), 7.76 (dd, *J* = 8.5, 1.5 Hz, 1H), 7.60 (d, *J* = 8.5 Hz, 1H), 7.17 (s, 1H), 5.56 (t, *J* = 9.0 Hz, 1H), 5.10 - 5.05 (m, 1H), 4.41 - 4.31 (m, 2H), 4.28 - 4.16 (m, 2H), 4.16 - 4.08 (m, 1H), 3.58 (s, 2H), 3.56 - 3.52 (m, 1H), 3.28 (s, 3H), 3.17 - 3.11 (m, 1H), 3.09 - 3.03 (m, 1H), 3.00 - 2.95 (m, 1H), 2.79 - 2.71 (m, 1H), 2.45 - 2.40 (m, 1H), 2.37 - 2.31 (m, 1H), 2.28 (s, 3H), 2.27 - 2.21 (m, 1H), 2.10 - 2.05 (m, 1H), 1.94 - 1.74 (m, 5H), 1.56 - 1.47 (m, 2H), 1.40 (d, *J =* 6.0 Hz, 3H), 1.25 - 1.20 (m, 1H), 1.10 - 1.05 (m, 4H), 0.95 - 0.82 (m, 6H), 0.57 - 0.52 (m, 1H), 0.36 (s, 3H).

### Compound 24'

ESI-MS (m/z): 863.4 [M+H]⁺; LC-MS retention time RT = 1.89 min. HPLC retention time RT = 13.82 min.

¹H NMR (500 MHz, DMSO-*d6*) δ 9.26 (d, *J* = 2.0 Hz, 1H), 8.56 - 0.50 (m, 2H), 8.40 (d, *J* = 2.0 Hz, 1H), 7.82 (s, 1H), 7.75 (dd, *J* = 8.5, 1.5 Hz, 1H), 7.56 (d, *J* = 8.5 Hz, 1H), 7.15 (s, 1H), 5.55 (t, *J* = 9.0 Hz, 1H), 5.10 - 5.05 (m, 1H), 4.30 - 4.18 (m, 2H), 4.03 - 3.98 (m, 2H), 3.88 - 3.85 (m, 1H), 3.70 - 3.65 (m, 1H), 3.61 - 3.50 (m, 2H), 3.32 - 3.30 (m, 1H), 3.19 - 3.14 (m, 1H), 3.12 (s, 3H), 3.10 - 3.02 (m, 2H), 2.80 - 2.75 (m, 1H), 2.40 - 2.32 (m, 2H), 2.29 (s, 3H), 2.27 - 2.21 (m, 1H), 2.15 - 2.10 (m, 1H), 1.94 - 1.75 (m, 5H), 1.57 - 1.47 (m, 2H), 1.26 (d, *J =* 6.0 Hz, 3H), 1.25 - 1.20 (m, 1H), 1.12 (t, *J* = 7.0 Hz, 3H), 1.07 (s, 3H), 0.96 - 0.82 (m, 4H), 0.59 - 0.53 (m, 1H), 0.49 (s, 3H).

### Example 25

### (1S,2S)-N-((6³S,4S,Z)-1¹-ethyl-1²-(2-((S)-1-methoxyethyl)-5-(5-((S)-1-methylpyrrolidin-2-yl)isoxazol-3-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

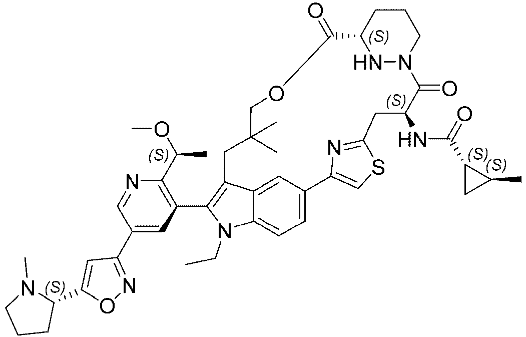

Example 25 was prepared by the following steps:

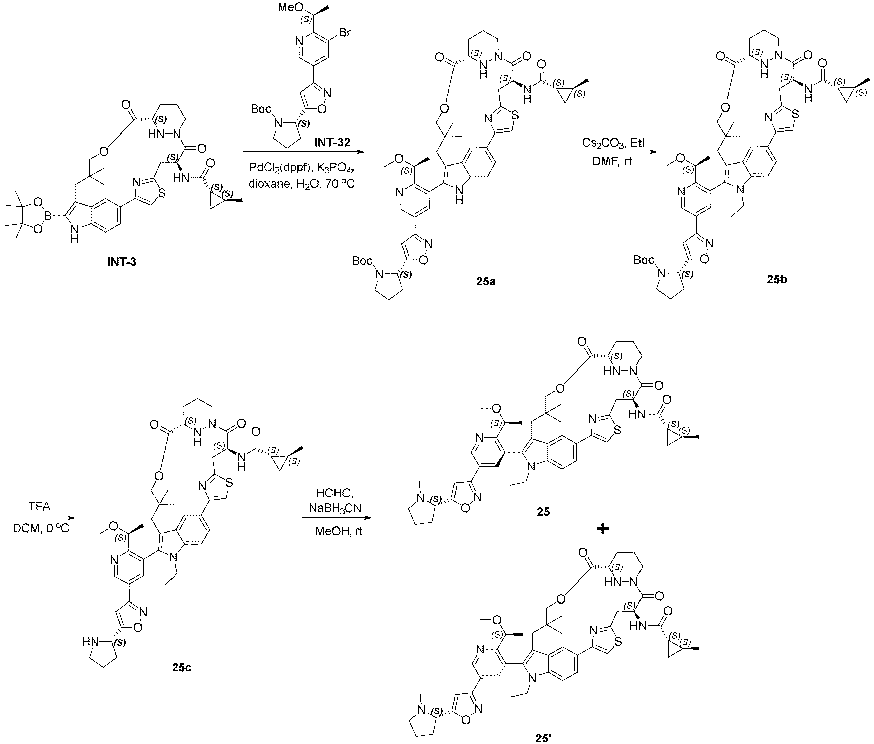

By replacing INT-7 in the synthesis step of compound 2 with INT-32, compound 25 and its diastereoisomer compound 25' can be obtained using similar methods and reaction steps. The absolute configurations of the two compounds are drawn based on the empirical assumption that compound 25 is the isomer with a longer retention time in LCMS or HPLC, while 25' is the isomer with a shorter retention time in LCMS or HPLC.

### Compound 25

ESI-MS (m/z): 863.5 [M+H]⁺; LC-MS retention time RT = 1.98 min. HPLC retention time RT = 14.36 min.

¹H NMR (500 MHz, DMSO-d6) δ 9.26 (d, *J* = 2.0 Hz, 1H), 8.54 (d, *J* = 1.5 Hz, 1H), 8.52 (d, *J* = 9.0 Hz, 1H), 8.40 (d, *J* = 2.0 Hz, 1H), 7.81 (s, 1H), 7.75 (dd, *J* = 9.0, 1.5 Hz, 1H), 7.56 (d, *J* = 9.0 Hz, 1H), 7.15 (s, 1H), 5.55 (t, *J =* 9.0 Hz, 1H), 5.10 - 5.05 (m, 1H), 4.27 - 4.18 (m, 2H), 4.06 - 3.95 (m, 2H), 3.90 - 3.81 (m, 1H), 3.70 - 3.65 (m, 1H), 3.61 (s, 1H), 3.59 - 3.53 (m, 2H), 3.19 - 3.15 (m, 1H), 3.12 (s, 3H), 3.09 - 3.03 (m, 2H), 2.80 - 2.73 (m, 1H), 2.40 - 2.32 (m, 2H), 2.28 (s, 3H), 2.27 - 2.23 (m, 1H), 2.14 - 2.09 (m, 1H), 1.95 - 1.78 (m, 5H), 1.54 - 1.48 (m, 2H), 1.26 (d, *J* = 6.0 Hz, 3H), 1.26 - 1.22 (m, 1H), 1.12 (t, *J* = 7.0 Hz, 3H), 1.07 (s, 3H), 0.92 (s, 3H), 0.90 - 0.86 (m, 1H), 0.58 - 0.53 (m, 1H), 0.49 (s, 3H).

### Compound 25'

ESI-MS (m/z): 863.5 [M+H]⁺. LC-MS retention time RT = 1.94 min. HPLC retention time RT = 13.91 min.

¹H NMR (500 MHz, DMSO-d6) δ 9.25 (d, *J* = 2.0 Hz, 1H), 8.53 - 8.48 (m, 2H), 8.25 (d, *J* = 2.0 Hz, 1H), 7.81 (s, 1H), 7.76 (dd, *J* = 8.5, 1.5 Hz, 1H), 7.60 (d, *J* = 8.5 Hz, 1H), 7.17 (s, 1H), 5.56 (t, *J* = 9.0 Hz, 1H), 5.10 - 5.05 (m, 1H), 4.38 - 4.32 (m, 2H), 4.28 - 4.16 (m, 3H), 4.15 - 4.10 (m, 1H), 3.60 - 3.52 (m, 3H), 3.28 (s, 3H), 3.18 - 3.11 (m, 2H), 3.08 - 3.03 (m, 1H), 3.02 - 2.97 (m, 2H), 2.80 - 2.73 (m, 1H), 2.48 - 2.43 (m, 1H), 2.36 - 2.32 (m, 1H), 2.29 (s, 3H), 2.26 - 2.22 (m, 1H), 2.10 - 2.05 (m, 1H), 1.91 - 1.82 (m, 3H), 1.82 - 1.75 (m, 2H), 1.52 - 1.47 (m, 2H), 1.40 (d, *J* = 6.0 Hz, 3H), 1.25 - 1.20 (m, 1H), 1.06 (s, 3H), 0.91 - 0.86 (m, 6H), 0.57 - 0.52 (m, 1H), 0.36 (s, 3H).

### Example 26

### (1r,2R,3S)-N-((6³S,4S,Z)-1¹-ethyl-1²-(2-((S)-1-methoxyethyl)-5-(5-((R)-4-methylmorpholin-2-yl)isoxazol-3-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

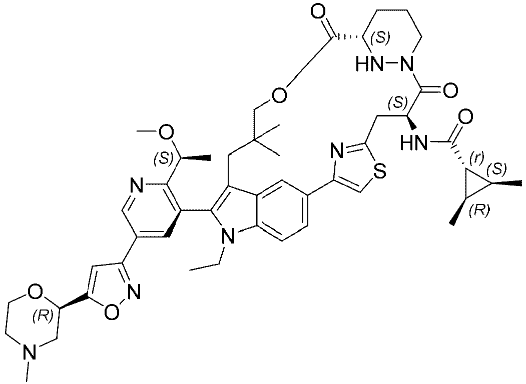

Example 26 was prepared by the following steps:

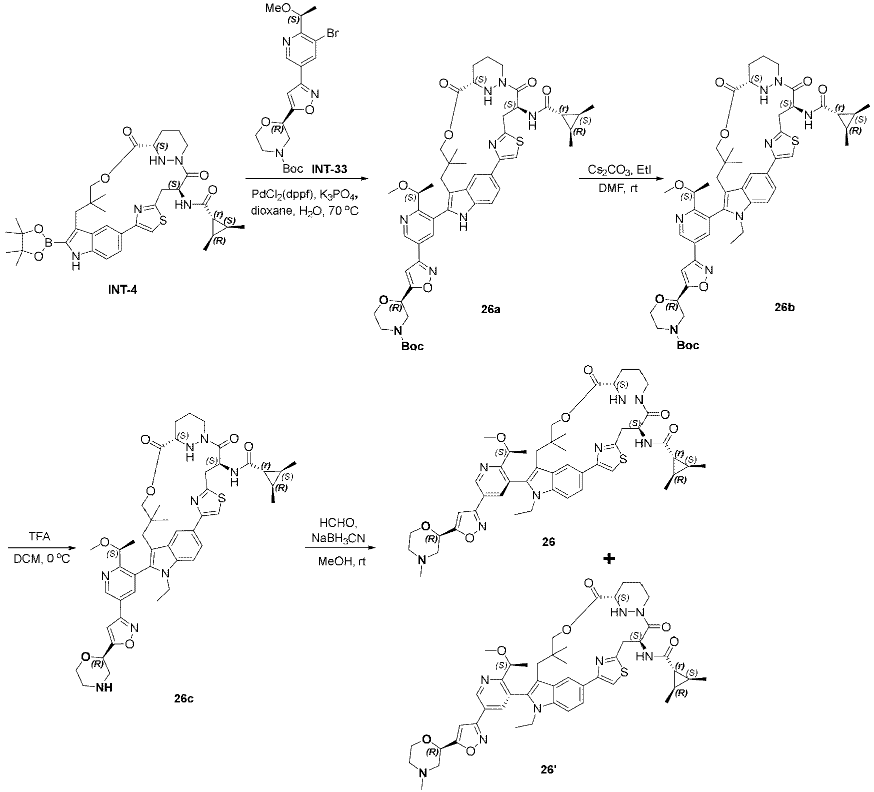

By replacing INT-7 in the synthesis step of compound 2 with INT-33 and INT-3 with INT-4, compound 26 and its diastereoisomer compound 26' can be obtained using similar methods and reaction steps. The absolute configurations of the two compounds are drawn based on the empirical assumption that compound 26 is the isomer with a longer retention time in LCMS or HPLC, while 26' is the isomer with a shorter retention time in LCMS or HPLC.

### Compound 26

ESI-MS (m/z): 893.4 [M+H]⁺. LC-MS retention time RT = 1.85 min. HPLC retention time RT= 13.87 min.

¹H NMR (500 MHz, DMSO-d6) δ 9.25 (d, *J =* 2.0 Hz, 1H), 8.52 (d, *J =* 1.5 Hz, 1H), 8.39 (d, *J =* 9.0 Hz, 1H), 8.27 (d, *J =* 2.0 Hz, 1H), 7.82 (s, 1H), 7.78 - 7.75 (m, 1H), 7.60 (d, *J =* 9.0 Hz, 1H), 7.32 (s, 1H), 5.56 (t, *J =* 9.0 Hz, 1H), 5.10 - 5.05 (m, 1H), 4.83 - 4.78 (m, 1H), 4.39 - 4.32 (m, 2H), 4.25 - 4.20 (m, 1H), 4.21 - 4.15 (m, 1H), 4.15 - 4.08 (m, 1H), 3.92 - 3.87 (m, 1H), 3.73 - 3.68 (m, 1H), 3.58 (s, 2H), 3.28 (s, 3H), 3.19 - 3.14 (m, 1H), 3.14 - 3.11 (m, 1H), 3.00 - 2.93 (m, 2H), 2.80 - 2.70 (m, 1H), 2.65 - 2.60 (m, 1H), 2.47 - 2.41 (m, 1H), 2.30 - 5.05 (m, 1H), 2.24 (s, 3H), 2.18 - 2.12 (m, 1H), 2.09 - 2.04 (m, 1H), 1.81 - 1.75 (m, 2H), 1.53 - 1.47 (m, 1H), 1.40 (d, *J* = 6.0 Hz, 3H), 1.26 - 1.20 (m, 2H), 1.19 - 1.14 (m, 2H), 1.08 (d, *J =* 5.5 Hz, 3H), 1.06 (d, *J* = 5.5 Hz, 3H), 0.93 - 0.85(m, 5H), 0.36 (s, 3H).

### Compound 26'

ESI-MS (m/z): 893.4 [M+H]⁺. LC-MS retention time RT = 1.84 min. HPLC retention time RT= 13.63 min.

¹H NMR (500 MHz, DMSO-d6) δ 9.27 (d, *J =* 2.0 Hz, 1H), 8.54 (d, *J =* 1.5 Hz, 1H), 8.42 - 8.39 (m, 2H), 7.83 (s, 1H), 7.77 - 7.74 (m, 1H), 7.57 (d, *J =* 8.5 Hz, 1H), 7.27 (s, 1H), 5.54 (t, *J =* 9.0 Hz, 1H), 5.07 - 5.02 (m, 1H), 4.84 - 4.80 (m, 1H), 4.26 - 4.20 (m, 2H), 4.07 - 4.03 (m, 1H), 4.03 - 3.96 (m, 1H), 3.91 - 3.87 (m, 1H), 3.87 - 3.80 (m, 1H), 3.74 - 3.68 (m, 1H), 3.68 - 3.65 (m, 1H), 3.59 - 3.55 (m, 1H), 3.32 - 3.28 (m, 1H), 3.19 - 3.15 (m, 1H), 3.13 (s, 3H), 3.10 - 3.05 (m, 1H), 2.98 - 2.95 (m, 1H), 2.80 - 2.72 (m, 1H), 2.66 - 2.60 (m, 1H), 2.40 - 2.35 (m, 1H), 2.31 - 2.25 (m, 1H), 2.25 (s, 3H), 2.19 - 2.15 (m, 1H), 2.14 - 2.09 (m, 1H), 1.79 (s, 2H), 1.57 - 1.48 (m, 1H), 1.26 (d, *J =* 6.0 Hz, 3H), 1.24 - 1.20 (m, 1H), 1.20 - 1.16 (m, 2H), 1.12 (t, *J* = 7.0 Hz, 3H), 1.10 (d, *J=* 6.0 Hz, 3H), 1.07 (d, *J=* 5.5 Hz, 3H), 0.92 (s, 3H), 0.48 (s, 3H).

### Example 27

### (1r,2R,3S)-N-((6³S,4S,Z)-1¹-ethyl-1²-(2-((S)-1-methoxyethyl)-5-(5-((S)-4-methylmorpholin-2-yl)isoxazol-3-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

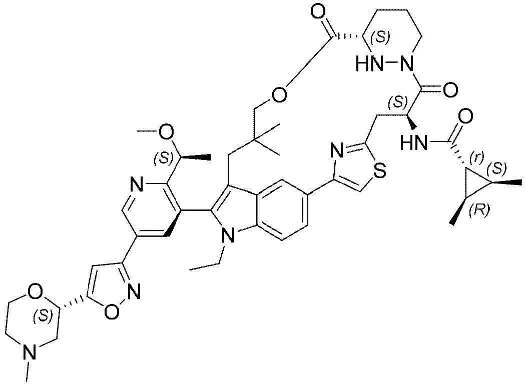

Example 27 was prepared by the following steps:

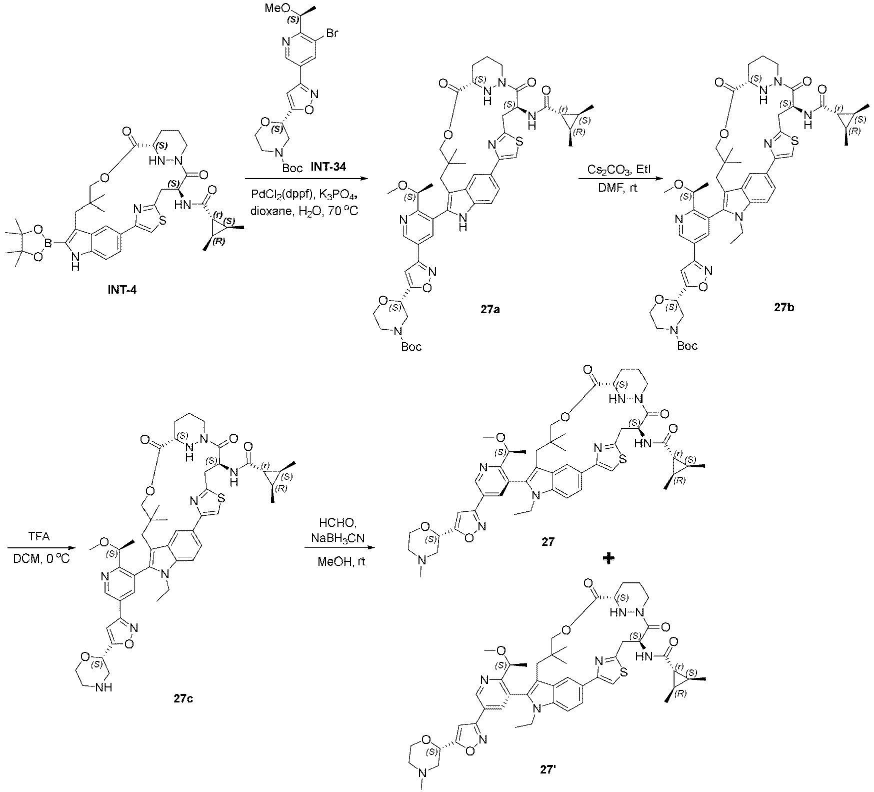

By replacing INT-7 in the synthesis step of compound 2 with INT-34 and INT-3 with INT-4, compound 27 and its diastereoisomer compound 27' can be obtained using similar methods and reaction steps. The absolute configurations of the two compounds are drawn based on the empirical assumption that compound 27 is the isomer with a longer retention time in LCMS or HPLC, while 27' is the isomer with a shorter retention time in LCMS or HPLC.

### Compound 27

ESI-MS (m/z): 893.4 [M+H]⁺. LC-MS retention time RT = 1.85 min. HPLC retention time RT= 13.88 min.

¹H NMR (500 MHz, DMSO-d6) δ 9.25 (d, *J =* 2.0 Hz, 1H), 8.52 (d, *J =* 1.5 Hz, 1H), 8.39 (d, *J =* 8.5 Hz, 1H), 8.27 (d, *J =* 2.0 Hz, 1H), 7.82 (s, 1H), 7.76 (dd, *J* = 8.5, 1.5 Hz, 1H), 7.60 (d, *J =* 8.5 Hz, 1H), 7.33 (s, 1H), 5.56 (t, *J =* 9.0 Hz, 1H), 5.09 - 5.04 (m, 1H), 4.85 - 4.80 (m, 1H), 4.39 - 4.32 (m, 2H), 4.26 - 4.20 (m, 2H), 4.19 - 4.15 (m, 1H), 4.14 - 4.07 (m, 1H), 3.91 - 3.86 (m, 1H), 3.73 - 3.68 (m, 1H), 3.58 (s, 2H), 3.28 (s, 3H), 3.18 - 3.11 (m, 2H), 2.99 - 2.94 (m, 2H), 2.78 - 2.73 (m, 1H), 2.66 - 2.61 (m, 1H), 2.47 - 2.41 (m, 1H), 2.30 - 2.26 (m, 1H), 2.25 (s, 3H), 2.18 - 2.11 (m, 1H), 2.09 - 2.04 (m, 1H), 1.81 - 1.76 (m, 2H), 1.54 - 1.48 (m, 1H), 1.40 (d, *J=* 6.0 Hz, 3H), 1.27 - 1.22 (m, 1H), 1.19 - 1.14 (m, 2H), 1.08 (d, *J* = 5.9 Hz, 3H), 1.06 (d, *J =* 5.5 Hz, 3H), 0.92 - 0.85 (m, 5H), 0.37 (s, 3H).

### Compound 27'

ESI-MS (m/z): 893.4 [M+H]⁺. LC-MS retention time RT = 1.84 min. HPLC retention time RT = 13.69 min.

¹H NMR (500 MHz, DMSO-d6) δ 9.27 (d, *J =* 2.0 Hz, 1H), 8.54 (s, 1H), 8.42 - 8.38 (m, 2H), 7.83 (s, 1H), 7.75 (dd, *J* = 8.5*,* 1.5 Hz, 1H), 7.56 (d, *J =* 8.5 Hz, 1H), 7.27 (s, 1H), 5.54 (t, *J* = 9.0 Hz, 1H), 5.07 - 5.02 (m, 1H), 4.85 - 4.80 (m, 1H), 4.27 - 4.20 (m, 2H), 4.07 - 4.03 (m, 1H), 4.03 - 3.96 (m, 1H), 3.92 - 3.88 (m, 1H), 3.88 - 3.81 (m, 1H), 3.74 - 3.69 (m, 1H), 3.70 - 3.65 (m, 1H), 3.59 - 3.54 (m, 1H), 3.56 - 3.30 (s, 2H), 3.19 - 3.15 (m, 1H), 3.13 (s, 3H), 3.10 - 3.05 (m, 1H), 3.00 - 2.95 (m, 1H), 2.80 - 2.73 (m, 1H), 2.65 - 2.60 (m, 1H), 2.40 - 2.35 (m, 1H), 2.25 (s, 3H), 2.19 - 2.15 (m, 1H), 2.14 - 2.09 (m, 1H), 1.79 (s, 2H), 1.56 - 1.48 (m, 1H), 1.27 (d, *J =* 6.0 Hz, 3H), 1.23 - 1.15 (m, 3H), 1.12 (t, *J* = 7.0 Hz, 3H), 1.10 (d, *J =* 6.0 Hz, 3H), 1.07 (d, *J =* 5.5 Hz, 3H), 0.92 (s, 3H), 0.48 (s, 3H).

### Example 28

### (1r,2R,3S)-N-((6³S,4S,Z)-1¹-ethyl-1²-(2-((S)-1-methoxyethyl)-5-(5-((S)-1-methylpyrrolidin-3-yl)isoxazol-3-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

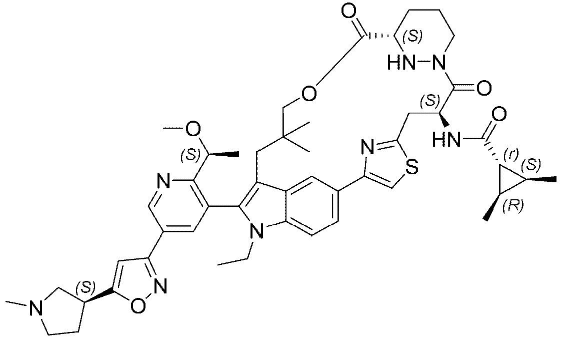

Example 28 was prepared by the following steps:

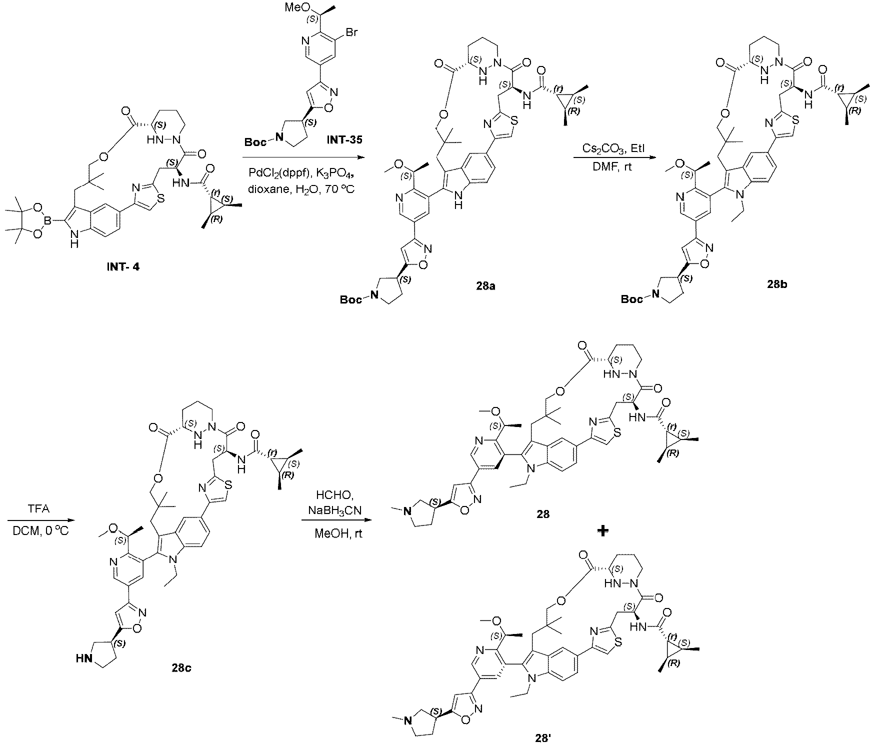

By replacing INT-7 in the synthesis step of compound 2 with INT-35 and INT-3 with INT-4, compound 28 and its diastereoisomer compound 28' can be obtained using similar methods and reaction steps. The absolute configurations of the two compounds are drawn based on the empirical assumption that compound 28 is the isomer with a longer retention time in LCMS or HPLC, while 28' is the isomer with a shorter retention time in LCMS or HPLC.

### Compound 28

ESI-MS (m/z): 877.4 [M+H]⁺. LC-MS retention time RT = 1.82 min.

### Compound 28'

ESI-MS (m/z): 877.4 [M+H]⁺. LC-MS retention time RT = 1.78 min.

### Example 29

### (1r,2R,3S)-N-((6³S,4S,Z)-1¹-ethyl-1²-(2-((S)-1-methoxyethyl)-5-(5-((R)-1-methylpyrrolidin-3-yl)isoxazol-3-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

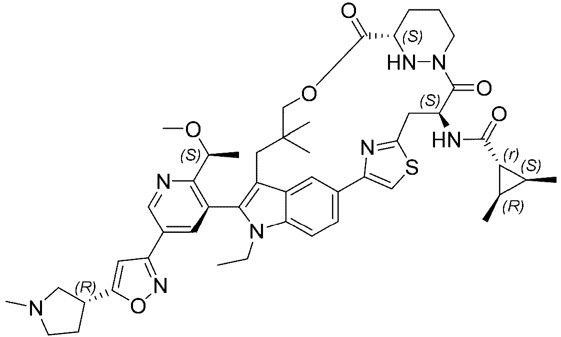

Example 29 was prepared by the following steps:

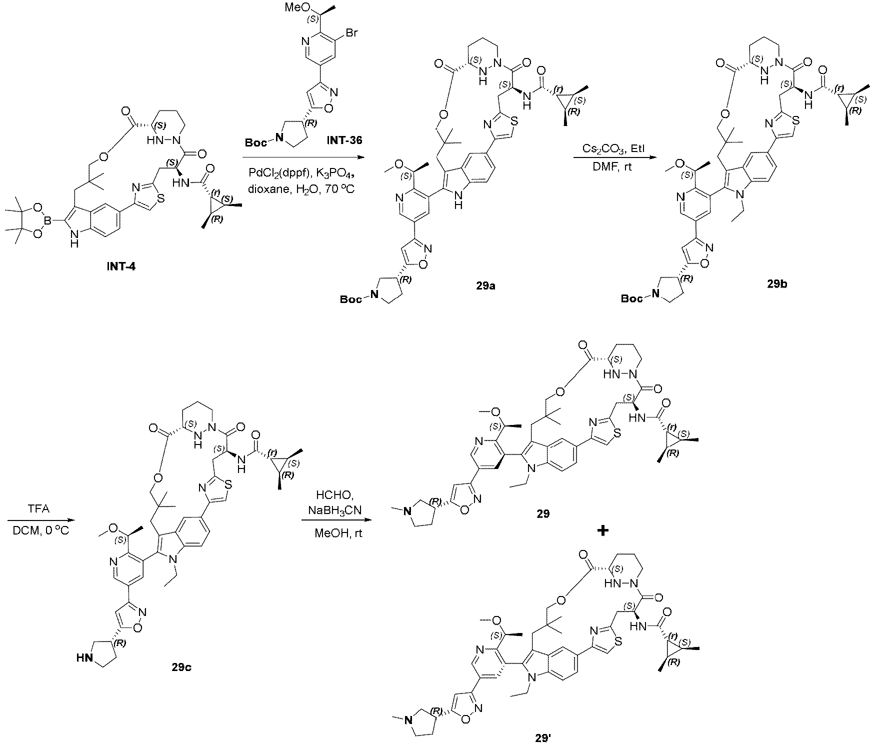

By replacing INT-7 in the synthesis step of compound 2 with INT-36 and INT-3 with INT-4, compound 29 and its diastereoisomer compound 29' can be obtained using similar methods and reaction steps. The absolute configurations of the two compounds are drawn based on the empirical assumption that compound 29 is the isomer with a longer retention time in LCMS or HPLC, while 29' is the isomer with a shorter retention time in LCMS or HPLC.

### Compound 29:

ESI-MS (m/z): 877.4 [M+H]⁺. LC-MS retention time RT = 1.87 min. HPLC retention time RT = 14.12 min.

¹H NMR (500 MHz, DMSO-d6) δ 9.22 (d, *J =* 2.0 Hz, 1H), 8.53 - 8.49 (m, 2H), 8.23 (d, *J =* 2.0 Hz, 1H), 7.82 (s, 1H), 7.78 - 7.75 (m, 1H), 7.60 (d, *J =* 8.5 Hz, 1H), 7.15 (s, 1H), 5.55 (t, *J =* 9.0 Hz, 1H), 5.09 - 5.03 (m, 1H), 4.37 - 4.33 (m, 2H), 4.25 - 4.19 (m, 3H), 4.14 - 4.08 (m, 1H), 3.59 - 3.55 (m, 3H), 3.28 (s, 3H), 3.00 - 2.95 (m, 2H), 2.75 - 2.71 (m, 2H), 2.61 - 2.57 (m, 3H), 2.42 - 2.40 (m, 1H), 2.37 - 2.35 (m, 1H), 2.28 - 2.27 (m, 3H), 2.04 - 1.99 (m, 2H), 1.80 - 1.76 (m, 3H), 1.40 (d, *J =* 6.0 Hz, 3H), 1.37 - 1.33 (m, 2H), 1.33 - 1.29 (m, 2H), 1.26 (s, 3H), 1.18 - 1.52 (m, 2H), 0.93 - 0.86 (m, 3H), 0.84 (t, *J =* 7.0 Hz, 3H), 0.36 (s, 3H).

### Compound 29'

ESI-MS (m/z): 877.4 [M+H]⁺. LC-MS retention time RT = 1.84 min. HPLC retention time RT = 13.74 min.

### Example 30

### (1S,2S)-N-((6³S,4S,Z)-1¹-ethyl-1²-(2-((S)-1-methoxyethyl)-5-(5-(1-methylpiperidin-4-yl)isoxazol-3-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

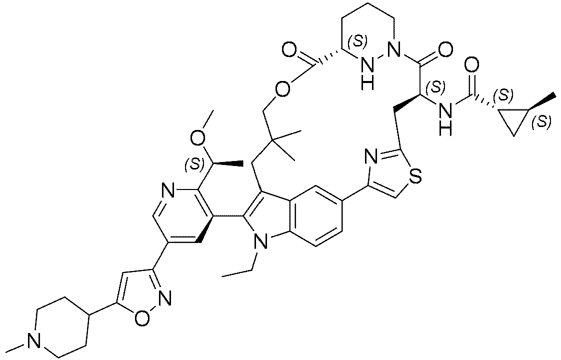

Example 30 was prepared by the following steps:

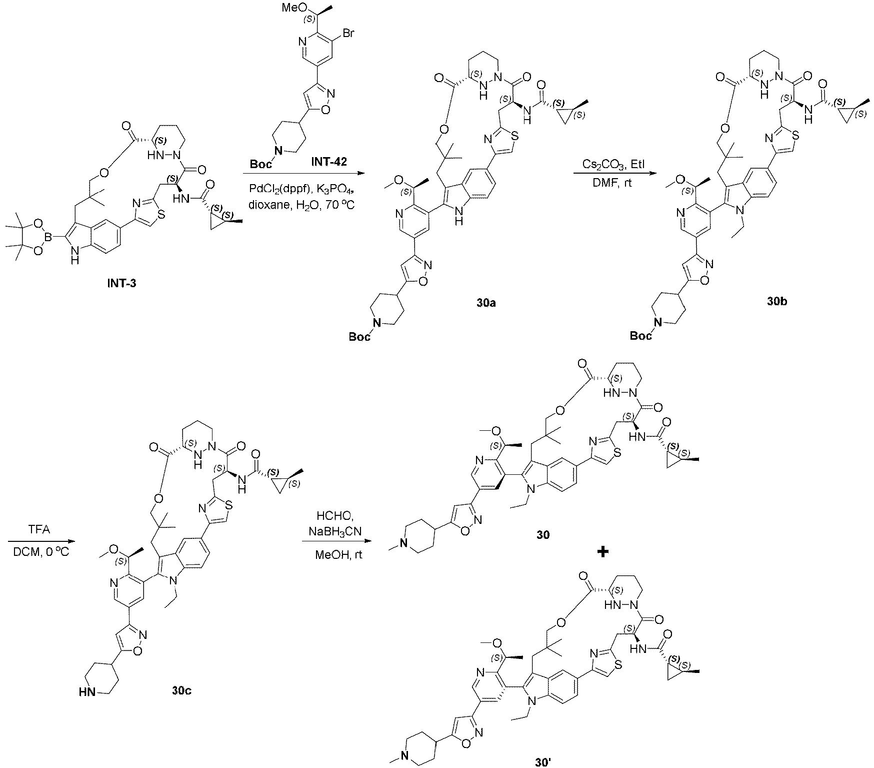

By replacing INT-7 in the synthesis step of compound 2 with INT-42 and INT-3 with INT-4, compound 30 and its diastereoisomer compound 30' can be obtained using similar methods and reaction steps. The absolute configurations of the two compounds are drawn based on the empirical assumption that compound 30 is the isomer with a longer retention time in LCMS or HPLC, while 30' is the isomer with a shorter retention time in LCMS or HPLC.

### Compound 30

ESI-MS (m/z): 891.5 [M+H]⁺; LC-MS retention time RT = 1.83 min.

### Compound 30'

ESI-MS (m/z): 891.5 [M+H]⁺; LC-MS retention time RT = 1.78 min.

### Example 31

### (1S,2S)-N-((6³S,4S,Z)-1¹-ethyl-1²-(2-((S)-1-methoxyethyl)-5-(5-(1-methylazetidin-3-yl)isoxazol-3-yl)pyridin-3-yl)-10,10-dimethyl-5,7-dioxo-6¹,6²,6³,6⁴,6⁵,6⁶-hexahydro-1¹H 8-oxa-2(4,2)-thiazola-1(5,3)-indola-6(1,3)-pyridazinacycloundecaphane-4-yl)-2-methylcyclopropane-1-carboxamide

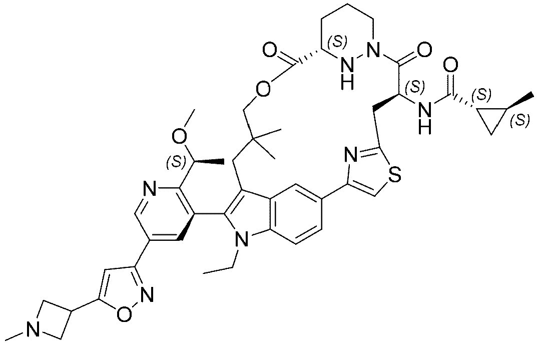

Example 31 was prepared by the following steps:

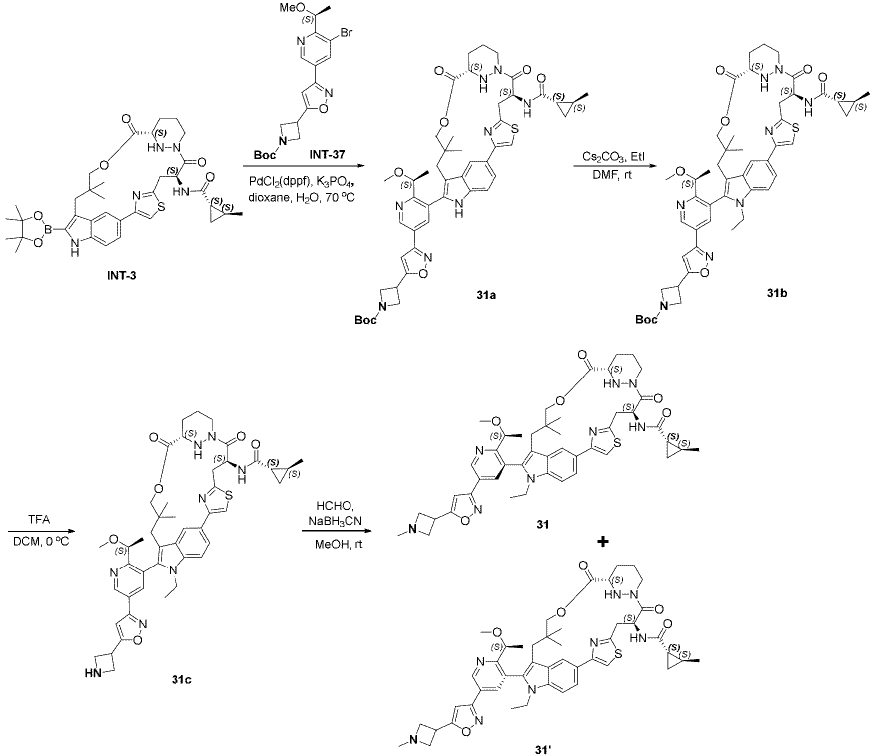

By replacing INT-7 in the synthesis step of compound 2 with INT-37, compound 31 and its diastereoisomer compound 31' can be obtained using similar methods and reaction steps. The absolute configurations of the two compounds are drawn based on the empirical assumption that compound 31 is the isomer with a longer retention time in LCMS or HPLC, while 31' is the isomer with a shorter retention time in LCMS or HPLC.

### Compound 31

ESI-MS (m/z): 863.5 [M+H]⁺; LC-MS retention time RT = 1.75 min.

### Compound 31'

ESI-MS (m/z): 863.5 [M+H]⁺; LC-MS retention time RT = 1.73 min.

### Example 32

### (1r,2R,3S)-N-((6³S,4S,Z)-1¹-ethyl-1²-(2-((S)-1-methoxyethyl)-5-(5-((S)-4-methylmorpholin-3-yl)isoxazol-3-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

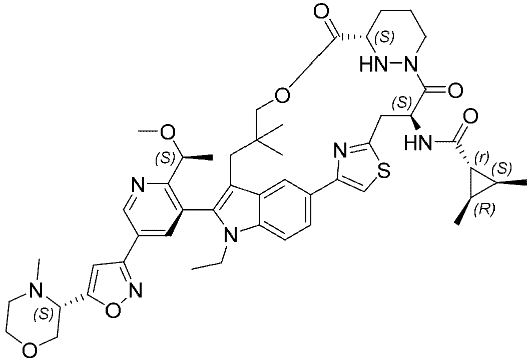

Example 32 was prepared by the following steps:

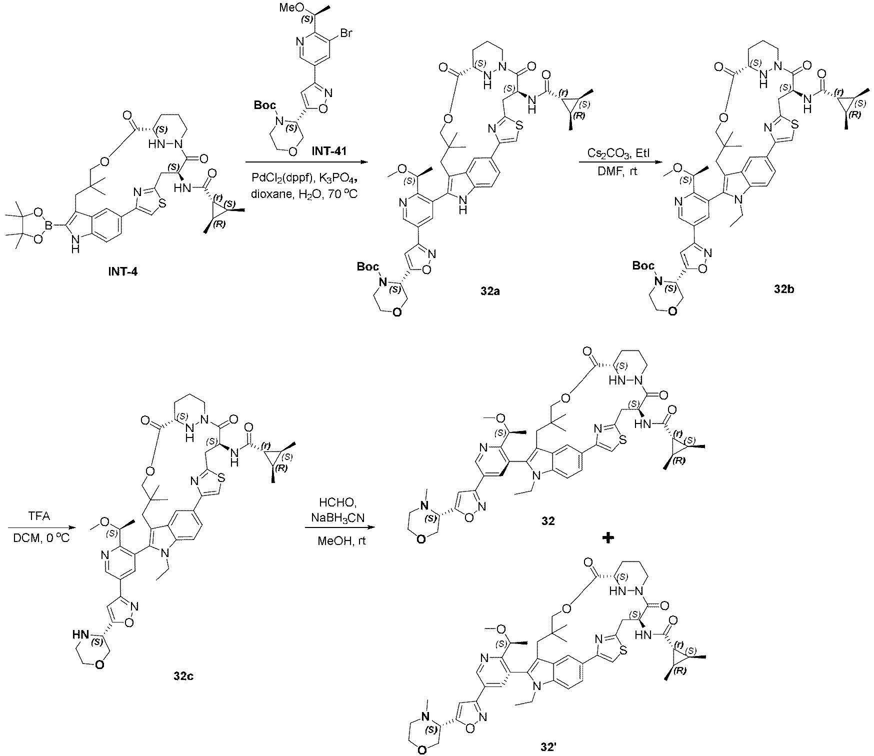

By replacing INT-7 in the synthesis step of compound 2 with INT-41 and INT-3 with INT-4, compound 32 and its diastereoisomer compound 32' can be obtained using similar methods and reaction steps. The absolute configurations of the two compounds are drawn based on the empirical assumption that compound 32 is the isomer with a longer retention time in LCMS or HPLC, while 32' is the isomer with a shorter retention time in LCMS or HPLC.

### Compound 32

ESI-MS (m/z): 893.5 [M+H]⁺; LC-MS retention time RT = 1.85 min. HPLC retention time RT = 13.59 min.

¹H NMR (500 MHz, DMSO-*d*₆) δ 9.26 (d, *J =* 2.5 Hz, 1H), 8.52 (d, *J =* 1.5 Hz, 1H), 8.39 (d, *J =* 9.0 Hz, 1H), 8.27 (d, *J =* 2.5 Hz, 1H), 7.82 (s, 1H), 7.80 - 7.74 (m, 1H), 7.60 (d, *J =* 8.6 Hz, 1H), 7.30 (d, *J =* 2.5 Hz, 1H), 5.56 (t, *J =* 9.0 Hz, 1H), 5.09 - 5.05 (m, 1H), 4.39 - 4.31 (m, 2H), 4.26 - 4.11 (m, 3H), 3.88 - 3.76 (m, 3H), 3.69 - 3.61 (m, 2H), 3.58 (d, *J =* 5.0 Hz, 3H), 3.33 - 3.29 (m, 1H), 3.28 (s, 3H), 3.18 - 3.12 (m, 1H), 3.02 - 2.96 (m, 1H), 2.82 - 2.71 (m, 2H), 2.47 - 2.42 (m, 1H), 2.36 - 2.29 (m, 1H), 2.14 (s, 3H), 1.80 - 1.75 (m, 2H), 1.55 - 1.48 (m, 1H), 1.40 (d, *J =* 6.0 Hz, 3H), 1.26 - 1.21 (m, 1H), 1.20 - 1.14 (m, 3H), 1.10 - 1.05 (m, 6H), 0.91 - 0.87 (m, 5H), 0.37 (s, 3H).

### Compound 32'

ESI-MS (m/z): 893.5 [M+H]⁺; LC-MS retention time RT = 1.83 min. HPLC retention time RT = 13.38 min.

¹H NMR (500 MHz, DMSO-*d*₆) δ 9.27 (t, *J* = 2.0 Hz, 1H), 8.55 (d, *J =* 1.5 Hz, 1H), 8.45 - 8.39 (m, 2H), 7.83 (s, 1H), 7.75 (dd, *J =* 8.5, 1.5 Hz, 1H), 7.56 (d, *J =* 8.5 Hz, 1H), 7.26 (d, *J =* 2.0 Hz, 1H), 5.54 (t, *J =* 9.0 Hz, 1H), 5.07 - 5.02 (m, 1H), 4.27 - 4.18 (m, 2H), 4.07 - 3.95 (m, 2H), 3.89 - 3.78 (m, 3H), 3.69 - 3.63 (m, 2H), 3.60 - 3.55 (m, 3H), 3.32 - 3.29 (m, 2H), 3.20 - 3.15 (m, 1H), 3.14 - 3.11 (m, 3H), 3.10 - 3.05 (m, 1H), 2.80 - 2.73 (m, 2H), 2.40 - 2.30 (m, 2H), 2.14 (d, *J =* 3.0 Hz, 3H), 1.86 - 1.73 (m, 2H), 1.56 - 1.47 (m, 1H), 1.28 - 1.24 (m, 3H), 1.23 - 1.16 (m, 3H), 1.13 - 1.05 (m, 9H), 0.92 (s, 3H), 0.49 (s, 3H).

### Example 33

### (1r,2R,3S)-N-((6³S,4S,Z)-1¹-ethyl-1²-(2-((S)-1-methoxyethyl)-5-(5-((R)-4-methylmorpholin-3-yl)isoxazol-3-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

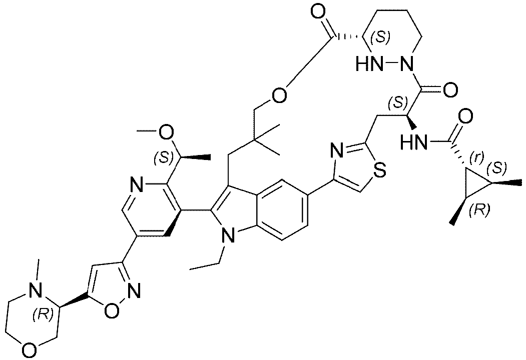

Example 33 was prepared by the following steps:

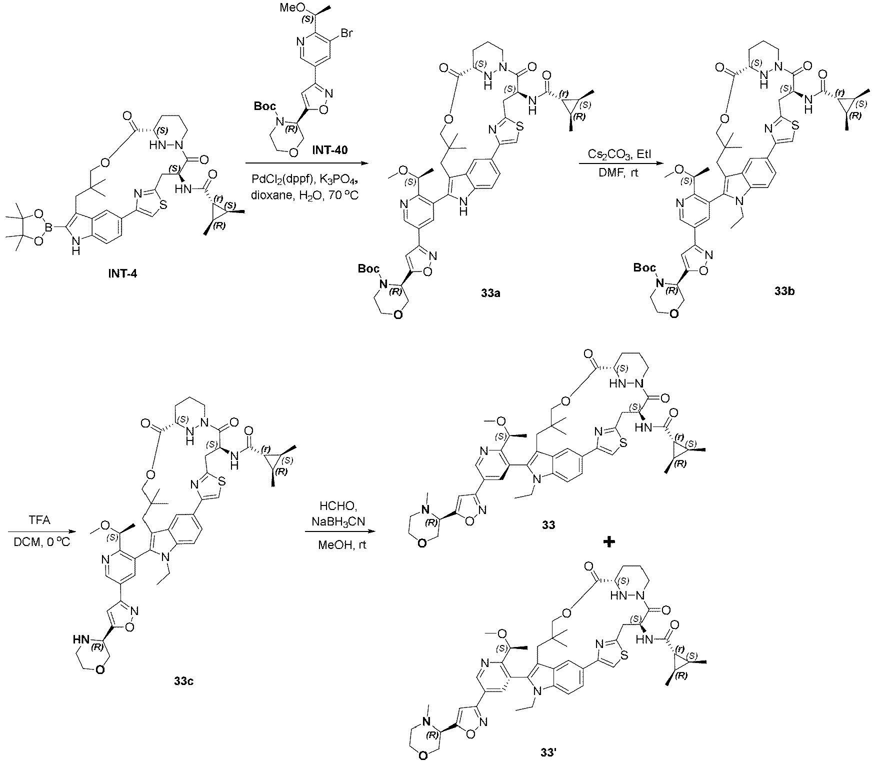

By replacing INT-7 in the synthesis step of compound 2 with INT-40 and INT-3 with INT-4, compound 33 and its diastereoisomer compound 33' can be obtained using similar methods and reaction steps. The absolute configurations of the two compounds are drawn based on the empirical assumption that compound 33 is the isomer with a longer retention time in LCMS or HPLC, while 33' is the isomer with a shorter retention time in LCMS or HPLC.

### Compound 33

ESI-MS (m/z): 893.5 [M+H]⁺; LC-MS retention time RT = 1.85 min. HPLC retention time RT = 13.58 min.

¹H NMR (500 MHz, DMSO-*d*₆) δ 9.26 (d, *J =* 2.5 Hz, 1H), 8.52 (d, *J* = 2.0 Hz, 1H), 8.39 (d, *J =* 9.0 Hz, 1H), 8.27 (d, *J =* 2.0 Hz, 1H), 7.82 (s, 1H), 7.77 (dd, *J =* 9.0, 2.0 Hz, 1H), 7.60 (d, *J* = 9.0 Hz, 1H), 7.30 (d, *J* = 2.5 Hz, 1H), 5.55 (t, *J =* 9.0 Hz, 1H), 5.09 - 5.04 (m, 1H), 4.45 - 4.30 (m, 2H), 4.29 - 4.07 (m, 3H), 3.86 - 3.77 (m, 2H), 3.64 - 3.53 (m, 5H), 3.28 (s, 3H), 3.18 - 3.12 (m, 2H), 3.00 - 2.96 (m, 1H), 2.82 - 2.71 (m, 3H), 2.38 - 2.28 (m, 2H), 2.14 (s, 3H), 2.09 - 1.98 (m, 2H), 1.80 - 1.75 (m, 2H), 1.53 - 1.42 (m, 2H), 1.40 (d, *J =* 6.0 Hz, 3H), 1.10 - 1.05 (m, 6H), 0.92 - 0.86 (m, 6H), 0.37 (s, 3H).

### Compound 33'

ESI-MS (m/z): 893.5 [M+H]⁺; LC-MS retention time RT = 1.83 min. HPLC retention time RT = 13.38 min.

¹H NMR (500 MHz, DMSO-*d*₆) δ 9.29 - 9.25 (m, 1H), 8.54 (d, *J =* 1.5 Hz, 1H), 8.45 - 8.38 (m, 2H), 7.83 (s, 1H), 7.75 (dd, *J =* 8.5, 1.5 Hz, 1H), 7.56 (d, *J =* 8.5 Hz, 1H), 7.26 (d, *J =* 1.5 Hz, 1H), 5.59 - 5.49 (m, 1H), 5.07 - 5.02 (m, 1H), 4.25 - 4.20 (m, 2H), 4.08 - 3.95 (m, 2H), 3.92 - 3.77 (m, 3H), 3.71 - 3.62 (m, 2H), 3.61 - 3.54 (m, 3H), 3.21 - 3.15 (m, 1H), 3.16 - 3.10 (m, 1H), 3.09 - 3.04 (m, 1H), 2.82 - 2.71 (m, 2H), 2.41 - 2.30 (m, 2H), 2.14 (d, *J=* 2.9 Hz, 4H), 1.82 - 1.75 (m, 2H), 1.54 - 1.49 (m, 1H), 1.29 - 1.24 (m, 3H), 1.14 - 1.08 (m, 6H), 1.07 (d, *J =* 6.0 Hz, 3H), 0.92 (s, 3H), 0.49 (s, 3H).

### Example 34 (1r,2R,3S)-N-((6³S,4S,Z)-1²-(5-(5-(1,3-dimethylazetidin-3-yl)isoxazol-3-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

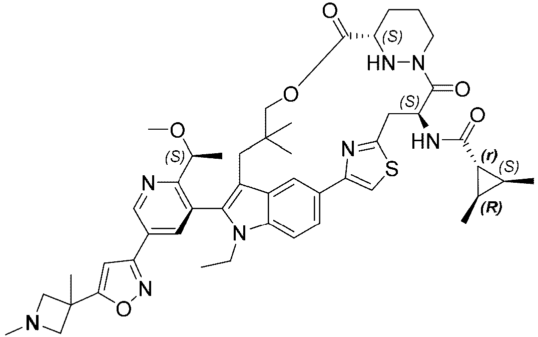

Example 34 was prepared by the following steps:

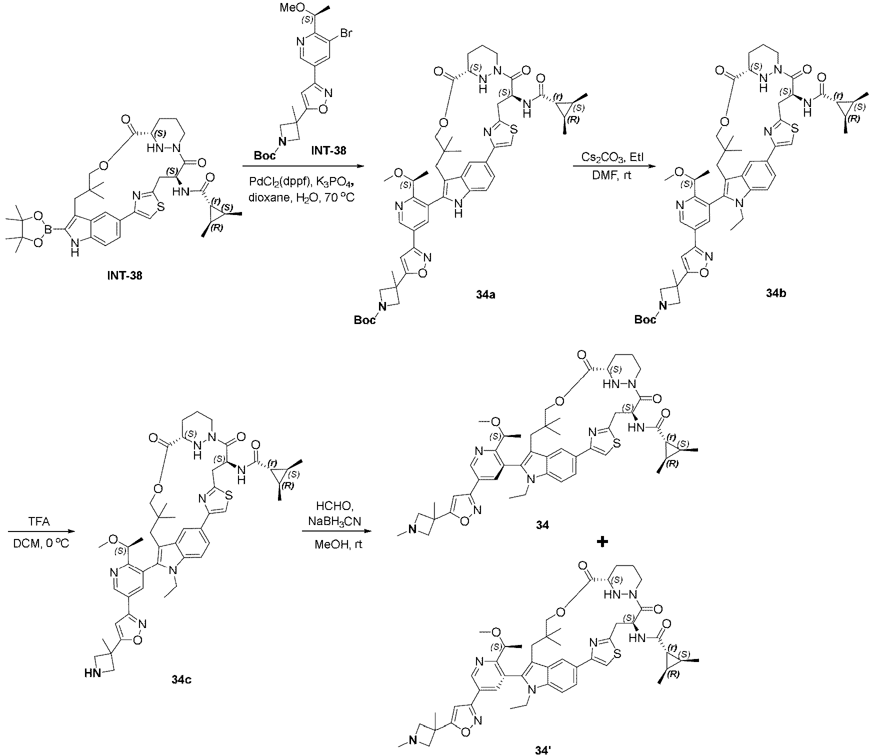

By replacing INT-7 in the synthesis step of compound 2 with INT-38 and INT-3 with INT-4, compound 34 and its diastereoisomer compound 34' can be obtained using similar methods and reaction steps. The absolute configurations of the two compounds are drawn based on the empirical assumption that compound 34 is the isomer with a longer retention time in LCMS or HPLC, while 34' is the isomer with a shorter retention time in LCMS or HPLC.

### Compound 34

ESI-MS (m/z): 877.4 [M+H]⁺; LC-MS retention time RT = 1.88 min. HPLC retention time RT = 14.13 min.

¹H NMR (500 MHz, DMSO-d6) δ 9.24 (d, *J =* 2.0 Hz, 1H), 8.52 (d, *J=* 1.0 Hz, 1H), 8.39 (d, *J* =9.0 Hz, 1H), 8.26 (d, *J =* 2.0 Hz, 1H), 7.82 (s, 1H), 7.78 - 7.75 (m, 1H), 7.60 (d, *J =* 9.0 Hz, 1H), 7.24 (s, 1H), 5.55 (t, *J =* 9.0 Hz, 1H), 5.09 - 5.03 (m, 1H), 4.40 - 4.31 (m, 2H), 4.40 - 4.31 (m, 2H), 4.15 - 4.09 (m, 1H), 3.58 (s, 2H), 3.42 - 3.38 (m, 2H), 3.28 (s, 3H), 3.25 (d, *J =* 7.0 Hz, 2H), 3.18 - 3.11 (m, 2H), 3.00 - 2.94 (m, 1H), 2.78 - 2.72 (m, 1H), 2.46 - 2.40 (m, 1H), 2.27 (s, 3H), 2.10 - 2.04 (m, 1H), 1.81 - 1.76 (m, 2H), 1.62 (s, 3H), 1.54 - 1.48 (m, 1H), 1.40 (d, *J =* 6.0 Hz, 3H), 1.26 - 1.15 (m, 4H), 1.09 (d, *J =* 6.0 Hz, 3H), 1.06 (d, *J =* 6.0 Hz, 3H), 0.91 - 0.87 (m, 5H), 0.37 (s, 3H).

### Compound 34'

ESI-MS (m/z): 877.4 [M+H]⁺; LC-MS retention time RT = 1.85 min. HPLC retention time RT = 13.83 min.

¹H NMR (500 MHz, DMSO-d6) δ 9.25 (d, *J =* 2.0 Hz, 1H), 8.55 (d, *J =* 1.0 Hz, 1H), 8.40 (t, *J =* 6.0 Hz, 2H), 7.83 (s, 1H), 7.77 - 7.73 (m, 1H), 7.57 (d, *J =* 8.5 Hz, 1H), 7.19 (s, 1H), 5.54 (t, *J =* 9.0 Hz, 1H), 5.07 - 5.03 (m, 1H), 4.26 - 4.18 (m, 2H), 4.10 - 4.05 (m, 1H), 4.01 - 3.97 (m, 1H), 3.91 - 3.84 (m, 1H), 3.70 - 3.65 (m, 1H), 3.57 - 7.53 (m, 1H), 3.43 - 3.38 (m, 3H), 3.26 (d, *J =* 7.0 Hz, 2H), 3.20 - 3.14 (m, 1H), 3.14 (s, 3H), 3.10 - 3.04 (m, 1H), 2.78 - 2.73 (m, 1H), 2.42 - 2.38 (m, 1H), 2.28 (s, 3H), 2.13 - 2.08 (m, 2H) 1.82 - 1.77 (m, 2H), 1.63 (s, 3H), 1.54 - 1.49 (m, 1H), 1.27 (d, *J =* 6.0 Hz, 3H), 1.23 (s, 1H), 1.20 - 1.13 (m, 2H), 1.12 (d, *J =* 7.0 Hz, 3H), 1.09 (d, *J =* 6.0 Hz, 3H), 1.06 (d, *J =* 6.0 Hz, 3H), 0.92 (s, 3H), 0.48 (s, 3H).

### Example 35

### (1S,2S)-N-((6³S,4S,Z)-1¹-ethyl-1²-(2-((S)-1-methoxyethyl)-5-(5-(morpholinomethyl)pyrazin-2-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

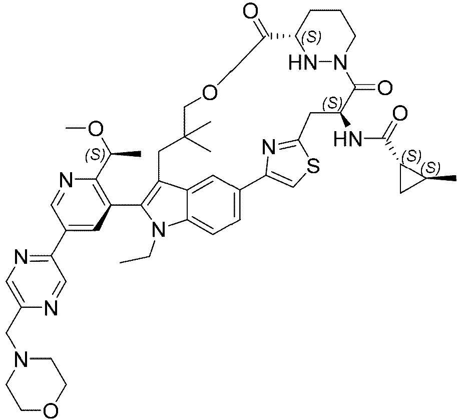

By replacing INT-6 in the synthesis step of compound 1 with INT-43 and INT-4 with INT-3, compound 35 can be obtained using similar methods and reaction steps. 35 is a relatively less polar compound with relatively longer LC-MS and HPLC retention times. According to experience, the in vitro cell activity of compound 35 is much better than that of its diastereomer 35', so the following examples will not further characterize the structure of the diastereomer.

### Compound 35

ESI-MS (m/z): 890.7 [M+H]⁺; LC-MS retention time RT = 1.84 min. HPLC retention time RT = 12.53 min

¹HNMR(500 MHz, DMSO-d6) δ 9.49 (d, *J* = 2.5 Hz, 1H), 9.37 (d, *J =* 1.5 Hz, 1H), 8.80 (d, *J =* 1.5 Hz, 1H), 8.54 - 8.45 (m, 3H), 7.82 (s, 1H), 7.77 (dd, *J =* 8.5, 1.5 Hz, 1H), 7.60 (d, *J =* 8.5 Hz, 1H), 5.56 (t, *J =* 9.0 Hz, 1H), 5.10 - 5.05 (m, 1H), 4.40 - 4.35 (m, 2H), 4.26 - 4.11 (m, 3H), 3.72 (s, 2H), 3.62 - 3.57 (m, 6H), 3.37 - 3.33 (m, 1H), 3.29 (s, 3H), 3.17 - 3.13 (m, 1H), 3.01 - 2.96 (m, 1H), 2.80-2.70 (m, 1H), 2.47 (s, 5H), 2.10 - 2.05 (m, 1H), 1.82 - 1.78 (m, 2H), 1.56 - 1.47 (m, 2H), 1.42 (d, *J =* 6.0 Hz, 3H), 1.10 - 1.05 (m, 4H), 0.91 - 0.85 (m, 7H), 0.57 - 0.53 (m, 1H), 0.37 (s, 3H).

### Example 36

### (1r,2R,3S)-N-((6³S,4S,Z)-1¹-ethyl-1²-(6-((S)-1-methoxyethyl)-1'-(2-morpholinoethyl)-2'-oxo-1',2'-dihydro-[3,4'-bipyridin]-5-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

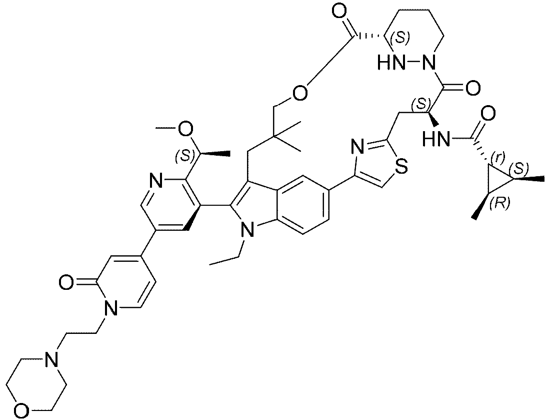

By replacing INT-6 in the synthesis step of compound 1 with INT-44, compound 36 can be obtained using similar methods and reaction steps. ESI-MS (m/z): 933.8 [M+H]⁺; LC-MS retention time RT=1.63 min.

¹H NMR (500 MHz, DMSO-*d*₆) δ 9.13 (d, *J =* 2.5 Hz, 1H), 8.53 - 8.50 (m, 1H), 8.38 (d, *J =* 9.0 Hz, 1H), 8.15 (d, *J =* 2.5 Hz, 1H), 7.82 (s, 1H), 7.80 - 7.78 (m, 1H), 7.77 - 7.74 (m, 1H), 7.61 - 7.58 (m, 1H), 6.90 - 6.87 (m, 1H), 6.76 - 6.71 (m, 1H), 5.56 (t, *J* = 9.0 Hz, 1H), 5.09 - 5.04 (m, 1H), 4.37 - 4.30 (m, 2H), 4.25 - 4.10 (m, 4H), 4.06 - 4.01 (m, 2H), 3.62 - 3.53 (m, 7H), 3.27 (s, 3H), 3.17 - 3.11 (m, 1H), 2.98 - 2.93 (m, 1H), 2.78 - 2.73 (m, 1H), 2.58 (t, *J =* 6.5 Hz, 2H), 2.45 - 2.42 (m, 4H), 2.10 - 2.03 (m, 1H), 1.80 - 1.73 (m, 4H), 1.54 - 1.48 (m, 1H), 1.40 (d, *J* = 6.0 Hz, 3H), 1.25 - 1.15 (m, 3H), 1.10 - 1.05 (m, 5H), 0.93 - 0.88 (m, 5H), 0.36 (s, 3H).

### Example 37

### (1r,2R,3S)-N-((6³S,4S,Z)-12-(5'-((1,1-dioxidothiomorpholino)methyl)-6-((S)-1-methoxyethyl)-[3,3'-bipyridin]-5-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

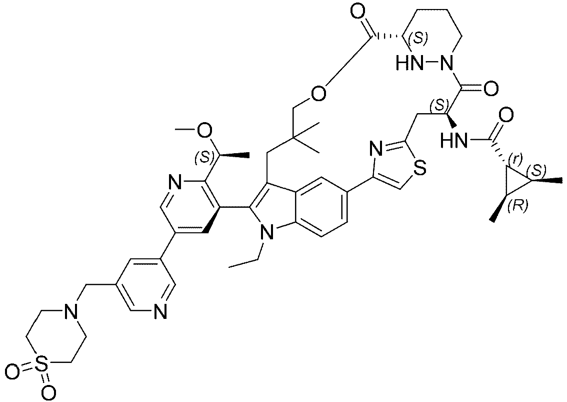

By replacing INT-6 in the synthesis step of compound 1 with INT-45 and INT-4 with INT-3, compound 37 can be obtained using similar methods and reaction steps. ESI-MS (m/z): 951.8 [M+H]⁺; LC-MS retention time RT = 1.67 min.

### Example 38

### (1S,2S)-N-((6³S,4S,Z)-1²-(5-(2-((1,1-dioxidothiomorpholino)methyl)pyrimidin-5-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

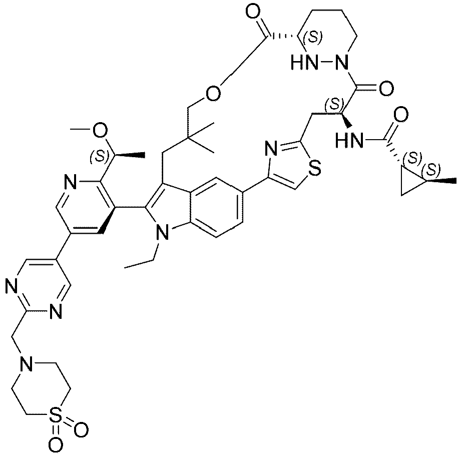

By replacing INT-6 in the synthesis step of compound 1 with INT-46 and INT-4 with INT-3, compound 38 can be obtained using similar methods and reaction steps. ESI-MS (m/z): 938.8 [M+H]⁺; LC-MS retention time RT = 1.58 min. HPLC retention time RT = 11.29 min

¹H NMR (500 MHz, DMSO) δ 9.32 - 9.27 (m, 2H), 9.22 (d, *J =* 2.5 Hz, 1H), 8.55 - 8.50 (m, 2H), 8.33 (d, *J =* 2.5 Hz, 1H), 7.82 (s, 1H), 7.78 - 7.75 (m, 1H), 7.60 (d, *J =* 8.5 Hz, 1H), 5.56 (t, *J* = 9.0 Hz, 1H), 5.10 - 5.05 (m, 1H), 4.41 - 4.31 (m, 2H), 4.27 - 4.11 (m, 4H), 4.03 (s, 2H), 3.61 - 3.55 (m, 2H), 3.29 (s, 3H), 3.17 - 3.14 (m, 1H), 3.113 - 3.09 (m, 8H), 2.99 - 2.93 (m, 1H), 2.79 - 2.72 (m, 1H), 2.10 - 2.04 (m, 1H), 1.82 - 1.74 (m, 2H), 1.55 - 1.47 (m, 2H), 1.41 (d, *J* = 6.0 Hz, 3H), 1.25 - 1.20(m, 1H), 1.09 - 1.03 (m, 4H), 0.96 - 0.88 (m, 6H), 0.87 - 0.84 (m, 1H), 0.57 - 0.50 (m, 1H), 0.36 (s, 3H).

### Example 39

### (1r,2R,3S)-N-((6³S,4S,Z)-1¹-ethyl-1²-(2-((S)-1-methoxyethyl)-5-(2-(morpholinomethyl)pyrimidin-5-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

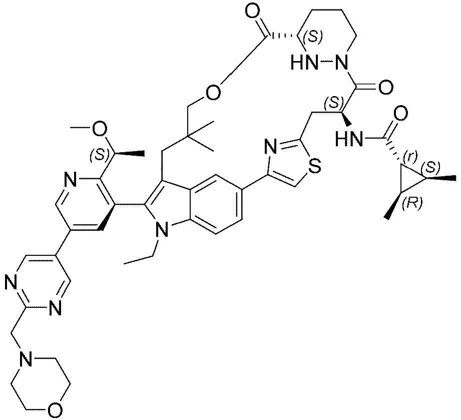

By replacing INT-6 in the synthesis step of compound 1 with INT-47, compound 39 can be obtained using similar methods and reaction steps. ESI-MS (m/z): 904.8 [M+H]⁺; LC-MS retention time RT=1.66 min, HPLC retention time RT=11.91 min.

¹H NMR (500 MHz, DMSO) δ 9.30 - 9.25 (m, 2H) 9.21 (d, *J =* 2.5 Hz, 1H), 8.52 (d, *J =* 1.5 Hz, 1H), 8.38 (d, *J =* 9.0 Hz, 1H), 8.32 (d, *J =* 2.5 Hz, 1H), 7.82 (s, 1H), 7.78 - 7.74 (m, 1H), 7.60 (d, *J* = 9.0 Hz, 1H), 5.56 (t, *J* = 9.0 Hz, 1H), 5.10 - 5.04 (m, 1H), 4.39 - 4.32 (m, 2H), 4.27 - 4.11 (m, 4H), 3.77 (s, 2H), 3.61 - 3.54 (m, 6H), 3.31 - 3.27 (m, 1H), 3.28 (s, 3H), 3.17 - 3.12 (m, 1H), 2.98 - 2.93 (m, 1H), 2.78 - 2.72 (m, 1H), 2.56 - 2.52 (m, 4H), 2.10 - 2.03 (m, 1H), 1.82 - 1.75 (m, 2H), 1.55 - 1.46 (m, 2H), 1.41 (d, *J =* 6.0 Hz, 3H), 1.20 - 1.14 (m, 2H), 1.10 - 1.04 (m, 6H), 0.92 (t, *J =* 7.0 Hz, 3H), 0.89 (s, 3H), 0.37 (s, 3H).

### Example 40

### (1S,2S)-N-((6³S,4S,Z)-1¹-ethyl-1²-(6-((S)-1-methoxyethyl)-1'-(2-morpholinoethyl)-6'-oxo-1',6'-dihydro-[3,3'-bipyridin]-5-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

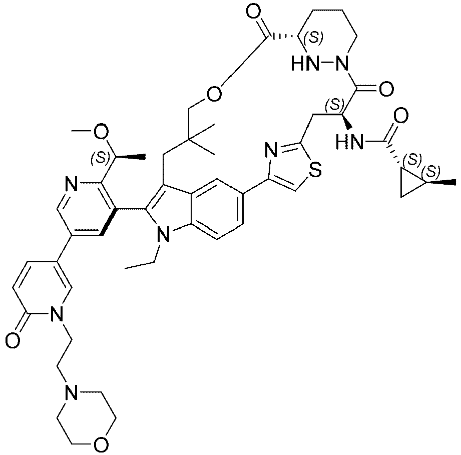

By replacing INT-6 in the synthesis step of compound 1 with INT-48 and INT-4 with INT-3, compound 40 can be obtained using similar methods and reaction steps. ESI-MS (m/z): 919.8 [M+H]⁺; LC-MS retention time RT = 1.59 min.

¹H NMR (500 MHz, DMSO-*d*₆) δ 9.01 (d, *J =* 2.5 Hz, 1H), 8.55 - 8.48 (m, 2H), 8.28 (d, *J* = 2.5 Hz, 1H), 8.06 (d, *J* = 2.5 Hz, 1H), 8.01 (dd, *J =* 9.0, 2.5 Hz, 1H), 7.81 (s, 1H), 7.75 (dd, *J =* 9.0, 2.0 Hz, 1H), 7.59 (d, *J =* 8.5 Hz, 1H), 6.53 - 6.49 (m, 1H), 5.55 (t, *J =* 9.0 Hz, 1H), 5.32 (t, *J =* 5.0 Hz, 1H), 5.11 - 5.05 (m, 1H), 4.46 - 4.12 (m, 5H), 4.06 (t, *J =* 6.0 Hz, 2H), 3.61 - 3.55 (m, 2H), 3.51 - 3.46 (m, 4H), 3.32 - 3.28 (m, 1H), 3.27 (s, 3H), 3.17 - 3.12 (m, 1H), 3.01 - 2.95 (m, 1H), 2.79 - 2.72 (m, 1H), 2.59 (t, *J =* 6.0 Hz, 2H), 2.48 - 2.39 (m, 4H), 2.04 - 1.94 (m, 3H), 1.82 - 1.75 (m, 2H), 1.55 - 1.43 (m, 4H), 1.39 (d, *J =* 6.0 Hz, 3H), 1.11 - 1.06 (m, 4H), 0.88 - 0.83 (m, 3H), 0.57 - 0.52 (m, 1H), 0.36 (s, 3H).

### Example 41

### (1S,2S)-N-((6³S,4S,Z)-1¹-ethyl-1²-(6'-((S)-1-methoxyethyl)-5-((4-methylpiperazin-1-yl)methyl)-[2,3'-bipyridin]-5'-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

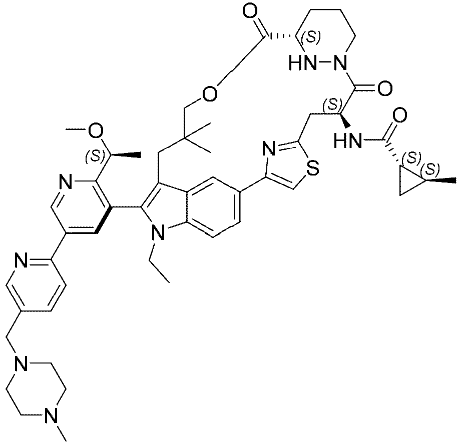

By replacing INT-7 in the synthesis step of compound 2 with INT-49, compound 41 can be obtained using similar methods and reaction steps. ESI-MS (m/z): 902.8 [M+H]⁺; LC-MS retention time RT = 1.70 min.

¹H NMR (500 MHz, DMSO-*d*₆) δ 9.43 (d, *J =* 2.5 Hz, 1H), 8.55 - 8.49 (m, 2H), 8.40 (d, *J =* 2.5 Hz, 1H), 8.05 (d, *J =* 7.5 Hz, 1H), 7.91 (t*, J =* 7.5 Hz, 1H), 7.82 (s, 1H), 7.78 - 7.74 (m, 1H), 7.60 (d, *J =* 8.5 Hz, 1H), 7.47 (d, *J* = 7.5 Hz, 1H), 5.57 (t, *J=* 9.0 Hz, 1H), 5.11 - 5.05 (m, 1H), 4.41 - 4.33 (m, 2H), 4.29 - 4.07 (m, 4H), 3.68 - 3.64 (m, 2H), 3.59 (s, 2H), 3.28 (s, 3H), 3.19 - 3.11 (m, 1H), 3.02 - 2.95 (m, 1H), 2.79 - 2.72 (m, 1H), 2.48 - 2.43 (m, 2H), 2.41 - 2.27 (m, 4H), 2.15 (s, 3H), 2.10 - 1.95 (m, 3H), 1.81 - 1.75 (m, 2H), 1.52 - 1.46 (m, 2H), 1.41 (d, *J=* 6.0 Hz, 3H), 1.11 - 1.06 (m, 4H), 0.92 - 0.84 (m, 8H), 0.59 - 0.52 (m, 1H), 0.38 (s, 3H).

### Example 42

### (1r,2R,3S)-N-((6³S,4S,Z)-1¹-ethyl-1²-(5'-((1-imino-1-oxido-1λ6-thiomorpholino)methyl)-6-((S)-1-methoxyethyl)-[3,3'-bipyridin]-5-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

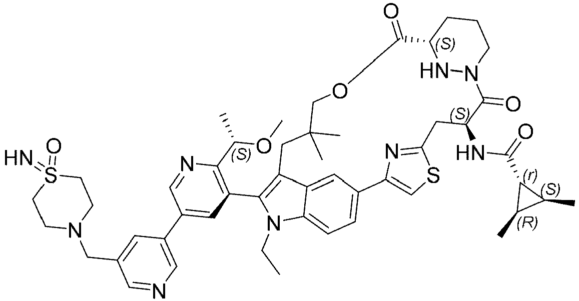

By replacing INT-7 in the synthesis step of compound 2 with INT-50 and replacing INT-4 with INT-3, compound 42 can be obtained using similar methods and reaction steps. ESI-MS (m/z): 950.8 [M+H]⁺; LC-MS retention time RT=1.56 min, HPLC retention time RT=10.91 min.

¹H NMR (500 MHz, DMSO-*d*₆) δ 9.16 (d, *J =* 2.5 Hz, 1H), 8.96 (d, *J =* 2.0 Hz, 1H), 8.61 (d, *J =* 2.0 Hz, 1H), 8.52 (d, *J =* 2.0 Hz, 1H), 8.38 (d, *J =* 9.0 Hz, 1H), 8.22 - 8.17 (m, 2H), 7.82 (s, 1H), 7.79 - 7.73 (m, 1H), 7.60 (d*, J =* 8.5 Hz, 1H), 5.56 (t, *J =* 9.0 Hz, 1H), 5.09 - 5.05 (m, 1H), 4.39 - 4.33 (m, 2H), 4.24 - 4.13 (m, 4H), 3.77 (s, 2H), 3.63 - 3.58 (m, 3H), 3.28 (s, 3H), 3.19 - 3.13 (m, 2H), 3.05 - 2.95 (m, 5H), 2.93 - 2.88 (m, 2H), 2.85 - 2.80 (m, 3H), 2.10 - 2.05 (m, 1H), 1.80 - 1.75 (m, 2H), 1.54 - 1.50 (m, 1H), 1.45 - 1.39 (m, 4H), 1.19 - 1.10 (m, 2H), 1.10 - 1.05 (m, 6H), 0.94 (t, *J* = 7.0 Hz, 3H), 0.89 (s, 3H), 0.38 (s, 3H).

### Example 43

### (1S,2S)-N-((6³S,4S,Z)-1¹-ethyl-1²-(2-((S)-1-methoxyethyl)-5-(2-methyl-2,3-dihydro-1H-pyrrolo[3,4-c]pyridin-6-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

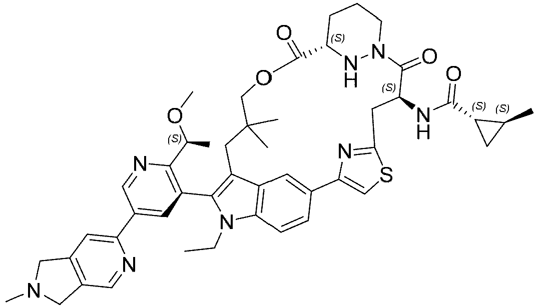

By replacing INT-7 in the synthesis step of compound 2 with INT-5l, compound 43 can be obtained using similar methods and reaction steps. ESI-MS (m/z): 845.8 [M+H]⁺; LC-MS retention time RT=1.76 min, HPLC retention time RT=12.62 min.

¹H NMR (500 MHz, DMSO-*d*₆) δ 9.42- 9.39 (m, 1H), 8.59 (s, 1H), 8.55 - 8.50 (m, 2H), 8.39 - 8.36 (m, 1H), 8.11 (s, 1H), 7.82 (s, 1H), 7.77 - 7.75 (m, 1H), 7.61 - 7.59 (m, 1H), 5.56 (t, *J* = 9.0 Hz, 1H), 5.12 - 5.07 (m, 1H), 4.38 - 4.33 (m, 2H), 4.25 - 4.14 (m, 3H), 3.89 (s, 3H), 3.58 (s, 2H), 3.29 (s, 3H), 3.17 - 3.12 (m, 1H), 3.01 - 2.98 (m, 1H), 2.82 - 2.71 (m, 1H), 2.46 - 2.42 (m, 1H), 2.08 - 2.06 (m, 1H), 2.03 - 1.94 (m, 2H), 1.80 - 1.75 (m, 2H), 1.56 - 1.45 (m, 2H), 1.41 (d, *J =* 6.0 Hz, 3H), 1.27 - 1.21 (m, 3H), 1.10 - 1.05 (m, 4H), 0.92 - 0.84 (m, 6H), 0.57 - 0.54 (m, 1H), 0.37 (s, 3H).

### Example 44

### (1r,2R,3S)-N-((6³S,4S,Z)-1²-(2-((S)-1-methoxyethyl)-5-(2-(morpholinomethyl)pyrimidin-5-yl)pyridin-3-yl)-10,10-dimethyl-5,7-dioxo-1¹-(2,2,2-trifluoroethyl)-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

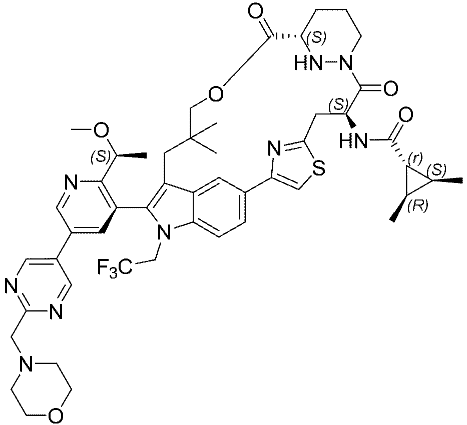

Compound 44 can be obtained by replacing INT-6 in the synthesis step of compound 1 with INT-47 and replacing iodoethane with 2,2,2-trifluoroethyl trifluoromethanesulfonate, using a similar method and reaction steps. ESI-MS (m/z): 958.8 [M+H]⁺; LC-MS retention time RT = 1.73 min.

¹H NMR (500 MHz, DMSO) δ 9.25 - 9.21 (m, 3H), 8.56 - 8.51 (m, 1H), 8.40 (d, *J* = 9.0 Hz, 1H), 8.36 -8.30 (m, 1H), 7.88 (s, 1H), 7.88 -7.83 (m, 1H), 7.81 -7.75 (m, 1H), 5.64 - 5.57 (m, 1H), 5.52 (t, *J =* 9.0 Hz, 1H), 5.07 -5.01 (m, 1H), 4.96 - 4.89 (m, 1H), 4.33 - 4.28 (m, 1H), 4.26 - 4.18 (m, 2H), 3.77 (s, 2H), 3.62 - 3.54 (m, 6H), 3.21 - 3.14 (m, 2H), 3.06 - 3.01 (m, 1H), 2.80 - 2.70 (m, 2H), 2.55 - 2.52 (m, 4H), 2.12 - 2.06 (m, 1H), 1.82 - 1.76 (m, 2H), 1.54 - 1.49 (m, 1H), 1.42 (d, *J =* 6.0 Hz, 3H), 1.26 - 1.20 (m, 2H), 1.20 - 1.13 (m, 3H), 1.11 - 1.06 (m, 6H), 0.93 (s, 3H), 0.88 - 0.81 (m, 1H), 0.32 (s, 3H).

### Example 45

### (1S,2S)-N-((6³S,4S,Z)-1¹-ethyl-1²-(2-((S)-1-methoxyethyl)-5-(6-methyl-6,7-dihydro-5H-pyrrolo[3,4-b]pyridin-3-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

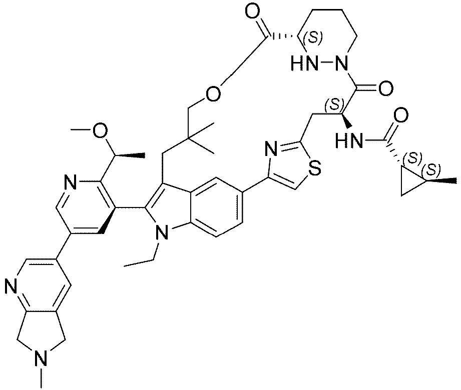

By replacing INT-7 in the synthesis step of compound 2 with INT-52, compound 45 can be obtained using similar methods and reaction steps. ESI-MS (m/z): 845.8 [M+H]⁺; LC-MS retention time RT=1.65 min, HPLC retention time RT=11.92 min.

¹H NMR (500 MHz, DMSO) δ 9.11 (d, *J =* 2.5 Hz, 1H), 8.80 (d, *J =* 1.5 Hz, 1H), 8.53 - 8.47 (m, 2H), 8.14 (d, *J =* 2.5 Hz, 1H), 8.15 - 8.09 (m, 1H), 7.80 (s, 1H), 7.77 - 7.74 (m, 1H), 7.59 (d, *J =* 8.5 Hz, 1H), 5.57 (t, *J =* 9.0 Hz, 1H), 5.10 - 5.05 (m, 1H), 4.39 - 4.30 (m, 2H), 4.27 - 4.21 (m, 1H), 4.20 - 4.11 (m, 2H), 3.94 (s, 2H), 3.89 (s, 2H), 3.62 - 3.56 (m, 2H), 3.28 (s, 3H), 3.18 - 3.09 (m, 2H), 2.98 - 2.92 (m, 1H), 2.79 - 2.72 (m, 1H), 2.54 (s, 3H), 2.10 - 2.04 (m, 1H), 1.83 - 1.75 (m, 2H), 1.55 - 1.47 (m, 2H), 1.41 (d, *J =* 6.0 Hz, 3H), 1.27 - 1.22 (m, 1H), 1.10 - 1.03 (m, 4H), 0.92 (t, *J =* 7.0 Hz, 3H), 0.90 - 0.83 (m, 4H), 0.57 - 0.52 (m, 1H), 0.37 (s, 3H).

### Example 46

### (1S,2S)-N-((6³S,4S,Z)-1¹-ethyl-1²-(5-(((R)-3-fluoropyrrolidin-1-yl)methyl)-6'-((S)-1-methoxyethyl)-[2,3'-bipyridin]-5'-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

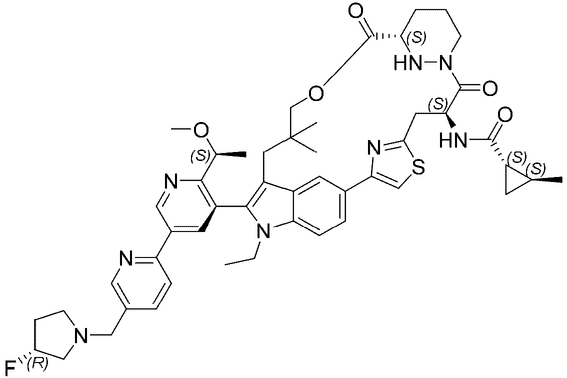

By replacing INT-7 in the synthesis step of compound 2 with INT-53, compound 46 can be obtained using similar methods and reaction steps. ESI-MS (m/z): 891.7 [M+H]⁺; LC-MS retention time RT=1.89 min, HPLC retention time RT=13.66 min.

¹H NMR (500 MHz, DMSO-*d*₆) δ 9.44 (d, *J =* 2.5 Hz, 1H), 8.65 (d, *J=* 2.0 Hz, 1H), 8.55 - 8.50 (m, 2H), 8.41 (d, *J=* 2.5 Hz, 1H), 8.19 - 8.13 (m, 1H), 7.88 - 7.85 (m, 1H), 7.82 (s, 1H), 7.78 - 7.74 (m, 1H), 7.62 - 7.58 (m, 1H), 5.56 *(t, J=* 9.0 Hz, 1H), 5.29 - 5.13 (m, 1H), 5.11 - 5.05 (m, 1H), 4.41 - 4.31 (m, 2H), 4.28 - 4.10 (m, 3H), 3.70 (s, 2H), 3.62 - 3.55 (m, 2H), 3.29 (s, 3H), 3.18 - 3.11 (m, 1H), 3.03 - 2.97 (m, 1H), 2.86 - 2.59 (m, 4H), 2.47 - 2.43 (m, 2H), 2.38 - 2.33 (m, 2H), 2.20 - 2.05 (m, 2H), 1.94 - 1.84 (m, 1H), 1.82 - 1.74 (m, 2H), 1.55 - 1.47 (m, 2H), 1.43 - 1.39 (m, 3H), 1.09 - 1.05 (m, 4H), 0.91 - 0.86 (m, 6H), 0.58 - 0.53 (m, 1H), 0.37 (s, 3H).

### Example 47

### (1S,2S)-N-((6³S,4S,Z)-1¹-ethyl-1²-(5-(((R)-3-hydroxypyrrolidin-1-yl)methyl)-6'-((S)-1-methoxyethyl)-[2,3'-bipyridin]-5'-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

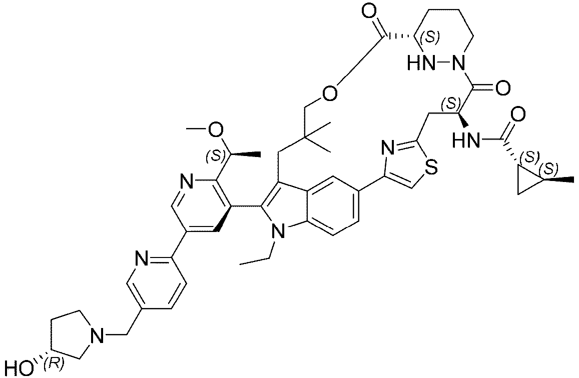

By replacing INT-7 in the synthesis step of compound 2 with INT-54, compound 47 can be obtained using similar methods and reaction steps. ESI-MS (m/z): 889.8 [M+H]⁺; LC-MS retention time RT=1.64 min, HPLC retention time RT=11.43 min.

¹H NMR (500 MHz, DMSO-*d*₆) δ 9.47 - 9.39 (m, 1H), 8.66 - 8.61 (m, 1H), 8.55 - 8.50 (m, 2H), 8.43 - 8.39 (m, 1H), 8.15 (d, *J* = 8.1 Hz, 1H), 7.88 - 7.84 (m, 1H), 7.82 (s, 1H), 7.79 - 7.74 (m, 1H), 7.62 - 7.56 (m, 1H), 5.56 (t, *J* = 9.2 Hz, 1H), 5.12 - 5.04 (m, 1H), 4.40 - 4.30 (m, 2H), 4.27 - 4.08 (m, 4H), 3.72 - 3.56 (m, 4H), 3.29 (s, 3H), 3.18 - 3.10 (m, 1H), 3.03 - 2.97 (m, 1H), 2.80 - 2.58 (m, 3H), 2.46 - 2.40 (m, 3H), 2.38 - 2.31 (m, 2H), 2.10 - 1.96 (m, 2H), 1.83 - 1.75 (m, 2H), 1.59 - 1.47 (m, 3H), 1.44 - 1.39 (m, 3H), 1.07 (s, 3H), 0.92 - 0.84 (m, 6H), 0.58 - 0.52 (m, 1H), 0.37 (s, 3H).

### Example 48

### (1r,2R,3S)-N-((6³S,4S,Z)-1'-(2,2-difluoroethyl)-1²-(2-((S)-1-methoxyethyl)-5-(2-(morpholinomethyl)pyrimidin-5-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

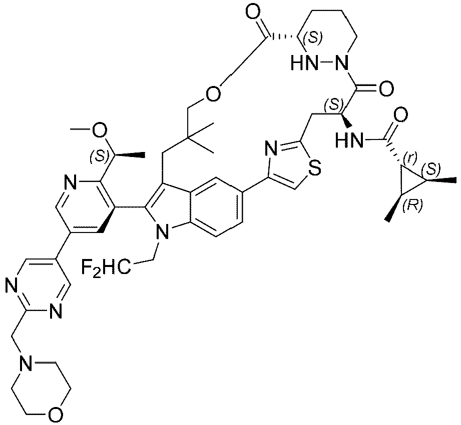

By replacing INT-6 in the synthesis step of compound 1 with INT-47 and replacing iodoethane with 1,1-difluoro-2-iodoethane, compound 48 can be obtained using a similar method and reaction steps. ESI-MS (m/z): 940.8 [M+H]⁺; LC-MS retention time RT = 1.63 min,

¹H NMR (500 MHz, DMSO) δ 9.28 - 9.23 (m, 2H), 9.21 (d, *J =* 2.5 Hz, 1H), 8.54 - 8.50 (m, 1H), 8.40 (d, *J* = 9.0 Hz, 1H), 8.30 - 8.25 (m, 1H), 7.86 (s, 1H), 7.83 - 7.77 (m, 1H), 7.71 (d, *J =* 9.0 Hz, 1H), 6.12 - 5.87 (m, 1H), 5.54 (t, *J* = 9.0 Hz, 1H), 5.10 - 5.04 (m, 1H), 5.00 - 4.94 (m, 1H), 4.60 - 4.54 (m, 1H), 4.36 - 4.28 (m, 2H), 4.26 - 4.18 (m, 2H), 3.77 (s, 2H), 3.70 - 3.66 (m, 1H), 3.60 - 3.56 (m, 4H), 3.46 - 3.42 (m, 1H), 3.29 (s, 3H), 3.17 - 3.12 (m, 2H), 3.02 - 2.96 (m, 1H), 2.77 - 2.72 (m, 1H), 2.65 - 2.60 (m, 1H), 2.39 - 2.33 (m, 1H), 2.11 - 2.06 (m, 1H), 1.82 - 1.76 (m, 2H), 1.54 - 1.49 (m, 1H), 1.42 (d, *J=* 6.0 Hz, 3H), 1.32 - 1.28 (m, 1H), 1.25 - 1.20 (m, 1H), 1.20 - 1.15 (m, 2H), 1.10 - 1.04 (m, 6H), 0.91 (s, 3H), 0.50 - 0.45 (m, 1H), 0.34 (s, 3H).

### Example 49

### (1S,2S)-N-((6³S,4S,Z)-1¹-ethyl-1²-(5-(5-((1-imino-1-oxido-1λ⁶-thiomorpholino)methyl)pyrimidin-2-yl)-2-((S)-1-methoxyethyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

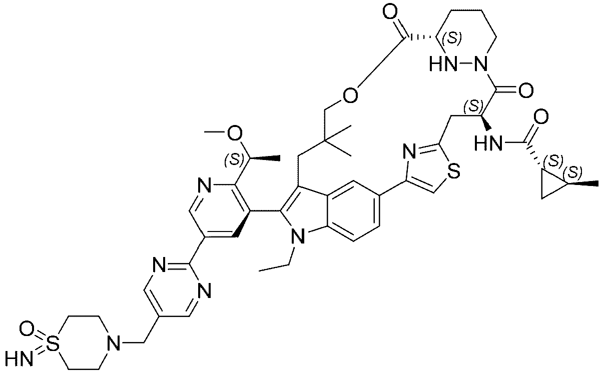

By replacing INT-7 in the synthesis step of compound 2 with INT-55, compound 49 can be obtained using similar methods and reaction steps. ESI-MS (m/z): 937.8 [M+H]⁺; LC-MS retention time RT=1.55 min, HPLC retention time RT=10.95 min.

¹H NMR (500 MHz, DMSO-*d*₆) δ 9.61 - 9.58 (m, 1H), 8.91 - 8.85 (m, 2H), 8.52 - 8.49 (m, 1H), 8.49 - 8.43 (m, 2H), 7.76 (s, 1H), 7.73 - 7.68 (m, 1H), 7.60 - 7.51 (m, 1H), 5.50 (t, *J =* 9.0 Hz, 1H), 5.07 - 4.99 (m, 1H), 4.36 - 4.27 (m, 2H), 4.21 - 4.04 (m, 3H), 3.73 (s, 2H), 3.52 (s, 2H), 3.23 (s, 3H), 3.11 - 3.04 (m, 6H), 2.96 - 2.89 (m, 3H), 2.82 - 2.77 (m, 2H), 2.72 - 2.65 (m, 1H), 2.36 - 2.29 (m, 1H), 2.04 - 1.98 (m, 1H), 1.75 - 1.67 (m, 2H), 1.46 - 1.41 (m, 2H), 1.35 (d, *J =* 6.0 Hz, 3H), 1.02 - 0.96 (m, 4H), 0.84 - 0.76 (m, 7H), 0.51 - 0.45 (m, 1H), 0.30 (s, 3H).

### Example 50

### (1S,2S)-N-((6³S,4S,Z)-1¹-ethyl-1²-(6'-((S)-1-methoxyethyl)-5-(((R)-3-(methylsulfonyl)pyrrolidin-1-yl)methyl)-[2,3'-bipyridin]-5'-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

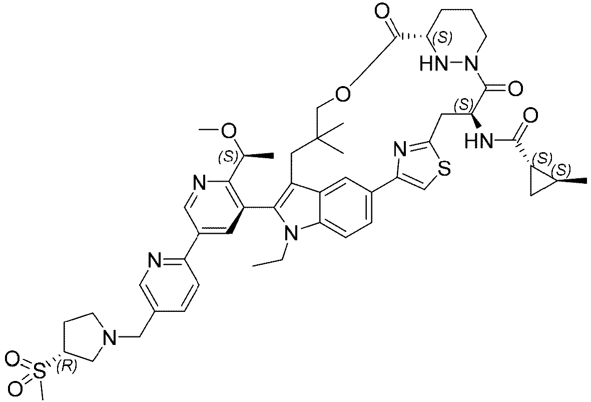

By replacing INT-7 in the synthesis step of compound 2 with INT-56, compound 50 can be obtained using similar methods and reaction steps. ESI-MS (m/z): 951.8 [M+H]⁺; LC-MS retention time RT=1.68 min, HPLC retention time RT=12.05 min.

¹H NMR (500 MHz, DMSO-*d*₆) δ 9.45 (s, 1H), 8.68 - 8.62 (m, 1H), 8.57 - 8.49 (m, 2H), 8.45 - 8.40 (m, 1H), 8.20 - 8.15 (m, 1H), 7.91 - 7.80 (m, 2H), 7.79 - 7.73 (m, 1H), 7.63 - 7.58 (m, 1H), 5.56 (t, *J* = 9.0 Hz, 1H), 5.12 - 5.07 (m, 1H), 4.42 - 4.08 (m, 5H), 3.84 - 3.67 (m, 3H), 3.63 - 3.54 (m, 2H), 3.29 (s, 3H), 3.18 - 3.10 (m, 2H), 3.03 - 2.73 (m, 6H), 2.66 - 2.62 (m, 1H), 2.45 - 2.40 (m, 1H), 2.38 - 2.35 (m, 1H), 2.20 - 2.03 (m, 3H), 1.84 - 1.74 (m, 2H), 1.55 - 1.47 (m, 2H), 1.41 (d, *J=* 6.0 Hz, 3H), 1.10 - 1.01 (m, 5H), 0.91 - 0.83 (m, 7H), 0.59 - 0.51 (m, 1H), 0.37 (s, 3H).

### Example 51

### (1S,2S)-N-((6³S,4S,Z)-1²-(5-(5-((1,1-dioxidothiomorpholino)methyl)pyrimidin-2-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

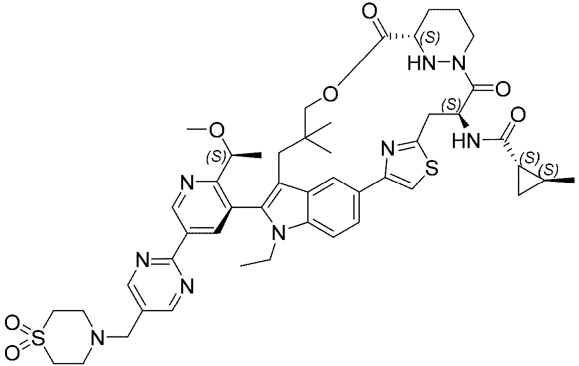

By replacing INT-7 in the synthesis step of compound 2 with INT-57, compound 51 can be obtained using similar methods and reaction steps. ESI-MS (m/z): 938.8 [M+H]⁺; LC-MS retention time RT=1.70 min, HPLC retention time RT=12.22 min.

¹H NMR (500 MHz, DMSO-*d*₆) δ 9.69 - 9.64 (m, 1H), 8.95 - 8.92 (m, 2H), 8.59 - 8.56 (m, 1H), 8.56 - 8.52 (m, 2H), 7.83 (s, 1H), 7.79 - 7.76 (m, 1H), 7.63 - 7.60 (m, 1H), 5.57 (t, *J =* 9.0 Hz, 1H), 5.12 - 5.07 (m, 1H), 4.42 - 4.35 (m, 2H), 4.26 - 4.12 (m, 3H), 3.80 (s, 2H), 3.59 (s, 2H), 3.30 (s, 3H), 3.19 - 3.11 (m, 6H), 3.05 - 2.99 (m, 1H), 2.97 - 2.91 (m, 4H), 2.80 - 2.73 (m, 1H), 2.46 - 2.40 (m, 1H), 2.10 - 2.04 (m, 1H), 1.83 - 1.75 (m, 2H), 1.54 - 1.48 (m, 2H), 1.42 (d, *J =* 6.0 Hz, 3H), 1.09 - 1.04 (m, 4H), 0.92 - 0.84 (m, 7H), 0.59 - 0.53 (m, 1H), 0.37 (s, 3H).

### Example 52

### (1S,2S)-N-((6³S,4S,Z)-1²-(5-(1,6-dimethyl-2-oxo-1,2,5,6,7,8-hexahydro-1,6-naphthyridin-3-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

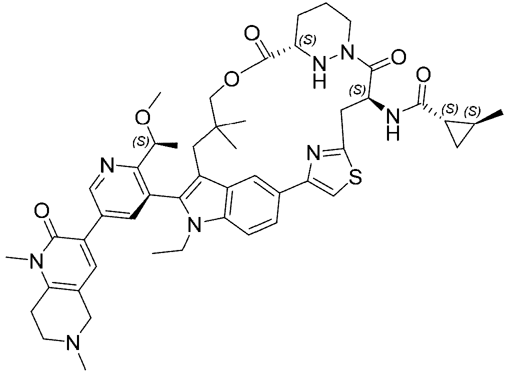

By replacing INT-7 in the synthesis step of compound 2 with INT-58, compound 52 can be obtained using similar methods and reaction steps. ESI-MS (m/z): 889.8 [M+H]⁺; LC-MS retention time RT=1.60 min, HPLC retention time RT=11.43 min.

¹H NMR (500 MHz, DMSO-*d*₆) δ 9.03 (d, *J =* 2.0 Hz, 1H), 8.53 (d, *J =* 9.0 Hz, 1H), 8.50 (d, *J* = 2.0 Hz, 1H), 8.19 (d, *J =* 2.0 Hz, 1H), 7.81 (s, 1H), 7.75 (dd, *J =* 9.0, 2.0 Hz, 1H), 7.69 (s, 1H), 7.58 (d*, J =* 9.0 Hz, 1H), 5.56 (t, *J =* 9.0 Hz, 1H), 5.11 - 5.03 (m, 1H), 4.37-4.29 (m, 2H), 4.26 - 4.13 (m, 3H), 3.58 (s, 2H), 3.47 (s, 3H), 3.28 (s, 3H), 3.16 - 3.11 (m, 1H), 2.97 - 2.94 (m, 1H), 2.86 - 2.83 (m, 2H), 2.79 - 2.72 (m, 1H), 2.69 - 2.59 (m, 2H), 2.36 (s, 3H), 2.09 - 2.07 (m, 1H), 2.02 - 1.96 (m, 1H), 1.80 - 1.73 (m, 2H), 1.52 - 1.45 (m, 2H), 1.39 (d, *J =* 6.0 Hz, 3H), 1.24 - 1.23 (m, 4H), 1.07 (s, 3H), 0.90 - 0.89 (m, 5H), 0.87 - 0.84 (m, 2H), 0.57 - 0.53 (m, 1H), 0.36 (s, 3H).

### Example 53

### (1S,2S)-N-((6³S,4S,Z)-1¹-ethyl-1²-(2-((S)-1-methoxyethyl)-5-(2-((4-methylpiperazin-1-yl)methyl)pyrimidin-5-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

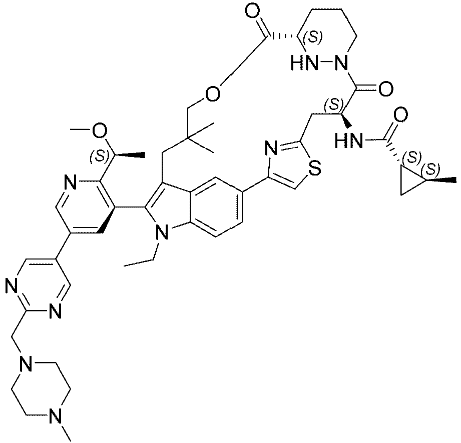

By replacing INT-7 in the synthesis step of compound 2 with INT-59, compound 53 can be obtained using similar methods and reaction steps. ESI-MS (m/z): 903.8 [M+H]⁺; LC-MS retention time RT = 1.53 min,

¹H NMR (500 MHz, DMSO) δ 9.27 (s, 2H), 9.21 (d, *J=* 2.5 Hz, 1H), 8.53 (d, *J=* 9.0 Hz, 1H), 8.51 (s, 1H), 8.32 (d, *J =* 2.5 Hz, 1H), 7.82 (s, 1H), 7.79 - 7.74 (m, 1H), 7.60 (d, *J= 9.0* Hz, 1H), 5.55 (t, *J= 9.0* Hz, 1H), 5.12 - 5.06 (m, 1H), 4.39 - 4.31 (m, 2H), 4.27 - 4.10 (m, 4H), 3.74 (s, 2H), 3.61 - 3.54 (m, 2H), 3.35 - 3.31 (m, 1H), 3.29 (s, 3H), 3.18 - 3.08 (m, 1H), 2.99 - 2.94 (m, 1H), 2.82 - 2.70 (m, 1H), 2.55 - 2.52 (m, 2H), 2.37 - 2.24 (m, 4H), 2.13 (s, 3H), 2.09 - 2.04 (m, 1H), 1.82 - 1.74 (m, 2H), 1.56 - 1.45 (m, 3H), 1.41 (d, *J=* 6.0 Hz, 3H), 1.09 -1.03 (m, 4H), 0.96 - 0.82 (m, 8H), 0.58 - 0.50 (m, 1H), 0.36 (s, 3H).

### Example 54

### (1S,2S)-N-((6³S,4S,Z)-1¹-ethyl-1²-(2-((S)-1-methoxyethyl)-5-(2-(4-methylpiperazin-1-yl)pyrimidin-5-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

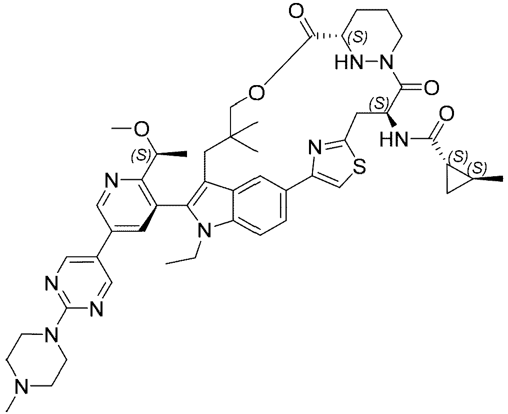

By replacing INT-7 in the synthesis step of compound 2 with INT-60, compound 54 can be obtained using similar methods and reaction steps. ESI-MS (m/z): 889.8 [M+H]⁺; LC-MS retention time RT=1.82 min, HPLC retention time RT=13.48 min.

¹H NMR (500 MHz, DMSO-*d*₆) δ 9.07 (d, *J =* 2.5 Hz, 1H), 8.87 (s, 2H), 8.55 - 8.49 (m, 2H), 8.10 (d, *J =* 2.5 Hz, 1H), 7.81 (s, 1H), 7.79 - 7.73 (m, 1H), 7.59 (d, *J =* 8.5 Hz, 1H), 5.56 (t, *J = 9.0* Hz, 1H), 5.11 - 5.05 (m, 1H), 4.36 - 4.28 (m, 2H), 4.26 - 4.12 (m, 4H), 3.85 - 3.76 (m, 4H), 3.61 - 3.55 (m, 2H), 3.35 - 3.31 (m, 1H), 3.27 (s, 3H), 3.18 - 3.12 (m, 1H), 2.99 - 2.93 (m, 1H), 2.79 - 2.73 (m, 1H), 2.45 - 2.37 (m, 4H), 2.24 (s, 3H), 2.10 - 2.03 (m, 1H), 1.82 - 1.73 (m, 2H), 1.54 - 1.45 (m, 2H), 1.39 (d, *J =* 6.0 Hz, 3H), 1.10 - 1.03 (m, 4H), 0.94 - 0.88 (m, 6H), 0.87 - 0.83 (m, 1H), 0.59 - 0.53 (m, 1H), 0.36 (s, 3H).

### Example 55

### (1S,2S)-N-((6³S,4S,Z)-1¹-ethyl-1²-(2-((S)-1-methoxyethyl)-5-(2-(piperidin-1-ylmethyl)pyrimidin-5-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

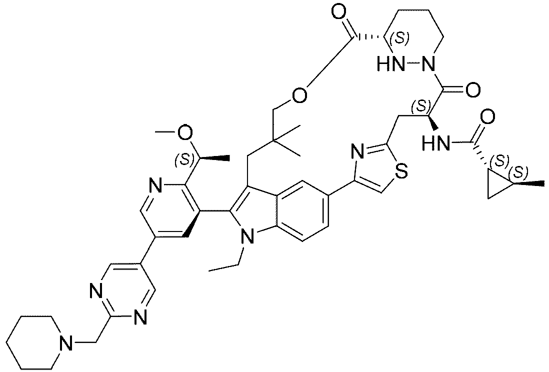

By replacing INT-7 in the synthesis step of compound 2 with INT-61, compound 55 can be obtained using similar methods and reaction steps. ESI-MS (m/z): 888.8 [M+H]⁺; LC-MS retention time RT=1.83 min, HPLC retention time RT=13.52 min.

¹H NMR (500 MHz, DMSO-*d*₆) δ 9.27 (s, 2H), 9.21 (d, *J =* 2.5 Hz, 1H), 8.56 - 8.48 (m, 2H), 8.32 (d, *J =* 2.5 Hz, 1H), 7.82 (s, 1H), 7.79 - 7.72 (m, 1H), 7.60 (d, *J =* 8.5 Hz, 1H), 5.56 (t, *J = 9.0* Hz, 1H), 5.11 - 5.05 (m, 1H), 4.39 - 4.33 (m, 2H), 4.26 - 4.12 (m, 3H), 3.72 (s, 2H), 3.63 - 3.52 (m, 2H), 3.33 - 3.30 (m, 1H), 3.29 (s, 3H), 3.16 - 3.12 (m, 1H), 2.97 - 2.92 (m, 1H), 2.80 - 2.71 (m, 1H), 2.49 - 2.45 (m, 3H), 2.10 - 2.05 (m, 1H), 1.82 - 1.77 (m, 2H), 1.55 - 1.45 (m, 7H), 1.40 (d, *J =* 6.0 Hz, 3H), 1.39 - 1.33 (m, 2H), 1.10 - 1.06 (m, 4H), 0.95 - 0.89 (m, 6H), 0.88 - 0.84 (m, 1H), 0.58 - 0.53 (m, 1H), 0.37 (s, 3H).

### Example 56

### (1S,2S)-N-((6³S,4S,Z)-1¹-ethyl-1²-(2-((S)-1-methoxyethyl)-5-(2-morpholinopyrimidin-5-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

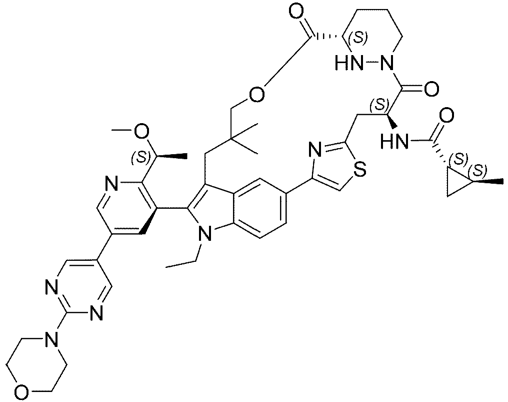

By replacing INT-7 in the synthesis step of compound 2 with INT-62, compound 56 can be obtained using similar methods and reaction steps. ESI-MS (m/z): 876.8 [M+H]⁺; LC-MS retention time RT=1.87 min, HPLC retention time RT=13.86 min.

### Example 57

### (1S,2S)-N-((6³S,4S,Z)-1¹-ethyl-1²-(2-((S)-1-methoxyethyl)-5-(2-methoxypyrimidin-5-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

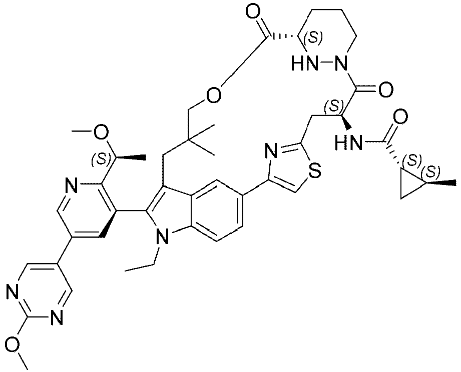

By replacing INT-7 in the synthesis step of compound 2 with INT-63, compound 57 can be obtained using similar methods and reaction steps. ESI-MS (m/z): 821.7 [M+H]⁺; LC-MS retention time RT=1.79 min

¹H NMR (500 MHz, DMSO-*d*₆) δ 9.15 (d, *J =* 2.5 Hz, 1H), 9.14 - 9.11 (m, 2H), 8.55 - 8.49 (m, 2H), 8.24 (d, *J =* 2.5 Hz, 1H), 7.82 (s, 1H), 7.80 - 7.75 (m, 1H), 7.61 (d, *J* = 8.5 Hz, 1H), 5.57 (t, *J* = 9.0 Hz, 1H), 5.12 - 5.07 (m, 1H), 4.41 - 4.31 (m, 2H), 4.30 - 4.12 (m, 3H), 3.99 (s, 3H), 3.64 - 3.55 (m, 2H), 3.37 - 3.33 (m, 1H), 3.29 (s, 3H), 3.19 - 3.12 (m, 1H), 3.03 - 2.93 (m, 1H), 2.81 - 2.72 (m, 1H), 2.13 - 2.04 (m, 1H), 1.85 - 1.74 (m, 2H), 1.56 - 1.48 (m, 2H), 1.41 (d, *J =* 6.0 Hz, 3H), 1.11 - 1.04 (m, 4H), 0.95 - 0.86 (m, 7H), 0.60 - 0.51 (m, 1H), 0.37 (s, 3H).

### Example 58

### (1S,2S)-N-((6³S,4S,Z)-1¹-ethyl-1²-(2-((S)-1-methoxyethyl)-5-(2-((4-(2-methoxyethyl)piperazin-1-yl)methyl)pyrimidin-5-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

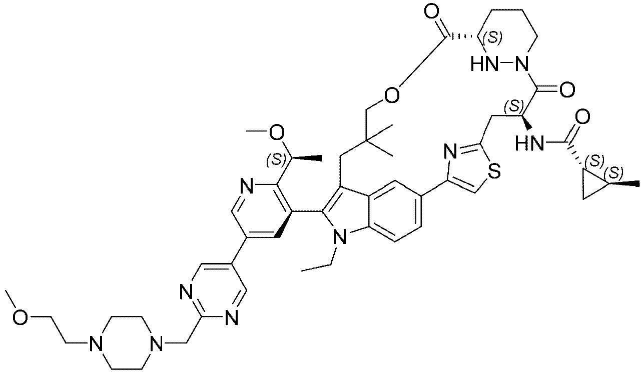

Example 58 was prepared by the following steps:

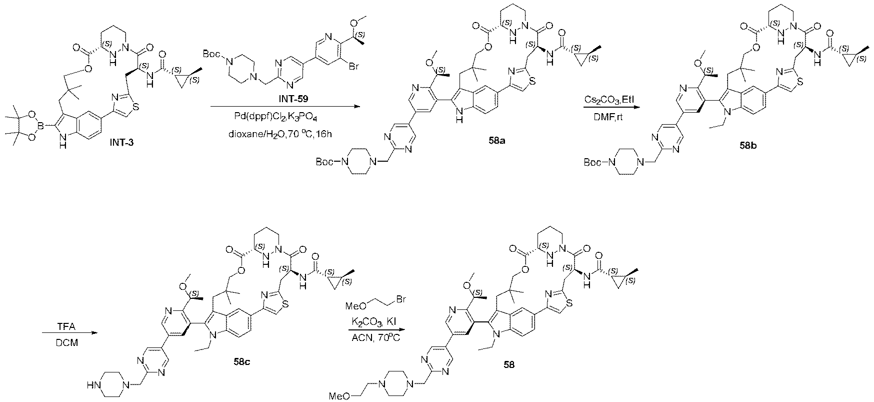

By replacing INT-7 in the synthesis step of compound 2 with INT-59, compound 58c can be obtained using similar methods and reaction steps. ESI-MS (m/z): 889.7 [M+H]⁺;
Step 4: Compound 58c (70 mg, 0.079 mmol) was dissolved in acetonitrile (5 mL), and 2-bromoethyl methyl ether (17 mg, 0.12 mmol), potassium carbonate (11 mg, 0.079 mmol), and potassium iodide (13 mg, 0.079 mmol) were added in sequence. The reaction mixture was stirred at 70 °C for 16 hours. After the reaction was complete, water (20 mL) was added to the reaction system, and the mixture was extracted with ethyl acetate (20 mL*2). The organic phases were combined, washed with saturated brine, dried over anhydrous sodium sulfate, filtered, and concentrated. The residue was purified by preparative liquid chromatography to obtain white solid compound 58 (5 mg, yield 6.7%). ESI-MS (m/z): 947.7 [M+H]⁺. LC-MS retention time RT=1.58 min, HPLC retention time RT=11.19 min.

¹H NMR (500 MHz, DMSO-*d*₆) δ 9.25 - 9.21 (m, 3H), 8.57 - 8.51 (m, 2H), 8.49 (d, *J =* 2.5 Hz, 1H), 7.83 (s, 1H), 7.78 - 7.73 (m, 1H), 7.58 (d, *J =* 8.5 Hz, 1H), 5.56 (t, *J* = 9.0 Hz, 1H), 5.11 - 5.06 (m, 1H), 4.27 - 4.20 (m, 2H), 4.08 - 3.96 (m, 2H), 3.94 - 3.88 (m, 1H), 3.76 (s, 2H), 3.72 - 3.65 (m, 1H), 3.60 - 3.55 (m, 1H), 3.41 (t, *J =* 5.9 Hz, 4H), 3.22 (s, 3H), 3.15 (s, 3H), 3.10 - 3.05 (m,1H), 2.82 - 2.75 (m, 1H), 2.48 - 2.39 (m, 6H), 2.17 - 2.10 (m, 1H), 1.85 - 1.77 (m, 2H), 1.55 - 1.48 (m, 2H), 1.43 - 1.40 (m, 1H), 1.30 (d, *J =* 6.0 Hz, 3H), 1.24 (s, 3H), 1.13 (t, *J =* 7.0 Hz, 3H), 1.09 - 1.06 (m, 4H), 0.93 (s, 3H), 0.90 - 0.84 (m, 2H), 0.59 - 0.55 (m, 1H), 0.53 (s, 3H).

### Example 59

### (1S,2S)-N-((6³S,4S,Z)-1²-(5-(2-(dimethylamino)pyrimidin-5-yl)-2-((S)-1-methoxyethyl)pyridin-3-yl)-1¹-ethyl-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

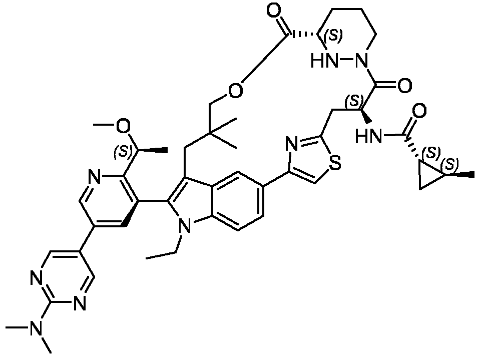

By replacing INT-7 in the synthesis step of compound 2 with INT-64, compound 59 can be obtained using similar methods and reaction steps. ESI-MS (m/z): 834.7 [M+H]⁺; LC-MS retention time RT=1.94 min, HPLC retention time RT=14.49 min.

¹H NMR (500 MHz, DMSO-*d*₆) δ 8.99 (d, *J =* 2.5 Hz, 1H), 8.80 - 8.77 (m, 2H), 8.46 - 8.41 (m, 2H), 8.02 (d, *J =* 2.5 Hz, 1H), 7.74 (s, 1H), 7.71 - 7.66 (m, 1H), 7.54 - 7.49 (m, 1H), 5.52 - 5.47 (m, 1H), 5.02 - 4.99 (m, 1H), 4.34 - 4.03 (m, 5H), 3.55 - 3.48 (m, 2H), 3.34 - 3.32 (m, 2H), 3.20 (s, 3H), 3.11 (s, 6H), 3.09 - 3.03 (m, 1H), 2.92 - 2.86 (m, 1H), 2.73 - 2.65 (m, 1H), 2.04 - 1.98 (m, 1H), 1.77 - 1.69 (m, 2H), 1.47 - 1.40 (m, 2H), 1.32 (d, *J =* 6.0 Hz, 3H), 1.00 (s, 3H), 0.97 - 0.96 (m, 1H), 0.87 - 0.82 (m, 6H), 0.79 - 0.77 (m, 1H), 0.49 - 0.46 (m, 1H), 0.29 (s, 3H).

### Example 60

### (1S,2S)-N-((6³S,4S,Z)-1¹-ethyl-1²-(2-((S)-1-methoxyethyl)-5-(2-((1-methylpiperidin-4-yl)oxy)pyrimidin-5-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

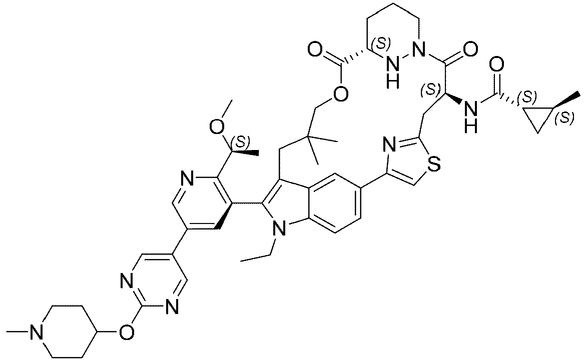

By replacing INT-7 in the synthesis step of compound 2 with INT-65, compound 60 can be obtained using similar methods and reaction steps. ESI-MS (m/z): 904.7 [M+H]⁺; LC-MS retention time RT = 1.69 min.

### Example 61

### (1S,2S)-N-((6³S,4S,Z)-1¹-ethyl-1²-(5-(2-(((S)-hexahydropyrazino [2,1-c][1,4]oxazin-8(1H)-yl)methyl)pyrimidin-5-yl)-2-((S)-1-methoxyethyl)pyridin-3-yl)-10,10-dimethyl-5,7-dioxo-6¹,6²,63,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

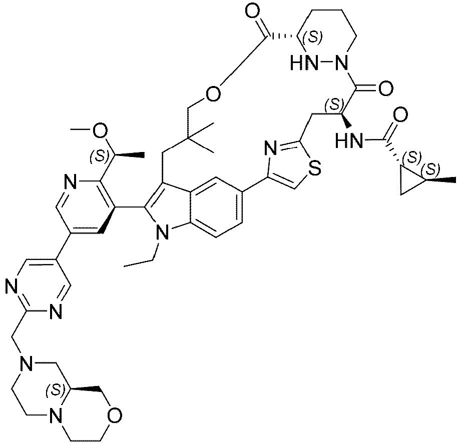

By replacing INT-7 in the synthesis step of compound 2 with INT-66, compound 60 can be obtained using similar methods and reaction steps. ESI-MS (m/z): 945.7 [M+H]⁺; LC-MS retention time RT=1.57 min, HPLC retention time RT=10.94 min.

### Example 62

### (1r,2R,3S)-N-((6³S,4S,Z)-1¹-ethyl-1²-(2-((S)-1-methoxyethyl)-5-(1-(((R)-1-methylpyrrolidin-2-yl)methyl)-1H-pyrazol-4-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2,3-dimethylcyclopropane-1-carboxamide

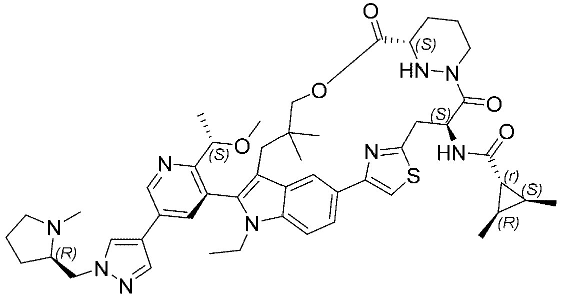

By replacing INT-7 in the synthesis step of compound 2 with INT-67 and INT-3 with INT-4, compound 62 can be obtained using similar methods and reaction steps. ESI-MS (m/z): 890.7 [M+H]⁺; LC-MS retention time RT=1.78 min, HPLC retention time RT=13.07 min.

¹H NMR (500 MHz, DMSO-*d*₆) δ 9.01 (d, *J =* 2.0 Hz, 1H), 8.52 - 8.49 (m, 1H), 8.45 (s, 1H), 8.37 (d, *J =* 9.0 Hz, 1H), 8.16 - 8.09 (m, 1H), 8.01 (d, *J =* 2.0 Hz, 1H), 7.81 (s, 1H), 7.77 - 7.73 (m, 1H), 7.58 (d, *J* = 9.0 Hz, 1H), 5.56 (t, *J =* 9.0 Hz, 1H), 5.10 - 5.03 (m, 1H), 4.36 - 4.10 (m, 7H), 3.62 - 3.55 (m, 2H), 3.25 (s, 3H), 3.17 - 3.11 (m, 2H), 2.99 - 2.93 (m, 1H), 2.79 - 2.72 (m, 2H), 2.47 - 2.42 (m, 1H), 2.10 - 2.05 (m, 1H), 1.82 - 1.75 (m, 3H), 1.70 - 1.63 (m, 2H), 1.56 - 1.48 (m, 2H), 1.38 (d, *J=* 6.0 Hz, 3H), 1.27 - 1.22 (m, 2H), 1.20 - 1.13 (m, 4H), 1.11 - 1.05 (m, 6H), 0.94 - 0.86 (m, 6H), 0.38 (s, 3H).

### Example 63

### (1S,2S)-N-((6³S,4S,Z)-1¹-ethyl-1²-(2-((S)-1-methoxyethyl)-5-(1-(1-methylpiperidin-4-yl)-1H-pyrazol-4-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

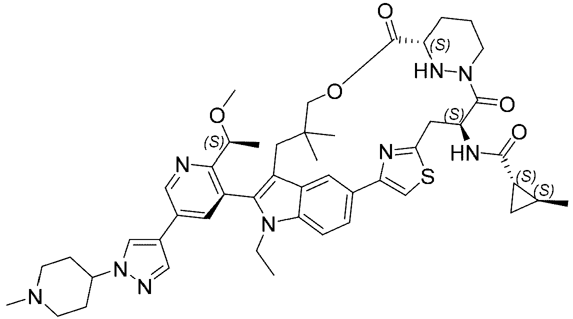

By replacing INT-7 in the synthesis step of compound 2 with INT-68, compound 63 can be obtained using similar methods and reaction steps. ESI-MS (m/z): 876.7 [M+H]⁺; LC-MS retention time RT=1.67 min, HPLC retention time RT=12.26 min.

¹H NMR (500 MHz, DMSO-*d*₆) δ 9.02 (d, *J =* 2.5 Hz, 1H), 8.57 - 8.53 (m, 2H), 8.48 (s, 1H), 8.15 (d, *J =* 2.5 Hz, 1H), 8.07 (s, 1H), 7.82 (s, 1H), 7.76 - 7.73 (m, 1H), 7.56 (d, *J =* 8.5 Hz, 1H), 5.56 (t, *J =* 9.0 Hz, 1H), 5.10 - 5.03 (m, 1H), 4.27 - 4.21 (m, 2H), 4.17 - 4.10 (m, 1H), 4.02 - 3.95 (m, 2H), 3.93 - 3.85 (m, 1H), 3.73 - 3.65 (m, 1H), 3.60 - 3.54 (m, 1H), 3.19 - 3.15 (m, 1H), 3.12 (s, 3H), 3.09 - 3.03 (m, 1H), 2.90 - 2.85 (m, 2H), 2.80 - 2.75 (m, 1H), 2.47 - 2.42 (m, 1H), 2.22 (s, 3H), 2.14 - 2.09 (m, 1H), 2.07 - 2.02 (m, 4H), 2.00 - 1.96 (m, 1H), 1.85 - 1.80 (m, 2H), 1.54 - 1.49 (m, 2H), 1.27 - 1.23 (m, 4H), 1.13 (d, *J =* 7.0 Hz, 3H), 1.09 - 1.05 (m, 4H), 0.92 (s, 3H), 0.93 - 0.85 (m, 2H), 0.58 - 0.54 (m, 1H), 0.51 (s, 3H).

### Example 64

### (1S,2S)-N-((6³S,4S,Z)-1¹-ethyl-1²-(2-((S)-1-methoxyethyl)-5-(1-(tetrahydro-2H-pyran-4-yl)-1H-pyrazol-4-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

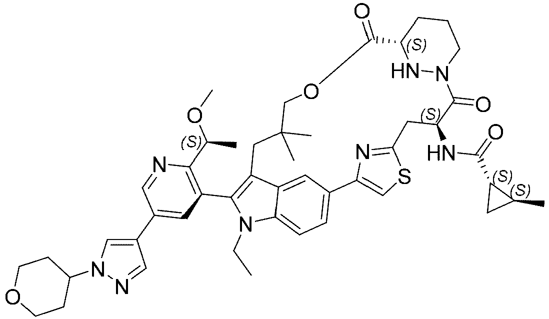

By replacing INT-7 in the synthesis step of compound 2 with INT-69, compound 64 can be obtained using similar methods and reaction steps. ESI-MS (m/z): 863.7 [M+H]⁺; LC-MS retention time RT=1.74 min, HPLC retention time RT=12.63 min.

¹H NMR (500 MHz, DMSO-*d*₆) δ 8.95 (d, *J =* 2.5 Hz, 1H), 8.49 - 8.45 (m, 2H), 8.42 (s, 1H), 8.08 (d, *J =* 2.5 Hz, 1H), 8.02 (s, 1H), 7.74 (s, 1H), 7.69 - 7.65 (m, 1H), 7.48 (d, *J =* 8.5 Hz, 1H), 5.49 (t, *J =* 9.0 Hz, 1H), 5.03 - 4.96 (m, 1H), 4.42 - 4.31 (m, 1H), 4.20 - 4.13 (m, 2H), 3.94 - 3.88 (m, 4H), 3.87 - 3.76 (m, 1H), 3.65 - 3.60 (m, 1H), 3.52 - 3.47 (m, 1H), 3.45 - 3.38 (m, 2H), 3.13 - 3.08 (m, 1H), 3.05 (s, 3H), 3.03 - 2.95 (m, 1H), 2.74 - 2.65 (m, 1H), 2.39 - 2.33 (m, 1H), 2.07 - 2.02 (m, 1H), 1.99 - 1.85 (m, 5H), 1.76 - 1.70 (m, 2H), 1.48 - 1.41 (m, 2H), 1.20 - 1.15 (m, 4H), 1.04 (t, *J* = 7.0 Hz, 3H), 1.02 - 0.96 (m, 3H), 0.85 (s, 3H), 0.83 - 0.79 (m, 1H), 0.51 - 0.46 (m, 1H), 0.43 (s, 3H).

### Example 65

### (1S,2S)-N-((6³S,4S,Z)-1¹-ethyl-1²-(2-((S)-1-methoxyethyl)-5-(1-((1-methylpiperidin-4-yl)methyl)-1H-1,2,3-triazol-4-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

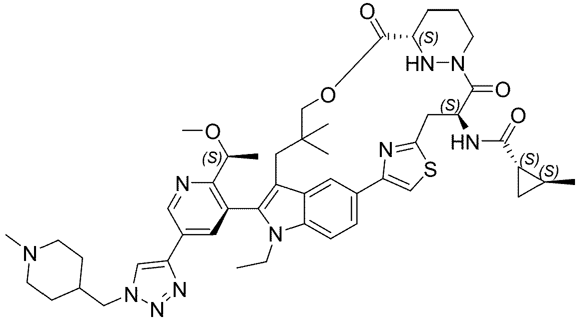

By replacing INT-7 in the synthesis step of compound 2 with INT-70, compound 65 can be obtained using similar methods and reaction steps. ESI-MS (m/z): 891.7 [M+H]⁺; LC-MS retention time RT=1.65 min, HPLC retention time RT=11.63 min.

### Example 66

### (1S,2S)-N-((6³S,4S,Z)-1¹-ethyl-1²-(2-((S)-1-methoxyethyl)-5-(2-(morpholinomethyl)pyrimidin-5-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

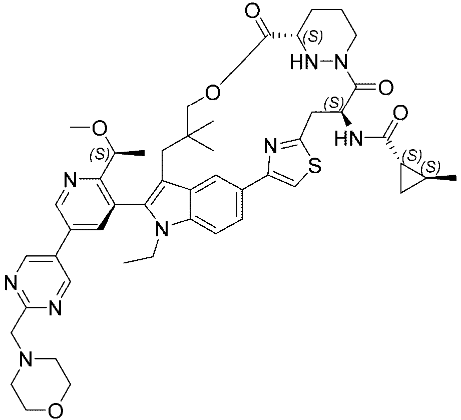

By replacing INT-6 in the synthesis step of compound 1 with INT-47 and INT-4 with INT-3, compound 65 can be obtained using similar methods and reaction steps. ESI-MS (m/z): 890.8 [M+H]⁺; LC-MS retention time RT=1.61 min, HPLC retention time RT=11.42 min.

¹H NMR (500 MHz, DMSO) δ 9.30 - 9.27 (m, 2H), 9.21 (d, *J =* 2.5 Hz, 1H), 8.53 - 8.48 (m, 2H), 8.31 (d, *J =* 2.5 Hz, 1H), 7.81 (s, 1H), 7.78 - 7.74 (m, 1H), 7.60 (d, *J =* 8.5 Hz, 1H), 5.56 (t, *J =* 9.0 Hz, 1H), 5.10 - 5.05 (m, 1H), 4.40 - 4.32 (m, 2H), 4.27 - 4.11 (m, 4H), 3.77 (s, 2H), 3.62 - 3.55 (m, 6H), 3.29 (s, 3H), 3.18 - 3.09 (m, 2H), 3.00 - 2.94 (m, 1H), 2.79 - 2.71 (m, 1H), 2.56 - 2.52 (m, 4H), 2.10 - 2.05 (m, 1H), 1.82 - 1.76 (m, 2H), 1.53 - 1.47 (m, 2H), 1.41 (d, *J =* 6.0 Hz, 3H), 1.10 - 1.05 (m, 4H), 0.95 - 0.88 (m, 6H), 0.87 - 0.84 (m, 1H), 0.57 - 0.52 (m, 1H), 0.37 (s, 3H).

### Biological screening of RAS inhibitors and results

### Test Example 1: In vitro cell proliferation inhibition test

Due to the diversity of RAS mutations and in order to evaluate the activity of compounds in different RAS mutation cell lines, we selected KRAS^{WT}, KRAS^{G12C}, KRAS^{G12D}, KRAS^{G12V} and BRAF mutation cell lines (see the table below) for in vitro activity evaluation and screening of compounds.

| **Cell Line** | **Histotype** | **Mutant** |
|---|---|---|
| NCI-H358 | Lung; Bronchiole | KRAS (p.G12C) |
| MIA PaCa-2 | Pancreas | KRAS (p.G12C) |
| LS513 | Large intestine; Cecum | KRAS (p.G12D) |
| AsPC-1 | Pancreas | KRAS (p.G12D) |
| HCC1588 | Lung | KRAS (p.G12D); BRAF (p.E204L) |
| SW480 | Large intestine; Colon | KRAS (p.G12V) |
| NCI-H727 | Lung; Bronchus | KRAS (p.G12V) |
| NCI-H520 | Lung | KRAS^{WT}; |
| HT-29 | Colon | KRAS^{WT}; BRAF (p.V600E) |

| | | |
|---|---|---|
| Protocol: CellTiter-Glo^{®} Cell Luminescent Viability Assay (Promega) | | |

According to the doubling time of different cell lines, different numbers of cells (1000-5000 cells/well) were seeded in 96-well plates containing 180 µl of corresponding culture medium and cultured overnight in a cell culture incubator at 37°C with 5% CO₂. On the second day, the test compound was pre-diluted 3-fold with culture medium, with the highest concentration being 100 µM, for a total of 10 concentration gradients; then 20 µl of culture medium containing different concentrations of the compound was added to the cells in the 96-well plate to ensure that the final concentration of the compound was up to 10 µM, with 10 concentration gradients of 3-fold dilution. After the cells and compounds were co-incubated for 72 h, the 96-well plate was removed from the incubator and equilibrated at room temperature for 30 min. Then, 25 µl of CellTiter-Glo^{®} Reagent was added to each well and mixed thoroughly. The mixture was incubated at room temperature for 10 min, and then 100 µl of the sample was transferred to a white 96-well plate (OptiPlateTM-96, PerkinElmer). The fluorescence signal value was read using a multi-function microplate reader (SpectraMax^{®} i3x, Molecular devices). The signal values were then standardized, and the curve was fitted using a four-parameter regression equation to calculate the half maximal inhibitory concentration (IC50) of the compound on the cell line.

**Table 3: Antiproliferative activity of the compounds of the present disclosure against KRAS mutant cell lines**

| Example | IC₅₀ (nM) | | Example | IC₅₀ (nM) | | Example | IC₅₀ (nM) | |
|---|---|---|---|---|---|---|---|---|
| | AsPC-1 (G12D) | SW480 (G12V) | | AsPC-1 (G12D) | SW480 (G12V) | | AsPC-1 (G12D) | SW480 (G12V) |
| **1** | 1.1 | 1.3 | **18'** | 486 | 32 | **39** | 0.57 | 0.48 |
| **1'** | 4772 | NT | **19** | 0.57 | 4.5 | **40** | 9.2 | 10 |
| **2** | 21 | 670 | **19'** | 33 | 16 | **41** | 2.6 | 3.7 |
| **2'** | 2495 | 820 | **20** | 3.4 | 3 | **42** | 3.0 | 3.5 |
| **3** | 0.18 | 0.33 | **20'** | 87 | NT | **43** | 17 | 3.8 |
| **3'** | 1231 | NT | **21** | 11 | 15 | **44** | 0.26 | 0.35 |
| **4** | 2.6 | 1.6 | **21'** | 1768 | NT | **45** | 5.3 | 6.2 |
| **4'** | 646 | NT | **22** | 11 | 10 | **46** | 6.1 | 4.1 |
| **5** | 6 | 10 | **22'** | >10000 | 161 | **47** | 2.9 | 6.1 |
| **5'** | 99 | NT | **23** | 42 | 117 | **48** | 0.54 | 0.51 |
| **6** | 13 | 23 | **23'** | 2368 | 1342 | **49** | 1.0 | 4.3 |
| **6'** | 1458 | NT | **24** | 26 | 19 | **50** | 8.5 | 7.8 |
| **7** | 128 | 159 | **24'** | >10000 | 3608 | **51** | 0.9 | 1.2 |
| **7'** | >10000 | NT | **25** | 20 | 22 | **52** | 0.21 | 1.2 |
| **8** | 0.7 | 1.4 | **25'** | 3419 | >10000 | **53** | 4.3 | 1.7 |
| **8'** | 236 | NT | **26** | 10 | 2.5 | **54** | 2.2 | 5.7 |
| **9** | 3.5 | 18 | **26'** | 13 | NT | **55** | 6.8 | 15 |
| **9'** | 836 | NT | **27** | 1.1 | 1.8 | **56** | 16 | 21 |
| **10** | 18 | 0.5 | **27'** | 7.7 | 50 | **57** | 5.6 | 4.6 |
| **10'** | 1799 | NT | **29** | 3.2 | 10 | **58** | 68 | 30 |
| **11** | 2.1 | 3.2 | **29'** | 5.3 | 11.4 | **59** | 3.0 | 6.2 |
| **11'** | 242 | 346 | **30** | 0.58 | 0.44 | **60** | 1.6 | 12 |
| **12** | 16 | 6.6 | **30'** | 60 | NT | **61** | 19 | 20 |
| **12'** | 814 | 319 | **31** | 1.6 | 2.0 | **62** | 0.54 | 3.9 |
| **13** | 2.5 | 2.0 | **31'** | 53 | NT | **63** | 48 | 71 |
| **13'** | 302 | 236 | **32** | 1.3 | 1.6 | **65** | 12 | 150 |
| **14** | 1.7 | 2.8 | **32'** | 33 | NT | **66** | 10 | 3.4 |
| **14'** | 1180 | 633 | **33** | 0.86 | 2.1 | | | |
| **15** | 3.0 | 6.0 | **33'** | 32 | NT | | | |
| **15'** | 1016 | NT | **34** | 0.16 | 0.17 | | | |
| **16** | 1.6 | 7.9 | **34'** | 116 | NT | | | |
| **16'** | 2282 | 265 | **35** | 4.0 | 2.0 | | | |
| **17** | 0.31 | 2.5 | **36** | 0.39 | 1.0 | | | |
| **17'** | 697 | 85 | **37** | 0.9 | 1.8 | | | |
| **18** | 0.45 | 1.4 | **38** | 66 | 23 | | | |

## Claims

1. A compound having a structure of Formula (I), or a pharmaceutically acceptable salt, isotope derivative, or stereoisomer thereof: 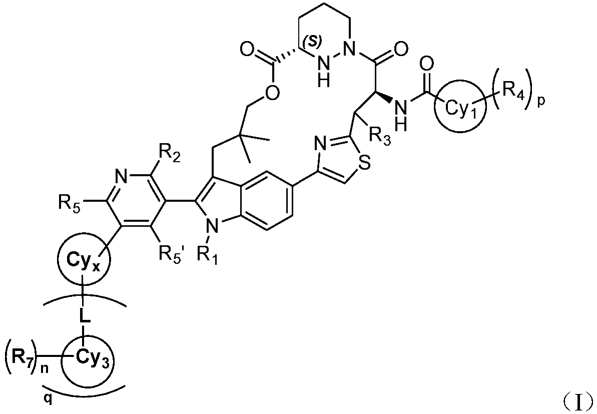
wherein, R₁ represents C₁-C₆ alkyl, -(C₁-C₆ alkylene)-(C₃-C₈ cycloalkyl), -(C₁-C₆ alkylene)-(4-8 membered heterocycloalkyl), -(C₁-C₆ alkylene)-ORa, -(C₁-C₆ alkylene)-SRa or -(C₁-C₆ alkylene)-NRaRa';
R₂ represents halogen, cyano, C₁-C₆ alkyl, -(C₀-C₆ alkylene)-(C₃-C₈ cycloalkyl), or -(C₀-C₆ alkylene)-(4-8 membered heterocycloalkyl), which is optionally substituted with 0, 1, or 2 substituents selected from -ORa, -SRa, or -NRaRa';
R₃ represents hydrogen, -O(C₀-C₆ alkylene)Ra, -S(C₀-C₆ alkylene)Ra, -N(C₀-C₆ alkylene)Ra(C₀-C₆ alkylene)Rₐ', which is optionally substituted with 0, 1, or 2 substituents selected from -ORa, -SRa, or -NRa Ra';
Cy₁ represents C₃-C₁₂ cycloalkyl or 4-12 membered heterocycloalkyl;
R₄ each independently represents hydrogen, halogen, oxo, C₁-C₆ alkyl, -(C₀-C₆ alkylene)(C₃-C₆) cycloalkyl, -(C₀-C₆ alkylene)(4-8 membered) heterocycloalkyl, -(C₀-C₆ alkylene)ORa, -(C₀-C₆ alkylene)SRa, -(C₀-C₆ alkylene)NRaRa', -(C₀-C₆ alkylene)CORa, -(C₀-C₆ alkylene)COORa, -(C₀-C₆ alkylene)CONRaRa', -(C₀-C₆ alkylene)NRaCORa', -(C₀-C₆ alkylene)OCONRaRa', -(C₀-C₆ alkylene) NRaCONRaRa', -(C₀-C₆ alkylene)SORa, -(C₀-C₆ alkylene)S(O)₂Ra, -(C₀-C₆ alkylene)NRaS(O)₂Ra', -(C₀-C₆ alkylene)CN, -(C₀-C₆ alkylene)(C6-C10 aryl) or -(C₀-C₆ alkylene)(5-12 membered heteroaryl); wherein two R₄ groups on two carbon atoms of Cy₁, together with the carbon atoms to which they are attached and the atoms between these two carbon atoms, may form a 3-8 membered ring, and the 3-8 membered ring may optionally contain 0, 1, 2 or 3 heteroatoms selected from N, O or S ; or two R₄ groups on the same carbon atom of Cy₁, together with the carbon atom to which they are attached, may form a 3-8 membered ring, and the 3-8 membered ring may optionally contain 0, 1, 2 or 3 heteroatoms selected from N, O or S;
R₅, R₅' each independently represents hydrogen, halogen, C₁-C₆ alkyl, -(C₀-C₆ alkylene)(C₃-C₈) cycloalkyl or -(C₀-C₆ alkylene)CN;
Cyₓ represents a Cy₂ optionally substituted with m R₆;
Cy₂ is selected from a C₆-C₁₂ aryl, a 5-12 membered heteroaryl, or an oxo-substituted saturated or partially saturated 5-12 membered heterocycloalkyl, wherein the C₆-C₁₂ aryl, the 5-12 membered heteroaryl, or the 5-12 membered heterocycloalkyl may be monocyclic or polycyclic fused rings;
R₆ is each independently selected from: hydrogen, halogen, oxo, C₁-C₆ alkyl, -(C₀-C₆ alkylene)(C₃-C₈ cycloalkyl), -(C₀-C₆ alkylene)(4-8 membered heterocycloalkyl), - (C₀-C₆) alkyleneCN, -(C₀-C₆ alkylene)C(O)NRaRa', -(C₀-C₆ alkylene) NRaC(O) Ra', -(C₀-C₆ alkylene)C(O)Ra, -(C₀-C₆ alkylene)C(O)ORa, -(C₀-C₆ alkylene)OC(O)NRaRa', -(C₀-C₆ alkylene)NRaC(O)ORa', -(C₀-C₆ alkylene)NRaC(O)NRaRa', -(C₀-C₆ alkylene)S(O)₂Ra, -P(O)RaRa', or -(C₀-C₆ alkylene)-ORa, -(C₀-C₆ alkylene)-SRa, or -(C₀-C₆ alkylene)-NRaRa', each optionally substituted with 0, 1, or 2 substituents; or two R₆ groups on two adjacent ring atoms of Cy₂ may together with said two ring atoms form a ring, the ring optionally comprising 0, 1, 2, 3, or 4 heteroatoms selected from N, O, and S, and optionally comprising 0, 1, 2, or 3 unsaturated bonds;
L represents -(C₀-C₆) alkylene-, -(C₀-C₃) alkylene-O-(C₀-C₃) alkylene-, -(C₀-C₃) alkylene-S-(C₀-C₃) alkylene-, or -(C₀-C₃) alkylene-NRa-(C₀-C₃) alkylene-; wherein, the -(C₀-C₆) alkylene- or -(C₀-C₃) alkylene is optionally substituted with 0, 1, 2, or 3 C₁-C₃ alkyl groups, or two C₁-C₃ alkyl substituents on the same carbon atom may form a 3-8 membered ring, which optionally contains 0, 1, 2, or 3 heteroatoms selected from N, O, or S;
Cy₃ is selected from C₃-C₁₂ cycloalkyl and 4- to 12-membered heterocycloalkyl, wherein when Cy₃ is a 4- to 12-membered heterocycloalkyl, Cy₃ may be a spiro ring, bridged ring, or fused ring, and when the ring contains a S or P atom, the S or P atom may optionally be oxidized to -S(O)₂-, -S(O)(NRa)- or -P(O)Ra;
R₇ is each independently selected from: hydrogen, halogen, oxo, C₁-C₆ alkyl, -(C₀-C₆ alkylene)(C₃-C₈ cycloalkyl), -(C₀-C₆ alkylene)(4-8 membered heterocycloalkyl), - (C₀-C₆) alkyleneCN, -(C₀-C₆ alkylene)ORa, -(C₀-C₆ alkylene)SRa, -(C₀-C₆ alkylene)NRaRa', -(C₀-C₆ alkylene)C(O)Ra, -(C₀-C₆ alkylene)C(O)ORa, -(C₀-C₆ alkylene)OC(O)NRaRa', -(C₀-C₆ alkylene)NRaC(O)ORa', -(C₀-C₆ alkylene)NRaC(O)NRaRa', -(C₀-C₆ alkylene)C(O)NRa Ra', -(C₀-C₆ alkylene)S(O)₂Ra, -(C₀-C₆ alkylene)S(O)(NH)Ra; alternatively, two R₇ groups on the same carbon atom of Cy₃ may form a spiro ring, which may optionally contain 0, 1, 2, 3, or 4 heteroatoms selected from N, O, and S; or two R₇ groups on adjacent ring atoms of Cy₃ may form a fused ring with said two ring atoms, which may optionally contain 0, 1, 2, 3, or 4 heteroatoms selected from N, O, and S; or two R₇ groups on non-adjacent ring atoms of Cy₃ may form a bridged ring with said two ring atoms, which may optionally contain 0, 1, 2, 3, or 4 heteroatoms selected from N, O, and S; and optionally, said spiro ring, fused ring, or bridged ring may contain 0, 1, 2, or 3 unsaturated bonds;
alternatively, R₆ and R₇ may together with L form a 4-8 membered ring, which may optionally contain 0, 1, 2, or 3 heteroatoms selected from N, O, and S;
wherein, p represents 0, 1, 2, 3 or 4;
q represents 0, 1 or 2;
m, n each independently represent 0, 1, 2, 3 or 4;
Ra and Ra' each independently represent hydrogen, C₁-C₆ alkyl, C₃-C₈ cycloalkyl, or 4-8 membered heterocycloalkyl; wherein, when Ra and Ra' are bonded to the same nitrogen atom, Ra, Ra', and the nitrogen atom together may form a 4-8 membered ring, which may optionally contain 0, 1, 2, or 3 heteroatoms selected from N, O, or S;
the alkyl, cycloalkyl, heterocycloalkyl and alkylene may be substituted by 0, 1, 2, 3, 4, 5 or 6 halogen atoms independently.

2. The compound having the structure of formula (I) or a pharmaceutically acceptable salt, isotope derivative, stereoisomer thereof according to claim 1, wherein, R₁ represents C₁-C₆ alkyl, -(C₁-C₆ alkylene)-(C₃-C₈ cycloalkyl) or -(C₁-C₆ alkylene)-(4-8 membered heterocycloalkyl); preferably, R₁ represents C₁-C₆ alkyl; more preferably, R₁ represents C₁-C₃ alkyl.

3. The compound having the structure of formula (I) or a pharmaceutically acceptable salt, isotope derivative, stereoisomer thereof according to any one of the preceding claims, wherein, R₂ represents C₁-C₆ alkyl, which may be optionally substituted by 0, 1 or 2 -ORa substituents; preferably, R₂ represents 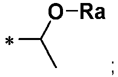 more preferably, R₂ represents 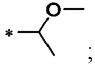 and even more preferably, R₂ represents 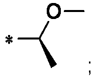 wherein * represents the site where R₂ is attached to the corresponding position in formula (I).

4. The compound having the structure of formula (I) or a pharmaceutically acceptable salt, isotope derivative, stereoisomer thereof according to any one of the preceding claims, wherein R₃ represents hydrogen or -O(C₁-C₆)alkyl, -O(C₀-C₆ alkylene)(C₃-C₈)cycloalkyl, or -O(C₀-C₆ alkylene)(4-8 membered)heterocycloalkyl, which is optionally substituted with 0 or 1 substituent selected from ORa, -SRa, or NRaRa'.

5. The compound having the structure of formula (I) or a pharmaceutically acceptable salt, isotope derivative, stereoisomer thereof according to any one of the preceding claims, wherein Cy₁ represents C₃-C₈ cycloalkyl or 4-8 membered heterocycloalkyl.

6. The compound having the structure of formula (I) or a pharmaceutically acceptable salt, isotope derivative, stereoisomer thereof according to any one of the preceding claims, wherein R₄ each independently represents hydrogen, halogen, C₁-C₆ alkyl, -(C₀-C₆ alkylene)ORₐ, -(C₀-C₆ alkylene)SRa, -(C₀-C₆ alkylene)NRaRa', -(Co-C₆ alkylene)CONRaRa', -(C₀-C₆ alkylene)NRaCORa', -(C₀-C₆ alkylene)OCONRaRa', -(C₀-C₆ alkylene)CN, or -(C₀-C₆ alkylene)(5-12 membered heteroaryl); or two R₄ groups on two carbon atoms of Cy₁, together with the carbon atoms to which they are attached and the atoms between these two carbon atoms, may form a 3-8 membered ring, which optionally contains 0, 1, 2, or 3 heteroatoms selected from N, O, or S; or two R₄ groups on the same carbon atom of Cy₁, together with the carbon atom to which they are attached, may form a 3-8 membered ring, which optionally contains 0, 1, 2, or 3 heteroatoms selected from N, O, or S.

7. The compound having the structure of formula (I) or a pharmaceutically acceptable salt, isotope derivative, stereoisomer thereof according to any one of the preceding claims, wherein R₄ each independently represents hydrogen, halogen, C₁-C₆ alkyl, -(C₀-C₆ alkylene)CONRaRa', or -(C₀-C₆ alkylene)(5-12 membered heteroaryl); or two R₄ groups on two carbon atoms of Cy₁, together with the carbon atoms to which they are attached and the atoms between these two carbon atoms, may form a 3-8 membered ring, which optionally contains 0, 1, 2, or 3 heteroatoms selected from N, O, or S; or two R₄ groups on the same carbon atom of Cy₁, together with the carbon atom to which they are attached, may form a 3-8 membered ring, which optionally contains 0, 1, 2, or 3 heteroatoms selected from N, O, or S.

8. The compound having the structure of formula (I) or a pharmaceutically acceptable salt, isotope derivative, stereoisomer thereof according to any one of the preceding claims, wherein R₅, R₅' each independently represents hydrogen or C₁-C₆ alkyl; more preferably, R₅, R₅' each independently represents hydrogen or methyl.

9. The compound having the structure of formula (I) or a pharmaceutically acceptable salt, isotope derivative, stereoisomer thereof according to any one of the preceding claims, wherein, Cy₂ is selected from a benzene ring, a 5-6 membered heteroaryl, or an oxo-substituted saturated or partially saturated 5-6 membered heterocyclic group; wherein the 5-6 membered heterocyclic group preferably contains a nitrogen atom.

10. The compound having the structure of formula (I) or a pharmaceutically acceptable salt, isotope derivative, stereoisomer thereof according to any one of the preceding claims, wherein, when Cy₂ represents a six-membered ring, Cy₂ is selected from:

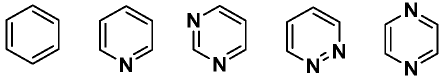

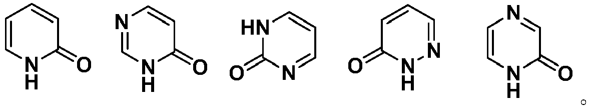

11. The compound having the structure of formula (I) or a pharmaceutically acceptable salt, isotope derivative, stereoisomer thereof according to any one of claims 1 to 8, wherein Cy₂ is selected from a 5-membered monocyclic heteroaromatic ring or a 9-membered fused heteroaromatic ring; wherein the 5-membered or 9-membered heterocyclic group preferably contains a nitrogen atom.

12. The compound having the structure of formula (I) or a pharmaceutically acceptable salt, isotope derivative, stereoisomer thereof according to any one of the preceding claims, wherein R₆ is each independently selected from: hydrogen, halogen, oxo, C₁-C₆ alkyl, -(C₀-C₆ alkylene)(C₃-C₈ cycloalkyl), -(C₀-C₆) alkylene-CN, -(C₀-C₆ alkylene)(4- to 8-membered heterocycloalkyl), -(C₀-C₆ alkylene)-C(O)NRaRa', -(Co-C₆ alkylene)-NRaC(O)Ra', -(C₀-C₆ alkylene)-S(O)₂Ra, or -P(O)RaRa' or -(C₀-C₆ alkylene)-ORa, -(C₀-C₆ alkylene)-SRa, or -(C₀-C₆ alkylene)-NRaRa', each optionally substituted with 0, 1, or 2 substituents; or as described in claim 1, two R₆ groups on adjacent ring atoms of Cy₂ may together with the ring atoms to which they are attached form a ring, which optionally contains 0, 1, 2, 3, or 4 heteroatoms selected from N, O, and S, and optionally contains 0, 1, 2, or 3 unsaturated bonds; when two R₆ groups form a ring containing unsaturated bonds, the ring is preferably an aromatic heterocycle.

13. The compound having the structure of formula (I) or a pharmaceutically acceptable salt, isotope derivative, stereoisomer thereof according to any one of the preceding claims, wherein L represents -(C₀-C₃) alkylene-, optionally substituted with 0, 1, 2, or 3 C₁-C₃ alkyl groups, or two C₁-C₃ alkyl substituents on the same carbon atom may form a 3- to 4-membered ring.

14. The compound having the structure of formula (I) or a pharmaceutically acceptable salt, isotope derivative, stereoisomer thereof according to any one of the preceding claims, wherein L represents -(C₀-C₁) alkylene-O-(C₀-C₁) alkylene-, -(C₀-C₁) alkylene-S-(C₀-C₁) alkylene-, or -(C₀-C₁) alkylene-NRa-(C₀-C₁) alkylene-, wherein the C₁ alkylene is optionally substituted with 0, 1, or 2 C₁-C₃ alkyl groups, or two C₁-C₃ alkyl substituents on the same carbon atom may form a 3-4 membered ring; more preferably, L represents -O-, -S-, or -NRa-.

15. The compound having the structure of formula (I) or a pharmaceutically acceptable salt, isotope derivative, stereoisomer thereof according to any one of the preceding claims, wherein Cy₃ is selected from 4- to 8-membered heterocycloalkyl, which may be a spirocyclic, bridged, or fused ring system, and when the ring contains an S or P atom, the S or P atom is optionally oxidized to -S(O)₂-, -S(O)(NRa)-, or - P(O)Ra-.

16. The compound having the structure of formula (I) or a pharmaceutically acceptable salt, isotope derivative, stereoisomer thereof according to any one of the preceding claims, wherein R₇ is each independently selected from: hydrogen, halogen, oxo, C₁-C₆ alkyl, -(C₀-C₆ alkylene)(4- to 8-membered heterocycloalkyl), -(C₀-C₆) alkylene-CN, -(C₀-C₆ alkylene)-ORa, -(C₀-C₆ alkylene)-SRa, -(C₀-C₆ alkylene)-NRaRa', -(C₀-C₆ alkylene)-C(O)Ra, -(C₀-C₆ alkylene)-OC(O)NRaRa', -(C₀-C₆ alkylene)-NRaC(O)ORa', -(C₀-C₆ alkylene)-NRaC(O)NRaRa', -(C₀-C₆ alkylene)-C(O)NRaRa', -(C₀-C₆ alkylene)-S(O)₂Ra, or -(C₀-C₆ alkylene)-S(O)(NH)Ra; or as described in claim 1, two R₇ groups may form a spirocyclic, bridged, or fused ring system, which optionally contains 0, 1, 2, 3, or 4 heteroatoms selected from N, O, and S, and optionally contains 0, 1, 2, or 3 unsaturated bonds; when two R' groups form a ring containing unsaturated bonds, the ring is preferably an aromatic heterocycle.

17. The compound having the structure of formula (I) or a pharmaceutically acceptable salt, isotope derivative, stereoisomer thereof according to any one of the preceding claims, wherein, when q is 0, one of R₆ contains at least one amino group, preferably a secondary amine or a tertiary amine, more preferably a tertiary amine.

18. The compound having the structure of formula (I) or a pharmaceutically acceptable salt, isotope derivative, stereoisomer thereof according to any one of the preceding claims, wherein, p is preferably 0, 1 or 2; or q is preferably 0 or 1; or m and n are each independently preferably 0, 1 or 2.

19. The compound having the structure of formula (I) or a pharmaceutically acceptable salt, isotope derivative, stereoisomer thereof according to any one of the preceding claims, wherein 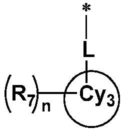 includes at least one secondary or tertiary amine; preferably, includes at least one tertiary amine; wherein * represents the site at which 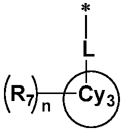 is connected to the corresponding position in formula (I).

20. The compound having the structure of formula (I) or a pharmaceutically acceptable salt, isotope derivative, stereoisomer thereof according to any one of the preceding claims, wherein the structure in formula (I) -Cy₁-(R₄)p is selected from the following: 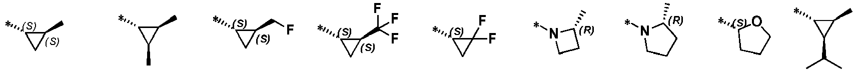 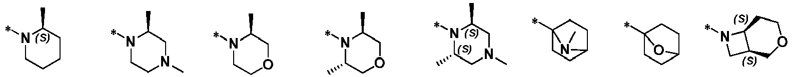 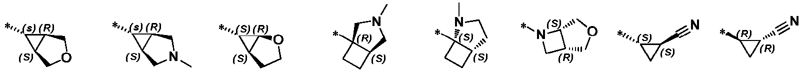 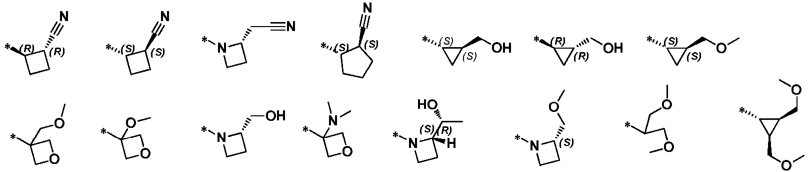 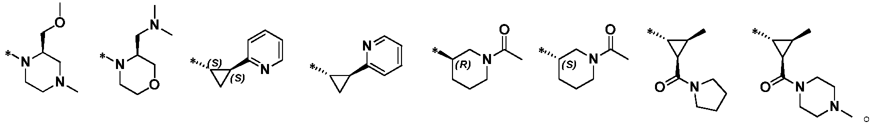 wherein * represents the site at which -Cy₁-(R₄)p is connected to the corresponding position in formula (I).

21. The compound having the structure of formula (I) or a pharmaceutically acceptable salt, isotope derivative, stereoisomer thereof according to any one of the preceding claims, wherein the structure in formula (I) 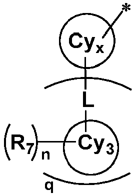 is selected from the following: 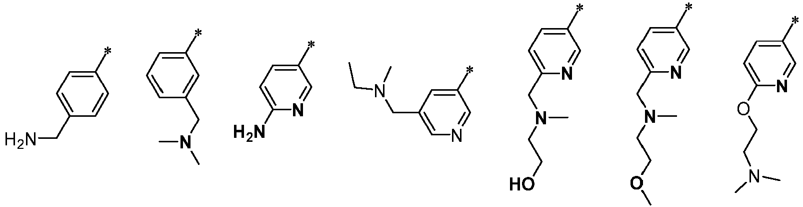 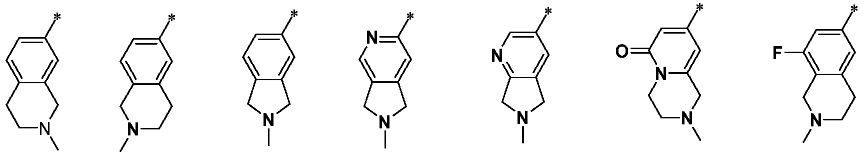 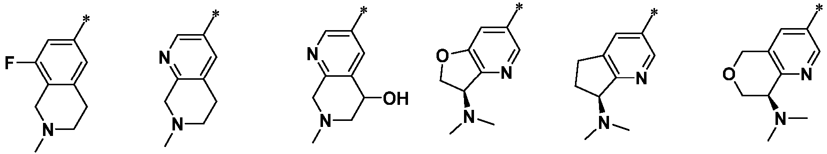 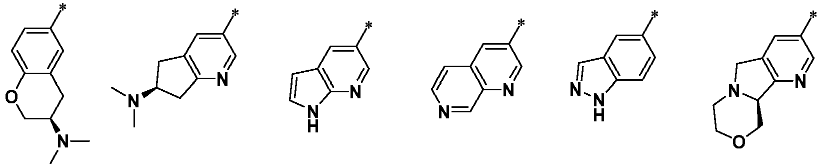 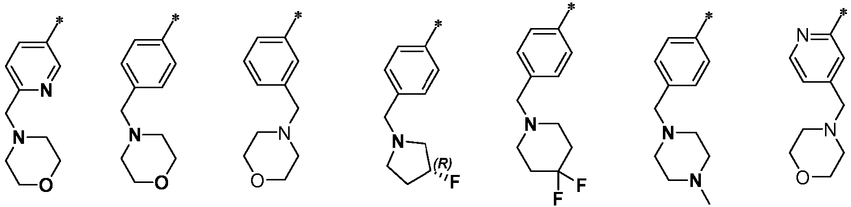 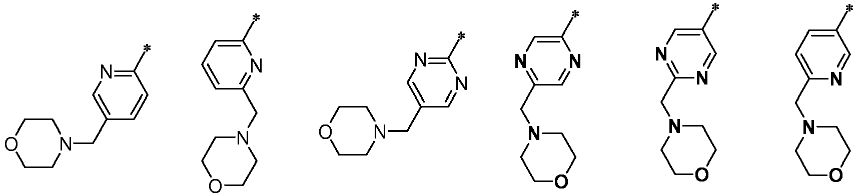 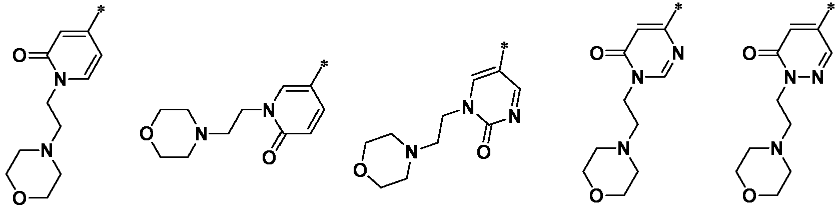 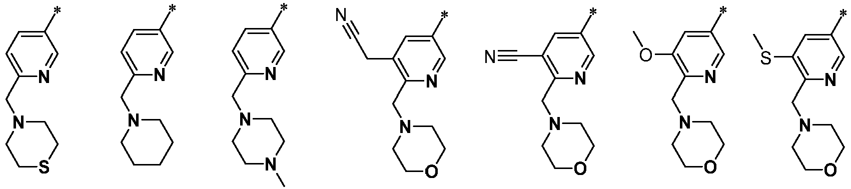 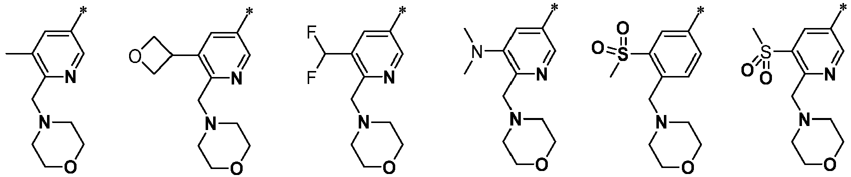 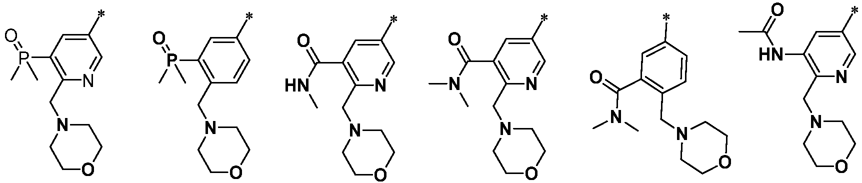 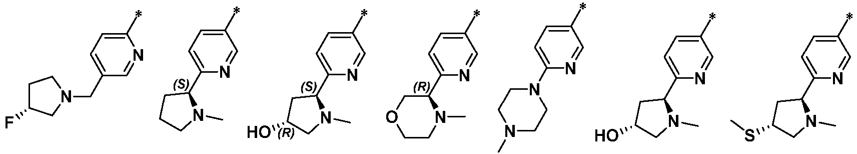 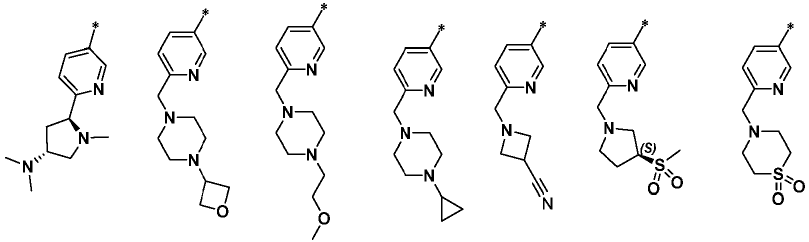 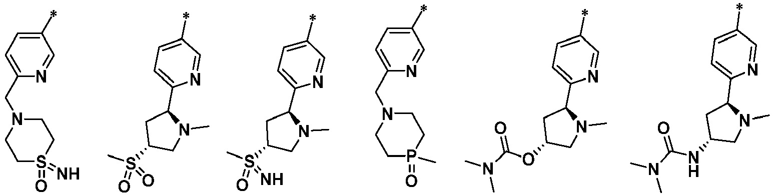 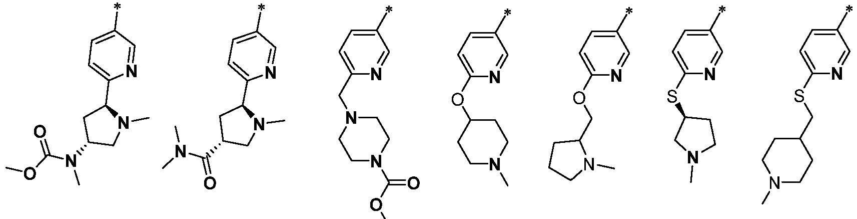 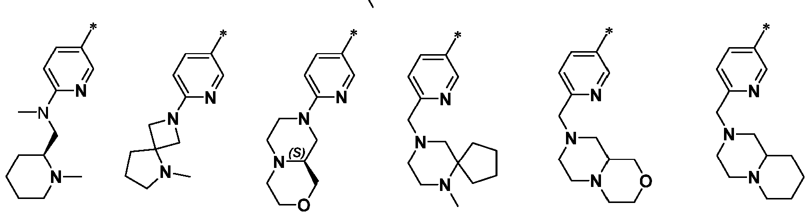 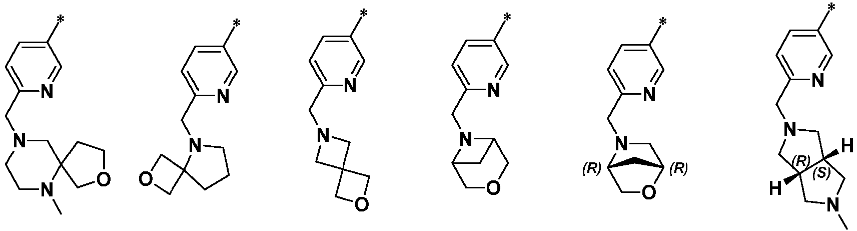 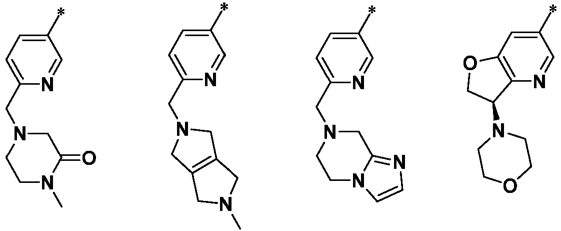 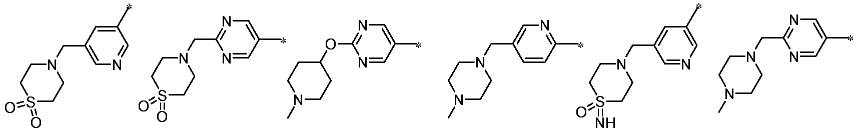 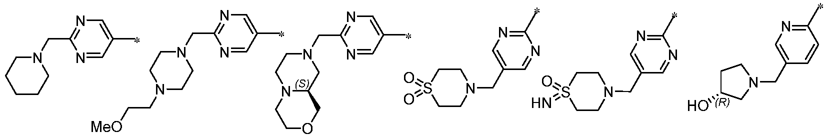 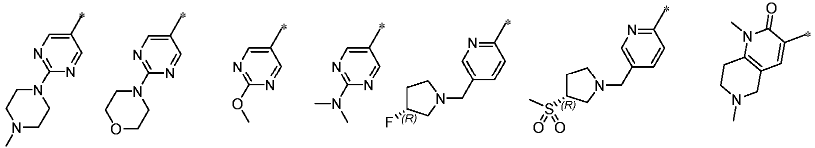 wherein * represents the site at which 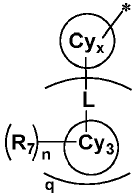 is connected to the corresponding position in Formula (I).

22. The compound having the structure of formula (I) or a pharmaceutically acceptable salt, isotope derivative, stereoisomer thereof according to any one of the preceding claims, wherein the structure in formula (I) the structure in formula (I) 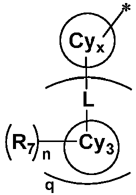 is selected from the following: is selected from the following: 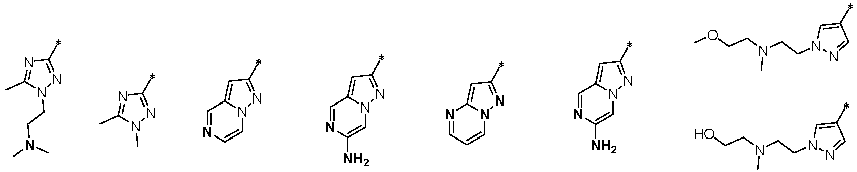 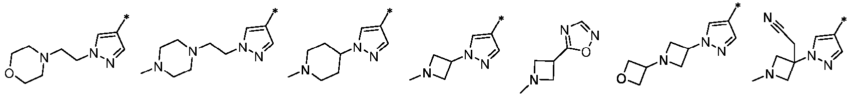 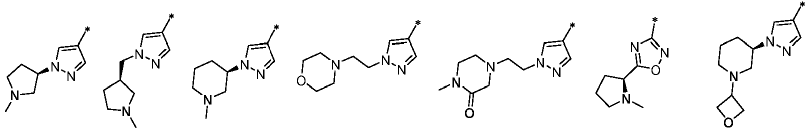 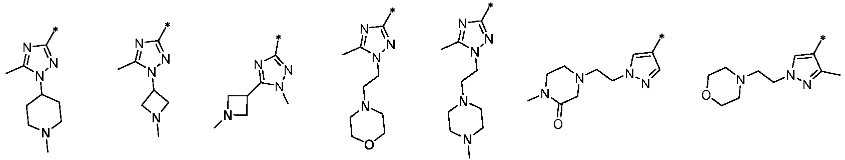 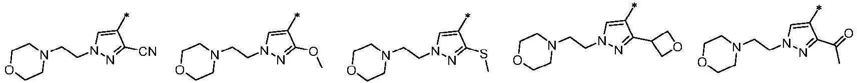 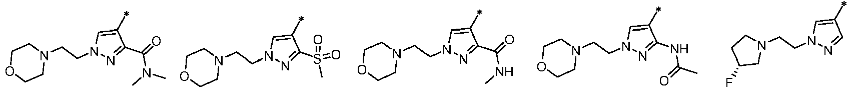 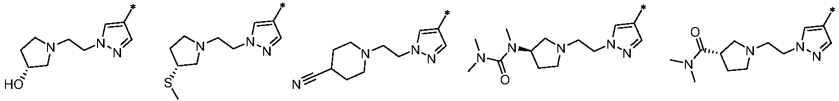 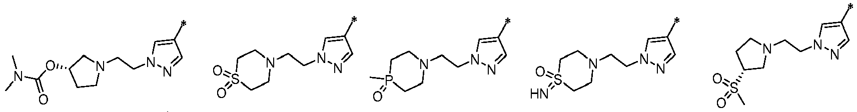 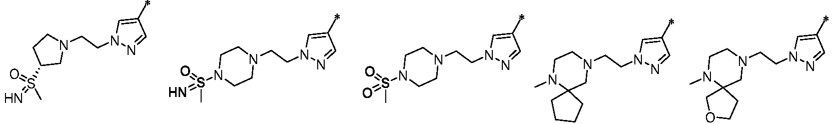 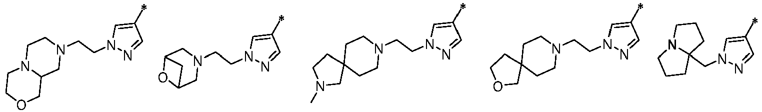 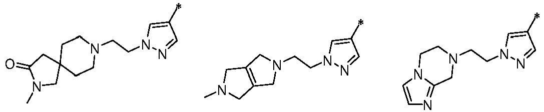 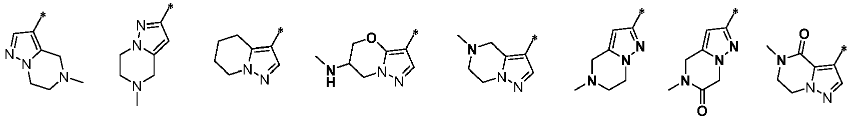 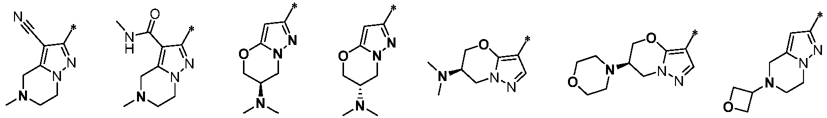 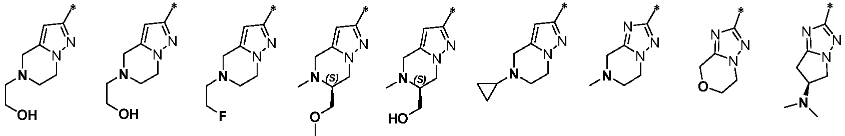 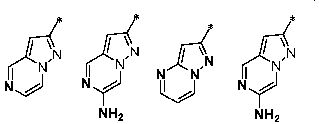 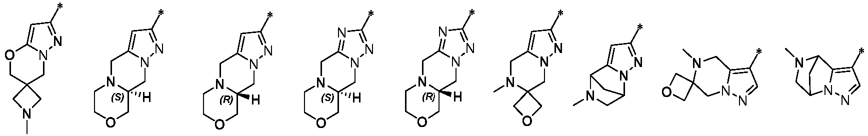 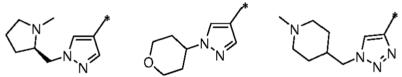 wherein, * represents the site at which 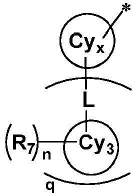 is connected to the corresponding position in formula (I).

23. The compound having the structure of formula (I) or a pharmaceutically acceptable salt, isotope derivative, stereoisomer thereof according to any one of the preceding claims, wherein the compound of formula (I) has the structure of formula (V):

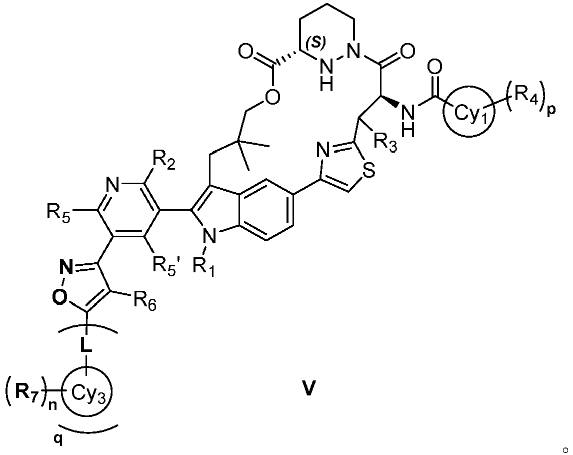

24. The compound having the structure of formula (V) or a pharmaceutically acceptable salt, isotope derivative, stereoisomer thereof according to any one of the preceding claims, wherein the structure of formula (V) 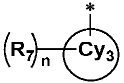 is selected from the following: 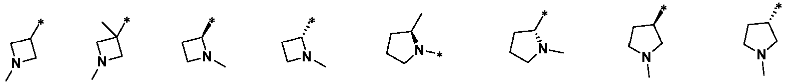 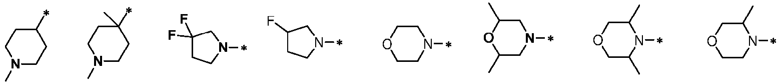 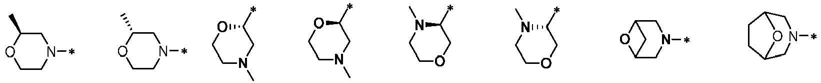 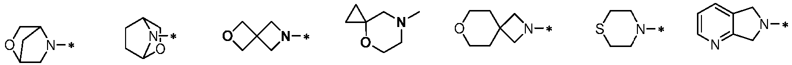 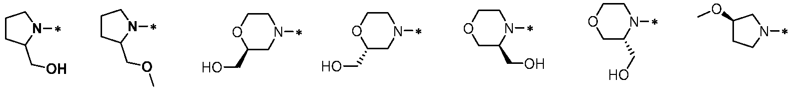 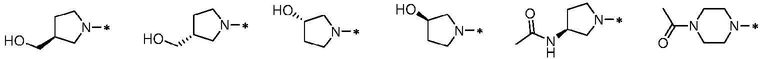 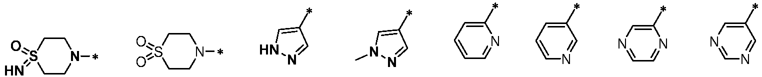 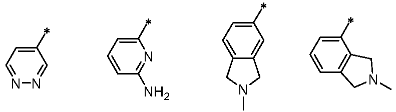 wherein * represents the site at which 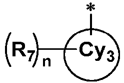 is connected to the corresponding position in Formula (V).

25. The compound having the structure of formula (I) or a pharmaceutically acceptable salt, isotope derivative, stereoisomer thereof as described in any of the preceding claims, wherein the compound of formula (I) has the structure of formula (II):

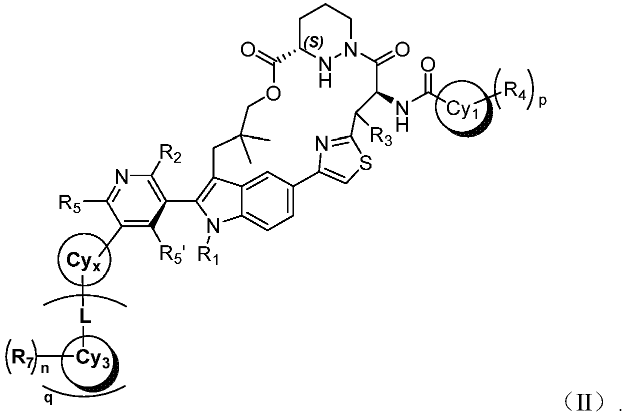

26. A compound having the following structure:

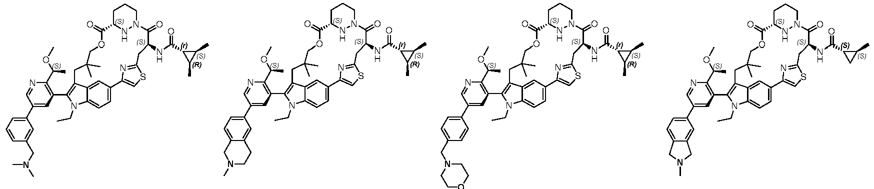

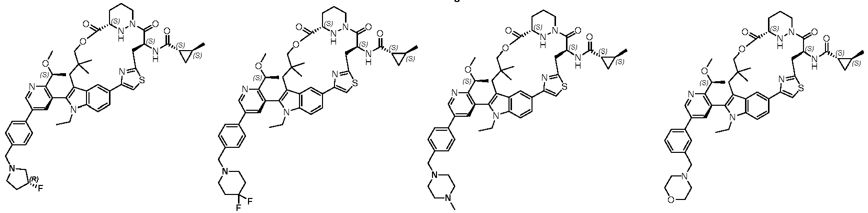

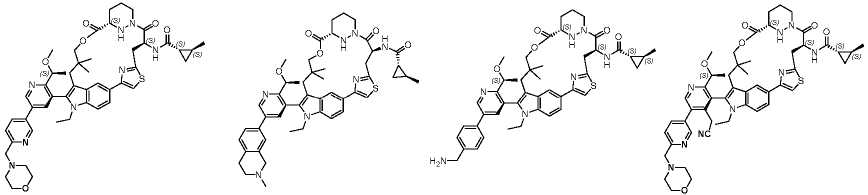

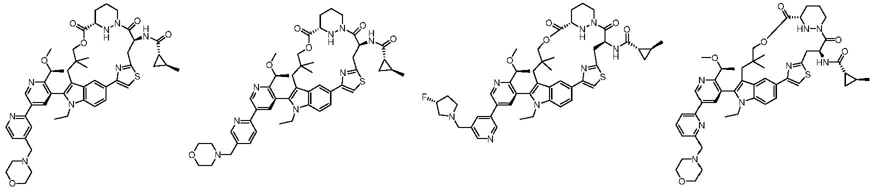

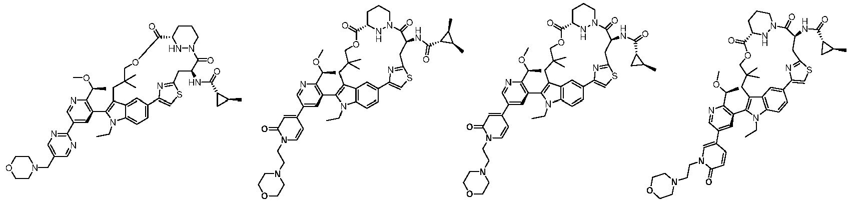

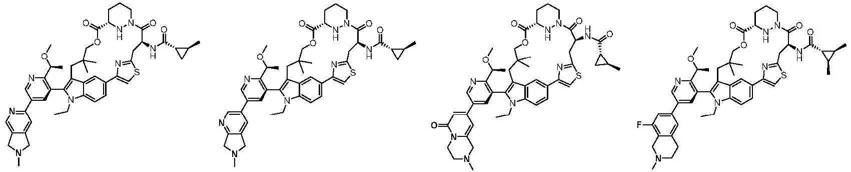

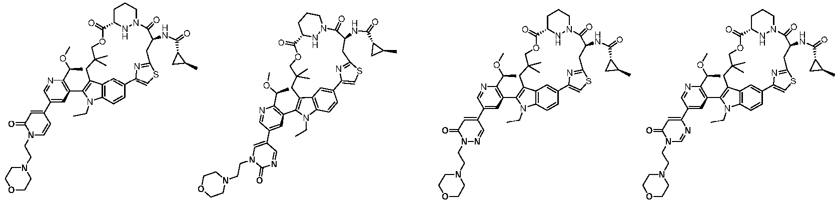

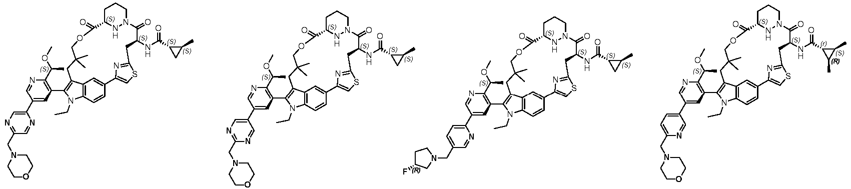

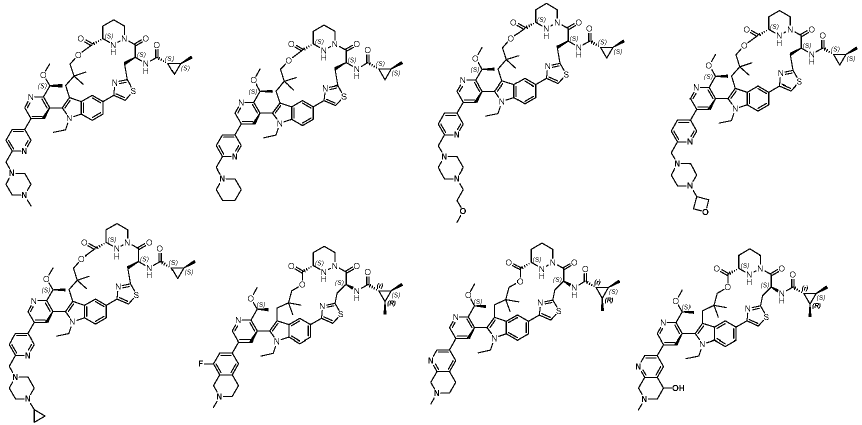

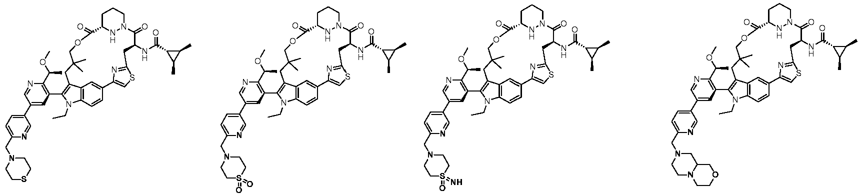

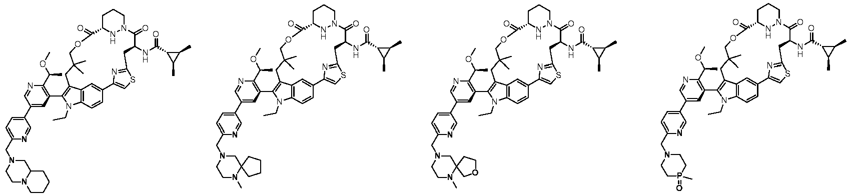

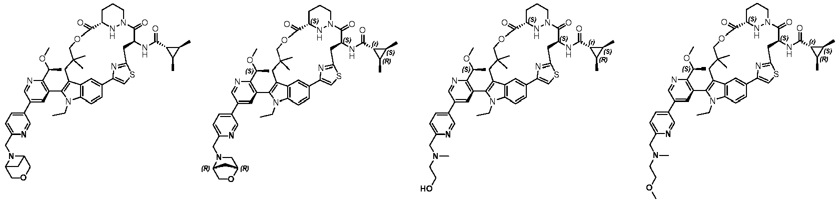

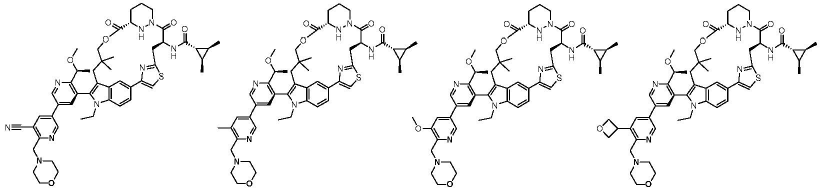

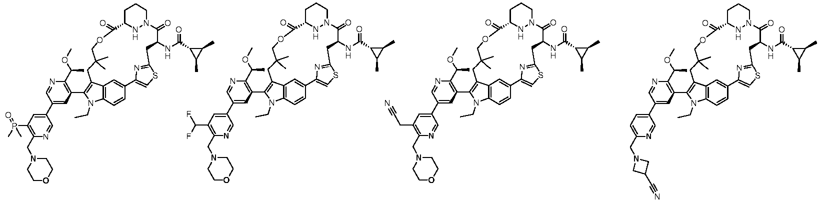

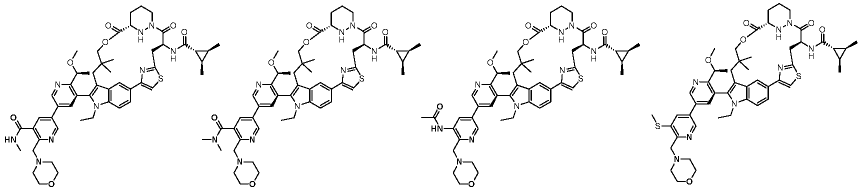

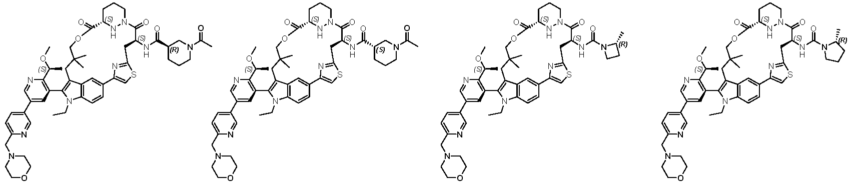

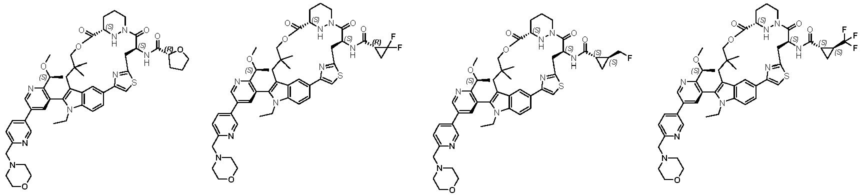

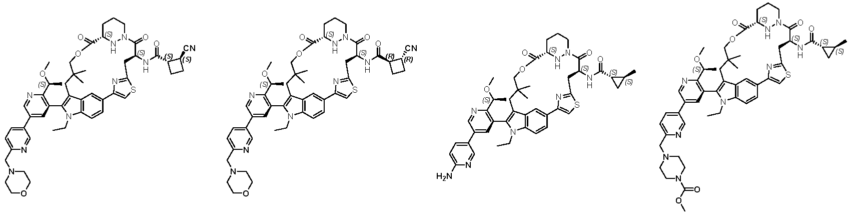

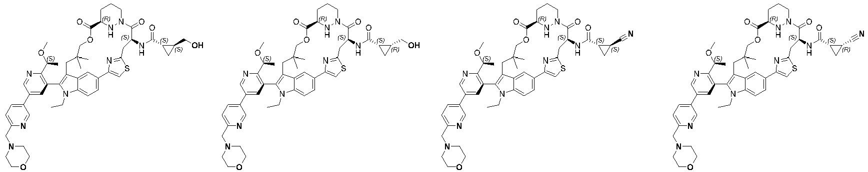

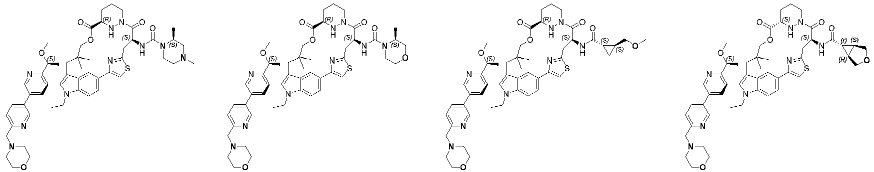

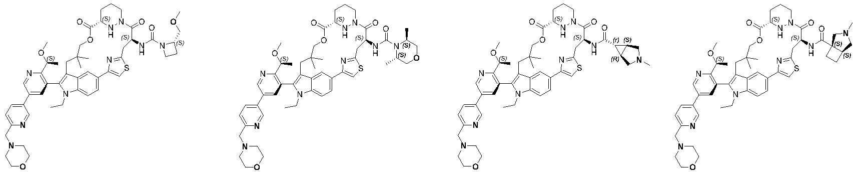

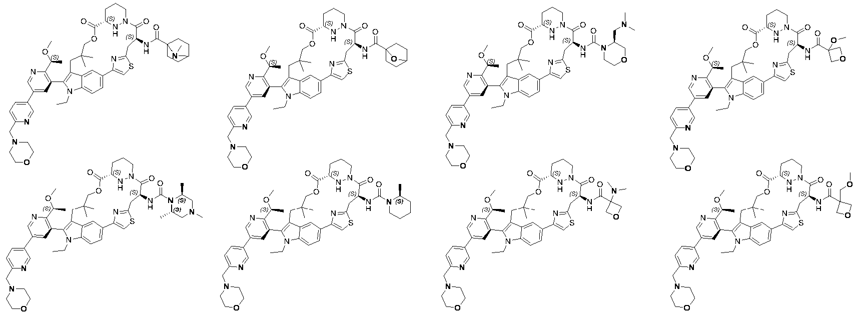

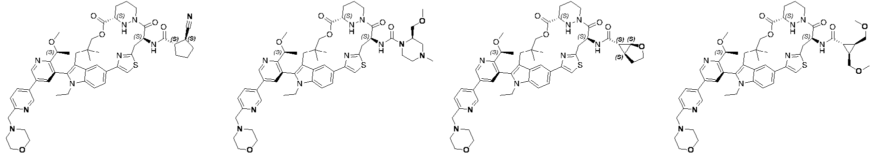

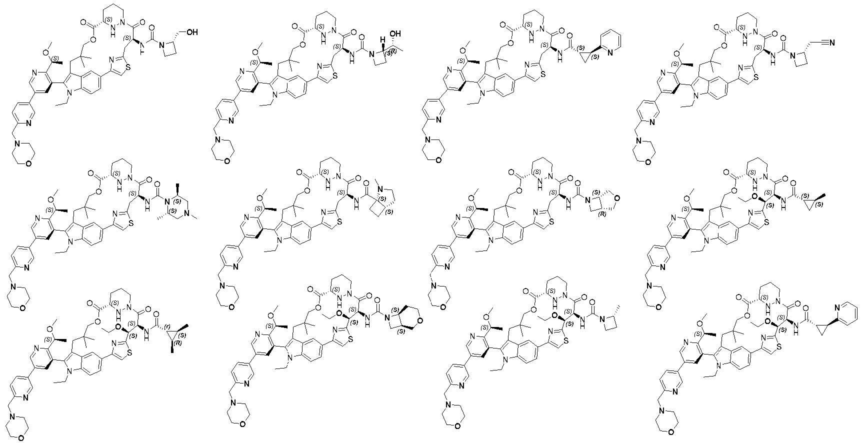

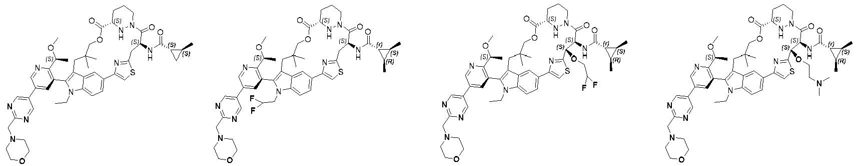

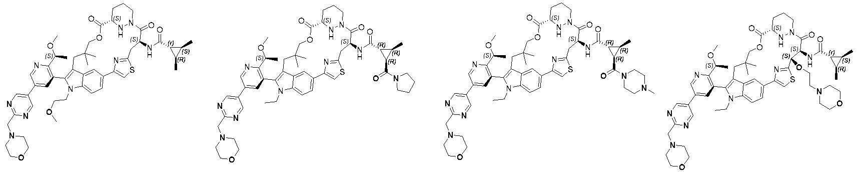

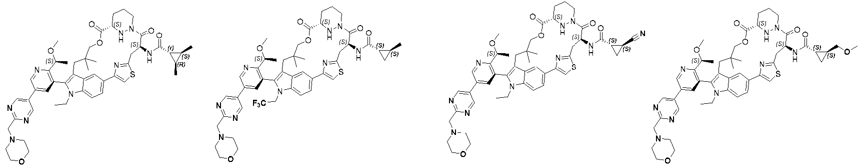

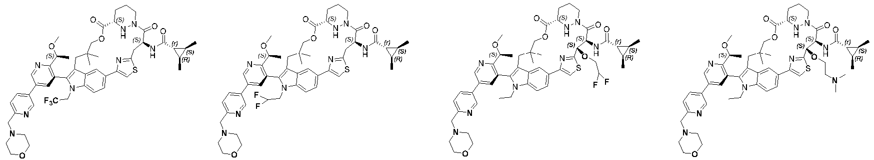

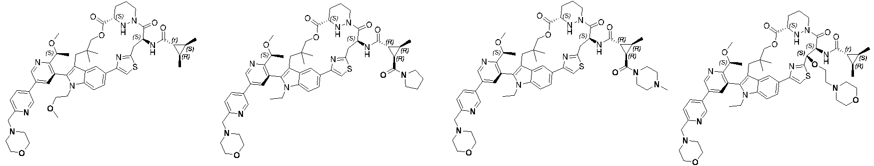

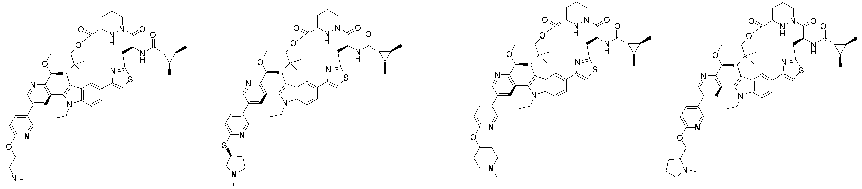

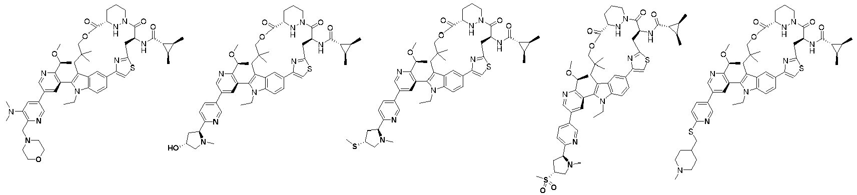

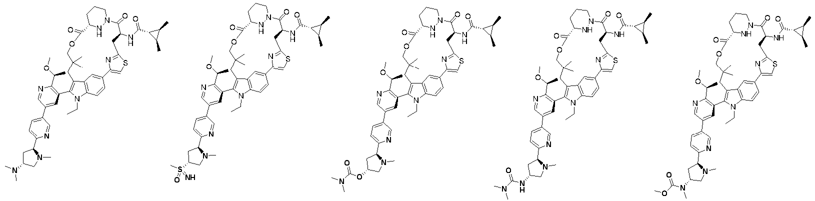

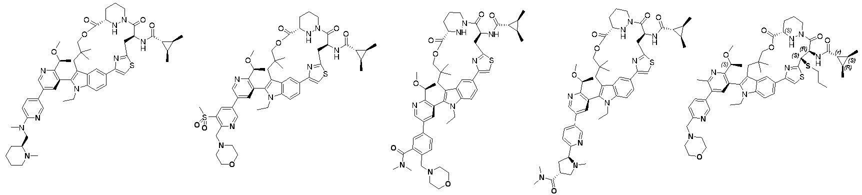

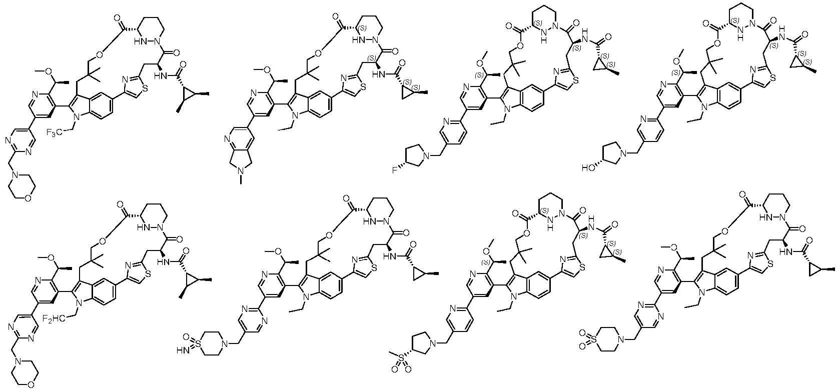

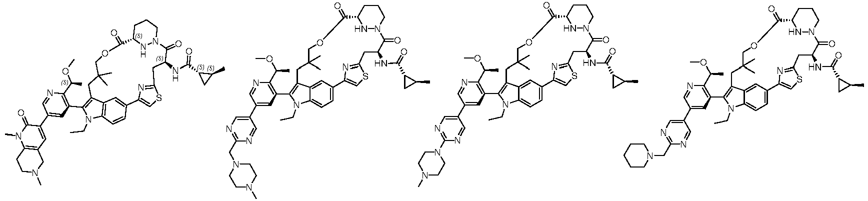

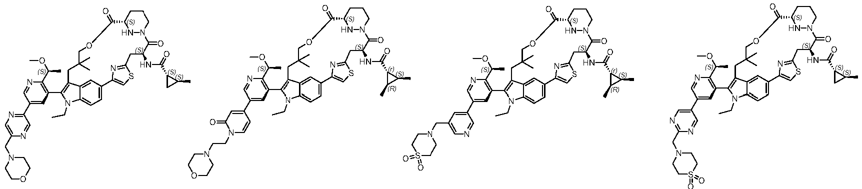

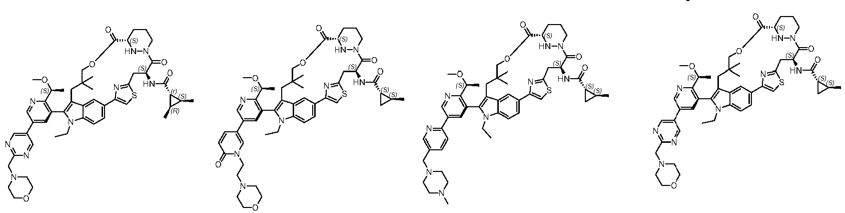

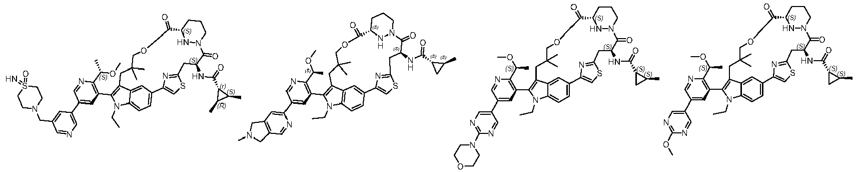

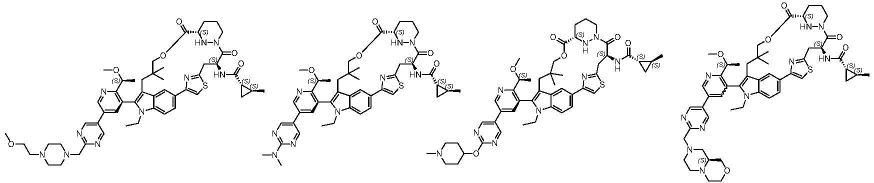

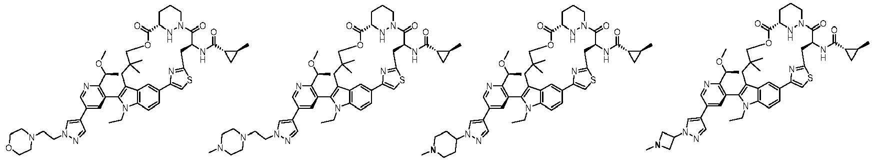

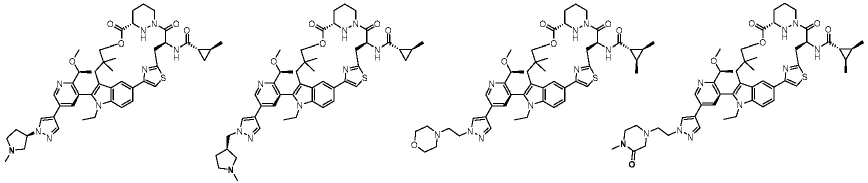

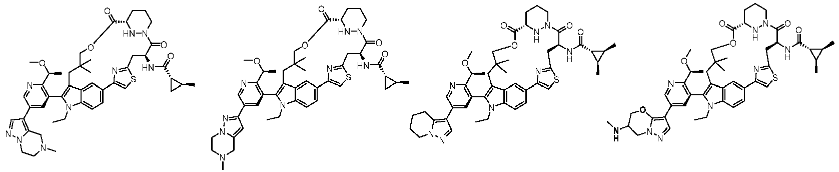

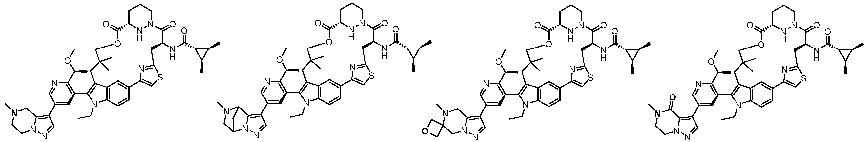

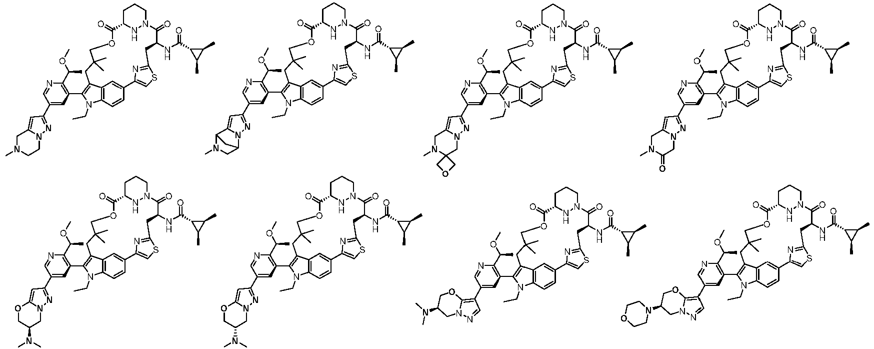

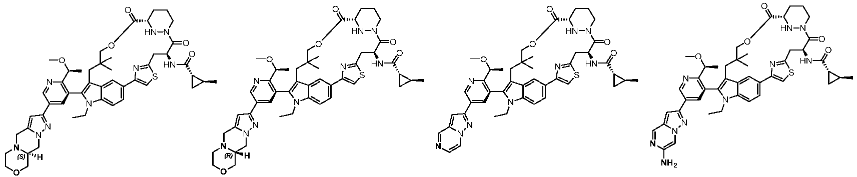

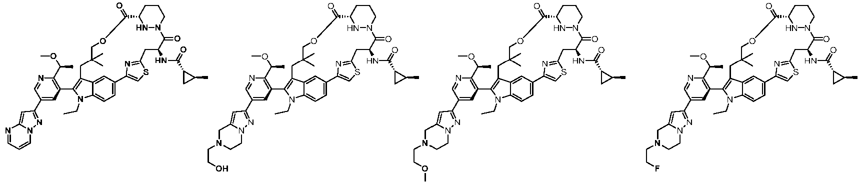

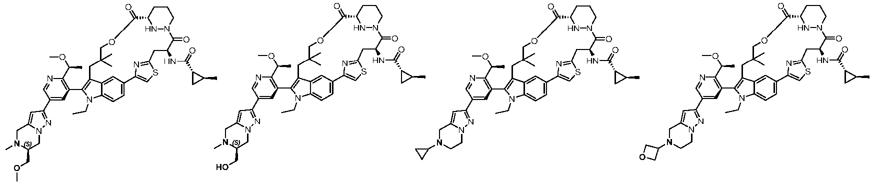

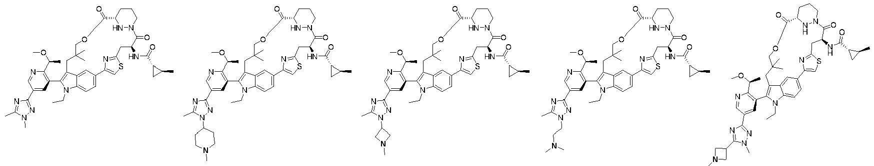

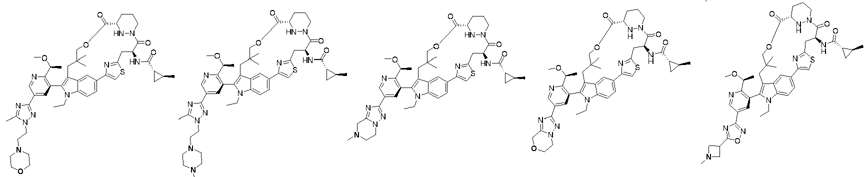

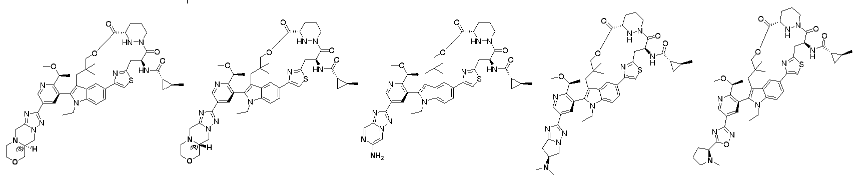

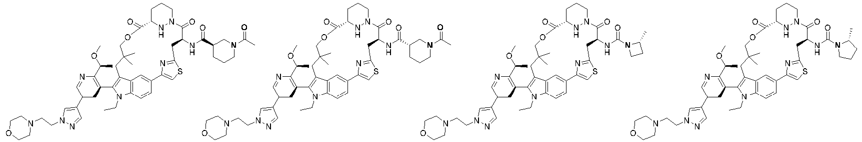

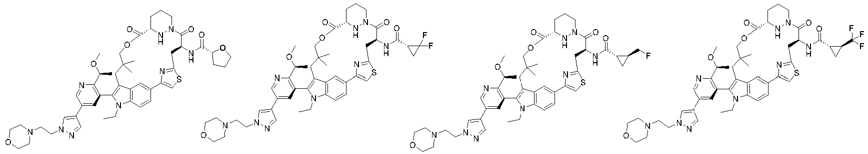

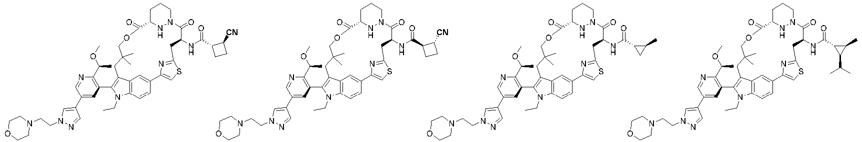

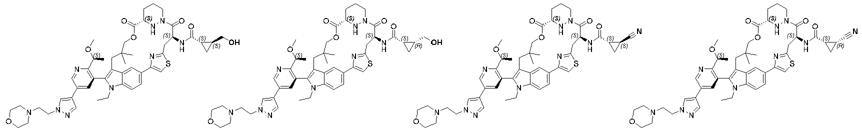

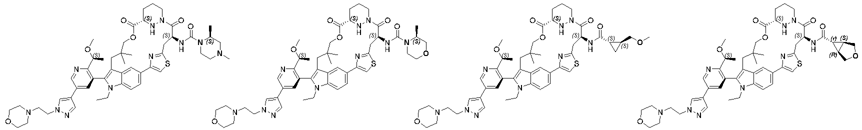

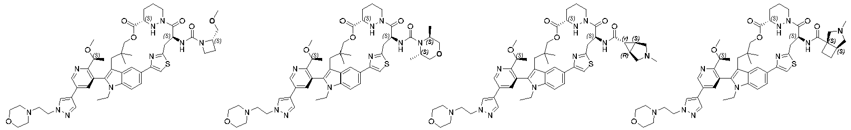

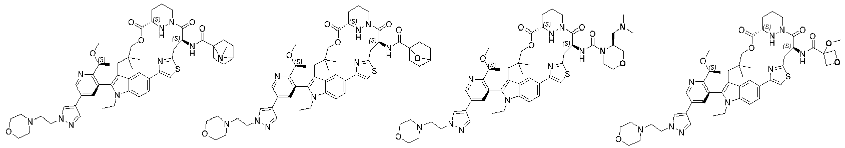

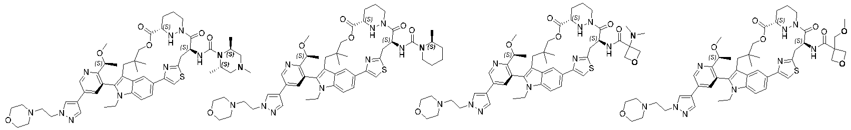

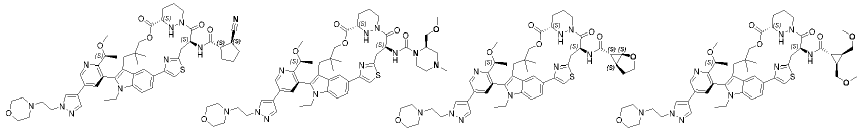

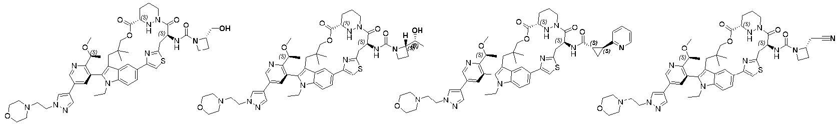

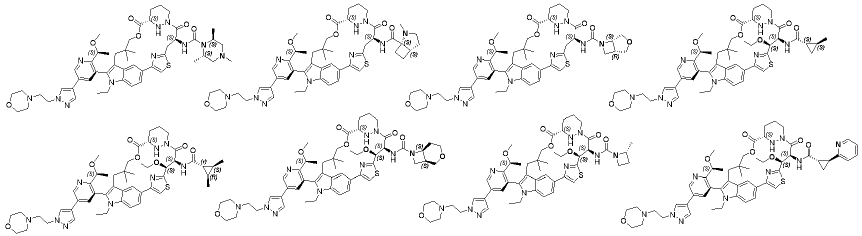

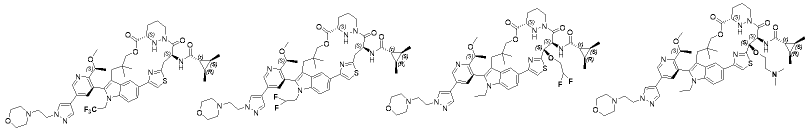

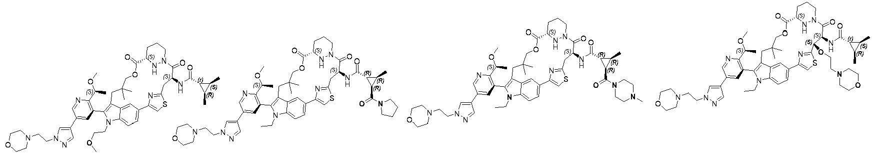

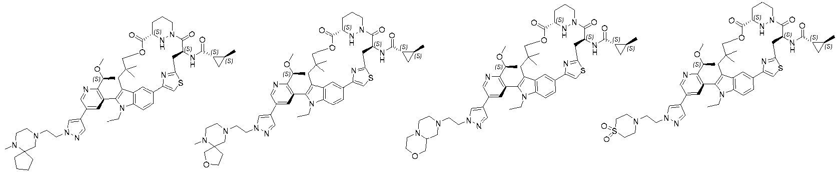

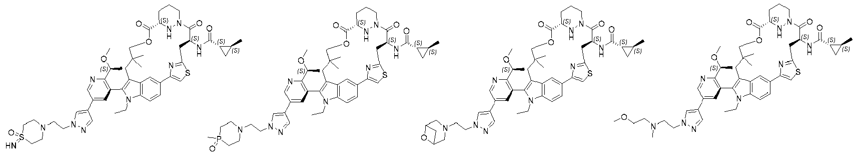

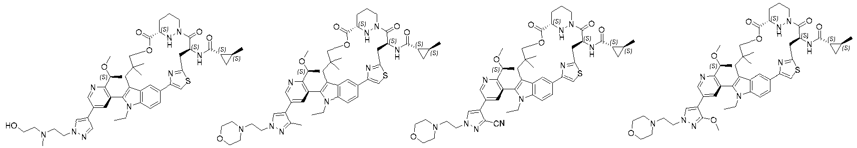

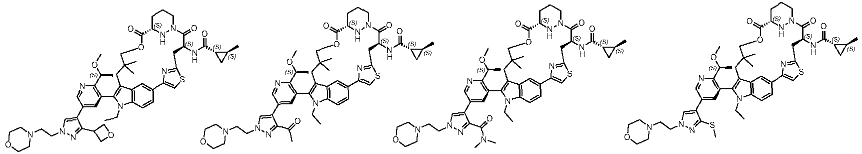

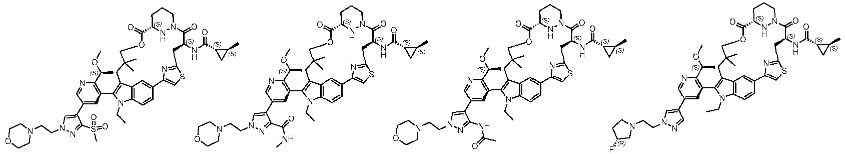

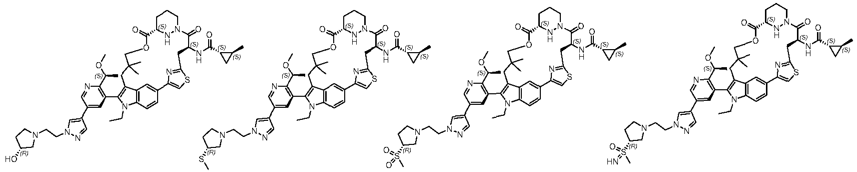

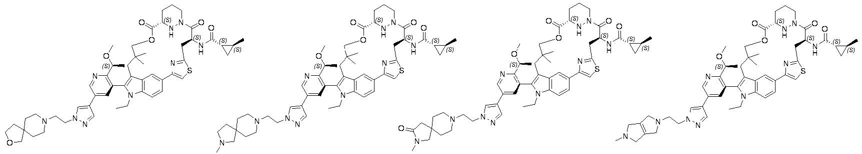

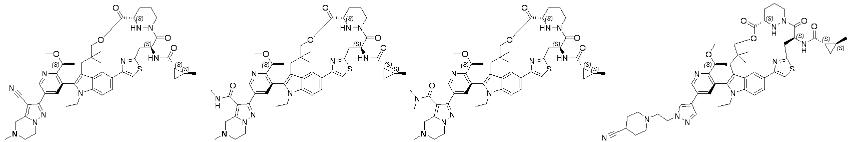

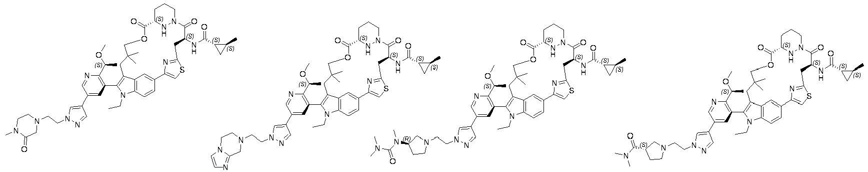

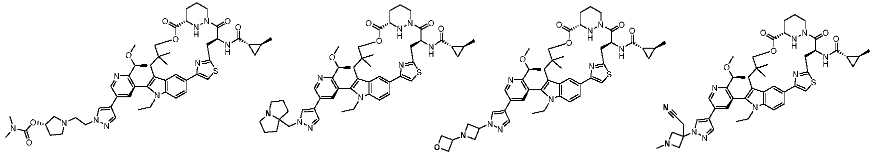

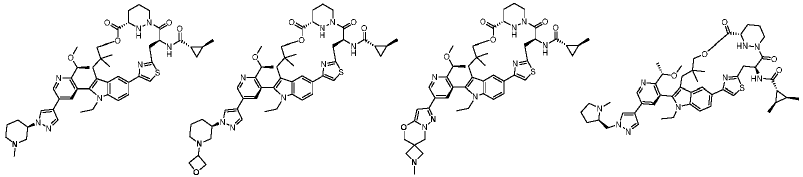

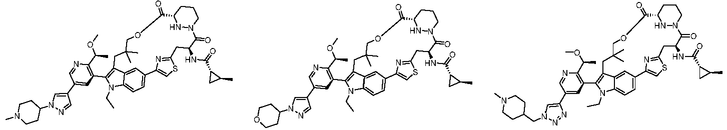

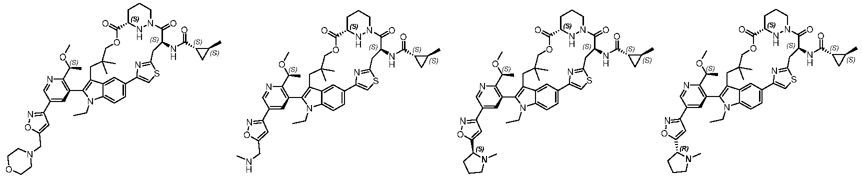

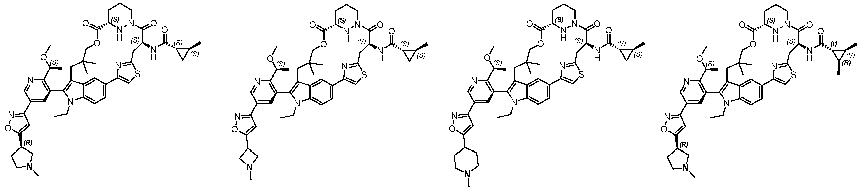

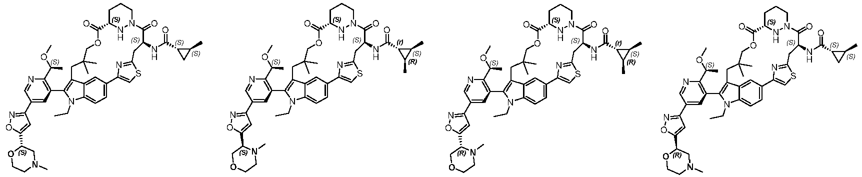

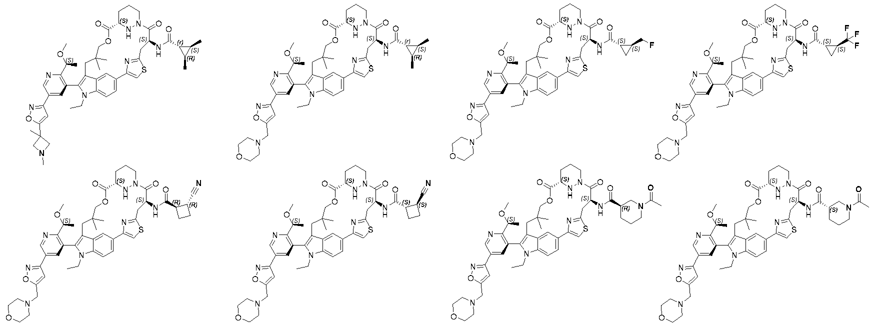

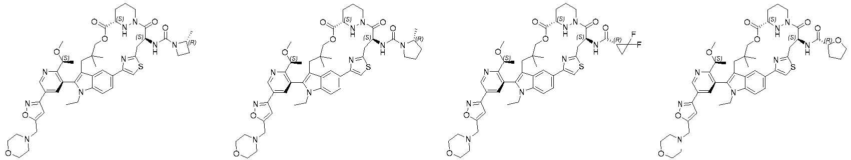

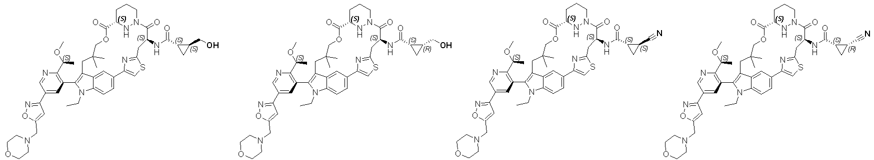

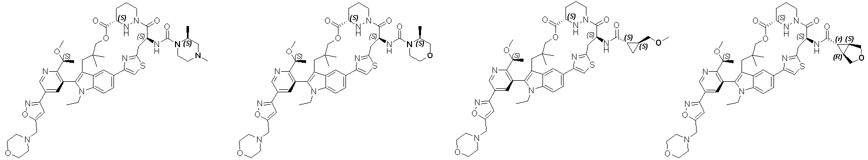

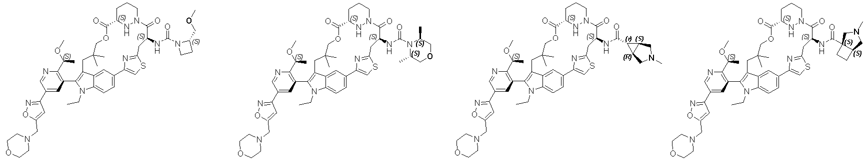

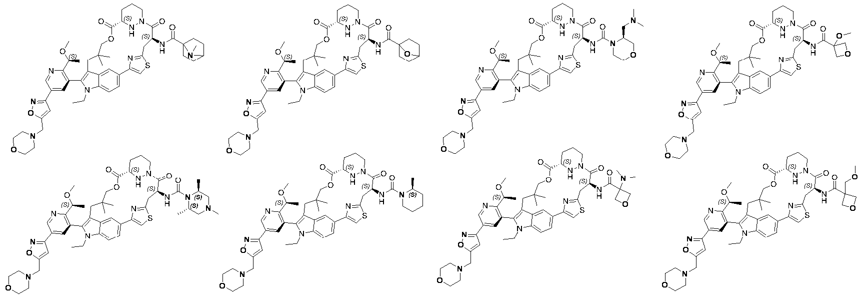

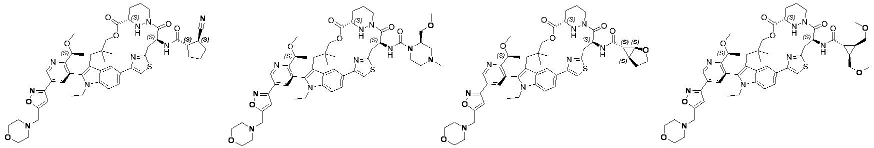

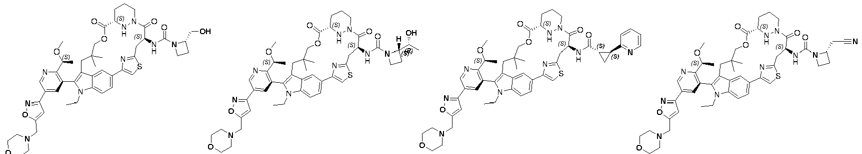

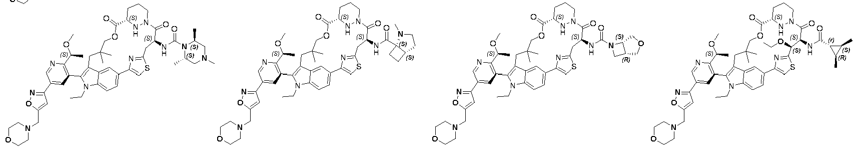

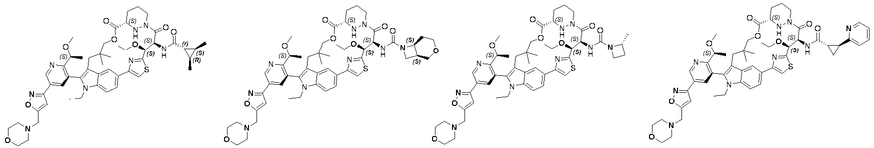

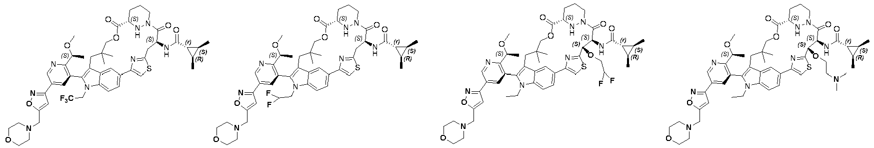

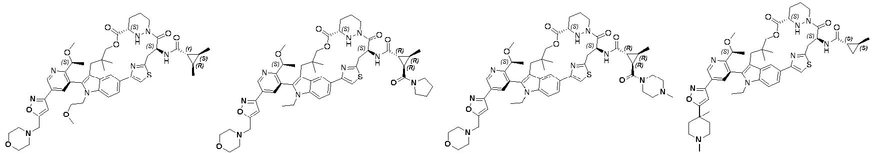

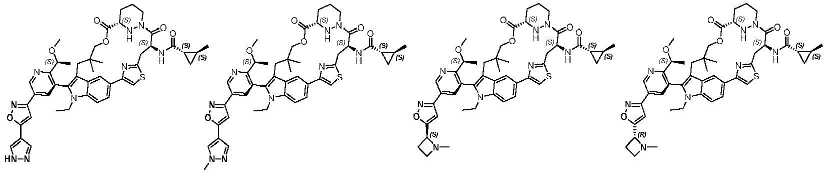

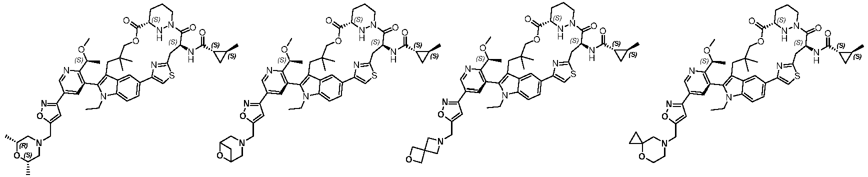

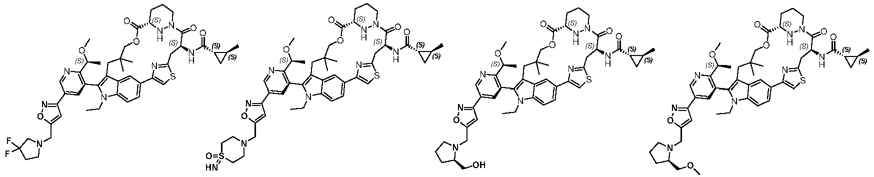

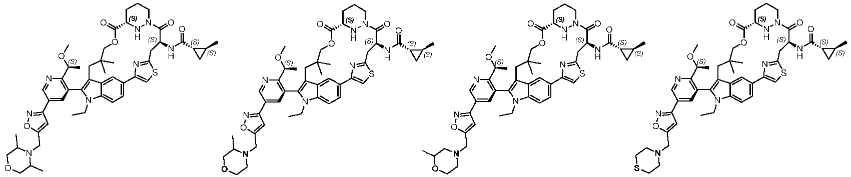

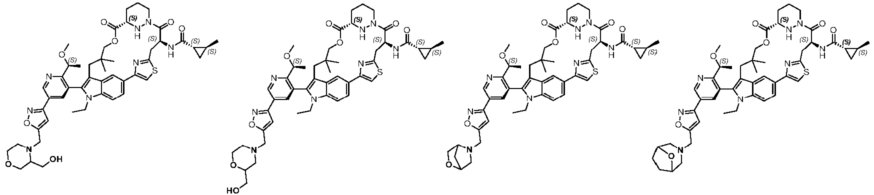

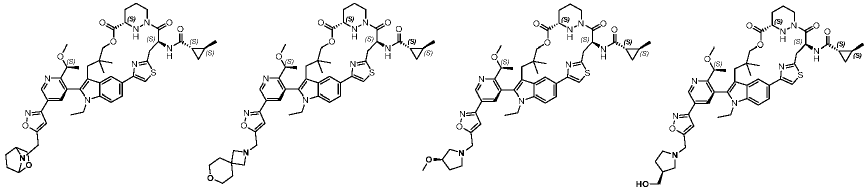

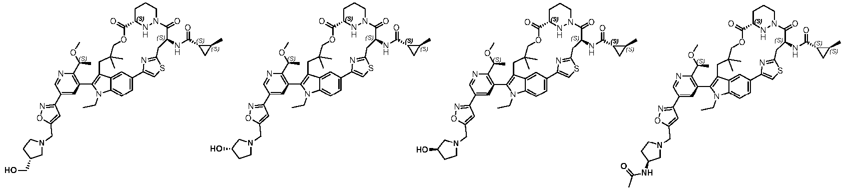

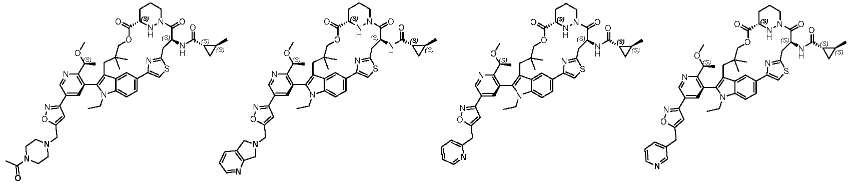

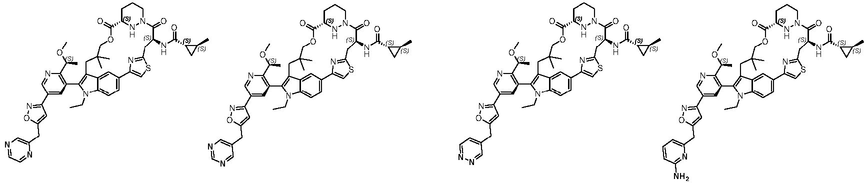

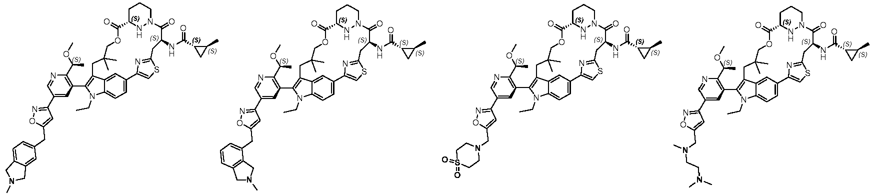

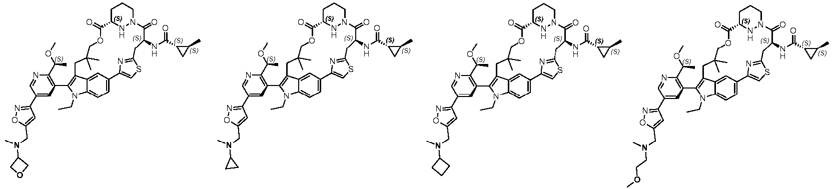

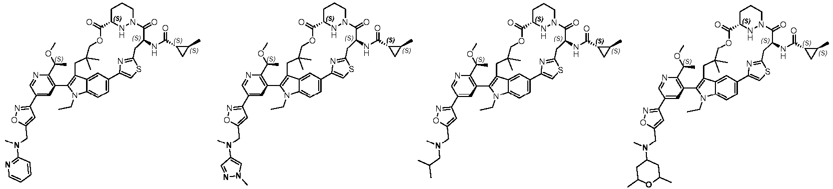

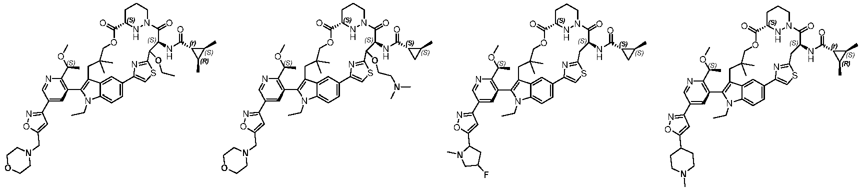

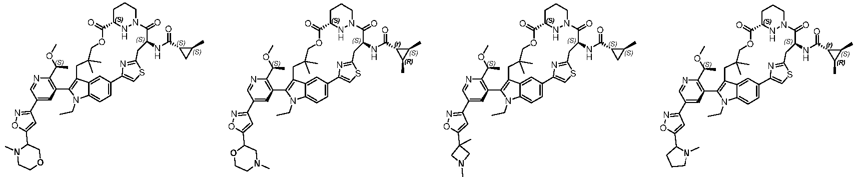

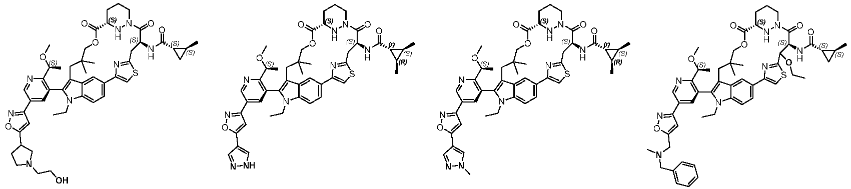

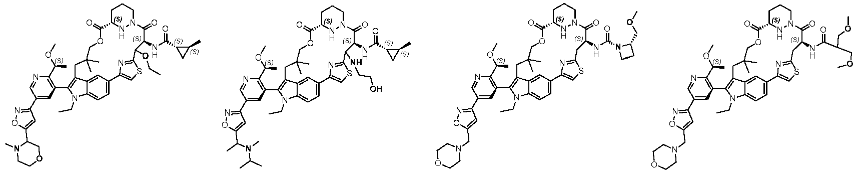

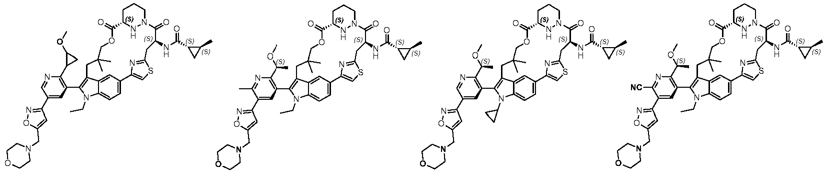

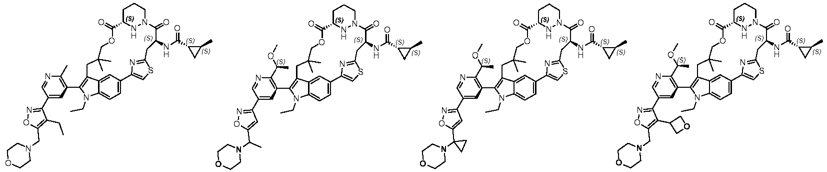

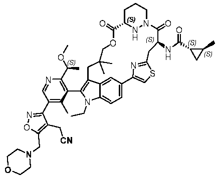

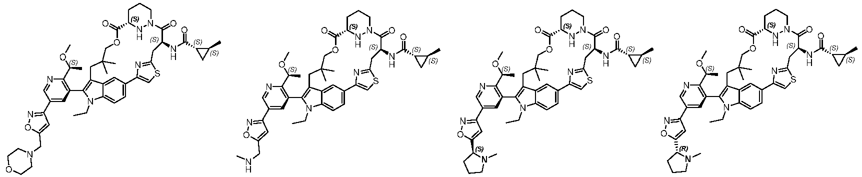

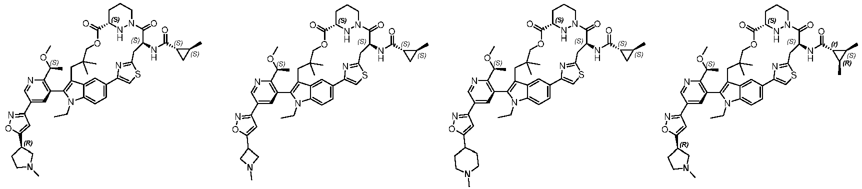

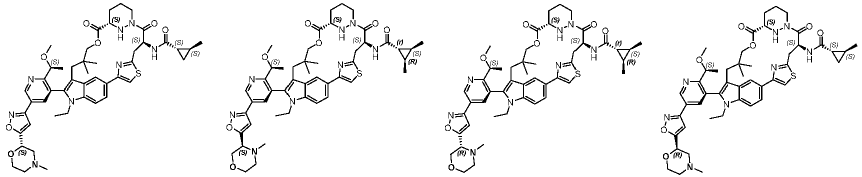

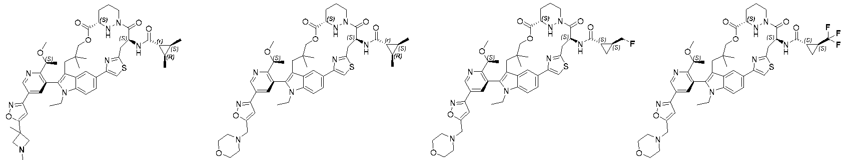

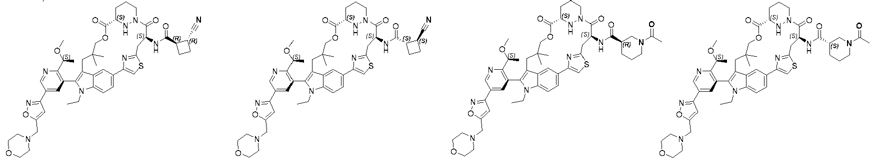

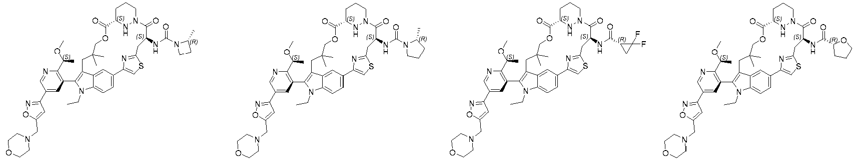

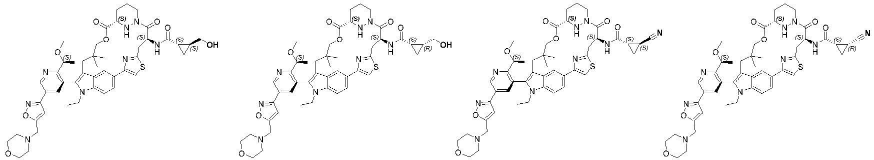

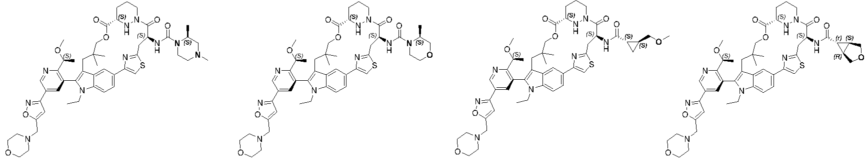

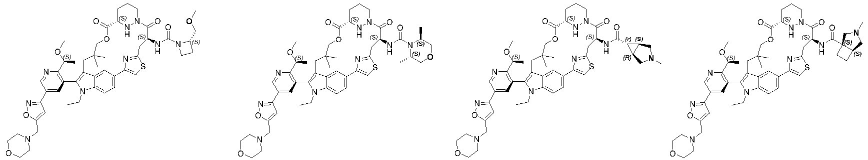

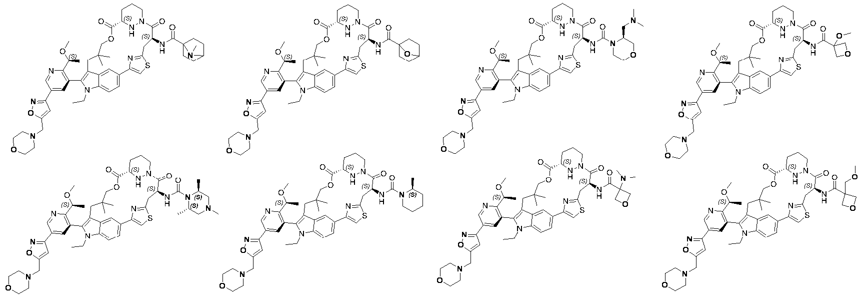

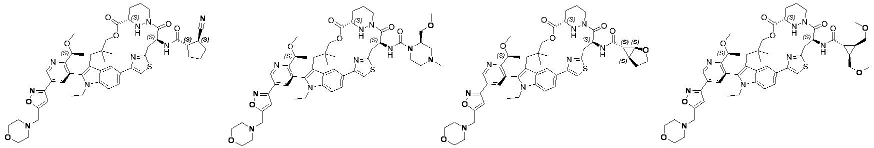

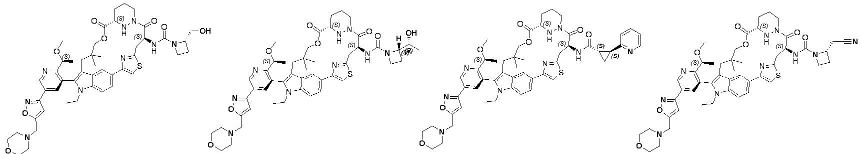

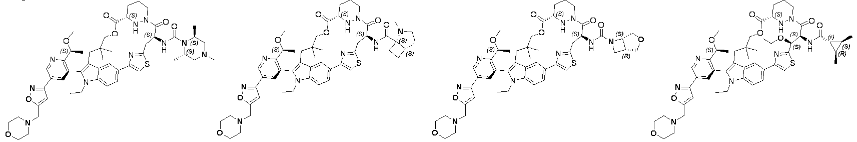

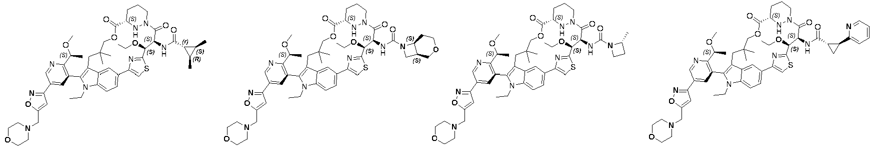

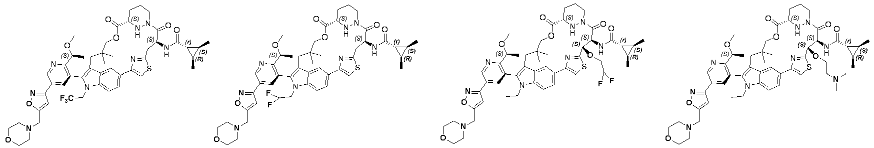

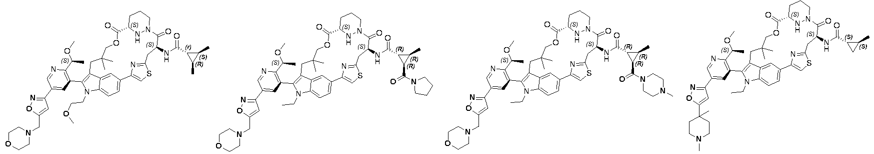

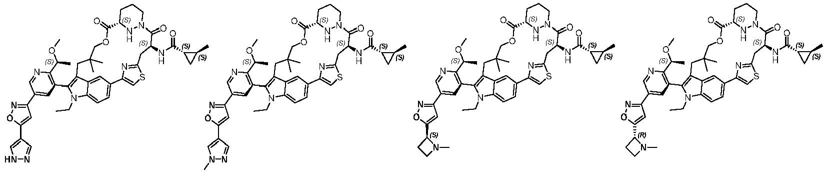

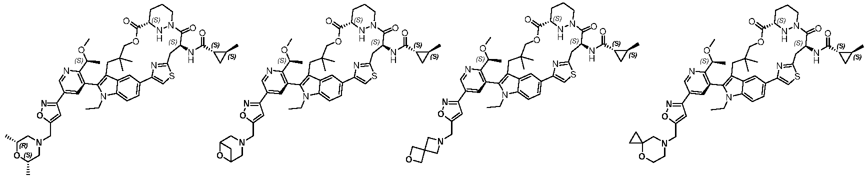

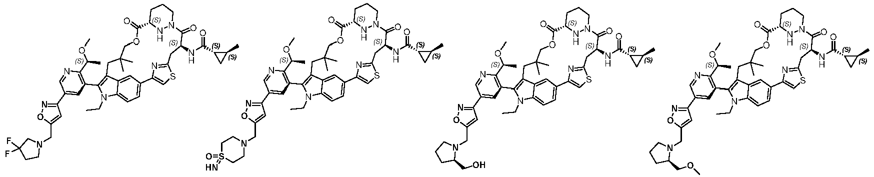

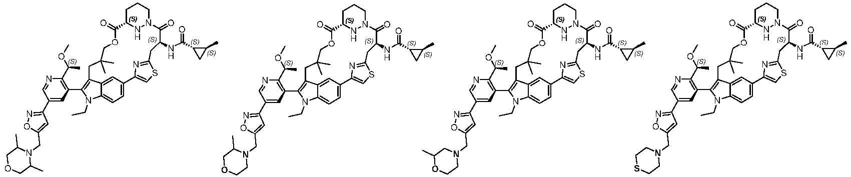

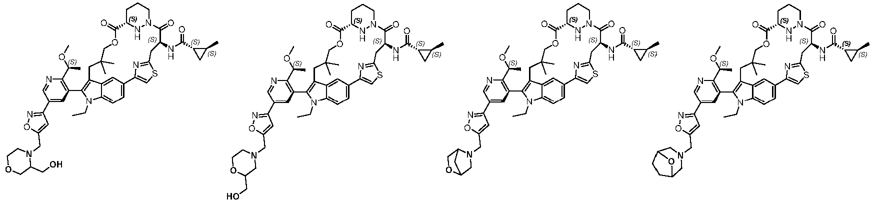

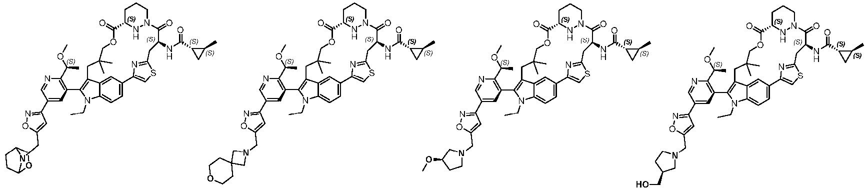

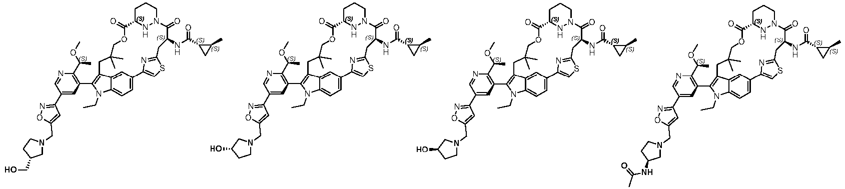

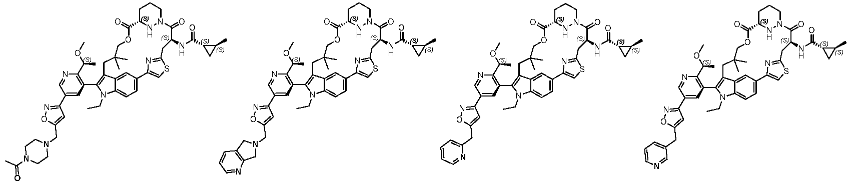

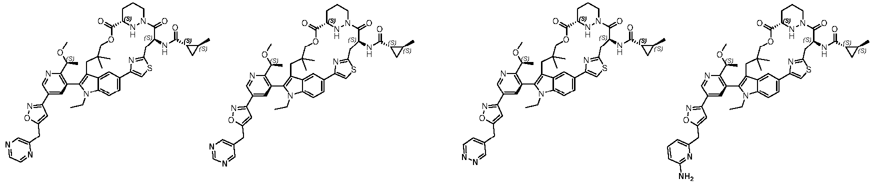

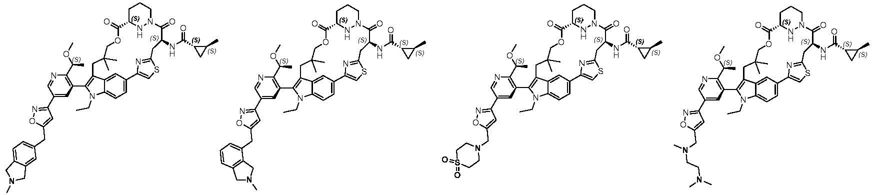

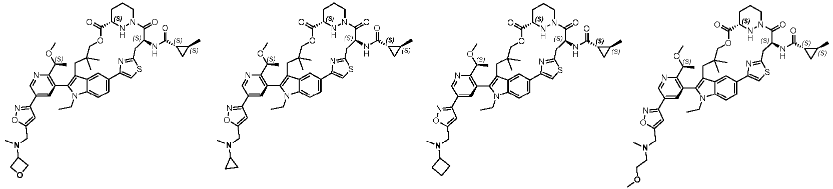

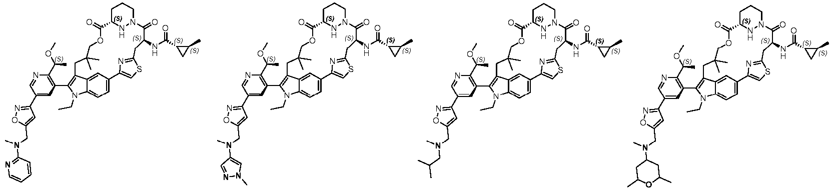

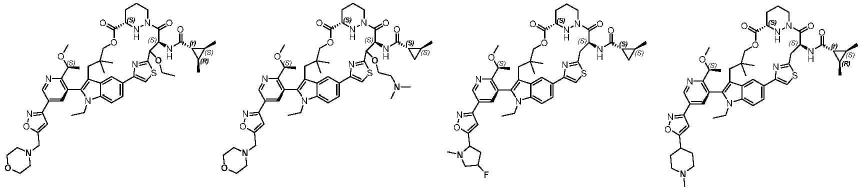

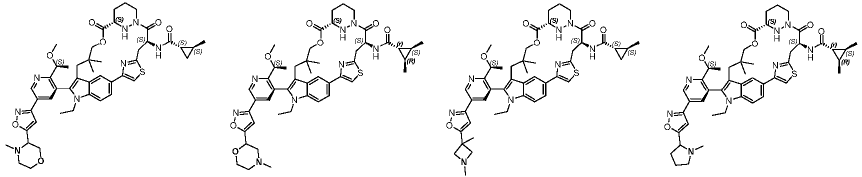

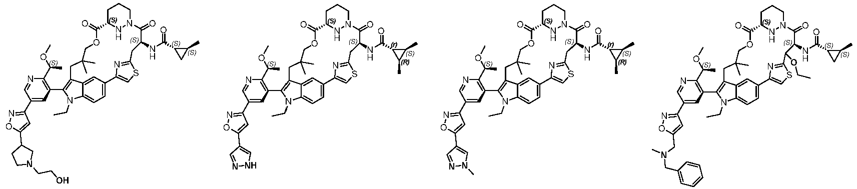

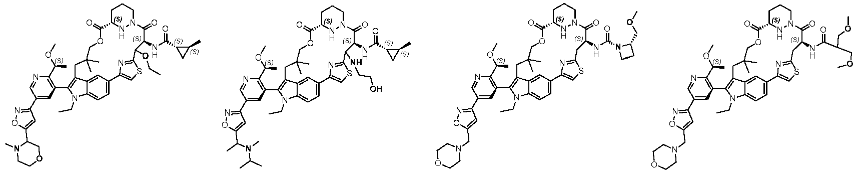

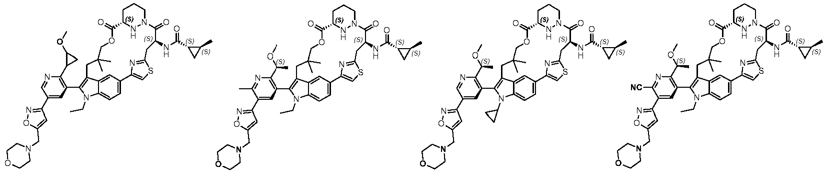

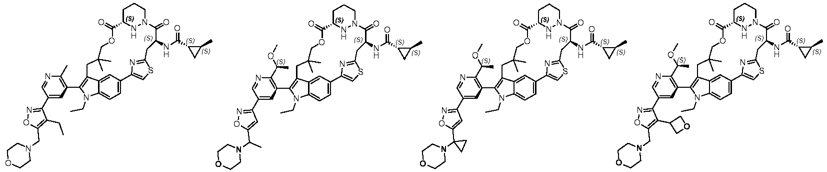

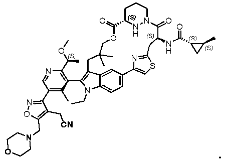

27. A pharmaceutical composition comprising the compound or a pharmaceutically acceptable salt, isotope derivative, or stereoisomer thereof according to any one of the preceding claims.

28. The use of the compound or a pharmaceutically acceptable salt, isotope derivative, or stereoisomer thereof according to any one of claims 1-26, or the pharmaceutical composition according to claim 27, in the preparation of a medicament for the prevention and/or treatment of cancer, tumors, inflammatory diseases, autoimmune diseases, or immune-mediated diseases.

29. A method for the prevention and/or treatment of cancer, tumors, inflammatory diseases, autoimmune diseases, or immune-mediated diseases, comprising administering a therapeutically effective amount of the compound or a pharmaceutically acceptable salt, isotope derivative, or stereoisomer thereof according to any one of claims 1-26, and/or the pharmaceutical composition according to claim 27, to a patient in need thereof.
